(19) Europäisches Patentamt / European Patent Office / Office européen des brevets

(11) EP 2 019 094 A1

(12) # EUROPEAN PATENT APPLICATION
published in accordance with Art. 153(4) EPC

(43) Date of publication:
**28.01.2009 Bulletin 2009/05**

(21) Application number: **07743623.6**

(22) Date of filing: **18.05.2007**

(51) Int Cl.:
*C07D 249/08* (2006.01) *A61K 31/4196* (2006.01)
*A61K 31/437* (2006.01) *A61K 31/4375* (2006.01)
*A61K 31/454* (2006.01) *A61K 31/4545* (2006.01)
*A61K 31/5377* (2006.01) *A61P 25/28* (2006.01)
*A61P 43/00* (2006.01) *C07D 401/10* (2006.01)
*C07D 401/14* (2006.01) *C07D 413/10* (2006.01)
*C07D 413/14* (2006.01) *C07D 471/04* (2006.01)

(86) International application number:
**PCT/JP2007/060188**

(87) International publication number:
**WO 2007/135970 (29.11.2007 Gazette 2007/48)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **19.05.2006 JP 2006140606**

(71) Applicant: **Eisai R&D Management Co., Ltd.**
**Tokyo 112-8088 (JP)**

(72) Inventors:
- **KIMURA, Teiji**
  **Tsukuba-shi**
  **Ibaraki 300-2635 (JP)**
- **KAWANO, Koki**
  **Tsukuba-shi**
  **Ibaraki 300-2635 (JP)**
- **DOI, Eriko**
  **Tsukuba-shi**
  **Ibaraki 300-2635 (JP)**
- **KITAZAWA,Noritaka**
  **Tsukuba-shi**
  **Ibaraki 300-2635 (JP)**
- **MIYAGAWA, Takehiko**
  **Tsukuba-shi**
  **Ibaraki 300-2635 (JP)**
- **SATO, Nobuaki**
  **Tsukuba-shi**
  **Ibaraki 300-2635 (JP)**
- **KANEKO, Toshihiko**
  **Tsukuba-shi**
  **Ibaraki 300-2635 (JP)**
- **SHIN, Kogyoku**
  **Tsukuba-shi**
  **Ibaraki 300-2635 (JP)**
- **ITO, Koichi**
  **Tsukuba-shi**
  **Ibaraki 300-2635 (JP)**
- **TAKAISHI, Mamoru**
  **Tsukuba-shi**
  **Ibaraki 300-2635 (JP)**
- **SASAKI, Takeo**
  **Tsukuba-shi**
  **Ibaraki 300-2635 (JP)**
- **HAGIWARA, Hiroaki**
  **Tsukuba-shi**
  **Ibaraki 300-2635 (JP)**

(74) Representative: **HOFFMANN EITLE**
**Patent- und Rechtsanwälte**
**Arabellastrasse 4**
**81925 München (DE)**

(54) **HETEROCYCLIC TYPE CINNAMIDE DERIVATIVE**

(57) Disclosed is a compound represented by the formula (I) below or a pharmacologically acceptable salt thereof. Also disclosed is a use of the compound or salt as a pharmaceutical product.

EP 2 019 094 A1

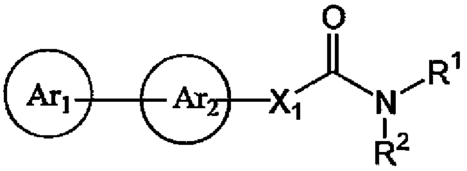

(I)

(In the formula, $Ar_1$ represents a triazolyl group or the like which may be substituted with a $C_{1-6}$ alkyl group or the like; $Ar_2$ represents a phenyl group or the like which may be substituted with a $C_{1-6}$ alkoxy group or the like; $X_1$ represents $-CR^3=CR^4-$ (wherein $R^3$ and $R^4$ respectively represent a $C_{1-6}$ alkyl group or the like); and $R^1$ and $R^2$ respectively represent a $C_{1-6}$ alkyl group or the like.)

## Description

TECHNICAL FIELD

The present invention relates to a pharmaceutical, more particularly, to an amyloid-β (hereinafter referred to as Aβ) production inhibitor effective for treatment of a neurodegenerative disease caused by Aβ such as Alzheimer's disease or Down's syndrome.

BACKGROUND ART

Alzheimer's disease is a disease characterized by degeneration and loss of neurons as well as formation of senile plaques and neurofibrillary degeneration. Currently, Alzheimer's disease is treated only with symptomatic treatment using a symptom improving agent typified by an acetylcholinesterase inhibitor, and a fundamental remedy to inhibit progression of the disease has not yet been developed. It is necessary to develop a method for controlling the cause of the onset of pathology in order to create a fundamental remedy for Alzheimer's disease.
It is assumed that Aβ-proteins as metabolites of amyloid precursor proteins (hereinafter referred to as APP) are highly involved in degeneration and loss of neurons and onset of symptoms of dementia (see Non-Patent Documents 1 and 2, for example). An Aβ-protein has, as main components, Aβ40 consisting of 40 amino acids and Aβ42 with two amino acids added at the C-terminal. The Aβ40 and Aβ42 are known to have high aggregability (see Non-Patent Document 3, for example) and to be main components of senile plaques (see Non-Patent Documents 3, 4 and 5, for example). Further, it is known that the Aβ40 and Aβ42 are increased by mutation in APP and presenilin genes which is observed in familial Alzheimer's disease (see Non-Patent Documents 6, 7 and 8, for example). Accordingly, a compound that reduces production of Aβ40 and Aβ42 is expected as a progression inhibitor or prophylactic agent for Alzheimer's disease.
Aβ is produced by cleaving APP by β-secretase and subsequently by γ-secretase. For this reason, attempts have been made to create γ-secretase and β-secretase inhibitors in order to reduce Aβ production. Many of these secretase inhibitors already known are, for example, peptides and peptide mimetics such as L-685,458 (see Non-Patent Document 9, for example) and LY-411575 (see Non-Patent Documents 10, 11 and 12, for example).

Non-Patent Document 1: Klein WL, and seven others, Alzheimer's disease-affected brain: Presence of oligomeric Aβ ligands (ADDLs) suggests a molecular basis for reversible memory loss, Proceeding National Academy of Science USA 2003, Sep 2;100(18), p.10417-10422.
Non-Patent Document 2: Nitsch RM, and sixteen others, Antibodies against β-amyloid slow cognitive decline in Alzheimer's disease, Neuron, 2003, May 22;38, p.547-554.
Non-Patent Document 3: Jarrett JT, and two others, The carboxy terminus of the β amyloid protein is critical for the seeding of amyloid formation: Implications for the pathogenesis of Alzheimer's disease, Biochemistry, 1993, 32(18), p.4693-4697.
Non-Patent Document 4: Glenner GG, and another, Alzheimer's disease: initial report of the purification and characterization of a novel cerebrovascular amyloid protein, Biochemical and biophysical research communications, 1984, May 16, 120(3), p.885-890.
Non-Patent Document 5: Masters CL, and five others, Amyloid plaque core protein in Alzheimer disease and Down syndrome, Proceeding National Academy of Science USA, 1985 Jun, 82(12), p.4245-4249.
Non-Patent Document 6: Gouras GK, and eleven others, Intraneuronal Aβ42 accumulation in human brain, American Journal of Pathology, 2000, Jan, 156(1), p.15-20.
Non-Patent Document 7: Scheuner D, and twenty others, Secreted amyloid β-protein similar to that in the senile plaques of Alzheimer's disease is increased in vivo by the presenilin 1 and 2 and APP mutations linked to familial Alzheimer's disease, Nature Medicine, 1996, Aug, 2(8), p.864-870.
Non-Patent Document 8: Forman MS, and four others, Differential effects of the swedish mutant amyloid precursor protein on β-amyloid accumulation and secretion in neurons and nonneuronal cells, The Journal of Biological Chemistry, 1997, Dec 19, 272(51), p.32247-32253.
Non-Patent Document 9: Shearman MS, and nine others, L-685,458, an Aspartyl Protease Transition State Mimic, Is a Potent Inhibitor of Amyloid β-Protein Precursor γ-Secretase Activity, Biochemistry, 2000, Aug 1, 39(30), p. 8698-8704.
Non-Patent Document 10: Shearman MS, and six others, Catalytic Site-Directed γ-Secretase Complex Inhibitors Do Not Discriminate Pharmacologically betweeen Notch S3 and β-APP Clevages, Biochemistry, 2003, Jun 24, 42 (24), p.7580-7586.
Non-Patent Document 11: Lanz TA, and three others, Studies of Aβ pharmacodynamics in the brain, cerebrospinal fluid, and plasma in young (plaque-free) Tg2576 mice using the γ-secretase inhibitor N2-[(2S)-2-(3,5-difluorophenyl)-2-hydroxyethanoyl]-N1-[(7S)-5-methyl-6-oxo-6,7-dihydro-5H-dibenzo[b,d]azepin-7-yl]-L-alaninamide (LY-411575),

The journal of pharmacology and experimental therapeutics, 2004, Apr, 309(1), p.49-55.
Non-Patent Document 12: Wong GT, and twelve others, Chronic treatment with the γ-secretase inhibitor LY-411,575 inhibits β-amyloid peptide production and alters lymphopoiesis and intestinal cell differentiation, The journal of biological chemistry, 2004, Mar 26, 279(13), p.12876-12882.

DISCLOSURE OF THE INVENTION

As described above, a compound that inhibits production of Aβ40 and Aβ42 from APP has been expected as a therapeutic or prophylactic agent for a disease caused by Aβ which is typified by Alzheimer's disease. However, a nonpeptidic compound having high efficacy which inhibits production of Aβ40 and Aβ42 has not yet been known. Accordingly, there is a need for a novel low-molecular-weight compound that inhibits production of Aβ40 and Aβ42.
As a result of extensive studies, the present inventors have found a nonpeptidic cinnamide compound that inhibits production of Aβ40 and Aβ42 from APP for the first time, and thus found a prophylactic or therapeutic agent for a disease caused by Aβ which is typified by Alzheimer's disease. This finding has led to the accomplishment of the present invention.
Specifically, the present invention relates to:

1) A compound represented by the formula (I):

[Formula 1]

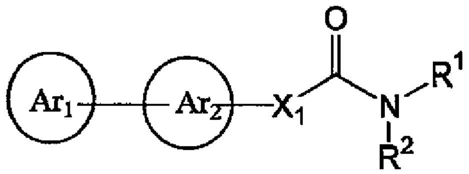

(I)

or a pharmacologically acceptable salt thereof,
wherein $Ar_1$ represents a triazolyl group or a tetrazolyl group which may be substituted with 1 to 3 substituents selected from Substituent Group A1 shown below;
$Ar_2$ represents a pyridinyl group, a pyrimidinyl group or a phenyl group which may be substituted with 1 to 3 substituents selected from Substituent Group A2 shown below;
$X_1$ represents (1) -C≡C- or (2) $-CR^3=CR^4-$ (wherein $R^3$ and
$R^4$ are the same or different and each represent a substituent selected from Substituent Group A3 shown below); and

(1) $R^1$ and $R^2$ are the same or different and each represent a group selected from Substituent Group A4 shown below; or $R^1$ and $R^2$, together with a nitrogen atom to which they are bonded, form:
(2-1) a 5- to 11-membered heterocyclic group which may be substituted with 1 to 4 substituents selected from Substituent Group A4 and is represented by the formula (II) :

[Formula 2]

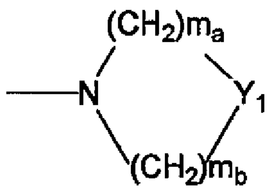

(II)

wherein $Y_1$ represents (1) -NH-, (2) -O-, (3) -S-, (4) -SO-, (5) $-SO_2-$, (6) $-CH_2-$, (7) -CO-, (8) -CONH-, (9) -NHCO-, (10) $-CR^5=CR^6-$ (wherein $R^5$ and $R^6$ are the same or different and each represent a substituent selected from

Substituent Group A4 shown below), (11) a single bond or (12) >C=CR$^{13}$R$^{14}$ (wherein R$^{13}$ and R$^{14}$ are the same or different and each represent a substituent selected from Substituent Group A4 shown below); and $m_a$ and $m_b$ are the same or different and each represent an integer of 0 to 4;

(2-2) a 6- to 20-membered non-aromatic heterocyclic group which may be substituted with 1 to 4 substituents selected from Substituent Group A4 and is represented by the formula (III):

[Formula 3]

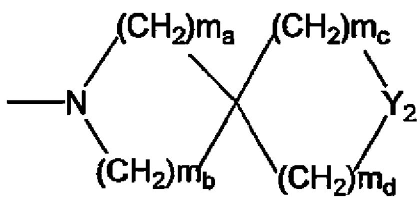

(III)

wherein $Y_2$ represents (1) -NH-, (2) -O-, (3) -S-, (4)- SO-, (5) -$SO_2$-, (6) -$CH_2$-, (7) -CO-, (8) -CONH-, (9) -NHCO-, (10) -$CR^{5a}$=$CR^{6a}$- (wherein $R^{5a}$ and $R^{6a}$ are the same or different and each represent a substituent selected from Substituent Group A4 shown below or $R^{5a}$ and $R^{6a}$, together with a carbon atom to which they are bonded, form a 6- to 14-membered aromatic hydrocarbon ring group or a 6- to 14-membered non-aromatic hydrocarbon ring group) or (11) a single bond; and $m_a$, $m_b$, $m_c$ and $m_d$ are the same or different and each represent an integer of 0 to 4;

(2-3) a 9- to 16-membered non-aromatic heterocyclic group which may be substituted with 1 to 4 substituents selected from Substituent Group A4 and is represented by the formula (IV):

[Formula 4]

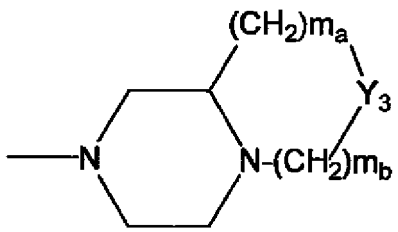

(IV)

wherein $Y_3$ represents (1) -NH-, (2) -O-, (3) -S-, (4) -SO-, (5) -$SO_2$-, (6) -$CH_2$-, (7) -CO-, (8) -CONH-, (9) -NHCO- or (10) a single bond; and $m_a$ and $m_b$ are as defined above;

(2-4) a group which may be substituted with 1 to 4 substituents selected from Substituent Group A4 and is represented by the following formula:

[Formula 5]

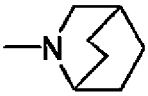,
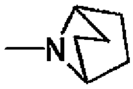,
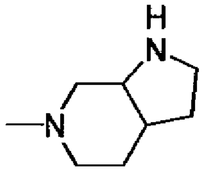
or
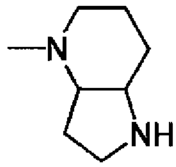

; or

(2-5) a group which may be substituted with 1 to 4 substituents selected from Substituent Group A4 and is represented by the following formula:

[Formula 6]

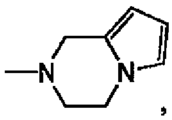,
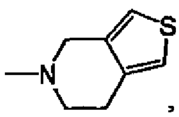,
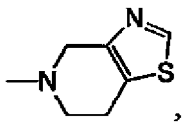,
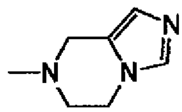,
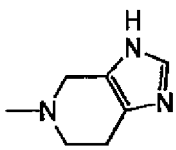,
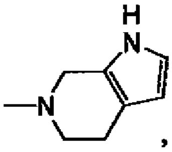,
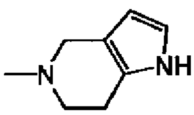,
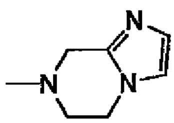,
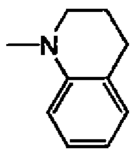,
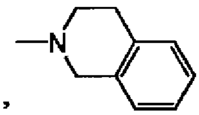,
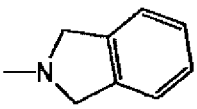
or
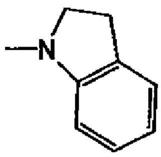

; or

$R^1$ and $R^2$, together with $-X_1$-CO-N, form:

(3-1) a cyclic group which may be substituted with 1 to 4 substituents selected from Substituent Group A4 and is represented by the formula (V):

[Formula 7]

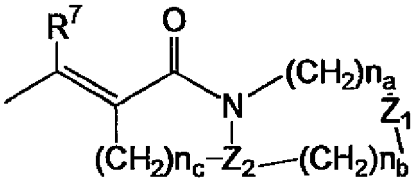 (V)

wherein $Z_1$ represents (1) -NH-, (2) -O-, (3) -S-, (4) -SO-, (5) $-SO_2-$, (6) $-CH_2-$, (7) -CO-, (8) -CONH-, (9) -NHCO- or (10) a single bond; $Z_2$ represents (1) a methine group or (2) a nitrogen atom; $R^7$ represents a substituent selected from Substituent Group A3 shown below; and $n_a$, $n_b$ and $n_c$ are the same or different and each represent an integer of 0 to 4;

(3-2) a cyclic group represented by the formula (VI):

[Formula 8]

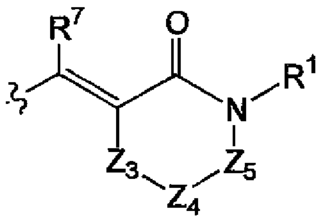

(VI)

wherein $Z_3$ represents (1) a single bond, (2) -CO-, (3) $-(CH_2)n_d-$ (wherein $n_d$ represents an integer of 1 to 3) or (4) $-CR^8R^9-$ (wherein $R^8$ and $R^9$ are the same or different and each represent a substituent selected from Substituent Group A4 shown below); $Z_4$ represents (1) a single bond, (2) -O-, (3) -NRCO-, (4) -CONR-, (5) -CSNR-, (6) -NRCS- (wherein R represents a substituent selected from Substituent Group A4 shown below) or (7) -S-; $Z_5$ represents (1) a single bond, (2) an imino group which may be substituted with a substituent selected from Substituent Group A4 shown below, (3) $-(CH_2)n_e-$(wherein $n_e$ represents an integer of 1 to 3), (4) $-CR^8R^9-$ (wherein $R^8$ and $R^9$ are as defined above) or (5) -O-; and $R^1$ and $R^7$ are as defined above; or

(3-3) a cyclic group which may be substituted with 1 to 4 substituents selected from Substituent Group A4 and is represented by the following formula:

[Formula 9]

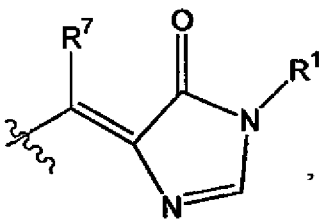

,

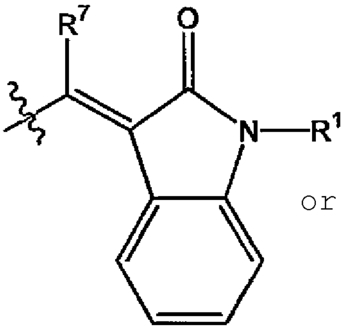

or

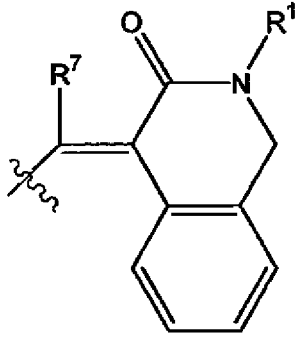

wherein $R^1$ and $R^7$ are as defined above

[Substituent Group A1: (1) a hydrogen atom, (2) a halogen atom, (3) a cyano group, (4) a nitro group, (5) a C3-8 cycloalkyl group, (6) a C2-6 alkenyl group, (7) a C2-6 alkynyl group, (8) a C1-6 alkoxy group, (9) a C3-8 cycloalkoxy group, (10) a formyl group, (11) a C1-6 alkylcarbonyl group and (12) a C1-6 alkyl group (wherein the C1-6 alkyl group may be substituted with 1 to 3 substituents selected from the group consisting of a halogen atom, a hydroxyl group, a cyano group, a C1-6 alkoxy group, a C3-8 cycloalkyl group and a C1-6 alkylcarbonyl group); Substituent Group A2: (1) a hydrogen atom, (2) a halogen atom, (3) a hydroxyl group, (4) a cyano group, (5) a C1-6 alkoxy group (wherein the C1-6 alkoxy group may be substituted with 1 to 3 substituents selected from the group consisting of a halogen atom, a cyano group, a C1-6 alkoxy group, a C2-6 alkenyl group, a C2-6 alkynyl group and a C3-8 cycloalkyl group), (6) a C3-8 cycloalkoxy group, (7) a C2-6 alkenyloxy group and (8) a C2-6 alkynyloxy group;

Substituent Group A3: (1) a hydrogen atom, (2) a halogen atom, (3) a 6- to 14-membered aromatic hydrocarbon

ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group; Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

2) The compound or pharmacologically acceptable salt thereof according to 1) above, wherein $Ar_1$ is a triazolyl group or a tetrazolyl group which may be substituted with 1 or 2 substituents selected from the group consisting of (1) a hydrogen atom, (2) a halogen atom, (3) a C3-8 cycloalkyl group, (4) a C2-6 alkenyl group, (5) a C2-6 alkynyl group and (6) a C1-6 alkyl group (wherein the C1-6 alkyl group may be substituted with 1 to 3 halogen atoms);
3) The compound or pharmacologically acceptable salt thereof according to 2) above, wherein $Ar_1$ is a triazolyl group which may be substituted with 1 or 2 substituents selected from the group consisting of (1) a hydrogen atom, (2) a halogen atom, (3) a C3-8 cycloalkyl group, (4) a C2-6 alkenyl group, (5) a C2-6 alkynyl group and (6) a C1-6 alkyl group (wherein the C1-6 alkyl group may be substituted with 1 to 3 halogen atoms);
4) The compound or pharmacologically acceptable salt thereof according to 2) above, wherein $Ar_1$ is a tetrazolyl group which may be substituted with 1 or 2 substituents selected from the group consisting of (1) a hydrogen atom, (2) a halogen atom, (3) a C3-8 cycloalkyl group, (4) a C2-6 alkenyl group, (5) a C2-6 alkynyl group and (6) a C1-6 alkyl group (wherein the C1-6 alkyl group may be substituted with 1 to 3 halogen atoms);
5) The compound or pharmacologically acceptable salt thereof according to 3) or 4) above, wherein $Ar_1$ is a triazolyl group or a tetrazolyl group which may be substituted with a C1-6 alkyl group;
6) The compound or pharmacologically acceptable salt thereof according to 1) above, wherein $Ar_2$ is a phenyl group which may be substituted with 1 to 3 substituents selected from the group consisting of (1) a hydrogen atom, (2) a halogen atom, (3) a hydroxyl group, (4) a cyano group, (6) a C1-6 alkoxy group (wherein the C1-6 alkoxy group may be substituted with 1 to 3 substituents selected from a C2-6 alkenyl group, a C2-6 alkynyl group and a C3-8 cycloalkyl group), (7) a C2-6 alkenyloxy group and (8) a C2-6 alkynyloxy group;
7) The compound or pharmacologically acceptable salt thereof according to any one of 1) to 6) above, wherein $Ar_2$ is a phenyl group which may be substituted with 1 to 3 substituents selected from the group consisting of (1) a hydrogen atom, (2) a halogen atom, (3) a cyano group and (4) a C1-6 alkoxy group;
8) The compound or pharmacologically acceptable salt thereof according to 1) above, wherein $X_1$ is -C≡C-;

9) The compound or pharmacologically acceptable salt thereof according to 1) above, wherein $X_1$ represents $-CR^3=CR^4-$ (wherein $R^3$ and $R^4$ are the same or different and each represent a substituent selected from Substituent Group A3 shown below)

[Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group; Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

10) The compound or pharmacologically acceptable salt thereof according to any one of 1) to 9) above, wherein $X_1$ is $-CR^{31}=CR^{41}-$ (wherein $R^{31}$ is a group selected from the group consisting of (1) a hydrogen atom, (2) a halogen atom, (3) a C1-6 alkyl group and (4) a C1-6 alkoxy group; and $R^{41}$ represents a substituent selected from the group consisting of (1) a hydrogen atom, (2) a halogen atom, (3) a 6- to 14-membered aromatic hydrocarbon ring group which may be substituted with 1 to 3 substituents selected from Substituent Group A5, (4) a 5- to 14-membered aromatic heterocyclic group which may be substituted with 1 to 3 substituents selected from Substituent Group A5 and (5) a C1-6 alkyl group (wherein the C1-6 alkyl group may be substituted with 1 to 3 substituents selected from the group consisting of a halogen atom, a hydroxyl group, a cyano group, a C3-8 cycloalkyl group, a C1-6 alkyl group, a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A5, a 5- to 14-membered aromatic heterocyclic group which may be substituted with 1 to 3 substituents selected from Substituent Group A5, a 5-to 14-membered non-aromatic heterocyclic group which may be substituted with 1 to 3 substituents selected from Substituent Group A5 and $-O-A^1$ (wherein $A^1$ represents a 6- to 14-membered aromatic hydrocarbon ring group which may be substituted with 1 to 3 substituents selected from Substituent Group A5 or a 5-to 14-membered aromatic heterocyclic group which may be substituted with 1 to 3 substituents selected from Substituent Group A5)))

[Substituent Group A5: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkyl group (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) an amino group (wherein the amino group may be substituted with a C1-6 alkyl group optionally having 1 to 5 halogen atoms)];

11) The compound or pharmacologically acceptable salt thereof according to 1), 9) or 10) above, wherein $X_1$ is

$-CR^{32}=CR^{42}-$ (wherein $R^{32}$ represents a hydrogen atom or a halogen atom; and $R^{42}$ represents a substituent selected from the group consisting of a hydrogen atom, a halogen atom, a C1-6 alkyl group (wherein the C1-6 alkyl group may be substituted with a C3-8 cycloalkyl group or a phenyl group) and a phenyl group);

12) The compound or pharmacologically acceptable salt thereof according to 1) above, wherein $R^1$ and $R^2$ are the same or different and each represent a group selected from Substituent Group A4 shown below [Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

13) The compound or pharmacologically acceptable salt thereof according to any one of 1) to 12) above, wherein $R^1$ is a group selected from Substituent Group A8 shown below and $R^2$ is a group selected from Substituent Group A6 shown below

[Substituent Group A6: (1) a hydrogen atom, (2) a C3-8 cycloalkyl group, (3) a C3-8 cycloalkoxy group, (4) a C1-6 alkyl group (wherein the C1-6 alkyl group may be substituted with 1 to 3 substituents selected from the group consisting of a halogen atom, a hydroxyl group, a cyano group, a C3-8 cycloalkyl group, a C3-8 cycloalkoxy group, a formyl group, a C1-6 alkylthio group, a hydroxyimino group, a C1-6 alkoxyimino group, a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7 shown below, a 5- to 14-membered aromatic heterocyclic group which may be substituted with 1 to 3 substituents selected from Substituent Group A7 shown below, a 5- to 14-membered non-aromatic heterocyclic group which may be substituted with 1 to 3 substituents selected from Substituent Group A7 and $-O-A^2$ (wherein $A^2$ represents a 6- to 14-membered aromatic hydrocarbon ring group which may be substituted with 1 to 3 substituents selected from Substituent Group A7 shown below or a 5- to 14-membered aromatic heterocyclic group which may be substituted with 1 to 3 substituents selected from Substituent Group A7 shown below)) and (5) a C1-6 alkoxy group (wherein the C1-6 alkoxy group may be substituted with 1 to 3 substituents selected from the group consisting of a halogen atom, a hydroxyl group, a cyano group, a C3-8 cycloalkyl group, a C3-8 cycloalkoxy group, a formyl group, a C1-6 alkylthio group, a hydroxyimino group, a C1-6 alkoxyimino group, a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7 shown below, a 5- to 14-membered aromatic heterocyclic group which may be substituted with 1 to 3 substituents selected from Substituent Group A7 shown below, a 5- to 14-membered non-aromatic heterocyclic group which may be substituted with 1 to 3 substituents selected from Substituent Group A7 and $-O-A^2$ (wherein $A^2$ is as defined above));

Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and $-O-A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which

may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above);

Substituent Group A8: (1) a hydrogen atom, (2) a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7, a 5-to 14-membered non-aromatic heterocyclic group which may be substituted with 1 to 3 substituents selected from Substituent Group A7 and -X-$A^2$ (wherein X represents an imino group, -O- or -S- and $A^2$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7)), (3) a 6- to 14-membered aromatic hydrocarbon ring group which may be substituted with 1 to 3 substituents selected from Substituent Group A7, (4) a 5- to 14-membered aromatic heterocyclic group which may be substituted with 1 to 3 substituents selected from Substituent Group A7 and (5) -X-$A^2$ (wherein X and $A^2$ are as defined above)];

14) The compound or pharmacologically acceptable salt thereof according to 1), 12) or 13), wherein $R^1$ is a C1-6 alkyl group (wherein the C1-6 alkyl group is a hydrogen atom, a C3-8 cycloalkoxy group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, a 6- to 14-membered aromatic hydrocarbon ring group which may be substituted with 1 to 3 substituents selected from Substituent Group A9, a 5- to 14-membered aromatic heterocyclic group which may be substituted with 1 to 3 substituents selected from Substituent Group A9 and -O-$A^4$ (wherein $A^4$ represents a 6- to 14-membered aromatic hydrocarbon ring group which may be substituted with 1 to 3 substituents selected from Substituent Group A9 or a 5- to 14-membered aromatic heterocyclic group which may be substituted with 1 to 3 substituents selected from Substituent Group A9)); and $R^2$ is (1) a hydrogen atom or (2) a C1-6 alkyl group (wherein the C1-6 alkyl group may be substituted with 1 to 3 substituents selected from the group consisting of a hydroxyl group, a C3-8 cycloalkyl group, a C3-8 cycloalkoxy group, a C1-6 alkylthio group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A9, a 5- to 14-membered aromatic heterocyclic group which may be substituted with 1 to 3 substituents selected from Substituent Group A9 and a 5- to 14-membered non-aromatic heterocyclic group which may be substituted with 1 to 3 substituents selected from Substituent Group A9)

[Substituent Group A9: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) -CO-$A^3$ (wherein $A^3$ represents a 6- to 14-membered aromatic hydrocarbon ring group), (10) a 6- to 14-membered aromatic hydrocarbon ring group which may be substituted with 1 to 3 substituents selected from Substituent Group A9 and (11) a 5- to 14-membered aromatic heterocyclic group which may be substituted with 1 to 3 substituents selected from Substituent Group A9];

15) The compound or pharmacologically acceptable salt thereof according to 1) above, wherein $R^1$ and $R^2$, together with a nitrogen atom to which they are bonded, form a 5- to 11-membered heterocyclic group which may be substituted with 1 to 4 substituents selected from Substituent Group A4 shown below and is represented by the formula (II):

[Formula 10]

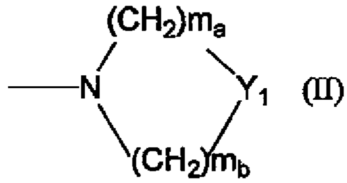

wherein $Y_1$ represents (1) -NH-, (2) -O-, (3) -S-, (4) -SO-, (5) $-SO_2-$, (6) $-CH_2-$, (7) -CO-, (8) -CONH-, (9) -NHCO-, (10) $-CR^5=CR^6-$ (wherein $R^5$ and $R^6$ are the same or different and each represent a substituent selected from Substituent Group A4 shown below), (11) a single bond or (12) $>C=CR^{13}R^{14}$ (wherein $R^{13}$ and $R^{14}$ are the same or different and each represent a substituent selected from Substituent Group A4 shown below); and $m_a$ and $m_b$ are the same or different and each represent an integer of 0 to 4

[Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

16) The compound or pharmacologically acceptable salt thereof according to any one of 1) to 15) above, wherein the 5- to 11-membered heterocyclic group is a piperidinyl group, a pyrrolidinyl group, an azepinyl group, an azocanyl group, a piperazinyl group, a 1,4-diazepanyl group, a morpholinyl group or a thiomorpholinyl group;

17) The compound or pharmacologically acceptable salt thereof according to 1), 15) or 16) above, wherein $R^1$ and $R^2$, together with a nitrogen atom to which are bonded, form a piperidinyl group, a pyrrolidinyl group, an azepinyl group, an azocanyl group, a piperazinyl group, a 1,4-diazepanyl group, a morpholinyl group or a thiomorpholinyl group which may be substituted with 1 to 3 substituents selected from the group consisting of (1) a hydrogen atom, (2) a halogen atom, (3) a hydroxyl group, (4) a formyl group, (5) a hydroxyimino group, (6) a C1-6 alkoxyimino group, (7) a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A7 shown below and a 5- to 14-membered aromatic heterocyclic group which may be substituted with 1 to 3 substituents selected from Substituent Group A7 shown below), (8) a 6- to 14-membered aromatic hydrocarbon ring group which may be substituted with 1 to 3 substituents selected from Substituent Group A7 shown below, (9) a 5- to 14-membered aromatic heterocyclic group which may be substituted with 1 to 3 substituents selected from Substituent Group A7 shown below, (10) $-O-A^2$ (wherein $A^2$ represents a 6- to 14-membered aromatic hydrocarbon ring group which may be substituted with 1 to 3 substituents selected from Substituent Group A7 shown below or a 5- to 14-membered aromatic heterocyclic group which may be substituted with 1 to 3 substituents selected from Substituent Group A7 shown below), (11) $-CO-A^2$ (wherein $A^2$ is as defined above) and (12) $=CH-A^2$ (wherein $A^2$ is as defined above)

[Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be

substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above)];

18) The compound or pharmacologically acceptable salt thereof according to 1), 15), 16) or 17), wherein $R^1$ and $R^2$, together with a nitrogen atom to which they are bonded, form a piperidinyl group, a pyrrolidinyl group, an azepinyl group, an azocanyl group, a piperazinyl group, a 1,4-diazepanyl group, a morpholinyl group or a thiomorpholinyl group which may be substituted with 1 to 4 substituents selected from the group consisting of (1) a hydrogen atom, (2) a halogen atom, (3) a hydroxyl group, (4) a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A10 shown below), (5) a 6- to 14-membered aromatic hydrocarbon ring group which may be substituted with 1 to 3 substituents selected from Substituent Group A10 shown below, (6) a 5- to 14-membered aromatic heterocyclic group which may be substituted with 1 to 3 substituents selected from Substituent Group A10 shown below, (7) -O-$A^6$ (wherein $A^6$ represents a 6- to 14-membered aromatic hydrocarbon ring group which may be substituted with 1 to 3 substituents selected from Substituent Group A10 shown below) and (8) =CH-$A^6$ (wherein $A^6$ is as defined above) [Substituent Group A10: (1) a hydrogen atom, (2) a halogen atom, (3) a C1-6 alkyl group (wherein the C1-6 alkyl group may be substituted with 1 to 5 halogen atoms), (4) a C1-6 alkoxy group and (5) a 6- to 14-membered aromatic hydrocarbon ring group];

19) The compound or pharmacologically acceptable salt thereof according to 1) above, wherein $R^1$ and $R^2$, together with a nitrogen atom to which they are bonded, form a 6- to 20-membered non-aromatic heterocyclic group which may be substituted with 1 to 4 substituents selected from Substituent Group A4 shown below and is represented by the formula (III):

[Formula 11]

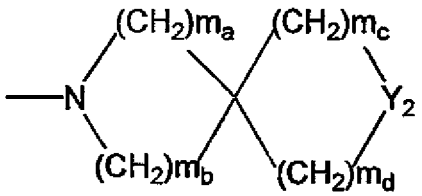

(III)

wherein $Y_2$ represents (1) -NH-, (2) -O-, (3) -S-, (4) -SO-, (5) -$SO_2$-, (6) -$CH_2$-, (7) -CO-, (8) -CONH-, (9) -NHCO-, (10) -$CR^5$= $CR^6$- (wherein $R^5$ and $R^6$ are the same or different and each represent a substituent selected from Substituent Group A4 shown below or $R^5$ and $R^6$, together with a carbon atom to which they are bonded, form a 6- to 14-membered aromatic hydrocarbon ring group or a 6- to 14-membered non-aromatic hydrocarbon ring group) or (11) a single bond; and $m_a$, $m_b$, $m_c$ and $m_d$ are the same or different and each represent an integer of 0 to 4 [Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected

from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

20) The compound or pharmacologically acceptable salt thereof according to 1) above, wherein $R^1$ and $R^2$, together with a nitrogen atom to which they are bonded, form a 9- to 16-membered non-aromatic heterocyclic group which may be substituted with 1 to 4 substituents selected from Substituent Group A4 and is represented by the formula (IV):

[Formula 12]

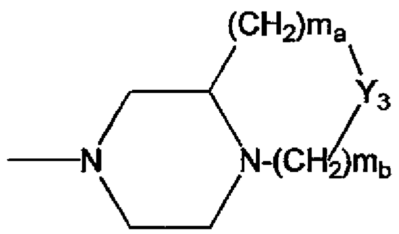

(IV)

wherein $Y_3$ represents (1) -NH-, (2) -O-, (3) -S-, (4) -SO-, (5) $-SO_2-$, (6) $-CH_2-$, (7) -CO-, (8) -CONH-, (9) -NHCO- or (10) a single bond; and $m_a$ and $m_b$ are the same or different and each represent an integer of 0 to 4 [Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

21) The compound or pharmacologically acceptable salt thereof according to 1) above, wherein $R^1$ and $R^2$, together with a nitrogen atom to which they are bonded, form a group which may be substituted with 1 to 4 substituents selected from Substituent Group A4 and is represented by the following formula:

[Formula 13]

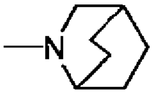, 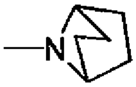, 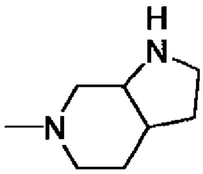 or 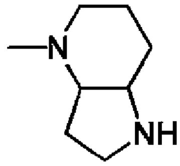

.

[Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

22) The compound or pharmacologically acceptable salt thereof according to 1) above, wherein $R^1$ and $R^2$, together with a nitrogen atom to which they are bonded, form a group which may be substituted with 1 to 4 substituents selected from Substituent Group A4 and is represented by the following formula:

[Formula 14]

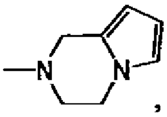, 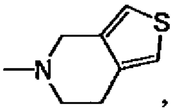, 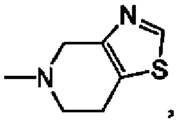, 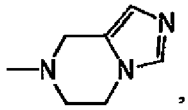, 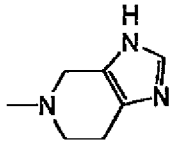, 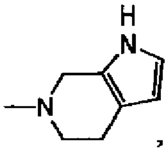, 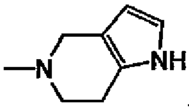, 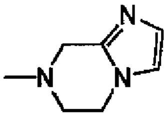, 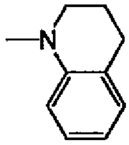, 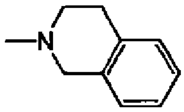,

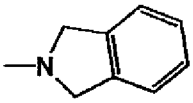 or 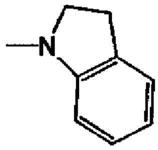

.

[Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

23) The compound or pharmacologically acceptable salt thereof according to any one of 1) to 22) above, wherein $R^1$ and $R^2$, together with a nitrogen atom to which they are bonded, form a group which may be substituted with 1 to 4 substituents selected from Substituent Group A4 and is represented by the following formula:

[Formula 15]

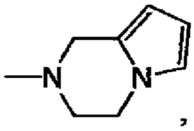, 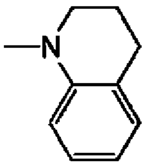, 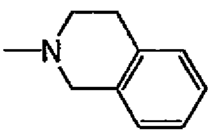, 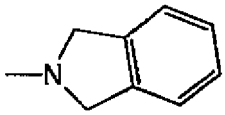

or 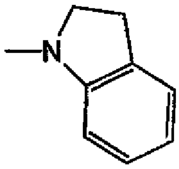

.

[Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

24) The compound or pharmacologically acceptable salt thereof according to 1), 22) or 23) above, wherein $R^1$ and $R^2$, together with a nitrogen atom to which they are bonded, form a group which may be substituted with 1 to 4 fluorine atoms;

25) The compound or pharmacologically acceptable salt thereof according to 1) above, wherein $R^1$ and $R^2$, together with $-X_1$-CO-N, form a cyclic group which may be substituted with 1 to 4 substituents selected from Substituent Group A4 and is represented by the formula (V):

[Formula 16]

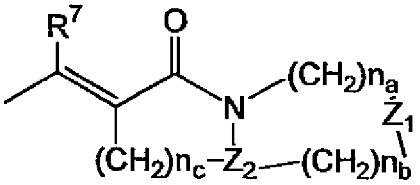 (V)

wherein $Z_1$ represents (1) -NH-, (2) -O-, (3) -S-, (4) -SO-, (5) $-SO_2-$, (6) $-CH_2-$, (7) -CO-, (8) -CONH-, (9) -NHCO- or

(10) a single bond; $Z_2$ represents (1) a methine group or (2) a nitrogen atom; $R^7$ represents a substituent selected from Substituent Group A3 shown below; and $n_a$, $n_b$ and $n_c$ are the same or different and each represent an integer of 0 to 4

[Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group; Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

26) The compound or pharmacologically acceptable salt thereof according to any one of 1) to 7) above, wherein $R^1$ and $R^2$, together with $-X_1$-CO-N, form a cyclic group which may be substituted with 1 to 4 substituents selected from Substituent Group A4 and is represented by the formula (VI):

[Formula 17]

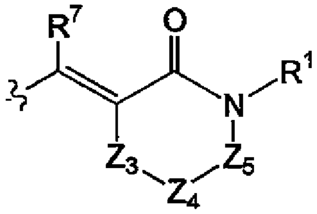

(VI)

wherein $Z_3$ represents (1) a single bond, (2) -CO-, (3) - $-CH_2)n_d$- (wherein $n_d$ represents an integer of 1 to 3) or (4) $-CR^8R^9$- (wherein $R^8$ and $R^9$ are the same or different and each represent a substituent selected from Substituent Group A4 shown below); $Z_4$ represents (1) a single bond, (2) -O-, (3) -NRCO-, (4) -CONR-, (5) -CSNR-, (6) -NRCS- (wherein R represents a substituent selected from Substituent Group A4 shown below) or (7) -S-; $Z_5$ represents (1) a single bond, (2) an imino group which may be substituted with a substituent selected from Substituent Group A4

shown below, (3) $-(CH_2)n_e-$(wherein $n_e$ represents an integer of 1 to 3), (4) $-CR^8R^9-$ (wherein $R^8$ and $R^9$ are as defined above) or (5) -O-; $R^1$ represents a substituent selected from Substituent Group A4; and $R^7$ represents a substituent selected from Substituent Group A3

[Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group; Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

27) The compound or pharmacologically acceptable salt thereof according to 1) or 26), wherein the formula (VI) represents a cyclic group which may be substituted with 1 to 4 substituents selected from Substituent Group A7 and is represented by the following formula:

[Formula 18]

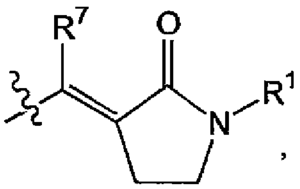
,
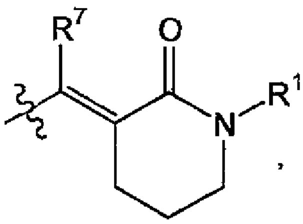
,
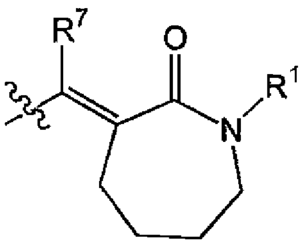

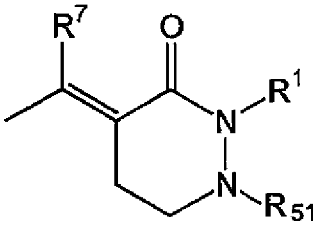
or
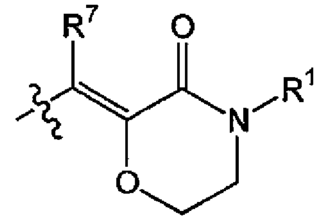

wherein $R^1$ and $R_{51}$ are the same or different and each represent a substituent selected from Substituent Group A4; and $R^7$ represents a substituent selected from Substituent Group A3

[Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group; Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above); Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6

alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above)];

28) The compound or pharmacologically acceptable salt thereof according to 1) above, wherein $R^1$ and $R^2$, together with -$X_1$-CO-N, form a cyclic group which may be substituted with 1 to 4 substituents selected from Substituent Group A4 and is represented by the following formula:

[Formula 19]

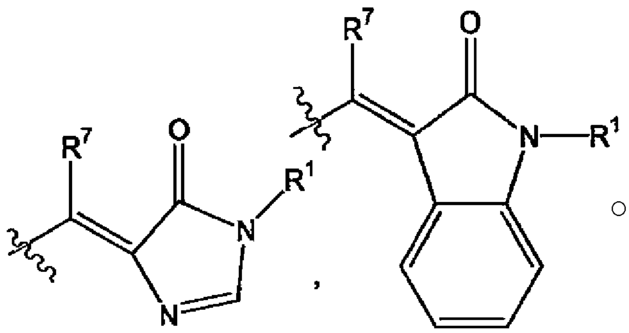

,

or

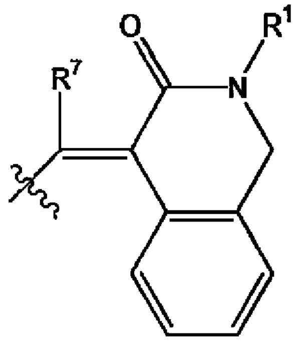

wherein $R^1$ and $R^7$ are as defined above

[Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

29) The compound or pharmacologically acceptable salt thereof according to any one of 1), 12), 13), 15) and 17) to 28) above, wherein $R^1$ is a substituent selected from Substituent Group A8

[Substituent Group A8: (1) a hydrogen atom, (2) a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6

alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7, a 5-to 14-membered non-aromatic heterocyclic group which may be substituted with 1 to 3 substituents selected from Substituent Group A7 and -X-$A^2$ (wherein X represents an imino group, -O- or -S- and $A^2$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7)), (3) a 6- to 14-membered aromatic hydrocarbon ring group which may be substituted with 1 to 3 substituents selected from Substituent Group A7, (4) a 5- to 14-membered aromatic heterocyclic group which may be substituted with 1 to 3 substituents selected from Substituent Group A7 and (5) -X-$A^2$ (wherein X and $A^2$ are as defined above); Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above)];

30) The compound or pharmacologically acceptable salt thereof according to any one of 1), 12), 13), 14), 15) and 17) to 29) above, wherein $R^1$ is a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A9, a 5- to 14-membered aromatic heterocyclic group which may be substituted with 1 to 3 substituents selected from Substituent Group A9, a 5-to 14-membered non-aromatic heterocyclic group which may be substituted with 1 to 3 substituents selected from Substituent Group A9 and -X-$A^4$ (wherein X represents an imino group, -O- or -S- and $A^4$ represents a 6- to 14-membered aromatic hydrocarbon ring group which may be substituted with 1 to 3 substituents selected from Substituent Group A9 or a 5- to 14-membered aromatic heterocyclic group which may be substituted with 1 to 3 substituents selected from Substituent Group A9))

[Substituent Group A9: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) -CO-$A^3$ (wherein $A^3$ represents a 6- to 14-membered aromatic hydrocarbon ring group), (10) a 6- to 14-membered aromatic hydrocarbon ring group which may be substituted with 1 to 3 substituents selected from Substituent Group A9 and (11) a 5- to 14-membered aromatic heterocyclic group which may be substituted with 1 to 3 substituents selected from Substituent Group A9];

31) The compound or pharmacologically acceptable salt thereof according to 1), 10), 26) or 28), wherein $R^1$ is

$-X_{21}-X_{22}-Ar_3$

(wherein $X_{21}$ represents 1) a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or

two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group) and a 5- to 14-membered non-aromatic heterocyclic group which may be substituted with 1 to 3 substituents selected from Substituent Group A7) or 2) a single bond; $X_{22}$ represents a single bond, an imino group which may be substituted with a substituent selected from Substituent Group A7, -O- or -S-; and $Ar_3$ represents a 6- to 14-membered aromatic hydrocarbon which may be substituted with 1 to 3 substituents selected from Substituent Group A7 or a 5- to 14-membered aromatic heterocyclic group which may be substituted with 1 to 3 substituents selected from Substituent Group A7) [Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above)];

32) The compound or pharmacologically acceptable salt thereof according to 1), 10), 26), 28) or 31) above, wherein $R^1$ is -$X_{21a}$-$X_{22a}$-$Ar_{3a}$

(wherein $X_{21a}$ represents a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms) and a 5- to 14-membered non-aromatic heterocyclic group which may be substituted with 1 to 3 substituents selected from Substituent Group A9); $X_{22a}$ represents a single bond or an oxygen atom; and $Ar_{3a}$ represents a 6- to 14-membered aromatic hydrocarbon ring group which may be substituted with 1 to 3 substituents selected from Substituent Group A9 or a 5- to 14-membered aromatic heterocyclic group which may be substituted with 1 to 3 substituents selected from Substituent Group A9)

[Substituent Group A9: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) -CO-$A^3$ (wherein $A^3$ represents a 6- to 14-membered aromatic hydrocarbon ring group), (10) a 6- to 14-membered aromatic hydrocarbon ring group which may be substituted with 1 to 3 substituents selected from Substituent Group A9 and (11) a 5- to 14-membered aromatic heterocyclic group which may be substituted with 1 to 3 substituents selected from Substituent Group A9];

33) The compound or pharmacologically acceptable salt thereof according to 32) above, wherein $Ar_{3a}$ is a 6- to 14-membered aromatic hydrocarbon ring group selected from the group consisting of a phenyl group, a naphthyl group and a fluorenyl group or a 5- to 14-membered aromatic heterocyclic group selected from the group consisting of a thienyl group, a pyridinyl group, a quinolinyl group, an isoquinolinyl group, an indolyl group, a benzothiazolyl group, a benzoxazolyl group and a furyl group, which may be substituted with 1 to 3 substituents selected from Substituent Group A9 [Substituent Group A9: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group

may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) -CO-$A^3$ (wherein $A^3$ represents a 6- to 14-membered aromatic hydrocarbon ring group), (10) a 6- to 14-membered aromatic hydrocarbon ring group which may be substituted with 1 to 3 substituents selected from Substituent Group A9 and (11) a 5- to 14-membered aromatic heterocyclic group which may be substituted with 1 to 3 substituents selected from Substituent Group A9];

34) The compound or pharmacologically acceptable salt thereof according to 1), wherein $R^1$ is a 6- to 14-membered non-aromatic hydrocarbon ring group or a 5- to 14-membered non-aromatic heterocyclic group represented by the formula (VII):

[Formula 20]

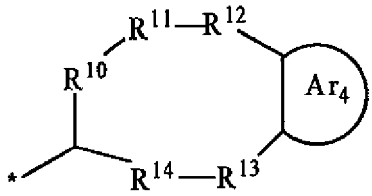

(VII)

wherein $R^{10}$ to $R^{14}$ represent 1) a single bond, 2) -CO-, 3) a methylene group which may be substituted with 1 or 2 substituents selected from Substituent Group A4, 4) -O-, 5) an imino group which may have a substituent selected from Substituent Group A4 or 6) -S-; and $Ar_4$ represents a 6- to 14-membered aromatic hydrocarbon ring group which may be substituted with 1 to 3 substituents selected from Substituent Group A4 shown below or a 5- to 14-membered aromatic heterocyclic group which may be substituted with 1 to 3 substituents selected from Substituent Group A4 shown below [Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above)];

35) The compound or pharmacologically acceptable salt thereof according to 34) above, wherein $Ar_4$ is a phenyl group or a 5- to 14-membered aromatic heterocyclic group selected from the group consisting of a pyridinyl group, a pyrimidinyl group, a pyrazinyl group, a thienyl group, an oxazolyl group, a pyrrolyl group, a thiazolyl group and a furyl group, which may be substituted with 1 to 3 substituents selected from the group consisting of a halogen atom, a C1-6 alkyl group (wherein the C1-6 alkyl group may be substituted with 1 to 3 substituents selected from the group consisting of a halogen atom and a C1-6 alkyl group), a C1-6 alkoxy group (wherein the C1-6 alkoxy group may be substituted with 1 to 3 halogen atoms), an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7, a 5-to 14-membered non-aromatic heterocyclic group which may be substituted with 1 to 3 substituents selected from Substituent Group A7 and -CO-$A^2$ (wherein $A^2$ represents a 6- to 14-membered aromatic hydrocarbon ring group

which may be substituted with 1 to 3 substituents selected from Substituent Group A7 shown below or a 5- to 14-membered aromatic heterocyclic group which may be substituted with 1 to 3 substituents selected from Substituent Group A7 shown below) [Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above)];

36) The compound or pharmacologically acceptable salt thereof according to 35) above, wherein $R^1$ is an indanyl group, an azaindanyl group, a tetrahydronaphthyl group, an azatetrahydronaphthyl group, a chromanyl group, an azachromanyl group, a tetrahydrobenzofuranyl group or a tetrahydrobenzothienyl group, which may be substituted with 1 to 3 substituents selected from the group consisting of (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 3 halogen atoms or C1-6 alkyl groups), (7) a C1-6 alkoxy group (wherein the C1-6 alkoxy group may be substituted with 1 to 3 halogen atoms), (8) an amino group (wherein the amino group may be substituted with a C1-6 alkyl group optionally having 1 to 5 halogen atoms) and (9) a 5- to 14-membered non-aromatic heterocyclic group;

37) A compound represented by the formula (VIII):

[Formula 21]

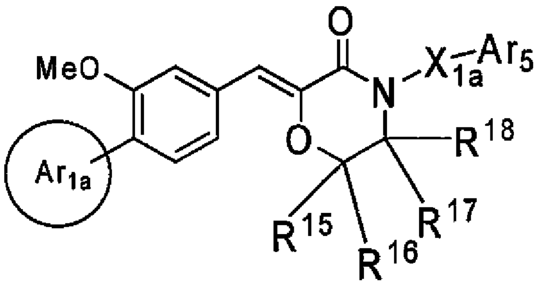

(VIII)

or a pharmacologically acceptable salt thereof,

wherein $Ar_{1a}$ represents a triazolyl group or a tetrazolyl group which may be substituted with a C1-6 alkyl group; and

(a) $R^{15}$, $R^{16}$, $R^{17}$ and $R^{18}$ are the same or different and each represent a hydrogen atom or a C1-6 alkyl group; $X_{1a}$ represents a C1-6 alkylene group (wherein the C1-6 alkylene group may be substituted with 1 to 3 hydroxyl groups or C1-6 alkyl groups (wherein the C1-6 alkyl group may be substituted with 1 to 3 hydroxyl groups)); and $Ar_5$ represents an aryl group, a pyridinyl group, an aryloxy group or a pyridinyloxy group which may be substituted with 1 to 3 substituents selected from Substituent Group All; or

(b) one of $R^{15}$ and $R^{16}$ and one of $R^{17}$ and $R^{18}$ are the same or different and each represent a hydrogen atom or a C1-6 alkyl group; the other of $R^{15}$ and $R^{16}$ and the other of $R^{17}$ and $R^{18}$, together with carbon atoms to which they are respectively bonded, form a C3-8 cycloalkyl group (wherein the C3-8 cycloalkyl group may be substituted with 1 to 3 substituents selected from Substituent Group A11); and $X_{1a}$ and $Ar_5$ are as defined in (a); or

(c) $Ar_5$-$X_{1a}$- represents a C3-8 cycloalkyl group (wherein one methylene group in the C3-8 cycloalkyl group may be substituted with an oxygen atom) condensed with a benzene ring (wherein the benzene ring may be substituted with 1 to 3 substituents selected from Substituent Group A11); and $R^{15}$, $R^{16}$, $R^{17}$ and $R^{18}$ are as defined in (a); or

(d) $Ar_5$-$X_{1a}$- and $R^{18}$, together with a nitrogen atom to which $Ar_5$-$X_{1a}$- is bonded and a carbon atom to which $R^{18}$ is bonded, form a 4- to 8-membered nitrogen-containing heterocyclic group (wherein one methylene group in the 4- to 8-membered nitrogen-containing heterocyclic group may be substituted with a methylene group or a vinylene group which may be substituted with 1 or 2 substituents selected from Substituent Group A11, an oxygen atom or an imino group which may be substituted with a C1-6 alkyl group or a C1-6 acyl group) which may be substituted with an aryl group or a pyridinyl group (wherein the aryl group or pyridinyl group may be substituted with 1 to 3 substituents selected from Substituent Group A11); and $R^{15}$, $R^{16}$ and $R^{17}$ are as defined in (a); or

(e) $R^{15}$ and $R^{16}$ form a C3-8 cycloalkyl group together; and $R^{17}$, $R^{18}$, $X_{1a}$ and $Ar_5$ are as defined in (a) and (c); or

(f) $R^{17}$ and $R^{18}$ form a C3-8 cycloalkyl group together; and $R^{15}$, $R^{16}$, $X_{1a}$ and $Ar_5$ are as defined in (a) and (c) [Substituent Group A11: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 5 halogen atoms or 1 to 3 C1-6 alkoxy groups), (7) an amino group which may be substituted with 1 or 2 C1-6 alkyl groups (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) a carbamoyl group which may be substituted with 1 or 2 C1-6 alkyl groups (wherein the C1-6 alkyl group may be substituted with 1 to 3 halogen atoms)];

38) The compound or pharmacologically acceptable salt thereof according to 37) above, wherein the compound is represented by the formula (VIII-a):

[Formula 22]

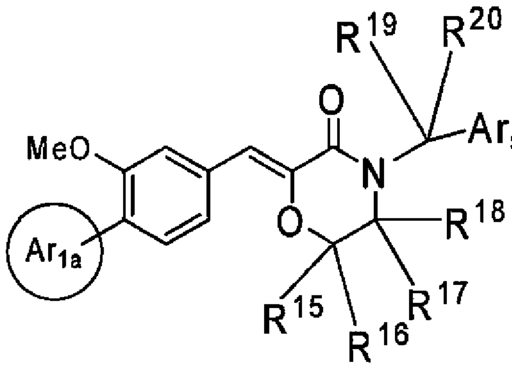

(VIII-a)

wherein $Ar_{1a}$ represents a triazolyl group or a tetrazolyl group which may be substituted with a C1-6 alkyl group; $R^{15}$, $R^{16}$, $R^{17}$ and $R^{18}$ are the same or different and each represent a hydrogen atom or a C1-6 alkyl group; $R^{19}$ and $R^{20}$ are the same or different and each represent a hydrogen atom or a C1-6 alkyl group (wherein the C1-6 alkyl group may be substituted with 1 to 3 hydroxyl groups); and $Ar_{5\text{-}a}$ represents a phenyl group or a pyridinyl group which may be substituted with 1 to 3 substituents selected from Substituent Group A11 [Substituent Group A11: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 5 halogen atoms or 1 to 3 C1-6 alkoxy groups), (7) an amino group which may be substituted with 1 or 2 C1-6 alkyl groups (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) a carbamoyl group which may be substituted with 1 or 2 C1-6 alkyl groups (wherein the C1-6 alkyl group may be substituted with 1 to 3 halogen atoms)];

39) The compound or pharmacologically acceptable salt thereof according to 38), wherein $Ar_{5\text{-}a}$ may be substituted with 1 to 3 halogen atoms;

40) The compound or pharmacologically acceptable salt thereof according to 37) above, wherein the formula (VIII) is represented by the formula (VIII-b):

[Formula 23]

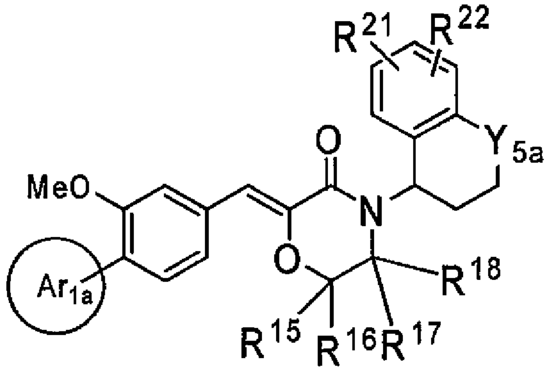

(VIII-b)

wherein $Ar_1$ represents a triazolyl group or a tetrazolyl group which may be substituted with a C1-6 alkyl group; $R^{15}$, $R^{16}$, $R^{17}$ and $R^{18}$ are the same or different and each represent a hydrogen atom or a C1-6 alkyl group;

$R^{21}$ and $R^{22}$ are the same or different and each represent a substituent selected from a hydrogen atom and Substituent Group All; and

$Y_{5a}$ represents a methylene group or an oxygen atom [Substituent Group A11: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 5 halogen atoms or 1 to 3 C1-6 alkoxy groups), (7) an amino group which may be substituted with 1 or 2 C1-6 alkyl groups (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) a carbamoyl group which may be substituted with 1 or 2 C1-6 alkyl groups (wherein the C1-6 alkyl group may be substituted with 1 to 3 halogen atoms)];

41) The compound or pharmacologically acceptable salt thereof according to 40) above, wherein $R^{21}$ and $R^{22}$ are the same or different and each represent a hydrogen atom, a halogen atom or a C1-6 alkoxy group;

42) The compound or pharmacologically acceptable salt thereof according to 37) above, wherein the formula (VIII) is represented by the formula (VIII-c):

[Formula 24]

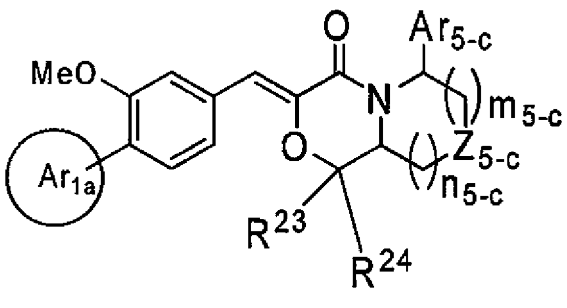

(VIII-c)

wherein $Ar_{1a}$ represents a triazolyl group or a tetrazolyl group which may be substituted with a C1-6 alkyl group;

$R^{23}$ and $R^{24}$ are the same or different and each represent a hydrogen atom or a C1-6 alkyl group;

$Ar_{5-c}$ represents a phenyl group or a pyridinyl group which may be substituted with 1 to 3 substituents selected from Substituent Group All;

$Z_{5-c}$ represents a methylene group or a vinylene group which may be substituted with 1 or 2 substituents selected from Substituent Group A11, an oxygen atom or an imino group which may be substituted with a C1-6 alkyl group or a C1-6 acyl group; and

$n_{5-c}$ and $m_{5-c}$ are the same or different and each represent an integer of 0 to 2

[Substituent Group A11: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 5 halogen atoms or 1 to 3 C1-6 alkoxy groups), (7) an amino group which may be substituted with 1 or 2 C1-6 alkyl groups (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) a carbamoyl group which may be substituted with 1 or 2 C1-6 alkyl groups (wherein the C1-6 alkyl group may be substituted with 1 to 3 halogen atoms)];

43) The compound or pharmacologically acceptable salt thereof according to 42), wherein $Z_{5-c}$ represents a methylene group (wherein the methylene group may be substituted with 1 or 2 substituents which are the same or different and are selected from the group consisting of a C1-6 alkyl group and a hydroxyl group); and $n_{5-c}$ and $m_{5-c}$ each represent 1;

44) The compound or pharmacologically acceptable salt thereof according to 42) or 43) above, wherein $Ar_{5-c}$ has 1 to 3 halogen atoms;

45) A compound represented by the formula (IX):

[Formula 25]

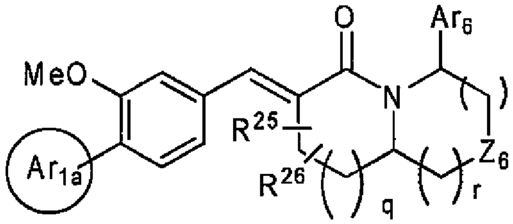

(IX)

or a pharmacologically acceptable salt thereof,

wherein $Ar_{1a}$ represents a triazolyl group or a tetrazolyl group which may be substituted with a C1-6 alkyl group; $Ar_6$ represents a phenyl group which may be substituted with 1 to 3 substituents selected from Substituent Group A12 or a pyridinyl group which may be substituted with 1 to 3 substituents selected from Substituent Group A12; $R^{25}$ and $R^{26}$ are the same or different and each represent a group selected from Substituent Group A12 shown below; $Z_6$ represents a methylene group or a vinylene group which may be substituted with 1 or 2 substituents selected from Substituent Group A11, an oxygen atom or an imino group which may be substituted with a C1-6 alkyl group or a C1-6 acyl group; and

p, q and r are the same or different and each represent an integer of 0 to 2

[Substituent Group A12: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 3 substituents selected from the group consisting of a halogen atom, a hydroxyl group, a cyano group, a C3-8 cycloalkyl group, a C1-6 alkoxy group and a C3-8 cycloalkoxy group), (7) a C1-6 alkoxy group (wherein the C1-6 alkoxy group may be substituted with 1 to 3 substituents selected from the group consisting of a halogen atom, a hydroxyl group, a cyano group, a C3-8 cycloalkyl group and a C3-8 cycloalkoxy group), (8) an amino group which may be substituted with 1 or 2 C1-6 alkyl groups (wherein the C1-6 alkyl group may be substituted with 1 to 3 halogen atoms) and (9) a carbamoyl group which may be substituted with 1 or 2 C1-6 alkyl groups (wherein the C1-6 alkyl group may be substituted with 1 to 3 halogen atoms)];

46) The compound or pharmacologically acceptable salt thereof according to 45) above, wherein $Z_6$ represents a methylene group (wherein the methylene group may be substituted with 1 or 2 substituents which are the same or different and are selected from the group consisting of a C1-6 alkyl group and a hydroxyl group); and p, q and r each represent 1;

47) The compound or pharmacologically acceptable salt thereof according to 46) above, wherein $Z_6$ represents a methylene group (wherein the methylene group may be substituted with 1 or 2 substituents which are the same or different and are selected from the group consisting of a C1-6 alkyl group and a hydroxyl group); p, q and r each represent 1; and $Ar_6$ represents a phenyl group substituted with 1 to 3 halogen atoms;

48) The compound or pharmacologically acceptable salt thereof according to 45) above, wherein $Z_6$ represents a methylene group (wherein the methylene group may be substituted with 1 or 2 substituents which are the same or different and are selected from the group consisting of a C1-6 alkyl group and a hydroxyl group); p and q each represent 1; and r represents 0;

49) The compound or pharmacologically acceptable salt thereof according to 48) above, wherein $Z_6$ represents a

methylene group (wherein the methylene group may be substituted with 1 or 2 substituents which are the same or different and are selected from the group consisting of a C1-6 alkyl group and a hydroxyl group); p and q each represent 1; r represents 0; and $Ar_6$ represents a phenyl group substituted with 2 or 3 halogen atoms;

50) The compound or pharmacologically acceptable salt thereof according to 45) above, wherein $Z_6$ represents an oxygen atom; and p, q and r each represent 1;

51) The compound or pharmacologically acceptable salt thereof according to 50) above, wherein $Z_6$ represents an oxygen atom; p, q and r each represent 1; and $Ar_6$ represents a phenyl group substituted with 1 to 3 halogen atoms;

52) The compound or pharmacologically acceptable salt thereof according to 45), 47), 49) or 51) above, wherein the halogen atom is a fluorine atom;

53) The compound or pharmacologically acceptable salt thereof according to 45) above, wherein $Z_6$ represents a methylene group (wherein the methylene group may be substituted with 1 or 2 substituents which are the same or different and are selected from the group consisting of a C1-6 alkyl group and a hydroxyl group); p represents 1; and q and r each represent 0;

54) The compound or pharmacologically acceptable salt thereof according to 45) above, wherein $Z_6$ represents a methylene group (wherein the methylene group may be substituted with 1 or 2 substituents which are the same or different and are selected from the group consisting of a C1-6 alkyl group and a hydroxyl group); p and r each represent 1; and q represents 0;

55) The compound or pharmacologically acceptable salt thereof according to 45) above, wherein $Z_6$ represents a methylene group (wherein the methylene group may be substituted with 1 or 2 substituents which are the same or different and are selected from the group consisting of a C1-6 alkyl group and a hydroxyl group); p represents 1; q represents 2; and r represents 0;

56) The compound or pharmacologically acceptable salt thereof according to 45) above, wherein $Z_6$ represents a methylene group (wherein the methylene group may be substituted with 1 or 2 substituents which are the same or different and are selected from the group consisting of a C1-6 alkyl group and a hydroxyl group); p and r each represent 1; and q represents 2;

57) The compound or pharmacologically acceptable salt thereof according to 45) above, wherein $Z_6$ represents a vinylene group (wherein the vinylene group may be substituted with 1 or 2 C1-6 alkyl groups); p represents 0; and q and r each represent 1;

58) The compound or pharmacologically acceptable salt thereof according to 45) above, wherein $Z_6$ represents a vinylene group (wherein the vinylene group may be substituted with 1 or 2 C1-6 alkyl groups); p and q each represent 1; and r represents 0;

59) A medicine comprising the compound or pharmacologically acceptable salt thereof according to any one of 1) to 58) above as an active ingredient;

60) A prophylactic or therapeutic agent for a disease caused by amyloid-β, comprising the compound or pharmacologically acceptable salt thereof according to any one of 1) to 58) above as an active ingredient; and

61) The prophylactic or therapeutic agent according to 59) above, wherein the disease caused by amyloid-β is Alzheimer's disease, dementia, Down's syndrome or amyloidosis.

The compound of the general formula (I), (VIII) or (IX) or pharmacologically acceptable salt thereof according to the present invention and the prophylactic or therapeutic agent for a disease caused by Aβ according to the present invention are novel inventions that have not yet been described in any documents.

Meanings of symbols, terms and the like used in the present specification will be explained and the present invention will be described in detail below.

In the present specification, a structural formula of a compound may represent a certain isomer for convenience. However, the present invention includes all isomers and isomer mixtures such as geometric isomers which can be generated from the structure of a compound, optical isomers based on asymmetric carbon, stereoisomers and tautomers. The present invention is not limited to the description of a chemical formula for convenience and may include any one of the isomers or mixtures thereof. Accordingly, the compound of the present invention may have an asymmetric carbon atom in the molecule and exist as an optically active compound or racemate, and the present invention includes each of the optically active compound and the racemate without limitations. Although crystal polymorphs of the compound may be present, the compound is not limited thereto as well and may be present as a single crystal form or a mixture of single crystal forms. The compound may be an anhydride or hydrate.

The "disease caused by Aβ" refers to a wide variety of diseases such as Alzheimer's disease (see Klein WL, and seven others, Alzheimer's disease-affected brain: Presence of oligomeric Aβ ligands (ADDLs) suggests a molecular basis for reversible memory loss, Proceeding National Academy of Science USA, 2003, Sep 2, 100(18), p.10417-10422; Nitsch RM, and sixteen others, Antibodies against β-amyloid slow cognitive decline in Alzheimer's disease, Neuron, 2003, May 22, 38(4), p.547-554: Jarrett JT, and two others, The carboxy terminus of the β amyloid protein is critical for the seeding of amyloid formation: Implications for the pathogenesis of Alzheimers' disease, Biochemistry, 1993, May

11, 32(18), p.4693-4697; Glenner GG, and another, Alzheimer's disease; initial report of the purification and characterization of a novel cerebrovascular amyloid protein, Biochemical and biophysical research communications, 1984, May 16, 120(3), p.885-890; Masters CL, and six others, Amyloid plaque core protein in Alzheimer disease and Down syndrome, Proceeding National Academy of Science USA, 1985, June, 82(12), p.4245-4249; Gouras GK, and eleven others, Intraneuronal Aβ42 accumulation in human brain, American journal of pathology, 2000, Jan, 156(1), p.15-20; Scheuner D, and twenty others, Secreted amyloid β-protein similar to that in the senile plaques of Alzheimer's disease is increased in vivo by the presenilin 1 and 2 and APP mutations linked to familial Alzheimer's disease, Nature Medicine, 1996, Aug, 2(8),p.864-870; Forman MS, and four others, Differential effects of the swedish mutant amyloid precursor protein on β-amyloid accumulation and secretion in neurons and nonneuronal cells, The journal of biological chemistry, 1997, Dec 19, 272(51), p.32247-32253, for example), senile dementia (see Blass JP, Brain metabolism and brain disease: Is metabolic deficiency the proximate cause of Alzheimer dementia? Journal of Neuroscience Research, 2001, Dec 1, 66 (5), p.851-856, for example), frontotemporal dementia (see Evin G, and eleven others, Alternative transcripts of presenilin-1 associated with frontotemporal dementia, Neuroreport, 2002, Apr 16, 13(5), p.719-723, for example), Pick's disease (see Yasuhara O, and three others, Accumulation of amyloid precursor protein in brain lesions of patients with Pick disease, Neuroscience Letters, 1994, Apr 25, 171(1-2), p.63-66, for example), Down's syndrome (see Teller JK, and ten others, Presence of soluble amyloid β-peptide precedes amyloid plaque formation in Down's syndrome, Nature Medicine, 1996, Jan, 2(1), p.93-95;Tokuda T, and six others, Plasma levels of amyloid β proteins Aβ1-40 and Aβ1-42 (43) are elevated in Down's syndrome, Annals of Neurology, 1997, Feb, 41(2), p.271-273, for example), cerebral angiopathy (see Hayashi Y, and nine others, Evidence for presenilin-1 involvement in amyloid angiopathy in the Alzheimer's disease-affected brain, Brain Research, 1998, Apr 13, 789(2), p.307-314; Barelli H, and fifteen others, Characterization of new polyclonal antibodies specific for 40 and 42 amino acid-long amyloid β peptides: their use to examine the cell biology of presenilins and the immunohistochemistry of sporadic Alzheimer's disease and cerebral amyloid angiopathy cases, Molecular Medicine, 1997, Oct, 3(10), p.695-707; Calhoun ME, and ten others, Neuronal overexpression of mutant amyloid precursor protein results in prominent deposition of cerebrovascular amyloid, Proceeding National Academy of Science USA, 1999, Nov 23, 96(24), p.14088-14093; Dermaut B, and ten others, Cerebral amyloid angiopathy is a pathogenic lesion in Alzheimer's Disease due to a novel presenilin-1 mutation, Brain, 2001, Dec, 124(12), p. 2383-2392, for example), hereditary cerebral hemorrhage with amyloidosis (Dutch type) (see Cras P, and nine others, Presenile Alzheimer dementia characterized by amyloid angiopathy and large amyloid core type senile plaques in the APP 692A1a-->Gly mutation, Acta Neuropathologica(Berl), 1998, Sep, 96(3), p.253-260; Herzig MC, and fourteen others, Aβ is targeted to the vasculature in a mouse model of hereditary cerebral hemorrhage with amyloidosis, Nature Neuroscience, 2004, Sep, 7(9), p.954-960; van Duinen SG, and five others, Hereditary cerebral hemorrhage with amyloidosis in patients of Dutch origin is related to Alzheimer disease, Proceeding National Academy of Science USA, 1987, Aug, 84(16), p.5991-5994; Levy E, and eight others, Mutation of the Alzheimer's disease amyloid gene in hereditary cerebral hemorrhage, Dutch type, Science, 1990, Jun 1, 248(4959), p.1124-1126, for example), cognitive impairment (see Laws SM, and seven others, Association between the presenilin-1 mutation Glu318Gly and complaints of memory impairment, Neurobiology of Aging, 2002, Jan-Feb, 23(1), p.55-58, for example), dysmnesia and learning disability (see Vaucher E, and five others, Object recognition memory and cholinergic parameters in mice expressing human presenilin 1 transgenes, Experimental Neurology, 2002 Jun, 175(2), p.398-406; Morgan D, and fourteen others, Aβ peptide vaccination prevents memory loss in an animal model of Alzheimer's disease, Nature, 2000 Dec 21-28, 408(6815), p.982-985; Moran PM, and three others, Age-related learning deficits in transgenic mice expressing the 751-amino acid isoform of human β-amyloid precursor protein, Proceeding National Academy of Science USA, 1995, June 6, 92(12), p.5341-5345, for example), amyloidosis, cerebral ischemia (see Laws SM, and seven others, Association between the presenilin-1 mutation Glu318Gly and complaints of memory impairment, Neurobiology of Aging, 2002, Jan-Feb, 23(1), p.55-58; Koistinaho M, and ten others, β-amyloid precursor protein transgenic mice that harbor diffuse Aβ deposits but do not form plaques show increased ischemic vulnerability: Role of inflammation, Proceeding National Academy of Science USA, 2002, Feb 5, 99(3), p.1610-1615; Zhang F, and four others, Increased susceptibility to ischemic brain damage in transgenic mice overexpressing the amyloid precursor protein, The journal of neuroscience, 1997, Oct 15, 17(20), p. 7655-7661, for example), vascular dementia (see Sadowski M, and six others, Links between the pathology of Alzheimer's disease and vascular dementia, Neurochemical Research, 2004, Jun, 29(6), p.1257-1266, for example), ophthalmoplegia (see O'Riordan S, and seven others, Presenilin-1 mutation(E280G), spastic paraparesis, and cranial MRI white-matter abnormalities, Neurology, 2002, Oct 8, 59(7), p.1108-1110, for example), multiple sclerosis (see Gehrmann J, and four others, Amyloid precursor protein (APP) expression in multiple sclerosis lesions, Glia, 1995, Oct, 15(2), p.141-51; Reynolds WF, and six others, Myeloperoxidase polymorphism is associated with gender specific risk for Alzheimer's disease, Experimental Neurology, 1999, Jan, 155(1), p.31-41, for example), head injury, skull injury (see Smith DH, and four others, Protein accumulation in traumatic brain injury, NeuroMolecular Medicine, 2003, 4(1-2), p.59-72, for example), apraxia (see Matsubara-Tsutsui M, and seven others, Molecular evidence of presenilin 1 mutation in familial early onset dementia, American journal of Medical Genetics, 2002, Apr 8, 114(3), p.292-298, for example), prion disease, familial amyloid neuropathy, triplet repeat disease (see Kirkitadze MD, and two others, Paradigm shifts in Alzheimer's disease

and other neurodegenerative disorders: the emerging role of oligomeric assemblies, Journal of Neuroscience Research, 2002, Sep 1, 69(5), p.567-577; Evert BO, and eight others, Inflammatory genes are upreglulated in expanded ataxin-3-expressing cell lines and spinocerebellar ataxia type 3 brains, The Journal of Neuroscience, 2001, Aug 1, 21(15), p. 5389-5396; Mann DM, and another, Deposition of amyloid(A4) protein within the brains of persons with dementing disorders other than Alzheimer's disease and Down's syndrome, Neuroscience Letters, 1990, Feb 5, 109(1-2), p.68-75, for example), Parkinson's disease (see Primavera J, and four others, Brain accumulation of amyloid-β in Non-Alzheimer Neurodegeneration, Jornal of Alzheimer's Disease, 1999, Oct, 1(3), p.183-193, for example), Lewy body dementia (see Giasson BI, and two others, Interactions of amyloidogenic proteins. NeuroMolecular Medicine, 2003, 4(1-2), p.49-58; Masliah E, and six others, β-amyloid peptides enhance α-synuclein accumulation and neuronal deficits in a trancgenic mouse model linking Alzheimer's disease and Parkinson's disease, Proceeding National Academy of Science USA, 2001, Oct 9, 98(21), p.12245-12250; Barrachina M, and six others, Amyloid-β deposition in the cerebral cortex in Dementia with Lewy bodies is accompanied by a relative increase in AβPP mRNA isoforms containing the Kunitz protease inhibitor, Neurochemistry International, 2005, Feb, 46(3), p.253-260; Primavera J, and four others, Brain accumulation of amyloid-β in Non-Alzheimer Neurodegeneration, Jornal of Alzheimer's Disease, 1999, Oct, 1(3), p.183-193, for example), parkinsonism-dementia complex (see Schmidt ML, and six others, Amyloid plaques in Guam amyotrophic lateral sclerosis/ parkinsonism-dementia complex contain species of Aβ similar to those found in the amyloid plaques of Alzheimer's disease and pathological aging, Acta Neuropathologica (Berl), 1998, Feb, 95(2), p.117-122; Ito H, and three others, Demonstration of β amyloid protein-containing neurofibrillary tangles in parkinsonism-dementia complex on Guam, Neuropathology and applied neurobiology, 1991, Oct, 17(5), p. 365-373, for example), frontotemporal dementia and parkinsonism linked to chromosome 17 (see Rosso SM, and three others, Coexistent tau andamyloid pathology in hereditary frontotemporal dementia with tau mutations, Annals of the New York academy of sciences, 2000, 920, p. 115-119, for example), argyrophilic grain dementia (see Tolnay M, and four others, Low amyloid(Aβ) plaque load and relative predominance of diffuse plaques distinguish argyrophilic grain disease from Alzheimer's disease, Neuropathology and applied neurobiology, 1999, Aug, 25(4), p.295-305, for example), Niemann-Pick disease (see Jin LW, and three others, Intracellular accumulation of amyloidogenic fragments of amyloid-β precursor protein in neurons with Niemann-Pick type C defects is associated with endosomal abnormalities, American Journal of Pathology, 2004, Mar, 164(3), p. 975-985, for example), amyotrophic lateral sclerosis (see Sasaki S, and another, Immunoreactivity of β-amyloid precursor protein in amyotrophic lateral sclerosis, Acta Neuropathologica(Berl), 1999, May, 97(5), p.463-468; Tamaoka A, and four others, Increased amyloid β protein in the skin of patients with amyotrophic lateral sclerosis, Journal of neurology, 2000, Aug, 247(8), p.633-635; Hamilton RL, and another, Alzheimer disease pathology in amyotrophic lateral sclerosis, Acta Neuropathologica, 2004, Jun, 107(6), p.515-522; Turner BJ, and six others, Brain β-amyloidaccumulation in transgenic mice expressing mutant superoxide dismutase 1, Neurochemical Research, 2004, Dec, 29(12), p.2281-2286, for example), hydrocephalus (see Weller RO, Pathology of cerebrospinal fluid and interstitial fluid of the CNS: Significance for Alzheimer disease, prion disorders and multiple sclerosis, Journal of Neuropathology and Experimental Neurology, 1998, Oct, 57(10), p.885-894; Silverberg GD, and four others, Alzheimer's disease, normal-pressure hydrocephalus, and senescent changes in CSF circulatory physiology: a hypothesis, Lancet neurology, 2003, Aug, 2(8), p.506-511; Weller RO, and three others, Cerebral amyloid angiopathy: Accumulation of Aβ in interstitial fluid drainage pathways in Alzheimer's disease, Annals of the New York academy of sciences, 2000, Apr, 903, p.110-117; Yow HY, and another, A role for cerebrovascular disease in determining the pattern of β-amyloid deposition in Alzheimer's disease, Neurology and applied neurobiology, 2002, 28, p.149; Weller RO, and four others, Cerebrovasculardisease is a major factor in the failure of elimination of Aβ from the aging human brain, Annals of the New York academy of sciences, 2002, Nov, 977, p.162-168, for example), paraparesis (see O'Riordan S, and seven others, Presenilin-1 mutation(E280G), spastic paraparesis, and cranial MRI white-matter abnormalities, Neurology, 2002, Oct 8, 59(7), p.1108-1110; Matsubara-Tsutsui M, and seven others, Molecular evidence of presenilin 1 mutation in familial early onset dementia, American journal of Medical Genetics, 2002, Apr 8, 114(3), p.292-298; Smith MJ, and eleven others, Variable phenotype of Alzheimer's disease with spastic paraparesis, Annals of Neurology, 2001, 49(1), p.125-129; Crook R, and seventeen others, A variant of Alzheimer's disease with spastic pararesis and unusual plaques due to deletion of exon 9 of presenilin 1, Nature Medicine, 1998, Apr;4(4), p.452-455, for example), progressive supranuclear palsy (see Barrachina M, and six others, Amyloid-β deposition in the cerebral cortex in Dementia with Lewy bodies is accompanied by a relative increase in AβPP mRNA isoforms containing the Kunitz protease inhibitor, Neurochemistry International, 2005, Feb, 46(3), p.253-260; Primavera J, and four others, Brain accumulation of amyloid-β in Non-Alzheimer Neurodegeneration, Jornal of Alzheimer's Disease, 1999, Oct, 1(3), p.183-193, for example), intracerebral hemorrhage (see Atwood CS, and three others, Cerebrovascular requirement for sealant, anti-coagulant and remodeling molecules that allow for the maintenance of vascular integrity and blood supply, Brain Research Reviews, 2003, Sep, 43(1), p.164-78; Lowenson JD, and two others, Protein aging: Extracellular amyloid formation and intracellular repair, Trends in cardiovascular medicine, 1994, 4 (1), p. 3-8, for example), convulsion (see Singleton AB, and thirteen others, Pathology of early-onset Alzheimer's disease cases bearing the Thr113-114ins presenilin-1 mutation, Brain, 2000, Dec, 123(Pt12), p.2467-2474, for example), mild cognitive impairment (see Gattaz WF, and four others, Platelet phospholipase A2 activity in Alzheimer's disease and mild cognitive

impairment, Journal of Neural Transmission, 2004, May, 111(5), p.591-601; Assini A, and fourteen others, Plasma levels of amyloid β-protein 42 are increased in women with mild cognitive impariment, Neurology, 2004, Sep 14, 63(5), p. 828-831, for example), arteriosclerosis (see De Meyer GR, and eight others, Platelet phagocytosis and processing of β-amyloid precursor protein as a mechanism of macrophage activation in atherosclerosis, Circulation Research, 2002, Jun 14, 90(11), p.1197-1204, for example).

Meanings of symbols, terms and the like describing the general formula (I) in the present specification will be explained and the present invention will be described in detail below.

The "6- to 14-membered cyclic aromatic hydrocarbon ring group", "5- to 14-membered aromatic heterocyclic group", "6- to 14-membered non-aromatic hydrocarbon ring group" and "5- to 14-membered non-aromatic heterocyclic group" in the formula (I) which are contained in the therapeutic or prophylactic agent for a disease caused by Aβ according to the present invention have the following meanings.

The "6- to 14-membered cyclic aromatic hydrocarbon ring group" refers to a monocyclic, bicyclic or tricyclic aromatic hydrocarbon group having 6 to 14 carbon atoms. Preferable examples of the group include 6- to 14-membered monocyclic, bicyclic or tricyclic aromatic hydrocarbon groups such as a phenyl group, an indenyl group, a naphthyl group, an azulenyl group, a heptalenyl group, a biphenyl group, a fluorenyl group, a phenalenyl group, a phenanthrenyl group and an anthracenyl group.

The "5- to 14-membered aromatic heterocyclic group" refers to a monocyclic, bicyclic or tricyclic aromatic heterocyclic group having 5 to 14 carbon atoms. Preferable examples of the group include (1) nitrogen-containing aromatic heterocyclic groups such as a pyrrolyl group, a pyridyl group, a pyridazinyl group, a pyrimidinyl group, a pyrazinyl group, a pyrazolinyl group, an imidazolyl group, an indolyl group, an isoindolyl group, an indolizinyl group, a purinyl group, an indazolyl group, a quinolyl group, an isoquinolyl group, a quinolizinyl group, a phthalazinyl group, a naphthyridinyl group, a quinoxalinyl group, a quinazolinyl group, a cinnolinyl group, a pteridinyl group, an imidazotriazinyl group, a pyrazinopyridazinyl group, an acridinyl group, a phenanthridinyl group, a carbazolyl group, a perimidinyl group, a phenanthrolinyl group and a phenacyl group, (2) sulfur-containing aromatic heterocyclic groups such as a thienyl group and a benzothienyl group, (3) oxygen-containing aromatic heterocyclic groups such as a furyl group, a pyranyl group, a cyclopentapyranyl group, a benzofuranyl group and an isobenzofuranyl group and (4) aromatic heterocyclic groups containing two or more hetero atoms selected from the group consisting of a nitrogen atom, a sulfur atom and an oxygen atom such as a thiazolyl group, an isothiazolyl group, a benzothiazolinyl group, a benzothiadiazolyl group, a phenothiazinyl group, an isoxazolyl group, a furazanyl group, a phenoxazinyl group, a pyrazoloxazolyl group, an imidazothiazolyl group, a thienofuryl group, a furopyrrolyl group and a pyridooxazinyl group.

The "6- to 14-membered non-aromatic hydrocarbon ring group" refers to a cyclic aliphatic hydrocarbon group having 6 to 14 carbon atoms. Examples of the group include cyclic aliphatic hydrocarbon groups having 6 to 14 carbon atoms such as a cyclopropyl group, a cyclobutyl group, a cyclopentyl group, a cyclohexyl group, a cycloheptyl group, a cyclooctyl group, a spiro[3.4]octanyl group, a decanyl group, an indanyl group, a 1-acenaphthenyl group, a cyclopentacyclooctenyl group, a benzocyclooctenyl group, an indenyl group, a tetrahydronaphthyl group, a 6,7,8,9-tetrahydro-5H-benzocycloheptenyl group and a 1,4-dihydronaphthalenyl group.

The "5- to 14-membered non-aromatic heterocyclic group" 1) has 5 to 14 ring-forming atoms, 2) contains 1 to 5 hetero atoms such as a nitrogen atom, -O- or -S- in the ring-forming atoms, and 3) may contain one or more carbonyl groups, double bonds or triple bonds in the ring, and refers not only to a 5-to 14-membered non-aromatic monocyclic heterocyclic group but also to a saturated heterocyclic group condensed with an aromatic hydrocarbon ring group or a saturated hydrocarbon ring group or saturated heterocyclic group condensed with an aromatic heterocyclic group. Specific examples of the 5- to 14-membered non-aromatic heterocyclic group include an azetidinyl ring, a pyrrolidinyl ring, a piperidinyl ring, an azepanyl ring, an azocanyl ring, a tetrahydrofuranyl ring, a tetrahydropyranyl ring, a morpholinyl ring, a thiomorpholinyl ring, a piperazinyl ring, a thiazolidinyl ring, a dioxanyl ring, an imidazolinyl ring, a thiazolinyl ring, a 1,2-benzopyranyl ring, an isochromanyl ring, a chromanyl ring, an indolinyl ring, an isoindolinyl ring, an azaindanyl group, an azatetrahydronaphthyl group, an azachromanyl group, a tetrahydrobenzofuranyl group, a tetrahydrobenzothienyl group, a 2,3,4,5-tetrahydrobenzo[b]thienyl group, a 3,4-dihydro-2H-benzo[b][1,4]dioxepinyl group, an indan-1-onyl group, a 6,7-dihydro-5H-cyclopentapyrazinyl group, a 6,7-dihydro-5H-[1]pyridinyl group, a 6,7-dihydro-5H-[1]pyridinyl group, a 5,6-dihydro-4H-cyclopenta[b]thienyl group, a 4,5,6,7-tetrahydrobenzo[b]thienyl group, a 3,4-dihydro-2H-naphthale-1-onyl group, a 2,3-dihydro-isoindol-1-onyl group, a 3,4-dihydro-2H-isoquinolin-1-onyl group and a 3,4-dihydro-2H-benzo[1,4]oxapinyl group.

Substituent Group A1, Substituent Group A2, Substituent Group A3, Substituent Group A4, Substituent Group A5, Substituent Group A6, Substituent Group A7, Substituent Group A8, Substituent Group A9 and Substituent Group A10 refer to the following groups.

Substituent Group A1 refers to (1) a hydrogen atom, (2) a halogen atom, (3) a cyano group, (4) a nitro group, (5) a C3-8 cycloalkyl group, (6) a C2-6 alkenyl group, (7) a C2-6 alkynyl group, (8) a C1-6 alkoxy group, (9) a C3-8 cycloalkoxy group, (10) a formyl group, (11) a C1-6 alkylcarbonyl group or (12) a C1-6 alkyl group (wherein the C1-6 alkyl group may be substituted with 1 to 3 substituents selected from the group consisting of a halogen atom, a hydroxyl

group, a cyano group, a C1-6 alkoxy group, a C3-8 cycloalkyl group and a C1-6 alkylcarbonyl group).

Substituent Group A2 refers to (1) a hydrogen atom, (2) a halogen atom, (3) a hydroxyl group, (4) a cyano group, (5) a C1-6 alkoxy group (wherein the C1-6 alkoxy group may be substituted with 1 to 3 substituents selected from the group consisting of a halogen atom, a cyano group, a C1-6 alkoxy group, a C2-6 alkenyl group, a C2-6 alkynyl group and a C3-8 cycloalkyl group), (6) a C3-8 cycloalkoxy group, (7) a C2-6 alkenyloxy group and (8) a C2-6 alkynyloxy group.

Substituent Group A3 refers to (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group.

Substituent Group A4 refers to (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 to 2 substituents selected from Substituent Group A4, (21) a carbamoyl group which may be substituted with 1 to 2 substituents selected from Substituent Group A4, (22) a 6- to 14-membered aromatic hydrocarbon ring group which may be substituted with 1 to 5 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4), (31) -CO-A (wherein A is as defined above) and (32) =CH-A (wherein A is as defined above).

Substituent Group A5 refers to (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkyl group (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) an amino group (wherein the amino group may be substituted with a C1-6 alkyl group optionally having 1 to 5 halogen atoms).

Substituent Group A6 refers to (1) a hydrogen atom, (2) a C3-8 cycloalkyl group, (3) a C3-8 cycloalkoxy group, (4) a C1-6 alkyl group (wherein the C1-6 alkyl group may be substituted with 1 to 3 substituents selected from the group consisting of a halogen atom, a hydroxyl group, a cyano group, a C3-8 cycloalkyl group, a C3-8 cycloalkoxy group, a formyl group, a C1-6 alkylthio group, a hydroxyimino group, a C1-6 alkoxyimino group, a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7 shown below, a 5- to 14-membered aromatic heterocyclic group which may be substituted with 1 to 3 substituents selected from Substituent Group A7 shown below, a 5- to 14-membered non-aromatic heterocyclic group which may be substituted with 1 to 3 substituents selected from Substituent Group A7 and -O-$A^2$ (wherein $A^2$ represents a 6- to 14-membered aromatic hydrocarbon ring group which may be substituted with 1 to 3 substituents selected from Substituent Group A7 shown below or a 5- to 14-membered aromatic heterocyclic group which may be substituted with 1 to 3 substituents selected from Substituent Group A7 shown below)) and (5) a C1-6 alkoxy group (wherein the C1-6 alkoxy group may be substituted with 1 to 3 substituents selected from the group consisting of a halogen atom, a hydroxyl group, a cyano group, a C3-8 cycloalkyl group, a C3-8 cycloalkoxy group, a formyl group, a C1-6 alkylthio group, a hydroxyimino group, a C1-6 alkoxyimino group, a C1-6 alkoxy group, an amino group (wherein

the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7 shown below, a 5- to 14-membered aromatic heterocyclic group which may be substituted with 1 to 3 substituents selected from Substituent Group A7 shown below, a 5- to 14-membered non-aromatic heterocyclic group which may be substituted with 1 to 3 substituents selected from Substituent Group A7 and -O-$A^2$ (wherein $A^2$ is as defined above)).

Substituent Group A7 refers to (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6-to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above).

Substituent Group A8 refers to (1) a hydrogen atom, (2) a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7, a 5- to 14-membered non-aromatic heterocyclic group which may be substituted with 1 to 3 substituents selected from Substituent Group A7 and -X-$A^2$ (wherein X represents an imino group, -O- or -S- and $A^2$ represents a 6- to 14-membered aromatic hydrocarbon ring group which may be substituted with 1 to 3 substituents selected from Substituent Group A7 or a 5-to 14-membered aromatic heterocyclic group which may be substituted with 1 to 3 substituents selected from Substituent Group A7)), (3) a 6- to 14-membered aromatic hydrocarbon ring group which may be substituted with 1 to 3 substituents selected from Substituent Group A7, (4) a 5- to 14-membered aromatic heterocyclic group which may be substituted with 1 to 3 substituents selected from Substituent Group A7 and (5) -X-$A^2$ (wherein X and $A^2$ are as defined above).

Substituent Group A9 refers to (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) -CO-$A^3$ (wherein $A^3$ represents a 6- to 14-membered aromatic hydrocarbon ring group), (10) a 6- to 14-membered aromatic hydrocarbon ring group which may be substituted with 1 to 3 substituents selected from Substituent Group A9 and (11) a 5- to 14-membered aromatic heterocyclic group which may be substituted with 1 to 3 substituents selected from Substituent Group A9.

Substituent Group A10 refers to (1) a hydrogen atom, (2) a halogen atom, (3) a C1-6 alkyl group (wherein the C1-6 alkyl group may be substituted with 1 to 5 halogen atoms), (4) a C1-6 alkoxy group and (5) a 6- to 14-membered aromatic hydrocarbon ring group.

The "halogen atom" refers to a fluorine atom, a chlorine atom, a bromine atom, an iodine atom or the like and is preferably a fluorine atom, a chlorine atom or a bromine atom.

The "C1-6 alkyl group" refers to an alkyl group having 1 to 6 carbon atoms. Preferable examples of the group include linear or branched alkyl groups such as a methyl group, an ethyl group, an n-propyl group, an i-propyl group, an n-butyl group, an i-butyl group, a tert-butyl group, an n-pentyl group, an i-pentyl group, a neopentyl group, an n-hexyl group, a 1-methylpropyl group, an 1,2-dimethylpropyl group, a 1-ethylpropyl group, a 1-methyl-2-ethylpropyl group, a 1-ethyl-2-methylpropyl group, a 1,1,2-trimethylpropyl group, a 1-methylbutyl group, a 2-methylbutyl group, a 1,1-dimethylbutyl group, a 2,2-dimethylbutyl group, a 2-ethylbutyl group, a 1,3-dimethylbutyl group, a 2-methylpentyl group and a

3-methylpentyl group.

The "C1-6 alkoxy group" refers to an alkyl group having 1 to 6 carbon atoms in which a hydrogen atom is replaced by an oxygen atom. Preferable examples of the group include a methoxy group, an ethoxy group, an n-propoxy group, an i-propoxy group, an n-butoxy group, an i-butoxy group, a sec-butoxy group, a tert-butoxy group, an n-pentoxy group, an i-pentoxy group, a sec-pentoxy group, a tert-pentoxy group, an n-hexoxy group, an i-hexoxy group, a 1,2-dimethylpropoxy group, a 2-ethylpropoxy group, a 1-methyl-2-ethylpropoxy group, a 1-ethyl-2-methylpropoxy group, a 1,1,2-trimethylpropoxy group, a 1,1,2-trimethylpropoxy group, a 1,1-dimethylbutoxy group, a 2,2-dimethylbutoxy group, a 2-ethylbutoxy group, a 1,3-dimethylbutoxy group, a 2-methylpentoxy group, a 3-methylpentoxy group and a hexyloxy group.

The "C1-6 alkylsulfonyl group" refers to an alkyl group having 1 to 6 carbon atoms in which one hydrogen atom is replaced by a sulfonyl group. Preferable examples of the group include a methanesulfonyl group and an ethanesulfonyl group.

The "amino group which may be substituted with a C1-6 alkyl group" refers to an amino group which may be substituted with an alkyl group having 1 to 6 carbon atoms. Preferable examples of the group include an amino group, a methylamino group, an ethylamino group, a propylamino group and a dimethylamino group.

The "C2-6 alkenyl group" refers to an alkenyl group having 2 to 6 carbon atoms. Preferable examples of the group include linear or branched alkenyl groups such as a vinyl group, an allyl group, a 1-propenyl group, an isopropenyl group, a 1-buten-1-yl group, a 1-buten-2-yl group, a 1-buten-3-yl group, a 2-buten-1-yl group and a 2-buten-2-yl group.

The "C2-6 alkynyl group" refers to an alkynyl group having 2 to 6 carbon atoms. Preferable examples of the group include linear or branched alkynyl groups such as an ethynyl group, a 1-propynyl group, a 2-propynyl group, a butynyl group, a pentynyl group and a hexynyl group.

The "C3-8 cycloalkyl group" refers to a cyclic alkyl group having 3 to 8 carbon atoms. Preferable examples of the group include a cyclopropyl group, a cyclobutyl group, a cyclopentyl group, a cyclohexyl group, a cycloheptyl group and a cyclooctyl group.

The "C1-6 alkylthio group" refers to an alkyl group having 1 to 6 carbon atoms in which one hydrogen atom is replaced by a sulfur atom. Preferable examples of the group include a methylthio group, an ethylthio group, an n-propylthio group, an i-propylthio group, an n-butylthio group, an i-butylthio group, a tert-butylthio group, an n-pentylthio group, an i-pentylthio group, a neopentylthio group, an n-hexylthio group and a 1-methylpropylthio group.

The "C1-6 alkylsulfinyl group" refers to an alkyl group having 1 to 6 carbon atoms in which one hydrogen atom is replaced by a sulfinyl group. Preferable examples of the group include a methylsulfinyl group, an ethylmethylsulfinyl group, an n-propylsulfinyl group, an i-propylsulfinyl group, an n-butylsulfinyl group, an i-butylsulfinyl group, a tert-butylsulfinyl group, an n-pentylsulfinyl group, an i-pentylsulfinyl group, a neopentylsulfinyl group, an n-hexylsulfinyl group and a 1-methylpropylsulfinyl group.

The "C1-6 alkylcarbonyl group" refers to an alkyl group having 1 to 6 carbon atoms in which one hydrogen atom is replaced by a carbonyl group. Preferable examples of the group include an acetyl group, a propionyl group and a butyryl group.

The "C3-8 cycloalkoxy group" refers to a cyclic alkyl group having 3 to 8 carbon atoms in which one hydrogen atom is replaced by an oxygen atom. Preferable examples of the group include a cyclopropoxy group, a cyclobutoxy group, a cyclopentoxy group, a cyclohexoxy group, a cycloheptyloxy group and a cyclooctyloxy group.

The "C3-8 cycloalkylthio group" refers to a cyclic alkyl group having 3 to 8 carbon atoms in which one hydrogen atom is replaced by a sulfur atom. Preferable examples of the group include a cyclopropylthio group, a cyclobutylthio group, a cyclopentylthio group, a cyclohexylthio group, a cycloheptylthio group and a cyclooctylthio group.

The "C1-6 alkoxyimino group" refers to an imino group in which a hydrogen atom is replaced by a C1-6 alkoxy group. Preferable examples of the group include a methoxyimino group and an ethoxyimino group.

The "C2-6 alkenyloxy group" refers to an alkenyl group having 2 to 6 carbon atoms in which one hydrogen atom is replaced by an oxygen atom. Preferable examples of the group include linear or branched alkenyloxy groups such as a vinyloxy group, an allyloxy group, a 1-propenyloxy group, an isopropenyloxy group, a 1-buten-1-yloxy group, a 1-buten-2-yloxy group, a 1-buten-3-yloxy group, a 2-buten-1-yloxy group and a 2-buten-2-yloxy group.

The "C2-6 alkynyloxy group" refers to an alkynyl group having 2 to 6 carbon atoms in which one hydrogen atom is replaced by an oxygen atom. Preferable examples of the group include linear or branched alkynyloxy groups such as an ethynyloxy group, a 1-propynyloxy group, a 2-propynyloxy group, a butynyloxy group, a pentynyloxy group and a hexynyloxy group.

The "C3-8 cycloalkylsulfinyl group" refers to a cyclic alkyl group having 3 to 8 carbon atoms in which one hydrogen atom is replaced by a sulfinyl group. Preferable examples of the group include a cyclopropylsulfinyl group, a cyclobutylsulfinyl group, a cyclopentylsulfinyl group, a cyclohexylsulfinyl group, a cycloheptylsulfinyl group and a cyclooctylsulfinyl group.

The "C3-8 cycloalkylsulfonyl group" refers to a cyclic alkyl group having 3 to 8 carbon atoms in which one hydrogen atom is replaced by a sulfonyl group. Preferable examples of the group include a cyclopropylsulfonyl group,

a cyclobutylsulfonyl group, a cyclopentylsulfonyl group, a cyclohexylsulfonyl group, a cycloheptylsulfonyl group and a cyclooctylsulfonyl group.

Preferable examples of the "hydroxyl group having a protecting group" include a methoxymethyl ether group, a tetrahydropyranyl ether group, a tert-butyl ether group, an allyl ether group, a benzoate group, an acetate group, a formate group, a crotonate group, a p-phenylbenzoate group, a pivaloate group, a tert-butyldimethylsilyl group, a tert-butyldiphenylsilyl group, a trityl group and a benzyl group.

A preferable example of the C1-6 alkoxy group in the "C1-6 alkoxy group (wherein the C1-6 alkoxy group may be substituted with 1 to 5 halogen atoms or the adjacent C1-6 alkoxy groups, together with a carbon atom to which they are bonded, may form a cyclic group)" is 1 to 5 halogen atoms; alternatively, the adjacent C1-6 alkoxy groups, together with a carbon atom to which they are bonded, may form a cyclic group. The phrase "the adjacent C1-6 alkoxy groups, together with a carbon atom to which they are bonded, may form a cyclic group" refers to a methylenedioxy group or an ethylenedioxy group, for example. Such a group is specifically represented by the following formula, for example.

[Formula 26]

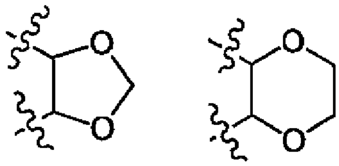

The substituent in the "C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)" is specifically represented by the following formula, for example.

[Formula 27]

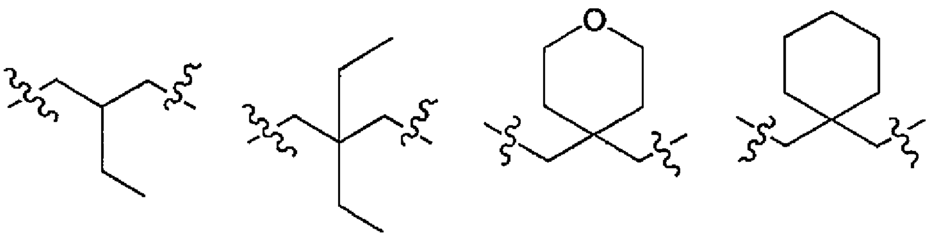

Next, the compound of the formula (I) of the present invention will be described.

In the compound of the formula (I) or pharmacologically acceptable salt thereof,

$Ar_1$ is preferably a triazolyl group or a tetrazolyl group which may be substituted with 1 to 2 substituents selected from Substituent Group A1, $Ar_1$ is more preferably a triazolyl group or a tetrazolyl group which may be substituted with 1 to 2 substituents selected from a hydrogen atom, a halogen atom, a C3-8 cycloalkyl group, a C2-6 alkenyl group, a C2-6 alkynyl group and a C1-6 alkyl group (wherein the C1-6 alkyl group may be substituted with 1 to 3 halogen atoms), and $Ar_1$ is most preferably a triazolyl group or a tetrazolyl group which may be substituted with 1 to 2 substituents selected from a hydrogen atom, a halogen atom, a C3-8 cycloalkyl group and a C1-6 alkyl group.

In the compound of the formula (I) or pharmacologically acceptable salt thereof,

$Ar_2$ is preferably a pyridinyl group, a pyrimidinyl group or a phenyl group which may be substituted with 1 to 3 substituents selected from Substituent Group A2,

$Ar_2$ is more preferably a pyridinyl group, a pyrimidinyl group or a phenyl group which may be substituted with 1 to 3 substituents selected from a hydrogen atom, a halogen atom, a cyano group, a hydroxyl group, a C1-6 alkoxy group

(wherein the C1-6 alkoxy group may be substituted with 1 to 3 substituents selected from the group consisting of a C2-6 alkenyl group, a C2-6 alkynyl group and a C3-8 cycloalkyl group), a C2-6 alkenyloxy group and a C2-6 alkynyloxy group, and

$Ar_2$ is most preferably a pyridinyl group, a pyrimidinyl group or a phenyl group which may be substituted with 1 to 3 substituents selected from a hydrogen atom, a halogen atom, a cyano group and a C1-6 alkoxy group.

In the compound of the formula (I) or pharmacologically acceptable salt thereof,

$X_1$ is preferably -C=C- or -$CR^3$=$CR^4$- (wherein $R^3$ and $R^4$ each represent a substituent selected from Substituent Group A3),

$X_1$ is more preferably -$CR^{31}$=$CR^{41}$- (wherein $R^{31}$ is a hydrogen atom, a halogen atom, a C1-6 alkyl group or a C1-6 alkoxy group; and $R^{41}$ is a hydrogen atom, a halogen atom, a 6- to 14-membered aromatic hydrocarbon ring group which may be substituted with 1 to 3 substituents selected from Substituent Group A5, a 5-to 14-membered aromatic heterocyclic group which may be substituted with 1 to 3 substituents selected from Substituent Group A5 or a C1-6 alkyl group (wherein the C1-6 alkyl group may have a substituent selected from a halogen atom, a hydroxyl group, a cyano group, a C3-8 cycloalkyl group, a C1-6 alkyl group, a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A5, a 5- to 14-membered aromatic heterocyclic group which may be substituted with 1 to 3 substituents selected from Substituent Group A5, a 5- to 14-membered non-aromatic heterocyclic group which may be substituted with 1 to 3 substituents selected from Substituent Group A5 and -O-$A^1$ (wherein $A^1$ represents a 6- to 14-membered aromatic hydrocarbon ring group which may be substituted with 1 to 3 substituents selected from Substituent Group A5 or a 5- to 14-membered aromatic heterocyclic group which may be substituted with 1 to 3 substituents selected from Substituent Group A5))), and

$X_1$ is most preferably -$CR^{32}$=$CR^{42}$- (wherein $R^{32}$ represents a hydrogen atom or a halogen atom; and $R^{42}$ represents a substituent selected from the group consisting of a hydrogen atom, a halogen atom, a C1-6 alkyl group (wherein the C1-6 alkyl group may be substituted with a C3-8 cycloalkyl group or a phenyl group) and a phenyl group).

In the compound of the formula (I) or pharmacologically acceptable salt thereof,

$R^1$ and $R^2$ are preferably taken together with a substituent selected from Substituent Group A4 and a nitrogen atom to which they are bonded to form a group such as a 5- to 11-membered heterocyclic group which may be substituted with 1 to 4 substituents selected from Substituent Group A4 and is represented by the formula (II), a 6- to 20-membered non-aromatic heterocyclic group which may be substituted with 1 to 4 substituents selected from Substituent Group A4 and is represented by the formula (III), a 9- to 16-membered non-aromatic heterocyclic group which may be substituted with 1 to 4 substituents selected from Substituent Group A4 and is represented by the formula (IV), a group which may be substituted with 1 to 4 substituents selected from Substituent Group A4 and is represented by the following formula:

[Formula 28]

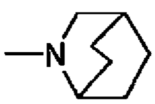
,
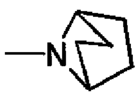
,
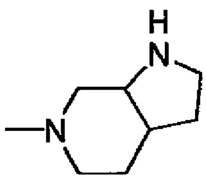
or
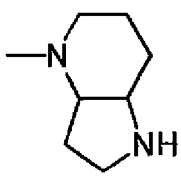

,

a group which may be substituted with 1 to 4 substituents selected from Substituent Group A4 and is represented by the following formula:

[Formula 29]

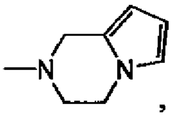, 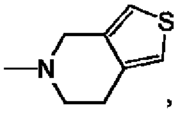, 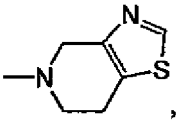, 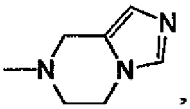, 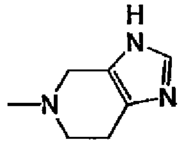,

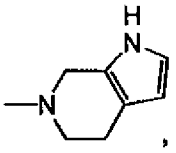, 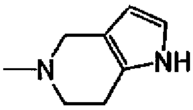, 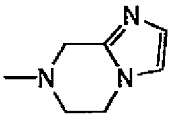, 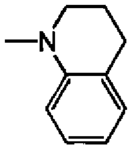, 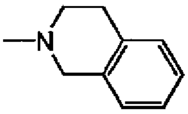,

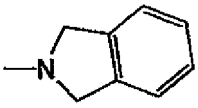 or 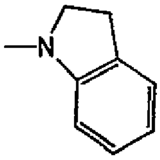

,

a cyclic group which may be substituted with 1 to 4 substituents selected from Substituent Group A4 and is represented by the formula (V), a cyclic group which may be substituted with 1 to 4 substituents selected from Substituent Group A4 and is represented by the formula (VI) or a group which may be substituted with 1 to 4 substituents selected from Substituent Group A4 and is represented by the following formula:

[Formula 30]

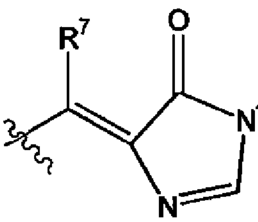, 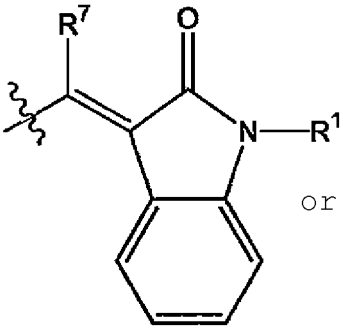 or 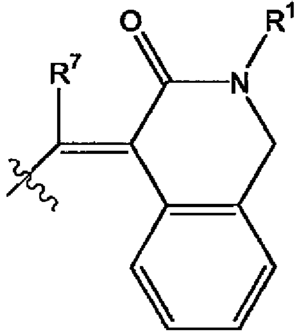

.

In the compound of the formula (I) or pharmacologically acceptable salt thereof,
preferably, $R^1$ and $R^2$ are each a substituent selected from Substituent Group A4,
more preferably, $R^1$ is a group selected from Substituent Group A8; and $R^2$ is a group selected from Substituent Group A6, and
most preferably, $R^1$ is a substituent selected from a C1-6 alkyl group (wherein the C1-6 alkyl group is a hydrogen atom, a C3-8 cycloalkoxy group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, a 6- to 14-membered aromatic hydrocarbon ring group which may be

substituted with 1 to 3 substituents selected from Substituent Group A9, a 5- to 14-membered aromatic heterocyclic group which may be substituted with 1 to 3 substituents selected from Substituent Group A9 and -O-$A^4$ (wherein $A^4$ represents a 6- to 14-membered aromatic hydrocarbon ring group which may be substituted with 1 to 3 substituents selected from Substituent Group A9 or a 5- to 14-membered aromatic heterocyclic group which may be substituted with 1 to 3 substituents selected from Substituent Group A9)); and $R^2$ is a hydrogen atom or a C1-6 alkyl group (wherein the C1-6 alkyl group may be substituted with 1 to 3 substituents selected from the group consisting of a hydroxyl group, a C3-8 cycloalkyl group, a C3-8 cycloalkoxy group, a C1-6 alkylthio group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A9, a 5- to 14-membered aromatic heterocyclic group which may be substituted with 1 to 3 substituents selected from Substituent Group A9 and a 5- to 14-membered non-aromatic heterocyclic group which may be substituted with 1 to 3 substituents selected from Substituent Group A9.

In the compound of the formula (I) or pharmacologically acceptable salt thereof,
the 5- to 11-membered heterocyclic group represented by the formula (II) which is formed by $R^1$ and $R^2$ together with a nitrogen atom to which they are bonded refers to a cyclic group containing 5 to 11 hetero atoms in total and is preferably a piperidinyl group, a pyrrolidinyl group, an azepinyl group, an azocanyl group, a piperazinyl group, a 1,4-diazepanyl group, a morpholinyl group or a thiomorpholinyl group, for example.
In the compound of the formula (I) or pharmacologically acceptable salt thereof, $R^1$ and $R^2$, together with a nitrogen atom to which they are bonded, preferably form a 5- to 11-membered heterocyclic group which may be substituted with 1 to 4 substituents selected from Substituent Group A4 and is represented by the formula (II).
In the compound of the formula (I) or pharmacologically acceptable salt thereof, $R^1$ and $R^2$, together with a nitrogen atom to which are bonded, more preferably form a 5- to 11-membered heterocyclic group represented by the formula (II) which may be substituted with 1 to 4 substituents selected from the group consisting of a hydrogen atom, a halogen atom, a hydroxyl group, a formyl group, a hydroxyimino group, a C1-6 alkoxyimino group, a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A7 and a 5- to 14-membered aromatic heterocyclic group which may be substituted with 1 to 3 substituents selected from Substituent Group A7), a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7, -O-$A^2$ (wherein $A^2$ represents a 6- to 14-membered aromatic hydrocarbon ring group which may be substituted with 1 to 3 substituents selected from Substituent Group A7 shown below or a 5- to 14-membered aromatic heterocyclic group which may be substituted with 1 to 3 substituents selected from Substituent Group A7 shown below), -CO-$A^2$ (wherein $A^2$ is as defined above) and =CH-$A^2$ (wherein $A^2$ is as defined above).
In the compound of the formula (I) or pharmacologically acceptable salt thereof, $R^1$ and $R^2$, together with a nitrogen atom to which they are bonded, most preferably form a 5- to 11-membered heterocyclic group represented by the formula (II) which may be substituted with 1 to 4 substituents selected from a hydrogen atom, a halogen atom, a hydroxyl group, a C1-6 alkyl group (wherein the C1-6 alkyl group may be substituted with 1 to 3 hydroxyl groups or 1 to 3 substituents selected from a 6- to 14-membered aromatic hydrocarbon ring group which may be substituted with 1 to 3 substituents selected from Substituent Group A10), a 6- to 14-membered aromatic hydrocarbon ring group which may be substituted with 1 to 3 substituents selected from Substituent Group A10, a 5- to 14-membered aromatic heterocyclic group which may be substituted with 1 to 3 substituents selected from Substituent Group A10, -O-$A^6$ (wherein $A^6$ represents a 6-to 14-membered aromatic hydrocarbon ring group which may be substituted with 1 to 3 substituents selected from Substituent Group A10) and =CH-$A^6$ (wherein $A^6$ is as defined above).

In the compound of the formula (I), the "6-to 20-membered non-aromatic heterocyclic group" represented by the formula (III) which is formed by $R^1$ and $R^2$ together with a nitrogen atom to which they are bonded refers to a spirocyclic group containing 6 to 20 hetero atoms in total which is represented by the formula (III). Such a group is preferably a substituent represented by the following formula, for example.

[Formula 31]

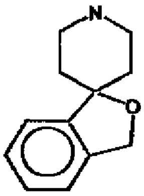

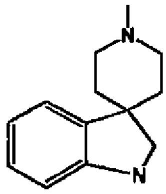

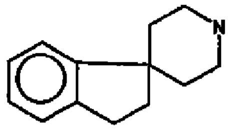

or

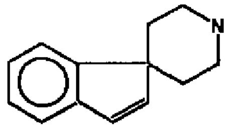

In the compound of the formula (I) or pharmacologically acceptable salt thereof, $R^1$ and $R^2$, together with a nitrogen atom to which they are bonded, preferably form a 6- to 20-membered non-aromatic heterocyclic group which may be substituted with 1 to 4 substituents selected from Substituent Group A4 and is represented by the formula (III).

In the compound of the formula (I) or pharmacologically acceptable salt thereof, $R^1$ and $R^2$, together with a nitrogen atom to which they are bonded, preferably form a 9- to 16-membered non-aromatic heterocyclic group which may be substituted with 1 to 4 substituents selected from Substituent Group A4 and is represented by the formula (IV). The "9- to 16-membered non-aromatic heterocyclic group" represented by the formula (IV) refers to a cyclic group containing 9 to 16 hetero atoms in total which is represented by the formula (IV).

In the compound of the formula (I) or pharmacologically acceptable salt thereof, $R^1$ and $R^2$, together with a nitrogen atom to which they are bonded, preferably form a group which may be substituted with 1 to 4 substituents selected from Substituent Group A4 and is represented by the following formula:

[Formula 32]

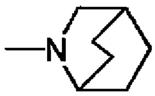,

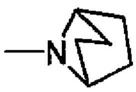,

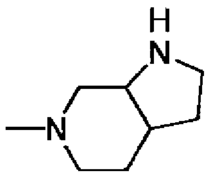

or

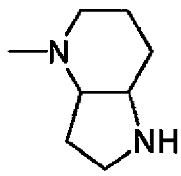

.

In the compound of the formula (I) or pharmacologically acceptable salt thereof, $R^1$ and $R^2$, together with a nitrogen atom to which they are bonded, preferably form a group which may be substituted with 1 to 4 substituents selected from Substituent Group A4 and is represented by the following formula:

[Formula 33]

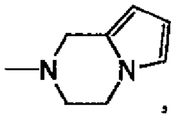, 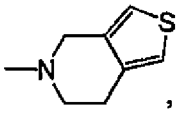, 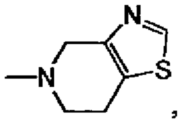, 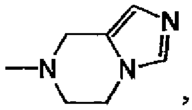, 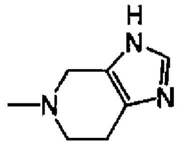, 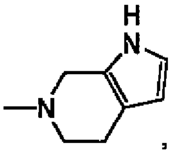, 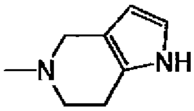, 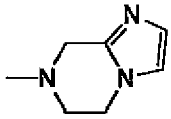, 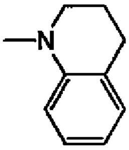, 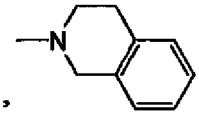,

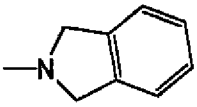 or 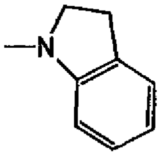

.

In the compound of the formula (I) or pharmacologically acceptable salt thereof, $R^1$ and $R^2$, together with a nitrogen atom to which they are bonded, more preferably form a group which may be substituted with 1 to 4 substituents selected from Substituent Group A4 and is represented by the following formula:

[Formula 34]

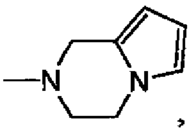, 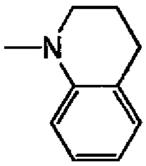, 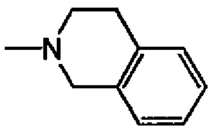, 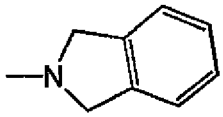

or 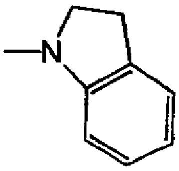

.

In the compound of the formula (I) or pharmacologically acceptable salt thereof, $R^1$ and $R^2$, together with a nitrogen atom to which they are bonded, preferably form a group which may be substituted with 1 to 4 substituents selected from Substituent Group A4.
In the compound of the formula (I) or pharmacologically acceptable salt thereof, $R^1$ and $R^2$, together with a nitrogen atom to which they are bonded, more preferably form a group which may be substituted with 1 to 4 fluorine atoms or the like.

In the compound of the formula (I) or pharmacologically acceptable salt thereof, $R^1$ and $R^2$, together with $-X_1$-CO-N, preferably form a cyclic group which may be substituted with 1 to 4 substituents selected from Substituent Group A4 and is represented by the formula (V), wherein $R^7$ represents a substituent selected from Substituent Group A3.

In the compound of the formula (I) or pharmacologically acceptable salt thereof, $R^1$ and $R^2$, together with $-X_1$-CO-N, preferably form a cyclic group which may be substituted with 1 to 4 substituents selected from Substituent

Group A4 and is represented by the formula (VI), wherein $R^1$ represents a substituent selected from Substituent Group A4 and $R^7$ represents a substituent selected from Substituent Group A3.

In the compound of the formula (I) or pharmacologically acceptable salt thereof, $R^1$ and $R^2$, together with $-X_1$-CO-N, more preferably form a cyclic group which may be substituted with 1 to 4 substituents selected from Substituent Group A7 and is represented by the following formula:

[Formula 35]

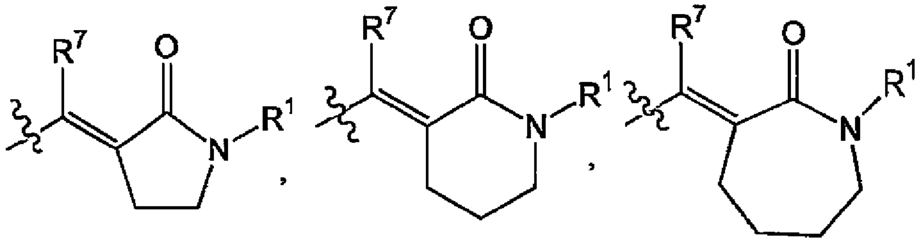

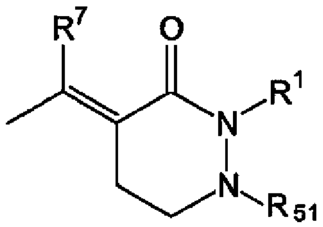

or

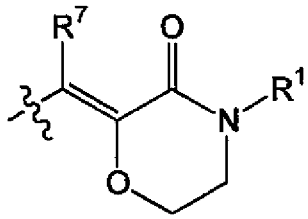

wherein $R^1$ and $R_{51}$ each represent a substituent selected from Substituent Group A4; and $R^7$ represents a substituent selected from Substituent Group A3.

In the compound of the formula (I) or pharmacologically acceptable salt thereof, $R^1$ in the cyclic group is preferably a substituent selected from Substituent Group A4.

In the compound of the formula (I) or pharmacologically acceptable salt thereof, $R^1$ in the cyclic group is more preferably a substituent selected from Substituent Group A8.

In the compound of the formula (I) or pharmacologically acceptable salt thereof, $R^1$ in the cyclic group is most preferably a substituent selected from a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A9, a 5- to 14-membered aromatic heterocyclic group which may be substituted with 1 to 3 substituents selected from Substituent Group A9, a 5- to 14-membered non-aromatic heterocyclic group which may be substituted with 1 to 3 substituents selected from Substituent Group A9 and $-X-A^4$ (wherein X represents an imino group, -O- or -S- and $A^4$ represents a 6- to 14-membered aromatic hydrocarbon ring group which may be substituted with 1 to 3 substituents selected from Substituent Group A9 or a 5-to 14-membered aromatic heterocyclic group which may be substituted with 1 to 3 substituents selected from Substituent Group A9)).

In the compound of the formula (I) or pharmacologically acceptable salt thereof, $R^1$ and $R^2$, together with $-X_1$-CO-N, preferably form a cyclic group which may be substituted with 1 to 4 substituents selected from Substituent Group A4 and is represented by the following formula:

[Formula 36]

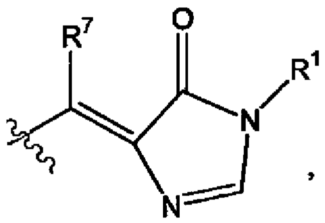
,
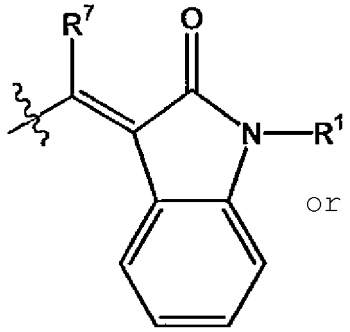
or
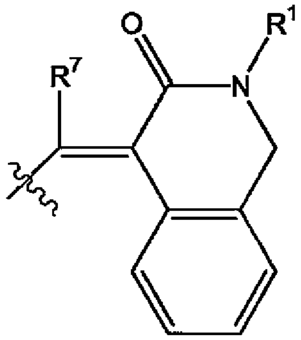

.

In the compound of the formula (I) or pharmacologically acceptable salt thereof, $R^1$ in the cyclic group is preferably a substituent which may be selected from Substituent Group A4.

In the compound of the formula (I) or pharmacologically acceptable salt thereof, $R^1$ in the cyclic group is more preferably a substituent which may be selected from Substituent Group A8.

In the compound of the formula (I) or pharmacologically acceptable salt thereof, $R^1$ in the cyclic group is most preferably a substituent selected from a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A9, a 5- to 14-membered aromatic heterocyclic group which may be substituted with 1 to 3 substituents selected from Substituent Group A9, a 5- to 14-membered non-aromatic heterocyclic group which may be substituted with 1 to 3 substituents selected from Substituent Group A9 and -X-$A^4$ (wherein X represents an imino group, -O- or -S- and $A^4$ represents a 6- to 14-membered aromatic hydrocarbon ring group which may be substituted with 1 to 3 substituents selected from Substituent Group A9 or a 5-to 14-membered aromatic heterocyclic group which may be substituted with 1 to 3 substituents selected from Substituent Group A9)).

In the compound of the formula (I) or pharmacologically acceptable salt thereof, $R^1$ in the formula (I), $R^1$ in the formula (VI) and $R^1$ in the cyclic group represented by the following formula:

[Formula 37]

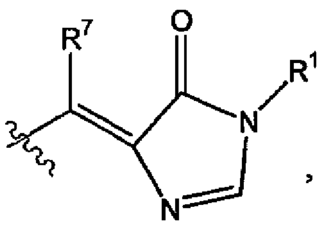
,
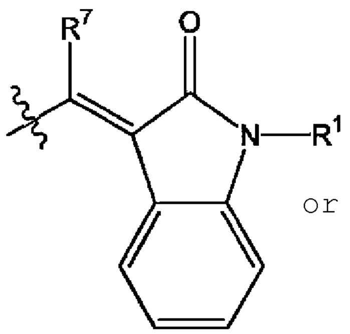
or
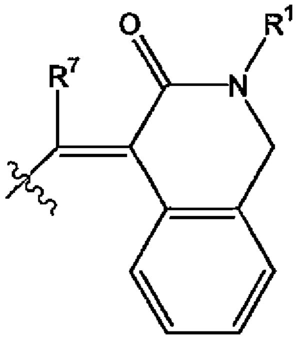

are each preferably $-X_{21}-X_{22}-Ar_3$
(wherein $X_{21}$ represents a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group) and a 5- to 14-membered non-aromatic heterocyclic group which may be substituted with 1 to 3 substituents selected from Substituent Group A7) or a single bond; $X_{22}$ represents a single bond, an imino group which may be substituted with a substituent selected from Substituent Group A7, -O- or -S-; and $Ar_3$ represents a 6- to 14-membered aromatic hydrocarbon which may be substituted with 1 to 3 substituents selected from Substituent Group A7 or a 5- to 14-membered aromatic heterocyclic group which may be substituted with 1 to 3 substituents selected from Substituent Group A7).

In the compound of the formula (I) or pharmacologically acceptable salt thereof, the $R^1$ is more preferably $-X_{21a}-X_{22a}-Ar_{3a}$ (wherein $X_{21a}$ represents a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms) and a 5- to 14-membered non-aromatic heterocyclic group which may be substituted with 1 to 3 substituents selected from Substituent Group A9); $X_{22a}$ represents a single bond or an oxygen atom; and $Ar_{3a}$ represents a 6-to 14-membered aromatic hydrocarbon ring group which may be substituted with 1 to 3 substituents selected from Substituent Group A9 or a 5- to 14-membered aromatic heterocyclic group which may be substituted with 1 to 3 substituents selected from Substituent Group A9).

$Ar_{3a}$ in the formula "$-X_{21a}-X_{22a}-Ar_{3a}$" represents a 6- to 14-membered aromatic hydrocarbon group or a 5-to 14-membered aromatic heterocyclic group, and preferably a group selected from a phenyl group, a naphthyl group and a fluorenyl group or a group selected from a thienyl group, a pyridinyl group, a quinolinyl group, an isoquinolinyl group, an indolyl group, a benzothiazolyl group, a benzoxazolyl group and a furyl group, for example.

In the compound of the formula (I) or pharmacologically acceptable salt thereof, $R^1$ is preferably a 6- to 14-membered non-aromatic hydrocarbon ring group or a 5- to 14-membered non-aromatic heterocyclic group represented by the formula (VII).

In the compound of the formula (I) or pharmacologically acceptable salt thereof, more preferably, when $R^1$ is represented by the formula (VII), $Ar_4$ is a group selected from the group consisting of a phenyl group, a pyridinyl group, a pyrimidinyl group, a pyrazinyl group, a thienyl group, an oxazolyl group, a pyrrolyl group, a thiazolyl group and a furyl group, which may be substituted with 1 to 3 substituents selected from the group consisting of a halogen atom, a C1-6 alkyl group (wherein the C1-6 alkyl group may be substituted with 1 to 3 substituents selected from the group consisting of a halogen atom and a C1-6 alkyl group), a C1-6 alkoxy group (wherein the C1-6 alkoxy group may be substituted with 1 to 3 halogen atoms), an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7, a 5- to 14-membered non-aromatic heterocyclic group which may be substituted with 1 to 3 substituents selected from Substituent Group A7 and $-CO-A^2$ (wherein $A^2$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7), for example.

In the compound of the formula (I) or pharmacologically acceptable salt thereof, most preferably, when $R^1$ is represented by the formula (VII), $Ar_4$ is an indanyl group, an azaindanyl group, a tetrahydronaphthyl group, an azatetrahydronaphthyl group, a chromanyl group, an azachromanyl group, a tetrahydrobenzofuranyl group or a tetrahydrobenzothienyl group, which may be substituted with 1 to 3 substituents selected from the group consisting of a halogen atom, a hydroxyl group, a cyano group, a C3-8 cycloalkyl group, a C3-8 cycloalkoxy group, a C1-6 alkyl group (wherein the C1-6 alkyl group may be substituted with 1 to 3 halogen atoms or C1-6 alkyl groups), a C1-6 alkoxy group (wherein the C1-6 alkoxy group may be substituted with 1 to 3 halogen atoms), an amino group (wherein the amino group may be substituted with a C1-6 alkyl group optionally having 1 to 5 halogen atoms) and a 5- to 14-membered non-aromatic heterocyclic group, for example.

Meanings of symbols, terms and the like describing the general formula (VIII) in the present specification will be explained and the present invention will be particularly described in detail below.

Substituent Group A11 refers to the following groups.
Substituent Group A11 refers to (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 5 halogen atoms or 1 to 3 C1-6 alkoxy groups), (7) an amino group which may be substituted with 1 or 2 C1-6 alkyl groups (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) a carbamoyl group which may be substituted with 1 or 2 C1-6 alkyl groups (wherein the C1-6 alkyl group may be substituted with 1 to 3 halogen atoms).

The "halogen atom", "C1-6 alkyl group", "C3-8 cycloalkyl group", "6- to 14-membered cyclic aromatic hydrocarbon ring group", "5- or 14-membered aromatic heterocyclic group", "C1-6 alkoxy group" and "C3-8 cycloalkoxy group" are as defined for the "general formula (I)".

The "C1-6 alkylene group" refers to an alkylene group having 1 to 6 carbon atoms. Preferable examples of the group include a methylene group, an ethylene group, a propylene group, a butylene group and a pentylene group.

A preferable example of the C1-6 alkyl group in the "C1-6 alkyl group (wherein the C1-6 alkyl group may be substituted with 1 to 3 hydroxyl groups)" is 1 to 3 hydroxyl groups.

A preferable example of the C1-6 alkylene group in the "C1-6 alkylene group (wherein the C1-6 alkylene group may be substituted with 1 to 3 hydroxyl groups or C1-6 alkyl groups (wherein the C1-6 alkyl group may be substituted with 1 to 3 hydroxyl groups))" is 1 to 3 hydroxyl groups or C1-6 alkyl groups (wherein the C1-6 alkyl group may be substituted with 1 to 3 hydroxyl groups).

The "aryl group" refers to a "6- to 14-membered cyclic aromatic hydrocarbon group" or a "5- to 14-membered aromatic heterocyclic group".

The "aryloxy group" refers to a group in which a hydrogen atom in the aromatic hydrocarbon ring of the "6- to 14-membered cyclic aromatic hydrocarbon group" or a hydrogen atom in the aromatic heterocycle of the "5- to 14-membered aromatic heterocyclic group" is replaced by an oxygen atom.

The "C3-8 cycloalkyl ring condensed with a benzene ring" is a ring of the following formula, for example.

[Formula 38]

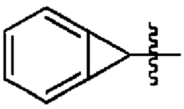 , 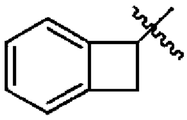 , 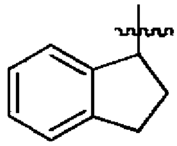 , 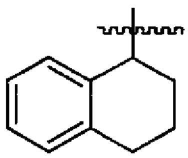

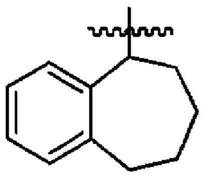 or 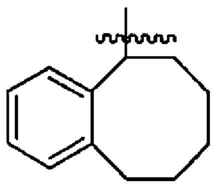

The "4- to 8-membered nitrogen-containing heterocyclic group" is a 4- to 8-membered heterocyclic group containing a nitrogen atom and is a group represented by the following formula, for example.

[Formula 39]

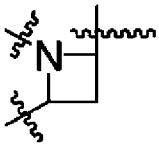 , 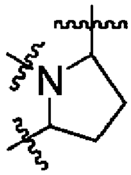 , 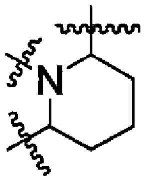 , 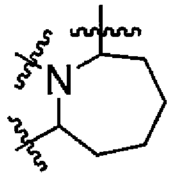 or 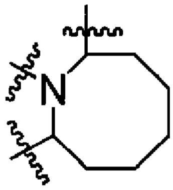

The "4- to 8-membered nitrogen-containing heterocyclic group which is formed together with a nitrogen atom and a carbon atom bonded and may be substituted with an aryl group or a pyridinyl group" is a group represented by the following formula, for example.

[Formula 40]

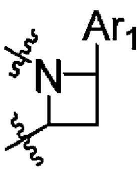 , 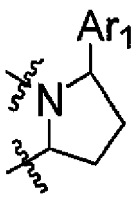 , 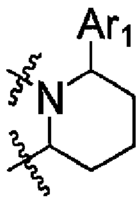 , 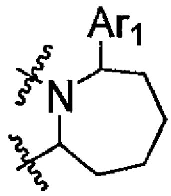 or 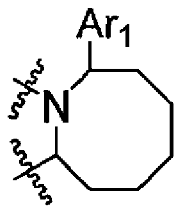

The "4- to 8-membered nitrogen-containing heterocyclic group (wherein one methylene group in the 4- to 8-membered nitrogen-containing heterocyclic group may be substituted with a methylene group or a vinylene group which may be substituted with 1 or 2 substituents selected from Substituent Group A11, an oxygen atom or an imino group which may be substituted with a C1-6 alkyl group or a C1-6 acyl group)" is a group specifically represented by the following formula, for example.

[Formula 41]

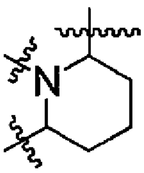 , 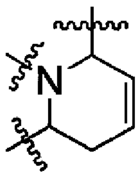 , 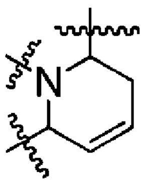 , 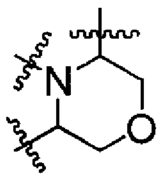 or 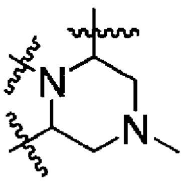

The "C3-8 cycloalkyl group formed by $R^{15}$ and $R^{16}$ together" is a group specifically represented by the following formula, for example.

[Formula 42]

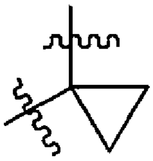,
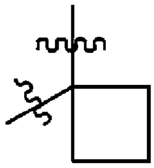,
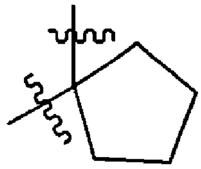

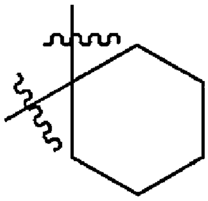,
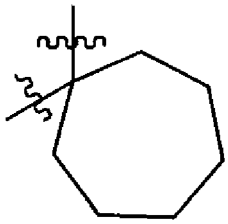
or
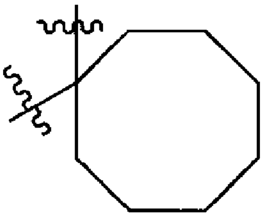

The "C3-8 cycloalkyl group formed by $R^{17}$ and $R^{18}$ together" is a group specifically represented by the following formula, for example.

[Formula 43]

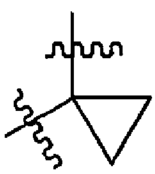,
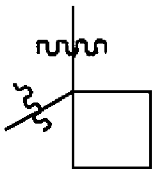,
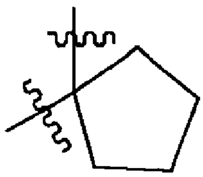

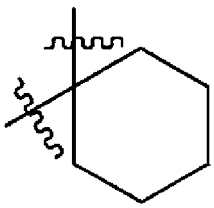,
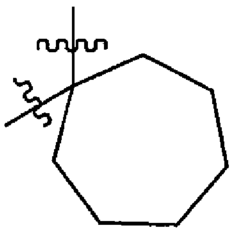
or
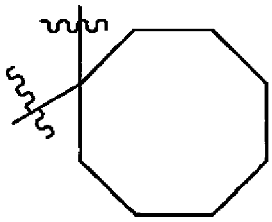

A preferable example of the C1-6 alkyl group in the "C1-6 alkyl group (wherein the C1-6 alkyl group may be substituted with 1 to 5 halogen atoms or 1 to 3 C1-6 alkoxy groups)" is 1 to 5 halogen atoms or 1 to 3 C1-6 alkoxy groups.

The "amino group which may be substituted with 1 or 2 C1-6 alkyl groups" refers to an amino group whose hydrogen atom(s) are replaced by 1 or 2 alkyl groups having 1 to 6 carbon atoms. Preferable examples of the group include a methylamino group, a dimethylamino group, an ethylamino group, a diethylamino group, an n-propylamino group and a di-n-propylamino group.

A preferable example of the C1-6 alkyl group in the "C1-6 alkyl group (wherein the C1-6 alkyl group may be substituted with 1 to 5 halogen atoms)" is 1 to 5 halogen atoms.

A preferable example of the C1-6 alkoxy group in the "C1-6 alkoxy group (wherein the C1-6 alkoxy group may be substituted with 1 to 5 halogen atoms)" is 1 to 5 halogen atoms.

The "carbamoyl group which may be substituted with 1 or 2 C1-6 alkyl groups" refers to a carbamoyl group whose hydrogen atom(s) are replaced by 1 or 2 alkyl groups having 1 to 6 carbon atoms. Preferable examples of the

group include a methylcarbamoyl group, a dimethylcarbamoyl group, an ethylcarbamoyl group, a diethylcarbamoyl group, an n-propylcarbamoyl group and a di-n-propylcarbamoyl group.

A preferable example of the C1-6 alkyl group in the "C1-6 alkyl group (wherein the C1-6 alkyl group may be substituted with 1 to 3 halogen atoms)" is 1 to 3 halogen atoms.

The "methylene group (wherein the methylene group may be substituted with 1 or 2 substituents which are the same or different and selected from the group consisting of a C1-6 alkyl group and a hydroxyl group)" is a group specifically represented by the following formula, for example.

[Formula 44]

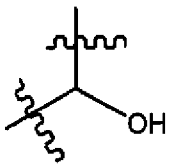 , 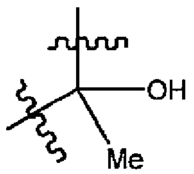 , 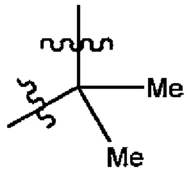 or 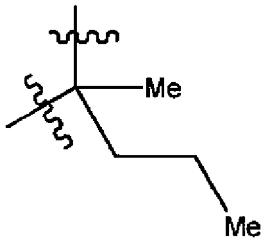

Next, the compound of the formula (VIII) of the present invention will be described.

In the compound of the formula (VIII) or pharmacologically acceptable salt thereof, $Ar_{1a}$ is preferably a triazolyl group or a tetrazolyl group which may be substituted with a C1-6 alkyl group.

The compound of the formula (VIII) or pharmacologically acceptable salt thereof is preferably such a compound or a pharmacologically acceptable salt thereof, wherein (a) $R^{15}$, $R^{16}$, $R^{17}$ and $R^{18}$ are the same or different and each represent a hydrogen atom or a C1-6 alkyl group;

$X_{1a}$ represents a C1-6 alkylene group (wherein the C1-6 alkylene group may be substituted with 1 to 3 hydroxyl groups or C1-6 alkyl groups (wherein the C1-6 alkyl group may be substituted with 1 to 3 hydroxyl groups)); and

$Ar_5$ represents an aryl group, a pyridinyl group, an aryloxy group or a pyridinyloxy group which may be substituted with 1 to 3 substituents selected from Substituent Group All; or

(b) one of $R^{15}$ and $R^{16}$ and one of $R^{17}$ and $R^{18}$ are the same or different and each represent a hydrogen atom or a C1-6 alkyl group; the other of $R^{15}$ and $R^{16}$ and the other of $R^{17}$ and $R^{18}$, together with carbon atoms to which they are respectively bonded, form a C3-8 cycloalkyl group (wherein the C3-8 cycloalkyl group may be substituted with 1 to 3 substituents selected from Substituent Group A11); and $X_{1a}$ and $Ar_5$ are as defined in (a); or

(c) $Ar_5$-$X_{1a}$- represents a C3-8 cycloalkyl group (wherein one methylene group in the C3-8 cycloalkyl group may be substituted with an oxygen atom) condensed with a benzene ring (wherein the benzene ring may be substituted with 1 to 3 substituents selected from Substituent Group A11); and $R^{15}$, $R^{16}$, $R^{17}$ and $R^{18}$ are as defined in (a); or

(d) $Ar_5$-$X_{1a}$- and $R^{18}$, together with a nitrogen atom to which $Ar_5$-$X_{1a}$- is bonded and a carbon atom to which $R^{18}$ is bonded, form a 4- to 8-membered nitrogen-containing heterocyclic group (wherein one methylene group in the 4- to 8-membered nitrogen-containing heterocyclic group may be substituted with a methylene group or a vinylene group which may be substituted with 1 or 2 substituents selected from Substituent Group A1, an oxygen atom or an imino group which may be substituted with a C1-6 alkyl group or a C1-6 acyl group) which may be substituted with an aryl group or a pyridinyl group (wherein the aryl group or pyridinyl group may be substituted with 1 to 3 substituents selected from Substituent Group A11); and $R^{15}$, $R^{16}$ and $R^{17}$ are as defined in (a); or

(e) $R^{15}$ and $R^{16}$ form a C3-8 cycloalkyl group together; and $R^{17}$, $R^{18}$, $X_{1a}$ and $Ar_5$ are as defined in (a) and (c); or

(f) $R^{17}$ and $R^{18}$ form a C3-8 cycloalkyl group together; and $R^{15}$, $R^{16}$, $X_{1a}$ and $Ar_5$ are as defined in (a) and (c), and is particularly preferably a compound of the formula (VIII-a) or a pharmacologically acceptable salt thereof, wherein $R^{15}$, $R^{16}$, $R^{17}$ and $R^{18}$ are the same or different and each represent a hydrogen atom or a C1-6 alkyl group; $R^{19}$ and $R^{20}$ are the same or different and each represent a hydrogen atom or a C1-6 alkyl group (wherein the C1-6 alkyl group may be substituted with 1 to 3 hydroxyl groups); and $Ar_{5\text{-}a}$ represents a phenyl group or a pyridinyl group which may be substituted with 1 to 3 substituents selected from Substituent Group All;

a compound of the formula (VIII-b) or a pharmacologically acceptable salt thereof, wherein $R^{15}$, $R^{16}$, $R^{17}$ and $R^{18}$ are the same or different and each represent a hydrogen atom or a C1-6 alkyl group; $R^{21}$ and $R^{22}$ are the same or different and each represent a substituent selected from a hydrogen atom and Substituent Group All; and Y represents

a methylene group or an oxygen atom; or
a compound of the formula (VIII-c) or a pharmacologically acceptable salt thereof, wherein $R^{23}$ and $R^{24}$ are the same or different and each represent a hydrogen atom or a C1-6 alkyl group; $Ar_{5-c}$ represents a phenyl group or a pyridinyl group which may be substituted with 1 to 3 substituents selected from Substituent Group All; $Z_{5-c}$ represents a methylene group or a vinylene group which may be substituted with 1 or 2 substituents selected from Substituent Group A11, an oxygen atom or an imino group which may be substituted with a C1-6 alkyl group or a C1-6 acyl group; and n represents an integer of 0 to 2.

Preferably, in the compound or pharmacologically acceptable salt thereof, $R^{15}$, $R^{16}$, $R^{17}$ and $R^{18}$ are the same or different and are each a hydrogen atom or a C1-6 alkyl group;
one of $R^{15}$ and $R^{16}$ and one of $R^{17}$ and $R^{18}$ are the same or different and each represent a hydrogen atom or a C1-6 alkyl group; and the other of $R^{15}$ and $R^{16}$ and the other of $R^{17}$ and $R^{18}$, together with carbon atoms to which they are respectively bonded, form a C3-8 cycloalkyl ring;
$R^{15}$ and $R^{16}$ form a C3-8 cycloalkyl group together; and $R^{17}$ and $R^{18}$ are the same or different and are each a hydrogen atom or a C1-6 alkyl group; or
$R^{15}$ and $R^{16}$ are the same or different and are each a hydrogen atom or a C1-6 alkyl group; and $R^{17}$ and $R^{18}$ form a C3-8 cycloalkyl group together.

In the compound or pharmacologically acceptable salt thereof, $Ar_5$ is preferably an aryl group, a pyridinyl group, an aryloxy group or a pyridinyloxy group which may be substituted with 1 to 3 substituents selected from Substituent Group A11, and
$Ar_5$ is more preferably a phenyl group or a pyridinyl group which may be substituted with 1 to 3 substituents selected from Substituent Group A11.

In the compound or pharmacologically acceptable salt thereof, the substituent for $Ar_5$ is preferably 1 to 3 substituents selected from Substituent Group A11, and
the substituent for $Ar_5$ is more preferably 1 to 3 halogen atoms.

In the compound or pharmacologically acceptable salt thereof, when $Ar_5$-$X_{1a}$- represents a C3-8 cycloalkyl group condensed with a benzene ring (wherein the benzene ring may be substituted with 1 to 3 substituents selected from Substituent Group A11), preferably, the substituent on the benzene ring is 1 to 3 substituents selected from Substituent Group A11, and more preferably, the substituents $R^{21}$ and $R^{22}$ on the benzene ring are the same or different and are 1 or 2 hydrogen atoms, halogen atoms or C1-6 alkoxy groups.

Meanings of symbols, terms and the like describing the general formula (IX) in the present specification will be explained and the present invention will be described in detail below.

Substituent Group A12 refers to (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 3 substituents selected from the group consisting of a halogen atom, a hydroxyl group, a cyano group, a C3-8 cycloalkyl group, a C1-6 alkoxy group and a C3-8 cycloalkoxy group), (7) a C1-6 alkoxy group (wherein the C1-6 alkoxy group may be substituted with 1 to 3 substituents selected from the group consisting of a halogen atom, a hydroxyl group, a cyano group, a C3-8 cycloalkyl group and a C3-8 cycloalkoxy group), (8) an amino group which may be substituted with 1 or 2 C1-6 alkyl groups (wherein the C1-6 alkyl group may be substituted with 1 to 3 halogen atoms) and (9) a carbamoyl group which may be substituted with 1 or 2 C1-6 alkyl groups (wherein the C1-6 alkyl group may be substituted with 1 to 3 halogen atoms).

The "halogen atom", "C1-6 alkyl group", "C3-8 cycloalkyl group", "C1-6 alkoxy group", "C3-8 cycloalkoxy group", "amino group which may be substituted with 1 or 2 C1-6 alkyl groups", "C1-6 alkyl group (wherein the C1-6 alkyl group may be substituted with 1 to 3 halogen atoms)", "carbamoyl group which may be substituted with 1 or 2 C1-6 alkyl groups", "C1-6 alkyl group (wherein the C1-6 alkyl group may be substituted with 1 to 3 halogen atoms)" and "methylene group (wherein the methylene group may be substituted with 1 or 2 substituents which are the same or different and are selected from the group consisting of a C1-6 alkyl group and a hydroxyl group)" are as defined for the "general formula (I)" or "general formula (VIII)".

The "C1-6 acyl group" is synonymous with a "C1-6 alkylcarbonyl group" and refers to an alkyl group having 1 to 6 carbon atoms in which one hydrogen atom is replaced by a carbonyl group. Preferable examples of the group include an acetyl group, a propionyl group and a butyryl group.

A preferable example of the C1-6 alkyl group in the "C1-6 alkyl group (wherein the C1-6 alkyl group may be substituted with 1 to 3 substituents selected from the group consisting of a halogen atom, a hydroxyl group, a cyano group, a C3-8 cycloalkyl group, a C1-6 alkoxy group and a C3-8 cycloalkoxy group)" is 1 to 3 substituents selected from the group consisting of a halogen atom, a hydroxyl group, a cyano group, a C3-8 cycloalkyl group, a C1-6 alkoxy group and a C3-8 cycloalkoxy group.

A preferable example of the C1-6 alkoxy group in the "C1-6 alkoxy group (wherein the C1-6 alkoxy group may be substituted with 1 to 3 substituents selected from the group consisting of a halogen atom, a hydroxyl group, a cyano

group, a C3-8 cycloalkyl group and a C3-8 cycloalkoxy group)" is 1 to 3 substituents selected from the group consisting of a halogen atom, a hydroxyl group, a cyano group, a C3-8 cycloalkyl group and a C3-8 cycloalkoxy group.

Next, the compound of the formula (IX) of the present invention will be described.

In the compound of the formula (IX) or pharmacologically acceptable salt thereof, $Ar_{1a}$ is preferably a triazolyl group or a tetrazolyl group which may be substituted with a C1-6 alkyl group.

In the compound of the formula (IX) or pharmacologically acceptable salt thereof, $Ar_6$ is preferably a phenyl group which may be substituted with 1 to 3 substituents selected from Substituent Group A12 or a pyridinyl group which may be substituted with 1 to 3 substituents selected from Substituent Group A12,

$Ar_6$ is more preferably a phenyl group substituted with 1 to 3 halogen atoms, and

$Ar_6$ is most preferably a phenyl group substituted with a fluorine atom.

In the compound of the formula (IX) or pharmacologically acceptable salt thereof, preferably, $R^{25}$ and $R^{26}$ are each a hydrogen atom, a halogen atom, a hydroxyl group, a cyano group, a C3-8 cycloalkyl group, a C3-8 cycloalkoxy group, a C1-6 alkyl group (wherein the C1-6 alkyl group may be substituted with 1 to 3 substituents selected from the group consisting of a halogen atom, a hydroxyl group, a cyano group, a C3-8 cycloalkyl group, a C1-6 alkoxy group and a C3-8 cycloalkoxy group), a C1-6 alkoxy group (wherein the C1-6 alkoxy group may be substituted with 1 to 3 substituents selected from the group consisting of a halogen atom, a hydroxyl group, a cyano group, a C3-8 cycloalkyl group and a C3-8 cycloalkoxy group), an amino group (wherein the amino group may be substituted with 1 or 2 C1-6 alkyl groups optionally substituted with 1 to 3 halogen atoms) and a carbamoyl group (wherein the carbamoyl group may be substituted with 1 or 2 C1-6 alkyl groups optionally substituted with 1 to 3 halogen atoms).

In the compound of the formula (IX) or pharmacologically acceptable salt thereof,

preferably, $Z_6$ represents a methylene group or a vinylene group which may be substituted with 1 or 2 substituents selected from Substituent Group A12, an oxygen atom or an imino group which may be substituted with a C1-6 alkyl group or a C1-6 acyl group; and p, q and r each represent an integer of 0 to 2,

preferably, $Z_6$ represents a methylene group (wherein the methylene group may be substituted with 1 or 2 substituents which are the same or different and are selected from the group consisting of a C1-6 alkyl group and a hydroxyl group); p represents 1; q represents 1; and r represents 1,

preferably, $Z_6$ represents a methylene group (wherein the methylene group may be substituted with 1 or 2 substituents which are the same or different and are selected from the group consisting of a C1-6 alkyl group and a hydroxyl group); p represents 1; q represents 1; and r represents 0,

preferably, $Z_6$ represents an oxygen atom; p represents 1; q represents 1; and r represents 1,

preferably, $Z_6$ represents a methylene group (wherein the methylene group may be substituted with 1 or 2 substituents which are the same or different and are selected from the group consisting of a C1-6 alkyl group and a hydroxyl group); p represents 1; q represents 0; and r represents 0,

preferably, $Z_6$ represents a methylene group (wherein the methylene group may be substituted with 1 or 2 substituents which are the same or different and are selected from the group consisting of a C1-6 alkyl group and a hydroxyl group); p represents 1; q represents 0; and r represents 1,

preferably, $Z_6$ represents a methylene group (wherein the methylene group may be substituted with 1 or 2 substituents which are the same or different and are selected from the group consisting of a C1-6 alkyl group and a hydroxyl group); p represents 1; q represents 2; and r represents 0,

preferably, $Z_6$ represents a methylene group (wherein the methylene group may be substituted with 1 or 2 substituents which are the same or different and are selected from the group consisting of a C1-6 alkyl group and a hydroxyl group); p represents 1; q represents 2; and r represents 1,

preferably, $Z_6$ represents a vinylene group (wherein the vinylene group may be substituted with 1 or 2 C1-6 alkyl groups); p represents 0; q represents 1; and r represents 1, and

preferably, $Z_6$ represents a vinylene group (wherein the vinylene group may be substituted with 1 or 2 C1-6 alkyl groups); p represents 1; q represents 1; and r represents 0.

Methods for preparing the compound of the general formula (I) of the present invention will be described below. The compound represented by the general formula (I):

[Formula 45]

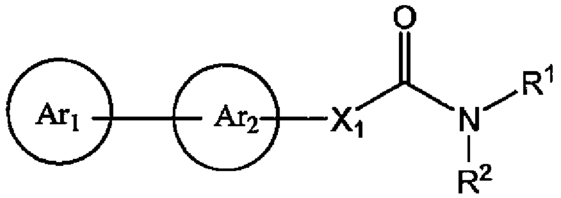

(I)

wherein $Ar_1$, $Ar_2$, $X_1$, $R^1$ and $R^2$ are as defined above, is synthesized according to a method such as the following General Preparation Method 1 to General Preparation Method 9, for example. It is obvious that, in order to prepare the compound of the present invention conveniently, the method comprises a protection reaction step and a deprotection reaction step appropriately, using a protecting group known to a person skilled in the art which is suitably selected for each step (see T. Greene et al., "Protective Groups in Organic Synthesis", John Wiley & Sons, Inc., New York, 1981, for example).

[General Preparation Method 1]

Typically used General Preparation Method 1 for the compound of the general formula (I) of the present invention will be described below.

[Formula 46]

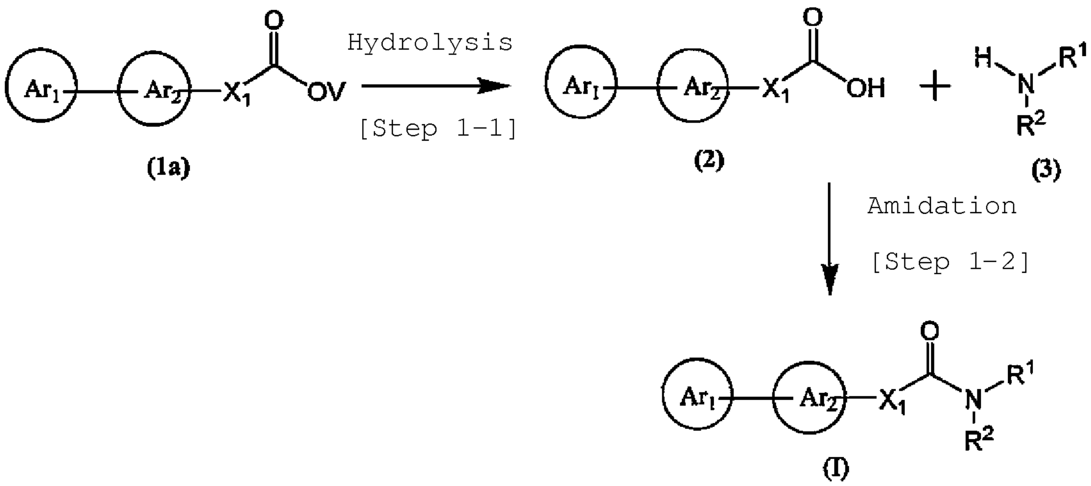


(1a)
(2)
(3)
(I)

In the formula, $Ar_1$, $Ar_2$ and $X_1$ are as defined above;
V represents a protecting group for a carboxyl group or the like such as a methyl group, an ethyl group, a benzyl group, an allyl group, a triphenylmethyl group, a tert-butyl group, a methoxymethyl group or a tert-butyldimethylsilyl group; and

(1) $R^1$ and $R^2$ each represent a group selected from Substituent Group A4 shown above; or $R^1$ and $R^2$, together with a nitrogen atom to which they are bonded, represent:
(2-1) a 5- to 11-membered non-aromatic heterocyclic group which may be substituted with 1 to 4 substituents selected from Substituent Group A4 shown above and is represented by the formula (II):

[Formula 47]

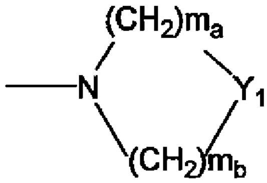

(II)

wherein $Y_1$ represents (1) -NH-, (2) -O-, (3) -S-, (4) -SO-, (5) $-SO_2-$, (6) $-CH_2-$, (7) -CO-, (8) -CONH-, (9) -NHCO-, (10) $-CR^5=CR^6-$ (wherein $R^5$ and $R^6$ each represent a substituent selected from Substituent Group A4 shown above), (11) a single bond or (12) $>C=CR^{13}R^{14}$ (wherein $R^{13}$ and $R^{14}$ each represent a substituent selected from Substituent Group A4 shown above); and $m_a$ and $m_b$ each represent an integer of 0 to 4; (2-2) a 6- to 20-membered non-aromatic heterocyclic group which may be substituted with 1 to 4 substituents selected from Substituent Group A4 shown above and is represented by the formula (III):

[Formula 48]

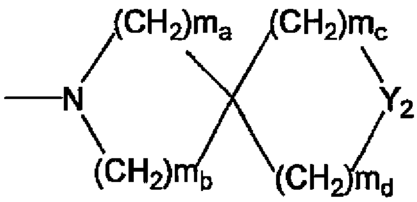

(III)

wherein $Y_2$ represents (1) -NH-, (2) -O-, (3) -S-, (4) -SO-, (5) $-SO_2-$, (6) $-CH_2-$, (7) -CO-, (8) -CONH-, (9) -NHCO-, (10) $-CR^5=CR^6-$ (wherein $R^5$ and $R^6$ each represent a substituent selected from Substituent Group A4 shown above or $R^5$ and $R^6$, together with a carbon atom to which they are bonded, form a 6- to 14-membered aromatic hydrocarbon ring group or a 6- to 14-membered non-aromatic hydrocarbon ring group) or (11) a single bond; and $m_a$, $m_b$, $m_c$ and $m_d$ each represent an integer of 0 to 4;

(2-3) a 9- to 16-membered non-aromatic heterocyclic group which may be substituted with 1 to 4 substituents selected from Substituent Group A4 shown above and is represented by the formula (IV):

[Formula 49]

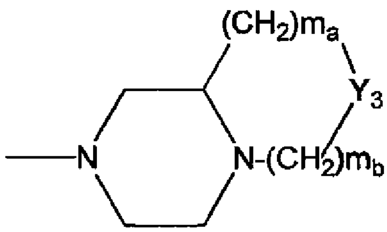

(IV)

wherein $Y_3$ represents (1) -NH-, (2) -O-, (3) -S-, (4) -SO-, (5) $-SO_2-$, (6) $-CH_2-$, (7) -CO-, (8) -CONH-, (9) -NHCO- or (10) a single bond; and $m_a$ and $m_b$ each represent an integer of 0 to 4;

(2-4) a group which may be substituted with 1 to 4 substituents selected from Substituent Group A4 shown above and is represented by the following formula:
[Formula 50]

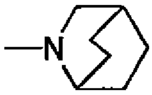,
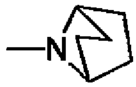,
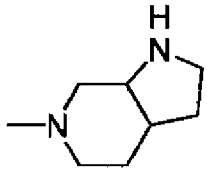
or
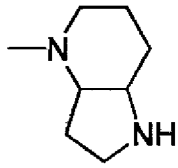

; or
(2-5) a group which may be substituted with 1 to 4 substituents selected from Substituent Group A4 shown above and is represented by the following formula:
[Formula 51]

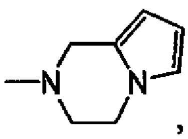,
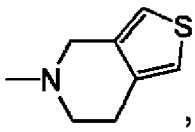,
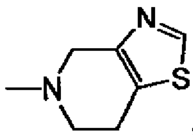,
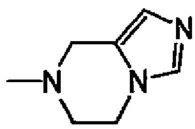,
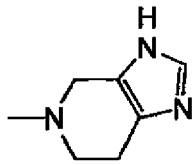,
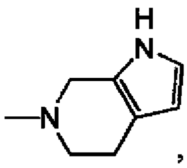,
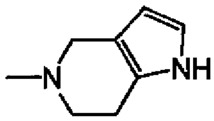,
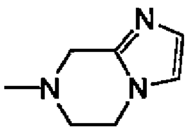,
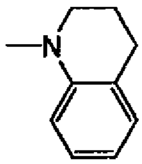,
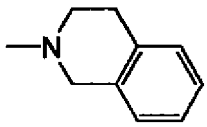,
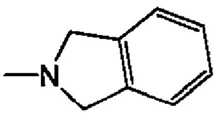
or
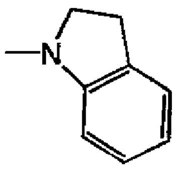

The above General Preparation Method 1 is an example of a method for preparing the compound of the general formula (I) comprising converting an ester compound (1a) into a carboxylic acid compound (2) by deprotection reaction in Step 1-1; and then reacting the carboxylic acid compound (2) with an amine compound (3) by amidation reaction.

[Preparation of carboxylic acid compound (2)]

The carboxylic acid compound (2) can be prepared from the ester compound (1a) according to Step 1-1, for example. Specifically, the deprotection reaction in Step 1-1 varies according to the starting material and is not particularly limited insofar as the conditions are similar to those in this reaction. A method known to a person skilled in the art may be used for the reaction (see T.W. Green, "Protective Groups in Organic Synthesis", John Wiley & Sons, Inc., 1981, p. 154-186). The reaction is preferably hydrolysis reaction of the ester compound. A method described in many known

documents may be used for the reaction (see Shin Jikken Kagaku Koza (New Courses in Experimental Chemistry), vol. 14, Yuki Kagobutsu No Gosei To Hannou (Synthesis and Reaction of Organic Compounds) [II], edited by The Chemical Society of Japan, Maruzen Co., Ltd., 1978, p.930-943, for example). Preferably, the desired carboxylic acid compound (2) can be obtained by carrying out reaction in the presence of 1.0 to 5.0 equivalents of a metal hydroxide (preferably sodium hydroxide, potassium hydroxide or lithium hydroxide, for example) with respect to the ester compound (1a), for example, using an aqueous solvent (a mixed solvent of water and methanol, ethanol or/and tetrahydrofuran or the like), for example at room temperature to 100°C. The carboxylic acid compound (2) can also be appropriately obtained under acidic conditions (preferably trifluoroacetic acid) depending on the corresponding ester compound (1a). Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

[Preparation of compound of general formula (I)]

The compound of the general formula (I) can be prepared from the carboxylic acid compound (2) according to Step 1-2. Specifically, the amidation reaction in Step 1-2 varies according to the starting material and is not particularly limited insofar as the conditions are similar to those in this reaction. A known method described in many documents may be used for the reaction (see Shin Jikken Kagaku Koza (New Courses in Experimental Chemistry), vol.14, Yuki Kagobutsu No Gosei To Hannou (Synthesis and Reaction of Organic Compounds) [II], edited by The Chemical Society of Japan, Maruzen Co., Ltd., 1978, p.1136-1162, for example). Preferable examples of the method include i) a method of converting the carboxylic acid compound (2) into an acid halide and then reacting the acid halide compound with an amine compound under basic conditions (see Shin Jikken Kagaku Koza (New Courses in Experimental Chemistry), vol. 14, Yuki Kagobutsu No Gosei To Hannou (Synthesis and Reaction of Organic Compounds) [II], edited by The Chemical Society of Japan, Maruzen Co., Ltd., 1978, p.1142-1145, for example); and ii) a method of reacting the carboxylic acid compound (2) with an amine compound using a condensing agent (see "Yukikagaku Jikken No Tebiki (Introduction to Organic Chemistry Experiments) [4]", Kagaku-Dojin Publishing Company, Inc., 1990, p.27-52, for example).

In the method i), the base, solvent and reaction temperature used vary according to the starting material and are not particularly limited. The amidation is preferably performed using, for example, (i) a method using pyridine, lutidine, quinoline, isoquinoline or the like as a basic solvent, (ii) a method using pyridine, triethylamine, N,N-diisopropylethylamine or the like as a base and using tetrahydrofuran, 1,4-dioxane or the like as a solvent not inhibiting the reaction and allowing the starting material to be dissolved therein to a certain extent or a mixed solvent thereof or (iii) a method using a two-layer partition system containing an alkaline solution, preferably a sodium hydroxide or potassium hydroxide solution, for example, as a base and a halogenated solvent, preferably methylene chloride or 1,2-dichloroethane, for example. The reaction temperature must be a temperature that can complete the reaction without promoting formation of a by-product and is preferably ice-cold temperature to 100°C. Under preferable reaction conditions, the reaction is completed in 1 to 24 hours, and the progress of the reaction can be monitored by a known chromatography technique.

The method for converting the carboxylic acid compound (2) into an acid halide varies according to the starting material and is not particularly limited insofar as the conditions are similar to those in this reaction. A known method may be used for the reaction. Preferably, a chlorinating agent such as thionyl chloride or oxalyl chloride can be used in an inert solvent such as methylene chloride, toluene or tetrahydrofuran. A catalytic amount of N,N-dimethylformamide or the like may be added to make the reaction proceed. The reaction temperature must be a temperature that can complete the reaction without promoting formation of an undesirable by-product, and is preferably ice-cold temperature to 100°C.

In the method ii), the condensing agent used varies according to the starting material and is not particularly limited. Preferably, 1.0 to 2.0 equivalents of 1,3-dicyclohexylcarbodiimide, 1-ethyl-3-(3'-dimethylaminopropyl)carbodiimide or benzotriazol-1-yloxytris(dimethylamino)phosphonium hexafluorophosphate is appropriately used with respect to the carboxylic acid compound (2). 1.0 to 2.0 equivalents of N-hydroxysuccinimide or N-hydroxybenzotriazole may be added in order to make the reaction efficiently proceed, for example. This reaction is preferably performed in the presence of a solvent from the viewpoint of operativity and stirring efficiency. The solvent used varies according to the starting material and the condensing agent used, and is not particularly limited insofar as it does not inhibit the reaction and allows the starting material to be dissolved therein to a certain extent. Preferable examples of the solvent include halogenated solvents such as methylene chloride and 1,2-dichloroethane, and polar solvents such as tetrahydrofuran and N,N-dimethylformamide. The reaction temperature must be a temperature that can complete the reaction without promoting formation of an undesirable by-product, and is preferably ice-cold temperature to 100°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization. Alternatively, the desired compound of the general formula (I) can be obtained by converting $R^1$ and $R^2$ by a conventional method using a technique known to a person skilled in the art after forming an amide bond. The desired compound of the general formula (I) can also be

obtained by appropriately modifying the substituents for $Ar_1$, $Ar_2$ and $X_1$.

[Preparation of amine compound (3)]

The amine compound (3) is commercially available or can be obtained by a technique known to a person skilled in the art. Preferable examples of the method include i) a method of converting a corresponding alcohol compound or alkyl halide compound into the amine compound by a known technique, ii) a method of converting a corresponding nitro compound, nitrile compound, oxime compound, azide compound or acid amide compound by a known reduction reaction, iii) a method of converting a corresponding carbonyl compound by a known reductive amination reaction and iv) a method of deprotecting a nitrogen atom protected by a protecting group to obtain the amine compound.

In the method i), the conversion can be performed by a method described in many known documents. For example, the amine compound is preferably obtained from the corresponding alcohol compound by Mitsunobu reaction (see O. Mitsunobu, "Synthesis", 1981, p.1, for example) or from an alkyl halide compound by the Gabriel method (see M.M.S. Gibson et al., "Angew. Chem.", 1968, vol.80, p.986, for example). (i) In Mitsunobu reaction, preferably, the desired amine compound can be efficiently obtained by two-stage reaction in which the corresponding alcohol compound is condensed with an imide compound using 1.0 to 3.0 equivalents of dialkyl azodicarboxylate in the presence of 1.0 to 3.0 equivalents of triphenylphosphine and is then treated with 1.0 to 3.0 equivalents of hydrazine, for example. The reaction temperature must be a temperature that can complete the reaction without promoting formation of an undesirable by-product. Preferably, the temperature is ice-cold temperature to 100°C for the first-stage condensation with an imide compound and is room temperature to 100°C for the second-stage hydrazine treatment. The solvent used in this reaction varies according to the starting material and the condensing agent used, and is not particularly limited insofar as it does not inhibit the reaction and allows the starting material to be dissolved therein to a certain extent. Diethyl ether or tetrahydrofuran is preferable for the first-stage reaction, for example, and methanol or ethanol is preferable for the second-stage reaction, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization. (ii) In the Gabriel method, preferably, the desired amine compound can be efficiently obtained by two-stage reaction in which the corresponding alkyl halide compound is condensed with an imide compound by a technique known to a person skilled in the art and is then treated with 1.0 to 3.0 equivalents of hydrazine. The reaction temperature must be a temperature that can complete the reaction without promoting formation of an undesirable by-product. Preferably, the temperature is ice-cold temperature to 100°C for the first-stage condensation with an imide compound and is 50 to 100°C for the second-stage hydrazine treatment. The solvent used in this reaction varies according to the starting material and the condensing agent used, and is not particularly limited insofar as it does not inhibit the reaction and allows the starting material to be dissolved therein to a certain extent. Diethyl ether, tetrahydrofuran or N, N-dimethylformamide is preferable for the first-stage reaction, for example, and methanol or ethanol is preferable for the second-stage reaction, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

In the method ii), a reduction reaction described in many known documents may be used (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., 1978, p.1333-1341, for example). Preferably, the desired amine compound can be obtained by a catalytic reduction method using a metal catalyst or a reduction method using a metal hydride, for example. (i) The catalytic reduction method is preferably performed in a hydrogen atmosphere at normal pressure to 100 atm. Preferable examples of the metal catalyst used in this reaction include platinum, platinum oxide, platinum black, Raney nickel and palladium-carbon. The solvent used in this reaction varies according to the starting material, and is not particularly limited insofar as it does not inhibit the reaction and allows the starting material to be dissolved therein to a certain extent. Preferable examples of the solvent include methanol, ethanol, diethyl ether, tetrahydrofuran, methylene chloride, chloroform and ethyl acetate. An acidic substance such as acetic acid or hydrochloric acid may be appropriately added in order to make the reaction efficiently proceed. (ii) In the reduction method using a metal hydride, preferably, the desired amine compound (3) can be efficiently obtained using lithium aluminum hydride or diborane. The solvent used in this reaction varies according to the starting material, and is not particularly limited insofar as it does not inhibit the reaction and allows the starting material to be dissolved therein to a certain extent. The solvent is preferably diethyl ether or tetrahydrofuran, for example. The reaction temperature must be a temperature that can complete the reaction without promoting formation of an undesirable by-product, and is preferably ice-cold temperature to 100°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography

technique or/and crystallization.

In the method iii), a reductive amination reaction known to a person skilled in the art may be used (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., 1978, p. 1380-1384, for example). (i) The desired amine compound is preferably obtained by a method of reacting the corresponding carbonyl compound and an amine compound using dehydration reaction by heating under reflux in the presence of an acid catalyst (such as preferably an inorganic acid such as hydrochloric acid or sulfuric acid; an organic acid such as methanesulfonic acid, p-toluenesulfonic acid or camphorsulfonic acid; or an organic acid salt such as pyridinium p-toluenesulfonate) and reducing the resulting imine compound by a metal hydride or the like such as lithium aluminum hydride or sodium borohydride. (ii) The desired amine compound is also preferably obtained by a method of treating the corresponding carbonyl compound and an amine compound in an inert solvent such as tetrahydrofuran in the presence of a Lewis acid catalyst (preferably titanium (IV) isopropoxide) and then reducing the resulting compound by a metal hydride such as sodium borohydride. (iii) Alternatively, the desired amine compound is preferably obtained by a method of reducing the carbonyl compound and 0.5 to 5.0 equivalents of an amine compound, for example, by a metal hydride such as sodium triacetoxyborohydride or sodium cyanoborohydride in an inert solvent such as methylene chloride, 1,2-dichloroethane, tetrahydrofuran, methanol or ethanol. An acidic substance such as acetic acid or hydrochloric acid may be appropriately added in order to make the reaction efficiently proceed. The progress of these reductive amination reactions can be monitored by a known chromatography technique. An undesirable by-product can be removed by a technique known to a person skilled in the art such as a conventional chromatography technique or/and crystallization.

In the method iv), a deprotection reaction described in many known documents may be used (see T.W. Green, "Protective Groups in Organic Synthesis", John Wiley & Sons, Inc., 1981, for example). The desired amine compound is preferably obtained from a corresponding carbamate compound (preferably a tert-butyl carbamate compound, a benzyl carbamate compound or a 9-fluorenylmethyl carbamate compound, for example), or is preferably obtained from a corresponding amide compound (preferably a formamide compound, an acetamide compound or a trifluoroacetamide compound, for example). Alternatively, the desired amine compound is preferably obtained from a corresponding imide compound by deprotection according to the Gabriel method. The conditions for the deprotection reaction vary according to the starting material and are not particularly limited insofar as the conditions are similar to those in this reaction. A known method may be used for the reaction. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

[General Preparation Method 2]

Typically used General Preparation Method 2 for the ester compound (1a) will be described below.

[Formula 52]

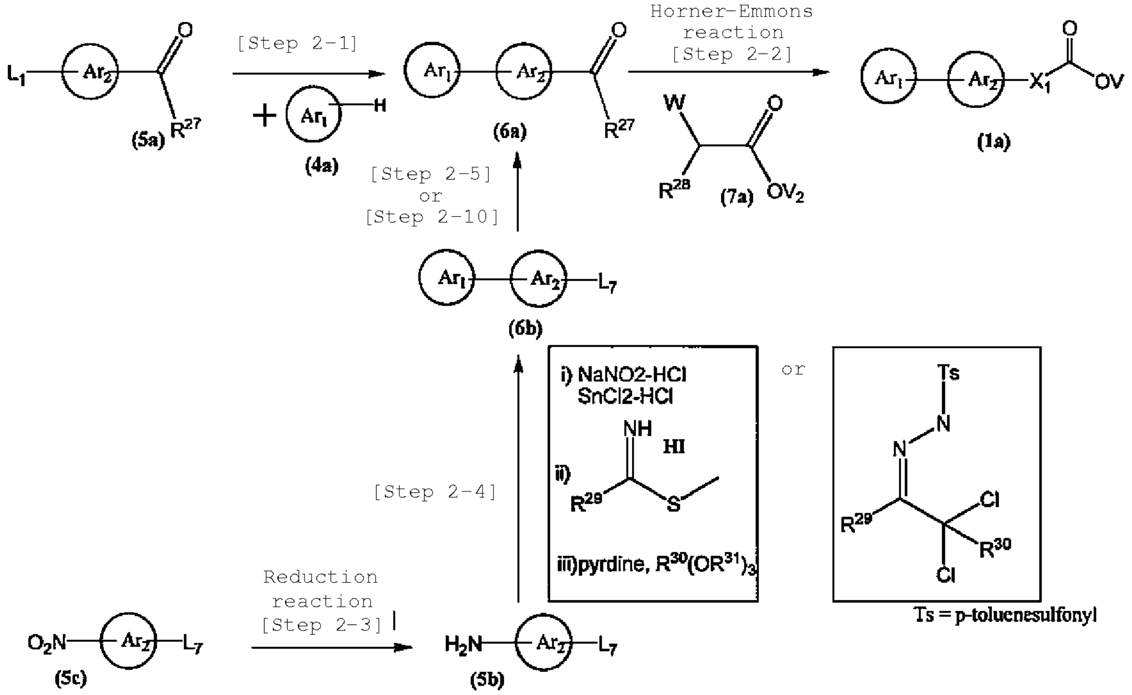


In the formula, $Ar_1$, $Ar_2$, $X_1$ and V are as defined above;
$V_2$ represents a protecting group for a carboxyl group such as a methyl group, an ethyl group, a benzyl group, an allyl group, a triphenylmethyl group, a tert-butyl group or a tert-butyldimethylsilyl group; $L_1$ represents a hydrogen atom or a leaving group such as a fluorine atom, a chlorine atom, a bromine atom, an iodine atom, a sulfonate such as a triflate, a trialkyltin group, boronic acid or a boronate ($B(OV_1)_2$); $L_7$ represents an ester such as a methyl ester, an ethyl ester or a benzyl ester or a cyano group; W represents a dimethylphosphonyl group, a diethylphosphonyl group, a diphenylphosphonyl group or a bis(2,2,2-trifluoroethyl)phosphonyl group;
$R^{31}$ represents a C1-6 alkyl group; $R^{29}$ and $R^{30}$ each represent a group selected from Substituent Group A1 shown below; and $R^{27}$ and $R^{28}$ each represent a group selected from Substituent Group A3 shown below. Substituent Group A1: (1) a hydrogen atom, (2) a halogen atom, (3) a cyano group, (4) a nitro group, (5) a C3-8 cycloalkyl group, (6) a C2-6 alkenyl group, (7) a C2-6 alkynyl group, (8) a C1-6 alkoxy group, (9) a C3-8 cycloalkoxy group, (10) a formyl group, (11) a C1-6 alkylcarbonyl group and (12) a C1-6 alkyl group (wherein the C1-6 alkyl group may be substituted with 1 to 3 substituents selected from the group consisting of a halogen atom, a hydroxyl group, a cyano group, a C1-6 alkoxy group, a C3-8 cycloalkyl group and a C1-6 alkylcarbonyl group).
Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group.

The ester compound (1a) can be obtained by a technique known to a person skilled in the art which varies according to the starting material. For example, the ester compound (1a) can be prepared as shown by the above reaction formula, but the preparation is not limited thereto. Specifically, the ester compound (1a) can be prepared by reacting a compound (4a) with a compound (5a) in Step 2-1 to obtain a carbonyl compound (6a); and then subjecting the carbonyl compound to Horner-Emmons reaction in Step 2-2, for example. Alternatively, the ester compound (1a) can be prepared from an amino compound (5b) as a starting material by forming $Ar_1$ in a compound (6b) through reaction in Step 2-4; then converting the compound (6b) into a compound (6a) according to Step 2-5 or Step 2-10; and subjecting the compound (6a) to reaction in Step 2-2.

[Preparation of compound (5a)]

The compound (5a) used in this step is commercially available or can be obtained by a technique known to a person skilled in the art. If not commercially available, the preferable compound (5a), wherein $L_1$ represents a fluorine atom, a chlorine atom or a bromine atom, can be obtained by oxidizing a corresponding alcohol compound by an oxidation reaction known to a person skilled in the art; or the carbonyl compound can be obtained by reducing an ester compound by a known reduction reaction.

[Preparation of compound (4a)]

The compound (4a) used in this step is commercially available or can be obtained by a technique known to a person skilled in the art. If not commercially available, the preferable compound (4a) can be prepared by a method known to a person skilled in the art (see (i) B.E. Huff et al., "Tetrahedron Letter", 1993, vol.50, p.8011-8014, for example, in the case of tetrazole; (ii) T. Vanek et al., "Collect. Czech. Chem. Commun." 1984, vol.49, p.2492, for example, in the case of [1,2,4]triazole; and (iii) J. Michel et al., "Tetrahedron Letter", 2001, vol.42, p.9117-9118, for example, in the case of [1,2,3]triazole).

[Preparation of carbonyl compound (6a)]

The carbonyl compound (6a) can be prepared from the compound (5a) as a starting material according to Step 2-1, for example. Specifically, Step 2-1 varies according to the starting material and is not particularly limited insofar as the conditions are similar to those in this reaction. A method known to a person skilled in the art may be used for the reaction. For example, the compound (4a) and the compound (5a) are preferably subjected to coupling reaction under basic conditions (see D.D. Davey et al., "J. Med. Chem.", 1991, vol.39, p.2671-2677, for example). Specifically, 2.0 to 5.0 equivalents of the compound (4a) is preferably used with respect to the compound (5a). Examples of the base used include sodium hydride, sodium hydroxide, potassium hydroxide, potassium carbonate, sodium carbonate, cesium carbonate and barium carbonate. 2.0 to 5.0 equivalents of the base is preferably used with respect to the compound (5a). The solvent used in this reaction varies according to the starting material, and is not particularly limited insofar as it does not inhibit the reaction and allows the starting material to be dissolved therein to a certain extent. Preferable examples of the solvent include acetonitrile, tetrahydrofuran, dimethyl sulfoxide, N,N-dimethylformamide and N-methylpyrrolidine. The reaction temperature must be a temperature that can complete the reaction without promoting formation of an undesirable by-product, and is preferably room temperature to 100°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

[Preparation of phosphonate compound (7a)]

[Formula 53]

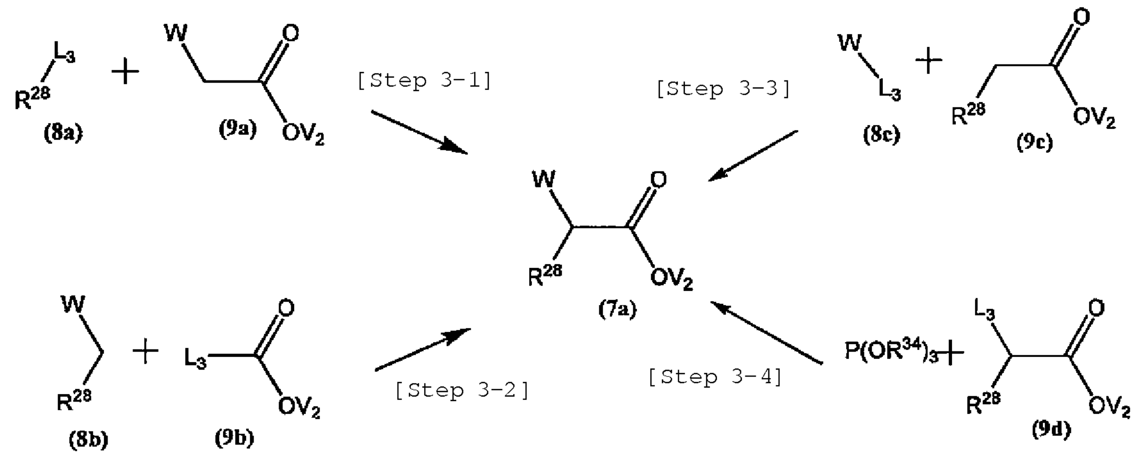


In the formula, $V_2$, W and $R^{28}$ are as defined above; $R^{34}$ represents a methyl group, an ethyl group, a phenyl group or a 2,2,2-trifluoroethyl group; and $L_3$ represents a chlorine atom, a bromine atom or an iodine atom.

The above reaction formula shows an example of a method for preparing the phosphonate compound (7a). Specifically, the phosphonate compound (7a) is commercially available or can be obtained by a method shown in the above Step 3-1 to Step 3-4 and known to a person skilled in the art (see C. Patois et al., "Synth. Commun.", 1991, vol. 22, p.2391; or J.A. Jackson et al., "J. Org. Chem.", 1989, vol.20, p.5556, for example). Step 3-1 is a step of obtaining the desired phosphonate compound (7a) by treating a phosphonate compound (9a) with 1.0 to 2.0 equivalents of an alkyl halide compound (8a) with respect to the phosphonate compound (9a) under basic conditions to introduce $R^{28}$, for example. Step 3-2 is a step of obtaining the desired phosphonate compound (7a) by treating a phosphonate compound (8b) with 1.0 to 2.0 equivalents of a halogenated formate compound (9b) under basic conditions. Step 3-3 is a step of obtaining the desired phosphonate compound (7a) by treating a phosphonic acid halide (8c) with 1.0 to 2.0 equivalents of an ester compound (9c) with respect to the phosphonic acid halide compound (8c) under basic conditions. Step 3-4 is a step of obtaining the desired phosphonate compound (7a) by treating an α-haloester compound (9d) with 1.0 to 10.0 equivalents of a trialkyl phosphite with respect to the α-haloester compound.

The base used in these steps varies according to the starting material. 1.0 to 1.5 equivalents of sodium hydride, n-butyl lithium, lithium diisopropylamide, lithium bis(trimethylsilyl)amide or sodium bis(trimethylsilyl)amide is preferably used, for example. The trialkyl phosphite used in this step is preferably trimethyl phosphite or triethyl phosphite. The solvent used in this step varies according to the starting material, and is not particularly limited insofar as it does not inhibit the reaction and allows the starting material to be dissolved therein to a certain extent. Preferable examples of the solvent include hexane, toluene, diethyl ether, tetrahydrofuran, N,N-dimethylformamide, hexamethylphosphoric triamide and a mixed solvent as described above. The reaction temperature must be a temperature that can complete the reaction without promoting formation of an undesirable by-product, and is preferably -78°C to 150°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

The desired phosphonate compound (7a) can be efficiently obtained by modification of $R^{28}$ by a technique known to a person skilled in the art.

The alkyl halide compound (8a), phosphonate compound (8b), phosphonic acid halide compound (8c), phosphonate compound (9a), halogenated formate compound (9b), ester compound (9c) and α-haloester compound (9d) used in this step are commercially available or can be obtained by a technique known to a person skilled in the art.

[Conversion of carbonyl compound (6a) into ester compound (1a)

Conversion of the carbonyl compound (6a) into the ester compound (1a) varies according to the starting material and can be performed by a known technique described in various documents (see H.O. House, "Modern synthetic reactions", W.A. Benjamin Inc., 1972, p.629-733; or W. Carrthers, "Some modern methods of organic synthsis", Cambridge University Press, 1986, p.125-144, for example). The carbonyl compound (6a) can be converted into the ester compound (1a) according to Step 2-2, for example. Specifically, Horner-Emmons reaction in Step 2-2 varies according

to the starting material and is not particularly limited insofar as the conditions are similar to those in this reaction. A method known to a person skilled in the art may be used for the reaction (see W.S. Wadsworth, Jr. "Org. Reactions.", 1997, vol.25, p.73, for example). Specifically, the carbonyl compound (6a) can be converted into the corresponding ester compound (1a) by condensation with the phosphonate compound (7a) under basic conditions. 1.0 to 2.0 equivalents of a base is preferably used with respect to the carboxylic acid compound (6a). Preferable examples of the base include sodium hydride, sodium hydroxide, potassium hydroxide, lithium hydroxide, n-butyllithium, lithium diisopropylamide, lithium bis(trimethylsilyl)amide, sodium bis(trimethylsilyl)amide, triethylamine and diisopropylethylamine. The solvent used in this reaction varies according to the starting material, and is not particularly limited insofar as it does not inhibit the reaction and allows the starting material to be dissolved therein to a certain extent. Preferable examples of the solvent include diethyl ether, tetrahydrofuran, dimethyl sulfoxide, toluene, benzene, ethanol and methanol. The reaction temperature must be a temperature that can complete the reaction without promoting formation of an undesirable by-product, and is preferably -78°C to 100°C, and more preferably -78°C to room temperature. Under preferable reaction conditions, the reaction is completed in 1 to 24 hours, and the progress of the reaction can be monitored by a known chromatography technique. In this reaction, a desired geometric isomer can be selectively prepared by appropriately selecting the phosphonate compound (5a), the base or/and the solvent. An undesirable by-product or geometric isomer can be removed by a technique known to a person skilled in the art such as a conventional chromatography technique or/and crystallization.

[Preparation of nitro compound (5c)]

The nitro compound (5c) used in this step is commercially available or can be obtained by a technique known to a person skilled in the art. If not commercially available, the preferable compound (5c) can be efficiently obtained from a corresponding precursor by a nitration reaction known to a person skilled in the art (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., 1978, p.1261-1300, for example).

[Preparation of amine compound (5b)]

The amine compound (5b) is commercially available or can be obtained by a technique known to a person skilled in the art. Preferably, the compound can be prepared from a nitro compound (5c) as a starting material according to Step 2-3, for example. Specifically, reduction reaction in Step 2-3 varies according to the starting material and is not particularly limited insofar as the conditions are similar to those in this reaction. A method known to a person skilled in the art may be used for the reaction (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., 1978, p.1333-1335, for example). The reaction is preferably a catalytic reduction method using a metal catalyst or a reduction method using a metal, for example. (i) The catalytic reduction method is preferably performed in a hydrogen atmosphere at normal pressure to 100 atm. Preferable examples of the metal catalyst used in this reaction include platinum, platinum oxide, platinum black, Raney nickel and palladium-carbon. The solvent used in this reaction varies according to the starting material, and is not particularly limited insofar as it does not inhibit the reaction and allows the starting material to be dissolved therein to a certain extent. Preferable examples of the solvent include methanol, ethanol, diethyl ether, tetrahydrofuran, methylene chloride, chloroform and ethyl acetate. An acidic substance such as acetic acid or hydrochloric acid may be appropriately added in order to make the reaction efficiently proceed. (ii) The reduction method using a metal preferably employs zinc, iron or tin, for example, and is preferably performed under acidic conditions such as hydrochloric acid, acetic acid or ammonium chloride.
The solvent used in these reactions varies according to the starting material, and is not particularly limited insofar as it does not inhibit the reaction and allows the starting material to be dissolved therein to a certain extent. Preferable examples of the solvent include methanol, ethanol and 2-propanol. The reaction temperature must be a temperature that can complete the reaction without promoting formation of an undesirable by-product, and is preferably room temperature to 100°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

[Preparation of compound (6b)]

The compound (6b) can be obtained by a technique known to a person skilled in the art. Preferably, the compound can be prepared from the amine compound (5b) as a starting material according to Step 2-4, for example. Specifically, in Step 2-4, i) when $Ar_1$ is [1,2,4]triazole, the amine compound (5b) can be efficiently converted into the compound (6b) by generating a diazonium salt using sodium nitrite and treating the diazonium salt with stannic chloride to prepare hydrazine in the first stage; condensing the hydrazine with a thioimidate in the second stage; and cyclizing

the condensate with an ortho ester in the presence of a base in the third stage. Preferably, in the first stage, the compound (5b) is reacted with 1.0 to 1.1 equivalents of sodium nitrite with respect to the compound (5b) in a hydrochloric acid solvent at -20°C to 0°C to prepare a diazonium salt, and then the diazonium salt is treated with 3.5 to 4.0 equivalents of tin chloride at the same temperature, for example. The thioimidate used in the second stage can be easily obtained by reacting a corresponding thioamide compound with 1.0 to 10.0 equivalents of methyl iodide in an ether solvent at room temperature. 1.0 to 1.1 equivalents of the thioimidate is preferably used with respect to the compound (5b). The reaction solvent is preferably an alcohol solvent such as methanol or ethanol. The reaction temperature is preferably ice-cold temperature to room temperature. In the third stage, 5 to 15 equivalents of the ortho ester is preferably reacted in the presence of 1.0 to 3.0 equivalents of a base, for example. The base used is potassium carbonate, triethylamine or pyridine, for example, and preferably pyridine. The solvent used in the present reaction varies according to the starting material, and is not particularly limited insofar as it does not inhibit the reaction and allows the starting material to be dissolved therein to a certain extent. The solvent is preferably toluene, tetrahydrofuran or dioxane, for example. The reaction temperature must be a temperature that can complete the reaction without promoting formation of an undesirable by-product, and is preferably room temperature to solvent reflux temperature. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization. ii) When $Ar_1$ is [1,2,3]triazole, the compound (6b) can be obtained by treating tosylhydrazone obtained from p-toluenesulfonylhydrazine and α,α-dichloroketone with the compound (5b) in an alcohol solvent by a known method (see K. Sakai et al., "Bull. Chem. Soc. Jpn.", 1986, vol.59, p. 179-183, for example).

[Preparation of compound (6a) from compound (6b)-1]

When the compound (6a) is an aldehyde compound, the aldehyde compound (6a) can be prepared from the compound (6b) as a starting material according to Step 2-5. Specifically, Step 2-5 varies according to the starting material and is not particularly limited insofar as the conditions are similar to those in this reaction. A method known to a person skilled in the art may be used for the reaction. For example, i) when $L_7$ is an alkyl ester group, a reduction reaction described in many known documents may be used (see Jikken Kagaku Koza (Courses in Experimental Chemistry), 4th edition, vol.21, Yuki Gosei (Organic Synthesis) [III], edited by The Chemical Society of Japan, Maruzen Co., Ltd., 1991, p.83-85, for example). Preferably, the desired aldehyde compound can be obtained by a reduction method using a metal hydride such as diisobutylaluminum hydride, for example. More preferably, the desired aldehyde compound can be efficiently obtained by a reduction method using lithium aluminum hydride or an aluminum hydride complex in the presence of an amine (see T. Abe et al., "Tetrahedron", 2001, vol.57, p.2701-2710, for example). For example, ii) when $L_7$ is a cyano group, a reduction reaction described in many known documents may be used. Preferably, the desired aldehyde compound can be obtained by a reduction method using a metal hydride such as sodium bis(2-methoxyethoxy) aluminum hydride or diisobutylaluminum hydride, for example (see Jikken Kagaku Koza (Courses in Experimental Chemistry), 4th edition, vol.21, Yuki Gosei (Organic Synthesis) [III], edited by The Chemical Society of Japan, Maruzen Co., Ltd., 1991, p.89-92, for example). Alternatively, for example, iii) when $L_7$ is an alkyl ester group, it is possible to use a two-step method of reducing the compound (1a) to an alcohol compound using a technique known to a person skilled in the art (see Jikken Kagaku Koza (Courses in Experimental Chemistry), 4th edition, vol.20, Yuki Gosei (Organic Synthesis) [II], edited by The Chemical Society of Japan, Maruzen Co., Ltd., 1992, p.1-29, for example) and then oxidizing the alcohol compound to an aldehyde (see Jikken Kagaku Koza (Courses in Experimental Chemistry), 4th edition, vol. 21, Yuki Gosei (Organic Synthesis) [III], edited by The Chemical Society of Japan, Maruzen Co., Ltd., 1991, p.2-22, for example).

In the method i), the base used in the reduction reaction varies according to the starting material and is not particularly limited. A secondary amine may be used as a base. Preferably, the desired aldehyde compound can be efficiently obtained using a linear or cyclic secondary alkylamine such as diethylamine or pyrrolidine. The solvent and reaction temperature used vary according to the starting material and are not particularly limited. The solvent is a solvent not inhibiting the reaction and allowing the starting material to be dissolved therein to a certain extent or a mixed solvent thereof. Preferably, an ether solvent such as tetrahydrofuran, 1,4-dioxane or diethyl ether or a non-polar solvent such as toluene or benzene can be used, for example. The reaction temperature must be a temperature that can complete the reaction without promoting formation of an undesirable by-product, and is preferably -78°C to room temperature. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

In the method ii), the solvent and reaction temperature used in the reaction vary according to the starting material and are not particularly limited. The solvent is a solvent not inhibiting the reaction and allowing the starting material to be dissolved therein to a certain extent or a mixed solvent thereof. Preferably, an ether solvent such as

tetrahydrofuran, 1,4-dioxane or diethyl ether or a non-polar solvent such as toluene or benzene can be used, for example. The reaction temperature must be a temperature that can complete the reaction without promoting formation of an undesirable by-product, and is preferably -78°C to room temperature. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

In the method iii), the solvent and reaction temperature used in the reduction step vary according to the starting material and are not particularly limited. The solvent is a solvent not inhibiting the reaction and allowing the starting material to be dissolved therein to a certain extent or a mixed solvent thereof. Preferably, an ether solvent such as tetrahydrofuran, 1,4-dioxane or diethyl ether or a non-polar solvent such as toluene or benzene can be used, for example. The reaction temperature must be a temperature that can complete the reaction without promoting formation of an undesirable by-product, and is preferably -78°C to room temperature. Under preferable reaction conditions, the reaction is completed in 1 to 24 hours, and the progress of the reaction can be monitored by a known chromatography technique. The solvent and reaction temperature used in the oxidation step vary according to the starting material and are not particularly limited. As a solvent not inhibiting the reaction and allowing the starting material to be dissolved therein to a certain extent or a mixed solvent thereof, an ether solvent such as tetrahydrofuran, 1,4-dioxane or diethyl ether; a halogenated solvent such as methylene chloride, 1,2-dichloroethane or chloroform; or a non-polar solvent such as toluene or benzene can be preferably used, for example. The reaction temperature must be a temperature that can complete the reaction without promoting formation of an undesirable by-product, and is preferably -78°C to 100°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

[Preparation of compound (6a) from compound (6b)-2]

When the compound (6a) is a ketone compound, the ketone compound (6a) can be prepared from the compound (6b) as a starting material according to Step 2-10. Specifically, Step 2-10 varies according to the starting material and is not particularly limited insofar as the conditions are similar to those in this reaction. A method known to a person skilled in the art may be used for the reaction. For example, i) when $L_7$ is an ester group, a reaction described in many known documents may be used (see "Teterahedron Letter", 1981, vol.22, p.3815-3818, for example). Preferably, the ketone compound (6a) can be efficiently obtained by converting the compound (6b) into carboxylic acid by the same method as in Step 1-1; converting the carboxylic acid to Weinreb amide by the same method as in Step 1-2; and then reacting the Weinreb amide with a Grignard reagent, an alkyl metal reagent, an aryl metal reagent or a metal enolate, for example. Alternatively, for example, ii) when $L_7$ is a cyano group, a reaction described in many known documents may be used (see Jikken Kagaku Koza (Courses in Experimental Chemistry), 4th edition, vol.21, Yuki Gosei (Organic Synthesis) [III], edited by The Chemical Society of Japan, Maruzen Co., Ltd., 1991, p.289-298, for example). Preferably, the desired ketone compound can be obtained by reacting a cyano group with a Grignard reagent and hydrolyzing the generated ketone imine salt, for example.

[General Preparation Method 3]

Typically used General Preparation Method 3 for the ester compound (1a) will be described below.

[Formula 54]

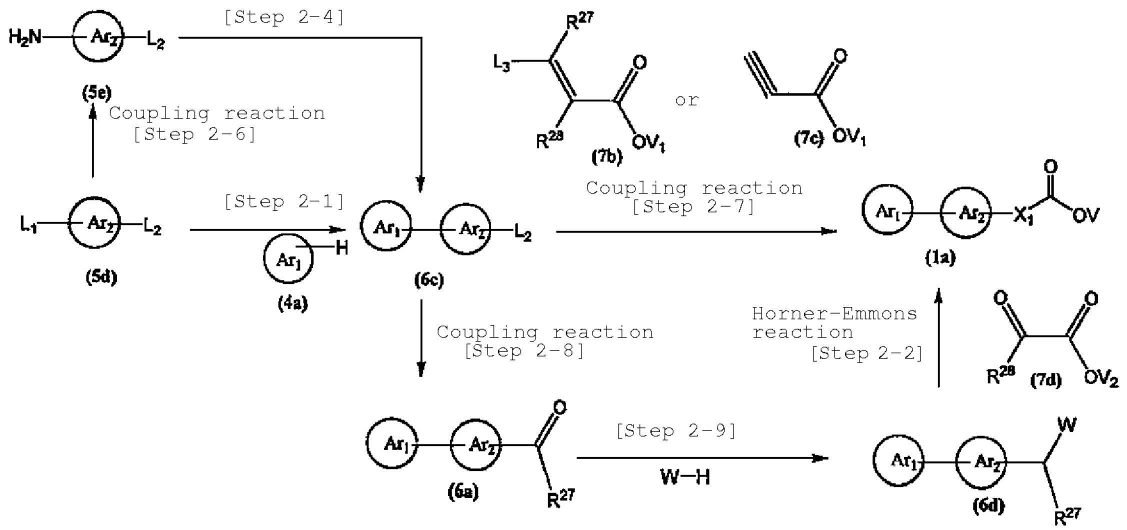


In the formula, $Ar_1$, $Ar_2$, $X_1$, $R^{27}$ and $R^{28}$ are as defined above;
V, $V_1$ and $V_2$ are the same or different and each represent a protecting group for a carboxyl group such as a methyl group, an ethyl group, a benzyl group, an allyl group, a triphenylmethyl group, a tert-butyl group or a tert-butyldimethylsilyl group; $L_1$, $L_2$ and $L_3$ each represent a hydrogen atom or a leaving group such as a fluorine atom, a chlorine atom, a bromine atom, an iodine atom, a sulfonate such as a triflate, a trialkyltin group, boronic acid or a boronate $(B(OV_1)_2)$; and W represents a dimethylphosphonyl group, a diethylphosphonyl group, a diphenylphosphonyl group or a bis(2,2,2-trifluoroethyl)phosphonyl group.

The ester compound (1a) can be prepared from an amino compound (5e) as a starting material by forming $Ar_1$ in a compound (6c) through reaction in Step 2-4; and then coupling the compound (6c) with a compound (7b) or (7c) according to Step 2-7. The ester compound (1a) can also be prepared by converting a compound (5d) as a starting material into a compound (6b) according to Step 2-1; and then subjecting the compound (6b) to Step 2-7. Alternatively, the ester compound (1a) can be prepared by converting a compound (6c) into the compound (6a) in Step 2-8; then converting the compound (6a) into a compound (6d) in Step 2-9; and reacting the compound (6d) with a compound (7d) by Horner-Emmons reaction in Step 2-2.

[Preparation of amine compound (5e)]

The preferable amine compound (5e) can be prepared according to coupling reaction in Step 2-6 from the compound (5d) as a starting material which is commercially available or can be obtained by a technique known to a person skilled in the art. Specifically, the coupling reaction in Step 2-6 varies according to the starting material and is not particularly limited insofar as the conditions are similar to those in this reaction. A method known to a person skilled in the art may be used for the reaction. Preferably, for example, it is possible to use a two-stage method of performing coupling reaction of benzophenone imine using a transition metal catalyst and then performing a known benzophenone removal reaction treatment (see S.L. Buchwald et al., "Tetrahedron Lett.", 1997, vol.38, p.6367-6370; or J.F. Hartwig et al., "J. Am. Chem. Soc.", 1998, vol.120, p.827-828, for example). In the coupling reaction of benzophenone imine, it is possible to use 0.01 to 0.2 equivalent of a catalyst with respect to the compound (5d). Preferable examples of the catalyst include known palladium complexes such as palladium (II) acetate, dichlorobis(triphenylphosphine)palladium (II), tetrakis (triphenylphosphine)palladium (0) and tris(dibenzylideneacetone)dipalladium (0); and known nickel catalysts such as (1,5-cyclooctadiene)nickel (0). Preferably, a phosphorus ligand (preferably triphenylphosphine, tri-o-tolylphosphine, tri-tert-butylphosphine, 2-(di-tert-butylphosphino)biphenyl, 2,2'-bis(diphenylphosphino)-1,1'-binaphthyl, 1,2-bis(diphenylphosphino)ethane or 1,1'-bis(diphenylphosphino)ferrocene, for example) is appropriately added in order to make the reaction efficiently proceed, for example. A preferable result may be achieved in the presence of a base. The base used is not particularly limited insofar as it is used in a coupling reaction similar to this reaction. Preferable examples of the base include sodium hydroxide, barium hydroxide, potassium fluoride, cesium fluoride, sodium carbonate, potassium carbonate, cesium carbonate, potassium phosphate and sodium tert-butoxide. This reaction is preferably performed in

the presence of a solvent from the viewpoint of handleability and stirring efficiency. The solvent used varies according to the starting material and the transition metal catalyst used, and is not particularly limited insofar as it does not inhibit the reaction and allows the starting material to be dissolved therein to a certain extent. Preferable examples of the solvent include acetonitrile, tetrahydrofuran, 1,4-dioxane, 1,2-dimethoxyethane, benzene, toluene, xylene, 1-methyl-2-pyrrolidone and N,N-dimethylformamide. The reaction temperature must be a temperature that can complete the coupling reaction, and is preferably room temperature to solvent reflux temperature. This reaction is performed preferably in an inert gas atmosphere, and more preferably in a nitrogen or argon atmosphere. A method known to a person skilled in the art may be used for the treatment after the second stage (see T.W. Green, "Protective Groups in Organic Synthesis", John Wiley & Sons, Inc., 1981). An undesirable by-product can be removed by a technique known to a person skilled in the art such as a conventional chromatography technique or/and crystallization.

In the preferable amine compound (5e), $L_2$ can be modified by a method known to a person skilled in the art, and a hydrogen atom in $L_2$ can be preferably converted into a halogen substituent (see Shin Jikken Kagaku Koza (New Courses in Experimental Chemistry), vol.14, Yuki Kagobutsu No Gosei To Hannou (Synthesis and Reaction of Organic Compounds) [I], edited by The Chemical Society of Japan, Maruzen Co., Ltd., 1977, p.354-360, for example).

[Preparation of compound (6c)]

The compound (6c) can be obtained by a technique known to a person skilled in the art. Preferably, the compound (6c) can be prepared from the compound (5d) as a starting material according to the above Step 2-1 or from the amine compound (5e) as a starting material according to the above Step 2-4, for example.

$L_2$ in the compound (6c) can be modified by a technique known to a person skilled in the art, and can be preferably converted into, for example, an iodine group (see S.L. Buchwald et al., "J. Am. Chem. Soc.", 2002, vol.124, p.14844-14845, for example), a lower alkyltin group (see J. Marti et al., "Synth. Commun.", 2000, vol.30, p.3023-3030, for example) or a boron group (see N. Miyaura et al., "J. Org. Chem.", 1995, vol.60, p.7508-7510, for example).

[Conversion of compound (6c) into ester compound (1a)]

The compound (6c) can be converted into the ester compound (1a) by a method known to a person skilled in the art. For example, the ester compound (1a) can be prepared from the compound (6c) together with the compound (7b) or the compound (7c) according to Step 2-7. Specifically, the coupling reaction in Step 2-7 varies according to the starting material and is not particularly limited insofar as the conditions are similar to those in this reaction. A method known to a person skilled in the art may be used for the reaction. Preferable examples of the method include Mizoroki-Heck reaction (see R.F. Heck, "Org. Reactions.", 1982, vol.27, p.345, for example), Suzuki-Miyaura reaction (see A. Suzuki, "Chem. Rev.", 1995, vol.95, p.2457, for example), Sonogashira reaction (see K. Sonogashira, "Comprehensive Organic Synthesis", 1991, vol.3, p.521) and Stille coupling reaction (see J.K. Stille, "Angew. Chem. Int. Ed. Engl.", 1986, vol.25, p.508, for example).

In the Mizoroki-Heck reaction, the halide or triflate compound (6c), wherein $L_2$ represents a chlorine atom, a bromine atom, an iodine atom or a triflate, is preferably coupled with 1.0 to 5.0 equivalents of the alkene compound (7b; wherein $L_3$ represents a hydrogen atom) with respect to the compound (6c) in the presence of 0.01 to 0.2 equivalent of a transition metal catalyst, for example. This reaction is preferably performed in the presence of a solvent from the viewpoint of handleability and stirring efficiency. The solvent used varies according to the starting material and the transition metal catalyst used, and is not particularly limited insofar as it does not inhibit the reaction and allows the starting material to be dissolved therein to a certain extent. Preferable examples of the solvent include acetonitrile, tetrahydrofuran, 1,4-dioxane, 1,2-dimethoxyethane, benzene, toluene, xylene, 1-methyl-2-pyrrolidone and N,N-dimethylformamide. The reaction temperature must be a temperature that can complete the coupling reaction, and is preferably room temperature to 150°C. This reaction is performed preferably in an inert gas atmosphere, and more preferably in a nitrogen or argon atmosphere. The transition metal catalyst is preferably a palladium complex, for example, and more preferably a known palladium complex such as palladium (II) acetate, dichlorobis(triphenylphosphine)palladium (II), tetrakis(triphenylphosphine)palladium (0) or tris(dibenzylideneacetone)dipalladium (0). It is also preferable to appropriately add a phosphorus ligand (preferably triphenylphosphine, tri-o-tolylphosphine, tri-tert-butylphosphine or 2-(di-tert-butylphosphino)biphenyl, for example) in order to make the reaction efficiently proceed. A preferable result may be achieved in the presence of a base. The base used is not particularly limited insofar as it is used in a coupling reaction similar to this reaction. Preferable examples of the base include triethylamine, N,N-diisopropylethylamine, N,N-dicyclohexylmethylamine and tetrabutylammonium chloride. Under preferable reaction conditions, the reaction is completed in 1 to 24 hours, and the progress of the reaction can be monitored by a known chromatography technique.

In the Suzuki-Miyaura reaction, the halide or triflate compound (6c), wherein $L_2$ represents a chlorine atom, a bromine atom, an iodine atom or a triflate, is preferably coupled with 1.0 to 2.0 equivalents of the boronic acid compound or boronate compound (7b; wherein $L_3$ represents $B(OH)_2$ or $B(OV_1)_2$) in the presence of 0.01 to 0.5 equivalent of a

transition metal catalyst with respect to the compound (6c), for example. This reaction is preferably performed in the presence of a solvent from the viewpoint of handleability and stirring efficiency. The solvent used varies according to the starting material and the transition metal catalyst used, and is not particularly limited insofar as it does not inhibit the reaction and allows the starting material to be dissolved therein to a certain extent. Preferable examples of the solvent include acetonitrile, tetrahydrofuran, 1,4-dioxane, 1,2-dimethoxyethane, benzene, toluene, xylene, 1-methyl-2-pyrrolidone, N,N-dimethylformamide, water and a mixed solvent thereof. The reaction temperature must be a temperature that can complete the coupling reaction, and is preferably room temperature to solvent reflux temperature. This reaction is performed preferably in an inert gas atmosphere, and more preferably in a nitrogen or argon atmosphere. Under preferable reaction conditions, the reaction is completed in 1 to 24 hours, and the progress of the reaction can be monitored by a known chromatography technique. The transition metal catalyst is preferably a known palladium complex, and more preferably a known palladium complex such as palladium (II) acetate, dichlorobis(triphenylphosphine)palladium (II), tetrakis(triphenylphosphine)palladium (0), or tris(dibenzylideneacetone)dipalladium (0). A phosphorus ligand (preferably triphenylphosphine, tri-o-tolylphosphine, tricyclohexylphosphine, or tri-tert-butylphosphine, for example) may be appropriately added in order to make the reaction efficiently proceed. A quaternary ammonium salt, preferably tetrabutylammonium chloride or tetrabutylammonium bromide, for example, may also be appropriately added in order to make the reaction efficiently proceed. In this reaction, a preferable result may be achieved in the presence of a base. The base used at this time varies according to the starting material, the solvent used and the like, and is not particularly limited. Preferable examples of the base include sodium hydroxide, barium hydroxide, potassium fluoride, cesium fluoride, sodium carbonate, potassium carbonate, cesium carbonate and potassium phosphate. Under preferable reaction conditions, the reaction is completed in 1 to 24 hours, and the progress of the reaction can be monitored by a known chromatography technique.

In this reaction, the desired coupling product (1a) can be efficiently obtained even when the compound (7b) is a halide or triflate compound (7b), wherein $L_3$ represents a chlorine atom, a bromine atom, an iodine atom or a triflate, for example, and the compound (6b) is a boronic acid compound or boronate compound (6b), wherein $L_2$ represents $B(OH)_2$ or $B(OV_1)_2$, for example.

The reaction conditions in the Sonogashira reaction vary according to the starting material, the solvent and the transition metal catalyst, and are not particularly limited insofar as the conditions are similar to those in this reaction. A method known to a person skilled in the art may be used for the reaction. An alkyne compound (7c) is preferably used as a starting material. Preferable examples of the solvent include acetonitrile, tetrahydrofuran, 1,4-dioxane, 1,2-dimethoxyethane, benzene, toluene, xylene, 1-methyl-2-pyrrolidone, N,N-dimethylformamide and dimethyl sulfoxide. More preferable examples of the solvent include tetrahydrofuran, 1,4-dioxane, 1-methyl-2-pyrrolidone and N,N-dimethylformamide. The reaction temperature must be a temperature that can complete the coupling reaction, and is preferably room temperature to solvent reflux temperature. This reaction is performed preferably in an inert gas atmosphere, and more preferably in a nitrogen or argon atmosphere. Under preferable reaction conditions, the reaction is completed in 1 to 24 hours, and the progress of the reaction can be monitored by a known chromatography technique. The transition metal catalyst is preferably a known palladium complex, and more preferably a known palladium complex such as palladium (II) acetate, dichlorobis(triphenylphosphine)palladium (II), tetrakis(triphenylphosphine)palladium (0), or tris(dibenzylideneacetone)dipalladium (0), for example. A phosphorus ligand (preferably triphenylphosphine, tri-o-tolylphosphine or tri-tert-butylphosphine, for example) may be appropriately added, for example, in order to make the reaction efficiently proceed. In the reaction, a metal halide or a quaternary ammonium salt such as copper (I) iodide, lithium chloride, tetrabutylammonium fluoride or silver (I) oxide may be added as necessary, for example. A preferable result may be achieved in the presence of a base. The base used here is not particularly limited insofar as it is used in a coupling reaction similar to this reaction. Preferable examples of the base include diethylamine, triethylamine, N,N-diisopropylethylamine, piperidine and pyridine.

In the Stille coupling reaction, 1.0 equivalent or more of the trialkyltin compound (6c), wherein $L_2$ represents a trialkyltin group, is preferably coupled with the halide or triflate compound (7b), wherein $L_3$ represents a chlorine atom, a bromine atom, an iodine atom or a triflate, in the presence of 0.01 to 0.2 equivalent of a transition metal catalyst. It is preferable to appropriately use 0.1 to 5.0 equivalents of copper (I) halide or/and lithium chloride in order to make the reaction efficiently proceed. Preferable examples of the solvent used in this reaction include toluene, xylene, N,N-dimethylformamide, N,N-dimethylacetamide, 1-methyl-2-pyrrolidone and dimethyl sulfoxide. The reaction temperature must be a temperature that can complete the coupling reaction, and is preferably room temperature to solvent reflux temperature. The preferable transition metal catalyst is a palladium complex, preferably a known palladium complex such as palladium (II) acetate, dichlorobis(triphenylphosphine)palladium (II), tetrakis(triphenylphosphine)palladium (0) or tris(dibenzylideneacetone)dipalladium (0), for example, and more preferably tetrakis(triphenylphosphine)palladium (0) or tris(dibenzylideneacetone)dipalladium (0), for example. This reaction is performed preferably in an inert gas atmosphere, and more preferably in a nitrogen or argon atmosphere. Under preferable reaction conditions, the reaction is completed in 1 to 24 hours, and the progress of the reaction can be monitored by a known chromatography technique.

[Preparation of compound (7b) and compound (7c)]

The compound (7b) and the compound (7c) used in this step are commercially available or can be obtained by a technique known to a person skilled in the art. If not commercially available, the preferable compound (7b), wherein $L_3$ represents $B(OH)_2$ or $B(OV_1)_2$; and $V_1$ is as defined above, can be efficiently obtained from a corresponding precursor by a coupling reaction known to a person skilled in the art, for example (see C.R. Deloge et al., "Bull. Soc. Chim. Fr.", 1992, vol.129, p.285-290, for example). Alternatively, the preferable compound (7b; wherein $L_3$ is a triflate) can be efficiently obtained from a corresponding precursor by a method known to a person skilled in the art, for example (see B. Dupre et al., "J. Org. Chem.", 1991, vol.56, p.3197-3198, for example).

[Preparation of compound (6a) from compound (6c)]

The carbonyl compound (6a) can be prepared from the compound (6c) as a starting material according to Step 2-8, for example. Specifically, Step 2-8 varies according to the starting material and is not particularly limited insofar as the conditions are similar to those in this reaction. A method known to a person skilled in the art may be used for the reaction. For example, it is possible to use a two-stage method of converting the compound (6b), wherein $L_2$ preferably represents a chlorine atom, a bromine atom, an iodine atom or a sulfonate such as a triflate, into a vinyl compound by Stille coupling reaction with a vinyltin compound; and then oxidizing the styrene compound by ozone oxidation reaction (see S.S. Chandran et al., "Bioorg. Med. Chem. Lett.", 2001, vol.11, p.1493-1496, for example). It is also possible to employ carbon monoxide insertion reaction using a transition metal catalyst (see T. Okano et al., "Bull. Chem. Soc. Jpn.", 1994, vol.67, p.2329-2332, for example).

[Preparation of ester compound (1a) from compound (6a)]

The ester compound (1a) can also be prepared by converting the carbonyl compound (6a) into the compound (6d); and then reacting the compound (6d) with the compound (7d) by Horner-Emmons reaction in Step 2-2, for example. A known technique described in many documents may be used for Step 2-9 to prepare a compound (6d), for example (see O. Pamies et al., "J. Org. Chem.", 2003, p.4815-4818, for example). Specifically, the carbonyl compound (6a) and a phosphoric acid compound such as diethyl phosphite are preferably used under basic conditions. 1.0 to 2.0 equivalents of a base is preferably used with respect to the carbonyl compound (6a). Preferable examples of the base include 1,8-diazabicyclo[5.4.0]undec-7-ene, triethylamine, pyridine and sodium methoxide. The solvent used in this reaction varies according to the starting material, and is not particularly limited insofar as it does not inhibit the reaction and allows the starting material to be dissolved therein to a certain extent. Preferable examples of the solvent include diethyl ether, tetrahydrofuran, dimethyl sulfoxide, toluene, benzene, ethanol and methanol. The reaction temperature must be a temperature that can complete the reaction without promoting formation of an undesirable by-product, and is preferably -78°C to 100°C, and more preferably -78°C to room temperature. Under preferable reaction conditions, the reaction is completed in 1 to 24 hours, and the progress of the reaction can be monitored by a known chromatography technique. An undesirable by-product formed in this reaction can be removed by a technique known to a person skilled in the art such as a conventional chromatography technique or/and crystallization. The prepared compound (6d) can be modified into a desired compound by a technique known to a person skilled in the art (see T.-J. Tsai, "Tetrahedron Letters", 1996, vol.37. no.5, p.629-632, for example).

[Preparation of compound (7d)]

The compound (7d) used in this step is commercially available or can be obtained by a technique known to a person skilled in the art. If not commercially available, the preferable compound (7d) can be obtained by oxidizing a corresponding alcohol compound by an oxidation reaction known to a person skilled in the art; or the $\alpha$-ketoester compound can be obtained by oxidizing an ester compound by a known oxidation reaction.

[General Preparation Method 4]

Typically used General Preparation Method 4 for the ester compound (1a) will be described below.
[Formula 55]

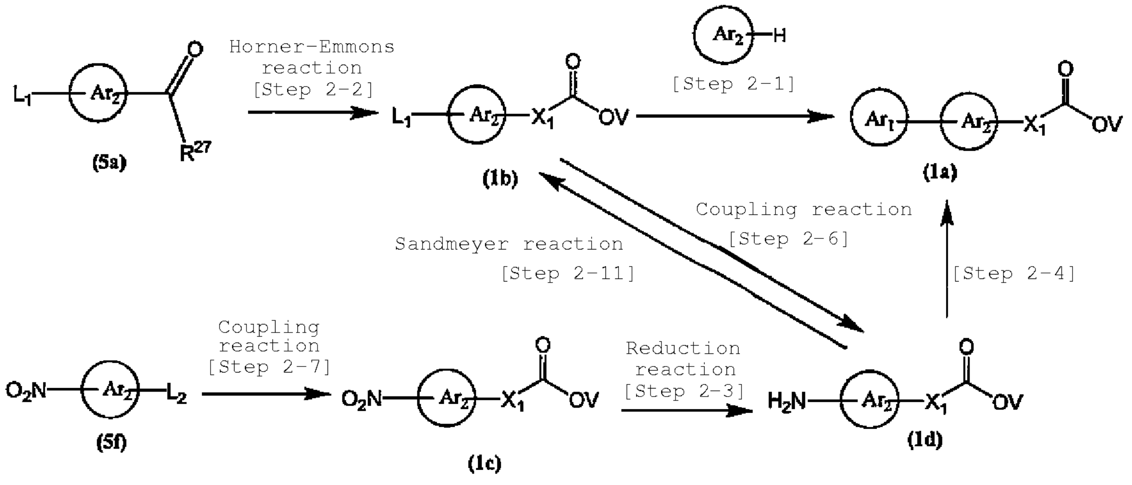


In the formula, $Ar_1$, $Ar_2$, $X_1$, $L_1$, $L_2$, $R^{27}$ and V are as defined above.

The above reaction formula shows an example of another method for preparing the ester compound (1a). Specifically, the reaction formula shows (i) a method of converting the above-described compound (5a) as a starting material into an ester compound (1b) according to the above Step 2-2; and then preparing the ester compound (1a) in the above Step 2-1, (ii) a method of converting an ester compound (1b) into an amine compound (1d) in Step 2-6; and then preparing the ester compound (1a) according to the above-described Step 2-4 or (iii) a method of preparing the ester compound (1a) from a nitro compound (5f) as a starting material in the above three Steps 2-7, 2-3 and 2-4. In addition, the reaction formula shows that (iv) an amine compound (1d) can be converted into the ester compound (1a) according to the above Step 2-1 through an ester compound (1b) by Sandmeyer reaction in Step 2-11.

[Preparation of nitro compound (5f)]

The nitro compound (5f) used in this step is commercially available or can be obtained by a technique known to a person skilled in the art. If not commercially available, the preferable compound (5f), wherein $L_2$ represents a fluorine atom, a chlorine atom, a bromine atom or an iodine atom, can be efficiently obtained from a corresponding precursor by a nitration reaction known to a person skilled in the art (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., 1978, p.1261-1300, for example).

[Conversion of ester compound (1b) into amine compound (1d)]

The ester compound (1b) can be converted into the amine compound (1d) by a method known to a person skilled in the art. Preferably, the same method as in the above Step 2-6 may be used, for example.

[Conversion of amine compound (1d) into ester compound (1b)]

Conversion of the amine compound (1d) into the ester compound (1b) varies according to the type of the material and is not particularly limited insofar as the conditions are similar to those in this reaction.
A method known to a person skilled in the art may be used for the conversion. Preferably, Sandmeyer reaction in Step 2-11 may be used, for example. The preferable ester compound (1b) can be efficiently obtained by a method known to a person skilled in the art (see Shin Jikken Kagaku Koza (New Courses in Experimental Chemistry), vol.14, Yuki Kagobutsu No Gosei To Hannou (Synthesis and Reaction of Organic Compounds) [I], edited by The Chemical Society of Japan, Maruzen Co., Ltd., 1977, p.383-388, for example).

[General Preparation Method 5]

Typically used General Preparation Method 5 for the compound of the general formula (I) of the present invention will be described below.

[Formula 56]

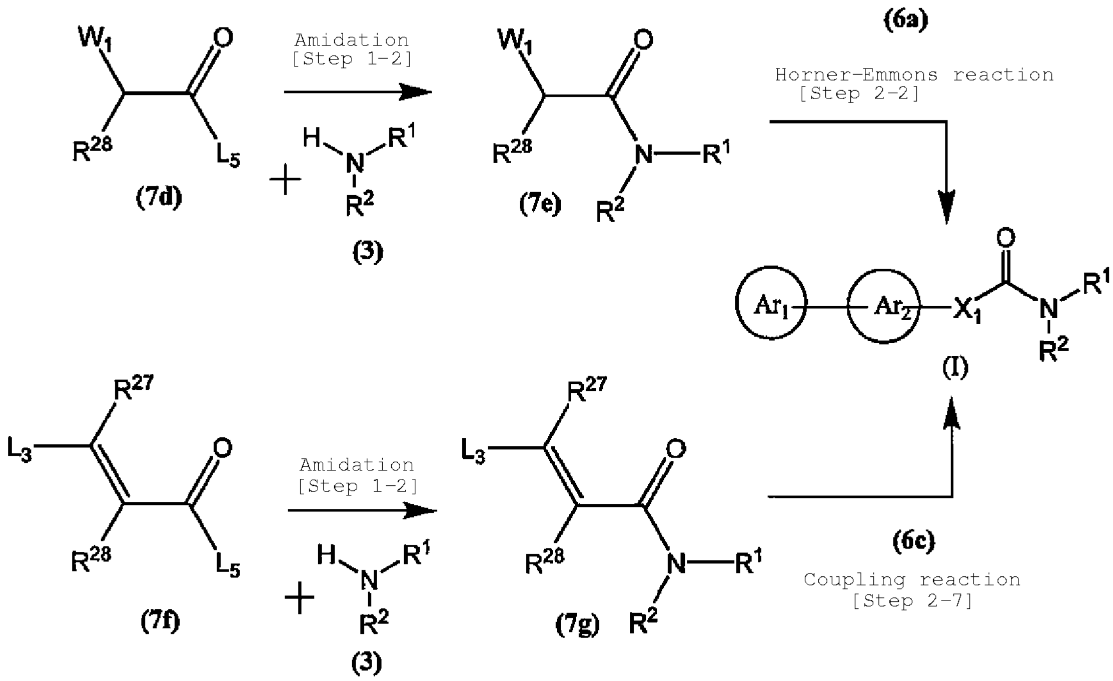


(7d) (3) (7e) (6a) (I) (7f) (3) (7g) (6c)

In the formula, $Ar_1$, $Ar_2$, $X_1$, $R^1$, $R^2$, $R^{27}$, $R^{28}$ and $L_3$ are as defined above; $W_1$ is as defined for W; and $L_5$ represents a hydroxyl group, a chlorine atom or a bromine atom.

The compound of the general formula (I) can be prepared by converting a compound (7d) into a compound (7e) according to the above Step 1-2; and then subjecting the compound (7e) to Step 2-2 together with the above-described carbonyl compound (6a), or by converting a compound (7f) into a compound (7g) according to the above Step 1-2; and then subjecting the compound (7g) to Step 2-7 together with the above-described compound (6c), for example.

[Preparation of compound (7d)]

The compound (7d) is commercially available or can be obtained by a technique known to a person skilled in the art. Preferably, the compound (7d) can be efficiently obtained from the above-described phosphonate (7a) as a starting material by the same deprotection reaction as in the above Step 1-1.

[Preparation of compound (7e)]

The compound (7e) is commercially available or can be prepared from the compound (7d) together with the above-described amine compound (3) through the same step as the above Step 1-2.

[Preparation of compound (7f)]

The compound (7f) is commercially available or can be obtained by a technique known to a person skilled in the art. Preferably, the compound (7f) can be efficiently obtained from the above-described compound (7b) as a starting material by the same deprotection reaction as in the above Step 1-1.

[Preparation of compound (7g)]

The compound (7g) is commercially available or can be prepared from the compound (7f) together with the above-described amine compound (3) through the same step as the above Step 1-2.

[General Preparation Method 6]

Typically used General Preparation Method 6 for the compound of the general formula (I) of the present invention will be described below.

[Formula 57]

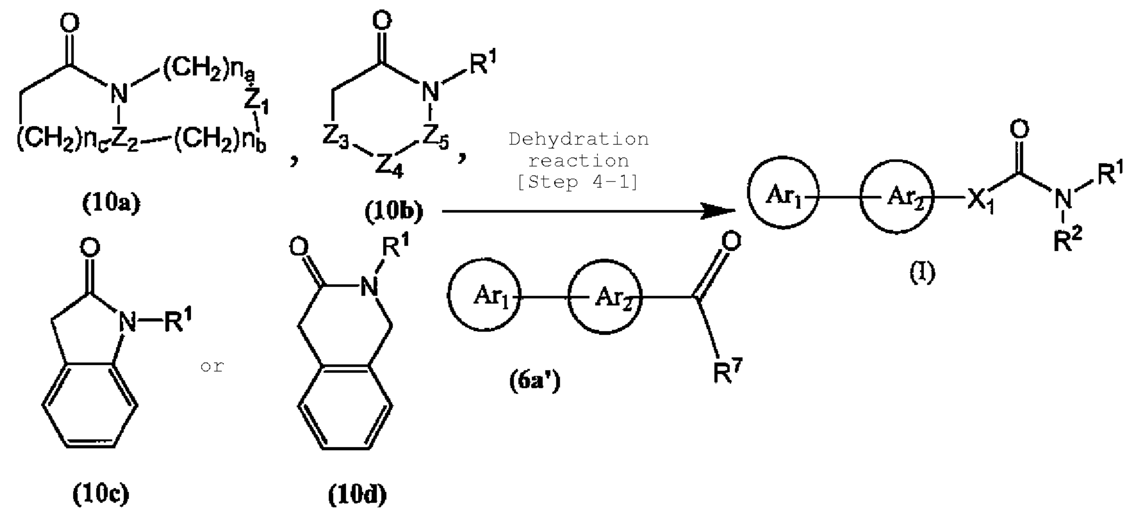


(10a), (10b), (10c) or (10d) (6a') (I)

In the formula, $Ar_1$, $Ar_2$ and $X_1$ are as defined above; and $R^1$ and $R^2$, together with $-X_1$-CO-N, form:

(3-1) a cyclic group which may be substituted with 1 to 4 substituents selected from Substituent Group A4 shown above and is represented by the formula (V):

[Formula 58]

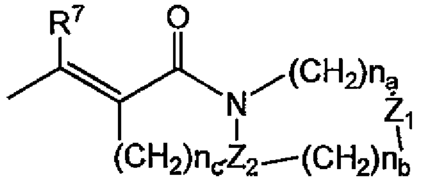
(V)

wherein $Z_1$ represents (1) NH, (2) -0-, (3) -S-, (4) -SO-, (5) $-SO_2-$, (6) $-CH_2-$, (7) -CO-, (8) -CONH-, (9) -NHCO- or (10) a single bond; $Z_2$ represents (1) a methine group or (2) a nitrogen atom; $R^7$ represents a substituent selected from Substituent Group A3 shown above; and na, $n_b$ and $n_c$ each represent an integer of 0 to 4;
(3-2) a cyclic group represented by the formula (VI):

[Formula 59]

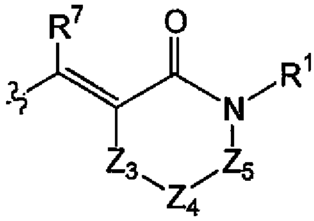

(VI)

wherein $Z_3$ represents (1) a single bond, (2) -CO-, (3) $-(CH_2)n_d-$ (wherein $n_d$ represents an integer of 1 to 3) or (4) $-CR^8R^9-$ (wherein $R^8$ and $R^9$ each represent a substituent selected from Substituent Group A4 shown above); $Z_4$ represents (1) a single bond, (2) -O-, (3) -NRCO-, (4) -CONR-, (5) -CSNR- or (6) -NRCS- (wherein R represents a substituent selected from Substituent Group A4 shown above); $Z_5$ represents (1) a single bond, (2) an imino group which may be substituted with a substituent selected from Substituent Group A4 shown above, (3) $-(CH_2)n_e-$ (wherein $n_e$ represents an integer of 1 to 3), (4) $-CR^8R^9-$ (wherein $R^8$ and $R^9$ are as defined above) or (5) -O-; and $R^1$ and $R^7$ are as defined above; or

(3-3) a cyclic group which may be substituted with 1 to 4 substituents selected from Substituent Group A4 shown above and is represented by the following formula:

[Formula 60]

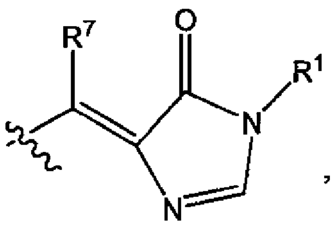

,

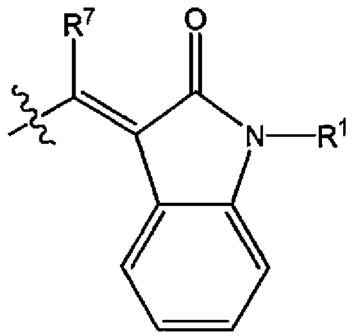

or

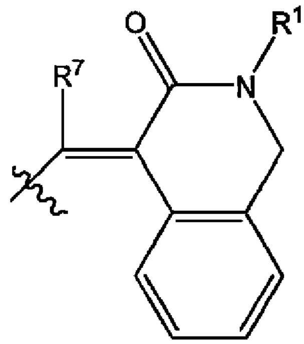

wherein $R^1$ and $R^7$ are as defined above.

The above reaction formula shows an example of a method for preparing the compound of the general formula (I) comprising dehydrating a compound (10a), a compound (10b), a compound (10c) or a compound (10d) as a starting material in Step 4-1 together with a carbonyl compound (6a'). Specifically, the dehydration reaction in Step 4-1 varies according to the starting material and is not particularly limited insofar as the conditions are similar to those in this reaction. A method known to a person skilled in the art may be used for the reaction. Preferably, the compound of the general formula (I) can be obtained by two steps of reacting the compound (10a), (10b), (10c) or (10d) treated under basic conditions with the carbonyl compound (6a') by aldol reaction to prepare an alcohol compound; and then eliminating a hydroxyl group by a known method, for example. Preferable examples of the base used in the first step of this method include sodium hydride, n-butyllithium, lithium diisopropylamide, lithium bis(trimethylsilyl)amide, sodium bis(trimethylsilyl) amide, sodium ethoxide and tert-butoxide. The equivalent of the base varies according to the starting material and is not limited, and is preferably 1.0 to 2.0 equivalents. Titanium (IV) isopropoxide or boron trifluoride may be added to make the reaction efficiently proceed, for example. The solvent used varies according to the starting material and the base, and is not particularly limited insofar as it does not inhibit the reaction and allows the starting material to be dissolved therein to a certain extent. Preferably examples of the solvent include diethyl ether and tetrahydrofuran. The reaction

temperature must be a temperature that can complete the reaction without promoting formation of an undesirable by-product, and is preferably -78°C to room temperature. The second-step dehydration reaction varies according to the starting material and is not particularly limited insofar as the conditions are similar to those in this reaction. A known method described in many documents may be used for the reaction. Preferable examples of the method include i) a method of treating an aldol adduct with an acid (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.194-196, for example) and ii) a method of converting an alcohol group of an aldol adduct into a leaving group such as a sulfonate group or a halogen atom, and then treating the adduct with a base (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.198-205, for example). The progress of the reaction can be monitored by a known chromatography technique. An undesirable by-product can be removed by a technique known to a person skilled in the art such as a conventional chromatography technique or/and crystallization.

The compound of the formula (I) can also be efficiently obtained by dehydration condensation of acidic hydrogen of the compound (10a), compound (10b), compound (10c) or compound (10d) with an oxygen atom of the carbonyl compound (6a') under basic conditions, for example (see H.O. House, "Modern synthetic reactions", W.A. Benjamin, Inc., 1972, p.629-653, for example). Preferable examples of the base used in this reaction include piperidine, pyrrolidine, sodium methoxide, sodium ethoxide, potassium tert-butoxide, sodium hydride, sodium hydroxide, potassium hydroxide, lithium hydroxide, sodium carbonate, potassium carbonate, calcium carbonate, cesium carbonate, n-butyllithium, lithium diisopropylamide, lithium bis(trimethylsilyl)amide and sodium bis(trimethylsilyl)amide. The equivalent of the base varies according to the base, starting material and solvent used and is not limited. The solvent used in this reaction varies according to the starting material and the base, and is not particularly limited insofar as it does not inhibit the reaction and allows the starting material to be dissolved therein to a certain extent. Preferable examples of the solvent include diethyl ether, tetrahydrofuran, benzene, toluene, xylene, methanol, ethanol and tert-butyl alcohol. The reaction temperature must be a temperature that can complete the reaction without promoting formation of an undesirable by-product, and is preferably -78°C to 150°C. Under preferable reaction conditions, the reaction is completed in 1 to 24 hours, and the progress of the reaction can be monitored by a known chromatography technique.

[Preparation of carbonyl compound (6a')]

The carbonyl compound (6a') can be prepared by the same method as for the above-described carbonyl compound (6a).

[Preparation of compound (10a), compound (10b), compound (11c) and compound (11d)]

The compound (10a), compound (10b), compound (11c) and compound (11d) are commercially available or can be prepared by a method known to a person skilled in the art. Preferably, the compounds can be efficiently prepared by introducing the $R^1$ group into secondary amide nitrogen under basic conditions, for example (see J.A. Campbell et al., "J. Org. Chem.", 1995, vol.60, p.4602-4616).

[General Preparation Method 7]

Typically used General Preparation Method 7 for the compound of the general formula (I) of the present invention will be described below.

[Formula 61]

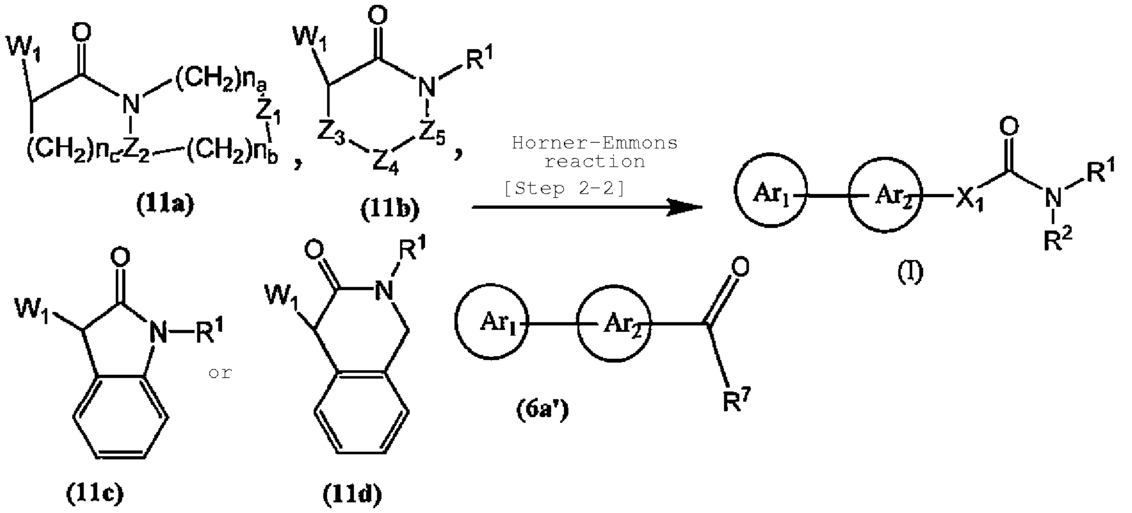


(11a), (11b), (11c), (11d), (6a'), (I)

In the formula, $Ar_1$, $Ar_2$, $X_1$, $R^1$, $R^2$, $R^7$, $Z_1$, $Z_2$, $Z_3$, $Z_4$, $Z_5$, $n_a$, $n_b$, $n_c$ and $W_1$ are as defined above.

The above reaction formula shows an example of a method for preparing the compound of the general formula (I) from a compound (11a), a compound (11b), a compound (11c) or a compound (11d) as a starting material together with the above-described carbonyl compound (6a') according to the above Step 2-2.

[Preparation of compound (11a), compound (11b), compound (11c) and compound (11d)]

The compound (11a), compound (11b), compound (11c) and compound (11d) are commercially available or can be prepared by a method known to a person skilled in the art. Preferably, the compounds can be prepared by halogenating a corresponding lactam compound as a starting material by a method known to a person skilled in the art (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.430-438, for example), and then reacting the compound with an alkyl phosphinite by Arbuzov reaction (see "Chemical Review", 1981, vol.81, p.415, for example) or with a metal phosphonite by Becker reaction (see "Journal of the American Chemical Society", 1945, vol.67, p.1180, for example). Alternatively, the compounds can be prepared from a lactam compound and a chlorophosphate in the presence of a base (see "The Journal of Organic Chemistry", 1989, vol.54, p.4750, for example).

The compound of the general formula (I) can also be prepared by reacting the above halogenated lactam with triarylphosphorus (such as triphenylphosphine) and then reacting the compound with a compound (6a') in the presence of a base.

[General Preparation Method 8]

Typically used General Preparation Method 8 for the compound of the general formula (I) of the present invention will be described below.

[Formula 62]

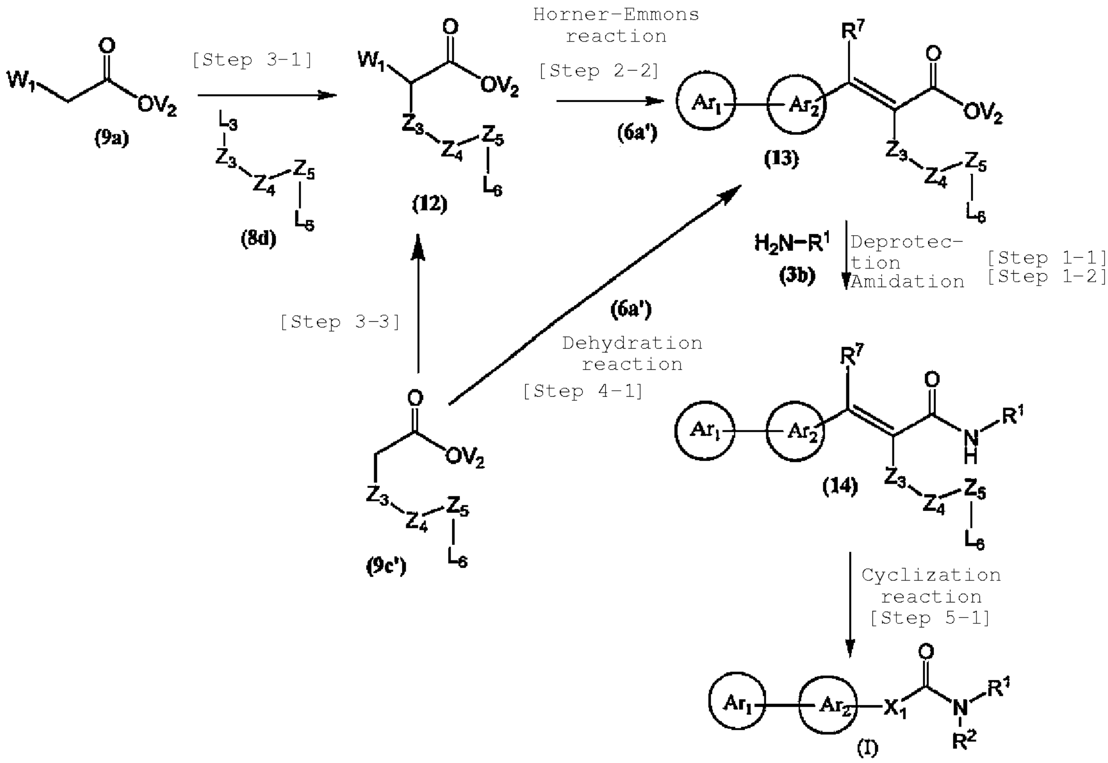


In the formula, $Ar_1$, $Ar_2$, $X_1$, $R^1$, $R^2$, $R^7$, $Z_3$, $Z_4$, $Z_5$, $L_3$, $W_1$ and $V_2$ are as defined above; and $L_6$ represents a group selected from Substituent Group A4 shown above.

The above reaction formula shows an example of a method for preparing the compound of the general formula (I) of the present invention comprising converting a phosphonate compound (12) together with the above-described carbonyl compound (6a') into a compound (13) according to Step 2-2; then converting the compound (13) into an amide compound (14) in the above two Steps 1-1 and 1-2; and cyclizing the amide compound (14) in Step 5-1.
Preferably, the substituent $L_6$ or $V_2$ is appropriately modified by a method known to a person skilled in the art in order to make the reaction efficiently proceed in each step. For example, when $L_6$ is a protected hydroxyl group, the hydroxyl group can be converted into a leaving group (such as a sulfonate group or a halogen group) by a method known to a person skilled in the art after deprotection.

[Preparation of compound (12)]

The compound (12) is commercially available or can be prepared by a method known to a person skilled in the art. Preferably, the compound can be prepared by reacting the phosphonic acid compound (9a) with a compound (8d) by the same method as in Step 3-1, for example. The compound can also be prepared from an ester compound (9c') by reaction in Step 3-3.

[Preparation of compound (8d)]

The compound (8d) is commercially available or can be prepared by a method known to a person skilled in the art.

[Preparation of compound (9c')]

The compound (9c') is commercially available or can be prepared by a method known to a person skilled in the art. If not commercially available, the compound can be obtained from a corresponding carboxylic acid compound by protection reaction according to a known technique, for example (see T.W. Green, "Protective Groups in Organic Synthesis", John Wiley & Sons, Inc., 1981, for example).

[Preparation of compound (13)]

The compound (13) can be prepared by reacting the phosphonate (12) with the carbonyl compound (6a') by reaction in Step 2-2, for example. The compound can also be prepared by reacting the ester compound (9c') with the carbonyl compound (6a') by the same reaction as in Step 4-1.

[Preparation of compound (14)]

The compound (13) can be prepared by deprotecting the compound (13) according to Step 1-1 and then reacting the carboxylic acid with an amine (3b) by amidation reaction in Step 1-2, for example.

[Preparation of amine compound (3b)]

The amine compound (3b) is commercially available or can be prepared by a method known to a person skilled in the art. Preferably, the compound can be prepared by the same method as in the above "Preparation of amine compound (3)", for example.

[Conversion of compound (14) into compound (I)]

Cyclization reaction from the compound (14) into the compound (I) in Step 5-1 varies according to the starting material and can be carried out by a method known to a person skilled in the art. For example, when $L_6$ is a sulfonate group or a halogen group, the cyclization can be carried out under basic conditions. The base is not particularly limited and is preferably sodium hydride, sodium methoxide, potassium tert-butoxide, lithium hydroxide or the like. 1.0 to 1.5 equivalents of the base is preferably used with respect to the compound (14). The reaction solvent is not particularly limited insofar as it does not inhibit the reaction and allows the starting material to be dissolved therein to a certain extent. Examples of the solvent include alcohol solvents such as methanol and ethanol; ether solvents such as tetrahydrofuran and dioxane; polar solvents such as dimethyl formamide and dimethyl sulfoxide; and toluene. A mixed solvent may also be used. Sodium iodide may be added to the reaction solution as necessary. The reaction temperature must be a temperature that can complete the reaction without promoting formation of an undesirable by-product, and is preferably -20°C to room temperature. The progress of the reaction can be monitored by a known chromatography technique. An undesirable by-product can be removed by a technique known to a person skilled in the art such as a conventional chromatography technique or/and crystallization.

[General Preparation Method 9]

Typically used General Preparation Method 9 for the compound of the general formula (I) of the present invention will be described below.
[Formula 63]

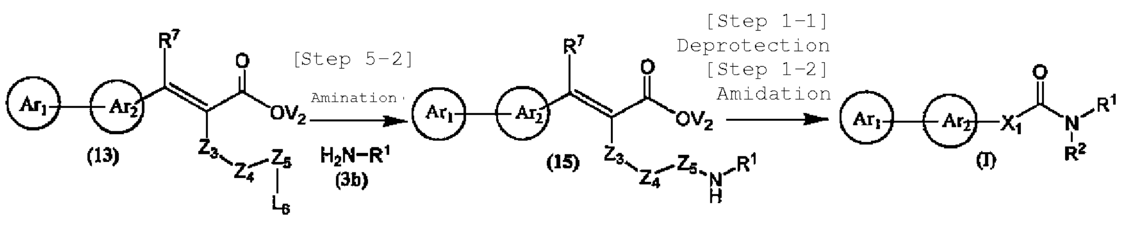


In the formula, $Ar_1$, $Ar_2$, $X_1$, $R^1$, $R^2$, $R^7$, $Z_3$, $Z_4$, $Z_5$ and $V_2$ are as defined above; and $L_6$ represents a group selected from Substituent Group A4 shown above.
The above reaction formula shows an example of a method for preparing the compound of the general formula (I) of the present invention comprising converting the ester compound (13) and the amine compound (3b) into a compound (15) in Step 5-2; deprotecting the ester in the above Step 1-1; and then forming an intramolecular amide bond in the above Step 1-2.

[Preparation of compound (15)]

Amination reaction from the ester compound (13) into the compound (15) in Step 5-1 varies according to the starting material and can be carried out by a method known to a person skilled in the art. i) When $L_6$ is a sulfonate group or a halogen group, the amination can be carried out under basic conditions, for example. The base is not particularly limited and is preferably potassium carbonate, sodium bicarbonate, cesium carbonate or the like. 1.0 to 3.0 equivalents of the base is preferably used with respect to the compound (13). The reaction solvent is not particularly limited insofar as it does not inhibit the reaction and allows the starting material to be dissolved therein to a certain extent. Examples of the solvent include ether solvents such as tetrahydrofuran and dioxane, acetonitrile, dimethylformamide and dimethyl sulfoxide. Sodium iodide may be added as necessary. The reaction temperature must be a temperature that can complete the reaction without promoting formation of an undesirable by-product, and is preferably room temperature to solvent reflux temperature. ii) When $L_6$ is a carbonyl group, a known reductive amination reaction may be used, for example (see Jikken Kagaku Koza (Courses in Experimental Chemistry), 4th edition, vol.20, Yuki Gosei (Organic Synthesis) [II], edited by The Chemical Society of Japan, Maruzen Co., Ltd., 1992, p.300-302, for example). The progress of the reaction can be monitored by a known chromatography technique. An undesirable by-product can be removed by a technique known to a person skilled in the art such as a conventional chromatography technique or/and crystallization.

Methods for preparing the compound of the general formula (VIII) of the present invention will be described below.

The compound represented by the general formula (VIII):

[Formula 64]

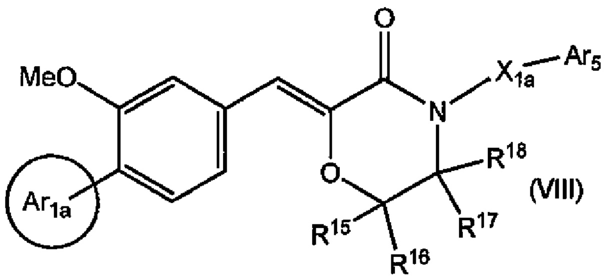

(VIII)

wherein $Ar_{1a}$, $R^{15}$, $R^{16}$, $R^{17}$, $R^{18}$, $X_{1a}$ and $Ar_5$ are as defined above, is synthesized according to a method such as the following General Preparation Method 10 to General Preparation Method 11, for example. It is obvious that, in order to prepare the compound of the present invention conveniently, the method comprises a protection reaction step and a deprotection reaction step appropriately, using a protecting group known to a person skilled in the art which is suitably selected for each step (see T. Greene et al., "Protective Groups in Organic Synthesis", John Wiley & Sons, Inc., New York, 1981, for example).

[General Preparation Method 10]

Typically used General Preparation Method 10 for the compound of the general formula (VIII) of the present invention will be described below.

[Formula 65]

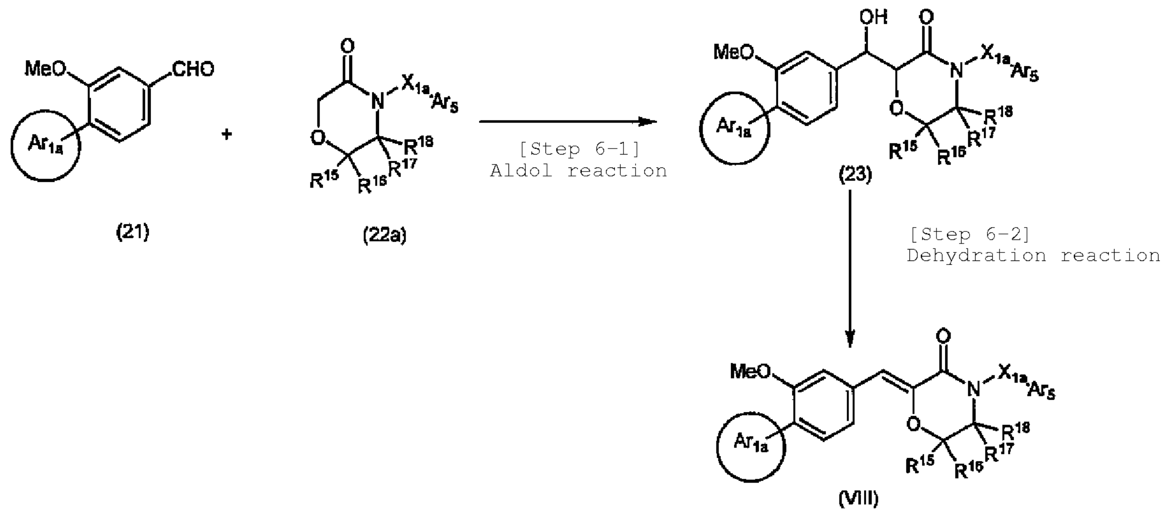


(21) (22a) (23) (VIII)

In the formula, $Ar_{1a}$, $Ar_5$, $R^{15}$, $R^{16}$, $R^{17}$, $R^{18}$ and $X_{1a}$ (which may have a protecting group when the $X_{1a}$ contains a hydroxyl group) are as defined above.

The above General Preparation Method 10 is an example of a method for preparing the compound of the general formula (VIII) comprising converting an aldehyde compound (21) and 1.0 to 3.0 equivalents of an oxomorpholine compound (22a) with respect to the aldehyde compound (1) into an aldol adduct (23) by aldol reaction in Step 6-1; and then dehydrating the adduct.

[Preparation of aldol adduct (23)]

The aldol adduct (23) can be prepared from the aldehyde compound (21) and the lactam compound (22a) according to Step 6-1, for example. Specifically, the aldol reaction in Step 1-1 varies according to the starting material and is not particularly limited insofar as the conditions are similar to those in this reaction. A method known to a person skilled in the art may be used for the reaction (see Jikken Kagaku Koza (Courses in Experimental Chemistry), 4th edition, vol.20, Yuki Gosei (Organic Synthesis) [II], edited by The Chemical Society of Japan, Maruzen Co., Ltd., July 1992, p. 94-100, for example). Examples of the method include (i) a method of converting the oxomorpholine compound (22a) into an alkali metal enolate by 1.0 to 5.0 equivalents of a base; and then reacting the enolate with the aldehyde compound (21) (see Jikken Kagaku Koza (Courses in Experimental Chemistry), 4th edition, vol.20, Yuki Gosei (Organic Synthesis) [II], edited by The Chemical Society of Japan, Maruzen Co., Ltd., 1992, p.97-98, for example) and (ii) a method of converting the oxomorpholine compound (22a) into an alkali metal enolate by 1.0 to 5.0 equivalents of a base; reacting the enolate with a silicon halide reagent (such as preferably trimethylchlorosilane or tert-butyldimethylchlorosilane) to once prepare silyl enol ether; and then reacting the ether with the aldehyde compound (21) in the presence of a Lewis acid (such as titanium tetrachloride, tin tetrachloride or boron trifluoride) (see Jikken Kagaku Koza (Courses in Experimental Chemistry), 4th edition, vol.20, Yuki Gosei (Organic Synthesis) [II], edited by The Chemical Society of Japan, Maruzen Co., Ltd., 1992, p.96-97, for example). Examples of the base for converting the oxomorpholine compound (22a) into an alkali metal enolate include lithium diisopropylamide, lithium bis(trimethylsilyl)amide, sec-butyllithium, sodium amide, sodium hydride, sodium methoxide and potassium tert-butoxide. Lithium diisopropylamide and sec-butyllithium are preferable. The solvent used is not particularly limited insofar as it does not inhibit the reaction and allows the starting material to be dissolved therein to a certain extent. An ether solvent such as tetrahydrofuran, 1,2-dimethoxyethane, 1,4-dioxane or diethyl ether, a non-polar solvent such as toluene or benzene or a mixed solvent thereof may be used, for example. The reaction temperature must be a temperature that can complete the reaction without promoting formation of an undesirable by-product, and is preferably -78°C to room temperature. Under preferable reaction conditions, the reaction is completed in 0.5 to 24 hours, and the progress of the reaction can be monitored by a known chromatography technique. An undesirable by-product can be removed by a technique known to a person skilled in the art such as a conventional chromatography technique, extraction or/and crystallization.

[Conversion of aldol adduct (23) into compound of general formula (VIII)]

The compound of the general formula (VIII) can be prepared by conversion of the aldol adduct (23) by dehydration reaction in Step 6-2. Specifically, the dehydration conditions in Step 6-2 vary according to the starting material and are not particularly limited insofar as the conditions are similar to those in this reaction. A known method described in many documents may be used for the reaction (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.194-226, for example). Preferable examples of the method include i) a method of treating the aldol adduct (23) with an acid (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.194-196, for example) and ii) a method of converting an alcohol group of the aldol adduct (23) into a leaving group such as a sulfonate group or a halogen atom, and then treating the adduct with a base (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.198-205, for example).

In the method i), the acid used varies according to the starting material and is not particularly limited. For example, 0.1 to 10 equivalents of an acid such as hydrochloric acid, sulfuric acid, phosphoric acid, potassium hydrogen sulfide, oxalic acid, p-toluenesulfonic acid, a boron trifluoride-ether complex, thionyl chloride or aluminum oxide is used with respect to the aldol adduct (23). A combination of an acid with an organic base such as pyridine may improve the reaction rate and the reaction yield in some cases. The solvent is not particularly limited insofar as it does not inhibit the reaction and allows the starting material to be dissolved therein to a certain extent. Examples of the solvent used include non-polar solvents such as toluene and benzene; polar solvents such as acetone, dimethyl sulfoxide and hexamethylphosphoramide; halogenated solvents such as methylene chloride; water; and mixed solvents thereof. The reaction may be performed without a solvent. The reaction temperature must be a temperature that can complete the reaction without promoting formation of an undesirable by-product, and is preferably room temperature to 200°C, for example. Under preferable reaction conditions, the reaction is completed in 0.5 to 24 hours, and the progress of the reaction can be monitored by a known chromatography technique. An undesirable by-product can be removed by a technique known to a person skilled in the art such as a conventional chromatography technique, extraction or/and crystallization.

Examples of the leaving group in the method ii) include a methanesulfonate group, a p-toluenesulfonate group, a chlorine group and a bromine group. The method of converting an alcohol group into a leaving group in the first step varies according to the starting material. A method known to a person skilled in the art may be used. For example, it is possible to use 1.0 to 2.0 equivalents of a sulfonating agent such as methanesulfonyl chloride or p-toluenesulfonyl chloride with respect to the aldol adduct (23) or 1.0 to 10 equivalents of a halogenating agent such as thionyl chloride with respect to the aldol adduct (23). A base may be added as necessary. The solvent is not particularly limited insofar as it does not inhibit the reaction. Examples of the solvent used include halogenated solvents such as methylene chloride; non-polar solvents such as toluene; ether solvents such as tetrahydrofuran and ethylene glycol dimethyl ether; and mixed solvents thereof. The reaction temperature must be a temperature that can complete the reaction without promoting formation of an undesirable by-product. The temperature is -78°C to solvent reflux temperature, for example, and is preferably -78°C to room temperature. An undesirable by-product can be removed by a technique known to a person skilled in the art such as a conventional chromatography technique, extraction or/and crystallization; however, the crude product may also be used for elimination reaction in the next step as is.

The elimination reaction in the second step is performed using 0.1 to 10 equivalents of a base with respect to the aldol adduct (23), for example. Examples of the base that can be used include organic bases such as diazabicycloundecene, pyridine and triethylamine; quaternary ammonium salts such as tetrabutylammonium hydroxide; alkali metal salts of alcohols such as sodium methoxide and potassium tert-butoxide; alkali metal hydroxides such as sodium hydroxide; alkali metal carbonates such as lithium carbonate and potassium carbonate; and organometallic reagents such as lithium diisopropylamide. The solvent is not particularly limited insofar as it does not inhibit the reaction and allows the starting material to be dissolved therein to a certain extent. Examples of the solvent used include halogenated solvents such as methylene chloride; non-polar solvents such as toluene; polar solvents such as acetonitrile, dimethylformamide and dimethyl sulfoxide; ether solvents such as tetrahydrofuran, 1,4-dioxane and ethylene glycol dimethyl ether; and mixed solvents thereof. An organic base such as pyridine may also be used as a solvent. The reaction temperature must be a temperature that can complete the reaction without promoting formation of an undesirable by-product, and is preferably -78°C to solvent reflux temperature, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

[Preparation of aldehyde compound (21)]

[Formula 66]

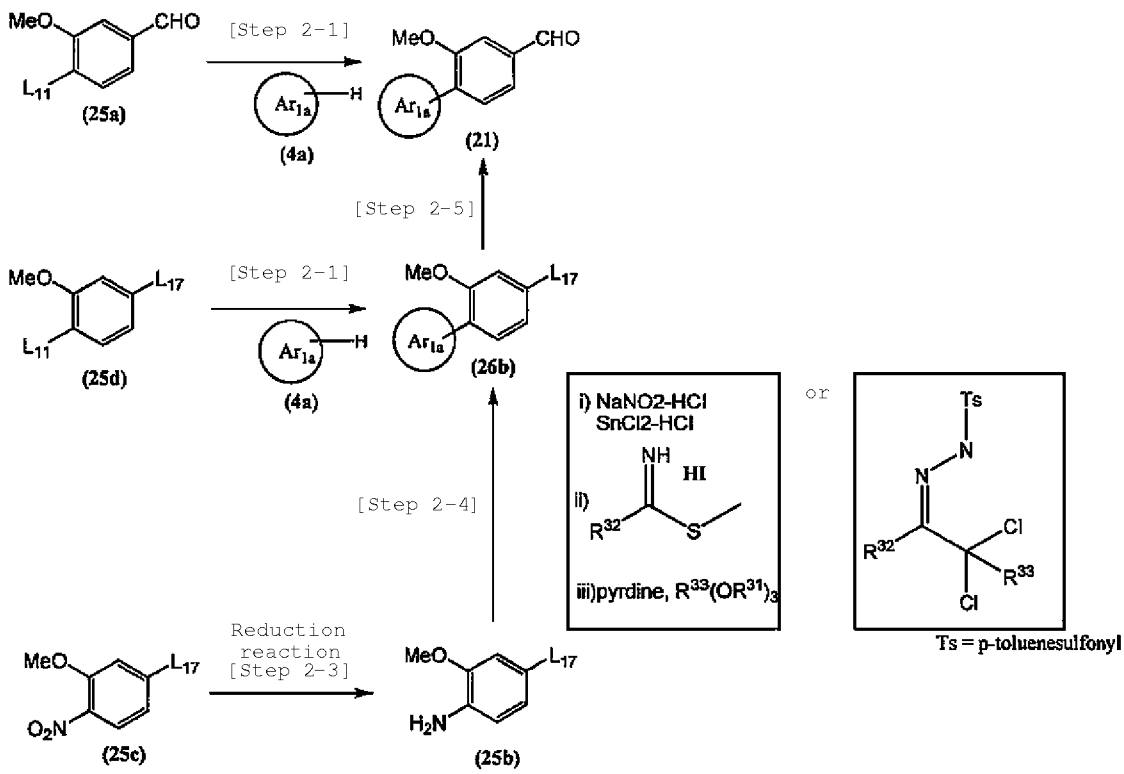


In the formula, $Ar_{1a}$ is as defined above; $L_{11}$ represents a fluorine atom, a chlorine atom, a bromine atom, an iodine atom, a sulfonate group such as a triflate group, a trialkyltin group, a boronic acid group or a boronate group; $L_{17}$ represents a C1-C4 alkoxycarbonyl group such as a methyl ester group, or a cyano group; $R^{31}$ represents a C1-C6 alkyl group; and $R^{32}$ and $R^{33}$ each represent hydrogen or a C1-6 alkyl group.

The aldehyde compound (21) can be obtained by a technique known to a person skilled in the art which varies according to the starting material. For example, the compound can be prepared as shown by the above reaction, but the preparation is not limited thereto. Specifically, the compound can be obtained by reacting an aldehyde compound (25a) with the compound (4a) in Step 2-1. The compound can also be obtained by subjecting a nitro compound (25c) as a starting material to reduction reaction in Step 2-3 and cyclization reaction in Step 2-4 and then converting the compound into an aldehyde in Step 2-5. Alternatively, the compound can be obtained by reacting a compound (25d) with the compound (4a) in Step 2-1 and then carrying out reaction in Step 2-5.

The aldehyde compound (25a), the nitro compound (25c) and the compound (25d) are commercially available or can be easily prepared by a method known to a person skilled in the art.

[Preparation of oxomorpholine compound (22a)]

[Formula 67]

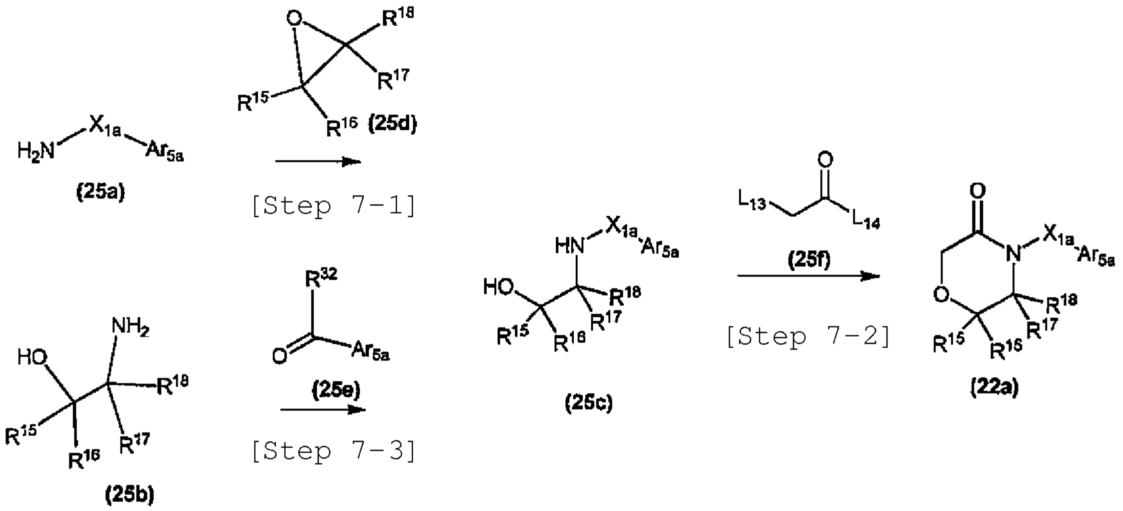


In the formula, $R^{15}$, $R^{16}$, $R^{17}$, $R^{18}$, $X_{1a}$ and $Ar_{5a}$ are as defined above; $R^{32}$ represents a hydrogen atom or a C1-5 alkylene group [wherein the C1-5 alkylene group may be substituted with 1 to 3 hydroxyl groups (which may have a protecting group when the $R^{32}$ contains a hydroxyl group) or C1-6 alkyl groups]; and $L_{13}$ and $L_{14}$ each represent a chlorine atom or a bromine atom.

The above reaction formula shows an example of a method for preparing the oxomorpholine compound (22a). Specifically, the reaction formula shows i) a method of converting the amine compound (25a) commercially available or prepared by a method known to a person skilled in the art as a starting material into the compound (25c) according to Step 7-1; and then forming an oxomorpholine ring in Step 7-2 or ii) a method of converting a compound (25b) and a carbonyl compound (25e) commercially available or prepared by a method known to a person skilled in the art into the compound (25c) according to Step 7-3; and then forming an oxomorpholine ring in Step 7-2.

[Preparation of compound (25a)]

The amine compound (25a) is commercially available or can be prepared by a method known to a person skilled in the art. If not commercially available, the compound can be prepared by a method described in a document and known to a person skilled in the art (see Shin Jikken Kagaku Koza (New Courses in Experimental Chemistry), vol. 14, Yuki Kagobutsu No Gosei To Hannou (Synthesis and Reaction of Organic Compounds) [III], edited by The Chemical Society of Japan, Maruzen Co., Ltd., 1978, p.1332-1399, for example). Examples of the method include (i) a method of converting a corresponding carbonyl derivative into the compound (25a) by reductive amination reaction, (ii) a method of reducing a corresponding carbonyl derivative to an alcohol derivative; then preparing an amine equivalent (preferably an azide group or an imide group, for example) from the alcohol derivative by a substitution reaction known to a person skilled in the art; and converting the amine equivalent into the compound (25a) by a conversion reaction known to a person skilled in the art, (iii) a method of converting a corresponding carbonyl derivative into an oxime derivative; and then reducing the oxime derivative to the compound (25a) by a reduction reaction known to a person skilled in the art and (iv) a method of converting a corresponding olefin compound into an alcohol derivative by oxidation reaction, preparing an amine equivalent (preferably an azide group or an imide group, for example) from the alcohol derivative by a substitution reaction known to a person skilled in the art; and then converting the amine equivalent into the compound (25a) by a conversion reaction known to a person skilled in the art. The compound (25a) can be efficiently obtained according to the synthesis method in the above "Preparation of amine compound (3)". The compound (25a) may be commercially available as an optically active compound or prepared by a method known to a person skilled in the art as an optically active compound (see "Angew. Chem. Int. Ed.", 2003, vol.42, p.5472-5474; "Tetrahedron", 1999, vol.55, p.7555-7562; "Chem. Rev", 1994, vol.94, p.2483-2547; and "Tetrahedron Letters", 1996, vol.37, p.3219-3222, for example). The compound of the present invention can be prepared as an optically active compound from this material as a starting material.

[Preparation of oxirane compound (25d)]

The oxirane compound (25d) is commercially available or can be prepared by a method known to a person skilled in the art. If not commercially available, the compound can be prepared by a method described in a document and known to a person skilled in the art (see Shin Jikken Kagaku Koza (New Courses in Experimental Chemistry), vol. 14, Yuki Kagobutsu No Gosei To Hannou (Synthesis and Reaction of Organic Compounds) [I], edited by The Chemical Society of Japan, Maruzen Co., Ltd., 1977, p.567-611, for example). The compound (25d) may be commercially available as an optically active compound or prepared by a method known to a person skilled in the art as an optically active compound (see K.B. Sharpless et al., "Comprehensive Organic Synthesis", B.M. Trost, Pergamon, 1991, vol.7, ch.3-2, for example). The compound of the present invention can be prepared as an optically active compound from this material as a starting material.

[Conversion of compound (25a) into compound (25c)]

Step 7-1 varies according to the starting material and is not particularly limited insofar as the conditions are similar to those in this reaction. A method known to a person skilled in the art may be used for the reaction. Preferable examples of the method include ring-opening reaction of an epoxide by an amine using the amine compound (25a) and 1.0 to 10 equivalents of the oxirane compound (25d) with respect to the compound (25a). A Lewis acid such as boron trifluoride, titanium tetraisopropoxide or lithium perchlorate may be added to the reaction solution as necessary (see "Synthesis", 2004, vol.10, p.1563-1565, for example). The solvent used is not particularly limited insofar as it does not inhibit the reaction and allows the starting material to be dissolved therein to a certain extent. Examples of the solvent that can be used include ether solvents such as diethyl ether and tetrahydrofuran; halogenated solvents such as methylene chloride, 1,2-dichloroethane and chloroform; non-polar solvents such as toluene and xylene; and mixed solvents thereof. A preferable result may be obtained without a solvent. The reaction temperature must be a temperature that can complete the reaction without promoting formation of an undesirable by-product, and is preferably room temperature to 300°C, for example. Under preferable reaction conditions, the reaction is completed in 0.5 to 24 hours, and the progress of the reaction can be monitored by a known chromatography technique. An undesirable by-product can be removed by a technique known to a person skilled in the art such as a conventional chromatography technique, extraction or/and crystallization.

[Preparation of compound (25f)]

The compound is commercially available or can be prepared by a method known to a person skilled in the art. The compound is preferably chloroacetyl chloride or bromoacetyl bromide, for example.

[Conversion of compound (25c) into oxomorpholine compound (22a)]

Step 8-2 varies according to the starting material and is not particularly limited insofar as the conditions are similar to those in this reaction. The reaction may be performed by a method known to a person skilled in the art. The reaction conveniently proceeds when vigorously stirring the compound (25c) and 1.0 to 10 equivalents of the compound (25f) with respect to the compound (25c) in a two-phase reaction solvent composed of an organic solvent and a basic solution, for example. The organic solvent is not particularly limited insofar as it does not inhibit the reaction and allows the starting material to be dissolved therein to a certain extent. Examples of the solvent that can be used include ether solvents such as diethyl ether; halogenated solvents such as methylene chloride, 1,2-dichloroethane and chloroform; and non-polar solvents such as toluene and xylene. The solvent is preferably a halogenated solvent. Examples of the basic solution that can be used include solutions of alkali metal salts such as sodium hydroxide, potassium hydroxide, sodium carbonate, potassium carbonate, cesium carbonate and sodium bicarbonate. The reaction temperature must be a temperature that can complete the reaction without promoting formation of an undesirable by-product. The temperature is preferably -78°C to room temperature, for example, and more preferably ice-cold temperature to room temperature. Under preferable reaction conditions, the reaction is completed in 0.5 to 24 hours, and the progress of the reaction can be monitored by a known chromatography technique. An undesirable by-product can be removed by a technique known to a person skilled in the art such as a conventional chromatography technique, extraction or/and crystallization.
The reaction may also conveniently proceed when mixing the compound (25c) with 1.0 to 10 equivalents of the compound (25f) with respect to the compound (25c) in an organic solvent in the presence of a base, for example. The base used varies according to the starting material and is not particularly limited. Examples of the base that can be used include alkali metal salts such as sodium hydroxide, potassium hydroxide, sodium carbonate, potassium carbonate, cesium carbonate and sodium bicarbonate; and organic bases such as diazabicycloundecene, pyridine, 4-N,N-dimethylaminopyridine and triethylamine. 2.0 to 10 equivalents of the base is preferably used with respect to the compound (25c). The solvent used is not particularly limited insofar as it does not inhibit the reaction and allows the starting material to be dissolved therein to a certain extent. Examples of the solvent that can be used include ether solvents such as diethyl ether and tetrahydrofuran; halogenated solvents such as methylene chloride, 1,2-dichloroethane and chloroform; and non-polar solvents such as toluene and xylene. The reaction temperature must be a temperature that can complete the reaction without promoting formation of an undesirable by-product, and is preferably -78°C to room temperature, for example. Under preferable reaction conditions, the reaction is completed in 0.5 to 24 hours, and the progress of the reaction can be monitored by a known chromatography technique. An undesirable by-product can be removed by a technique known to a person skilled in the art such as a conventional chromatography technique, extraction or/and crystallization.

[Preparation of compound (25b)]

The compound (25b) is commercially available or can be prepared by a method known to a person skilled in the art. If not commercially available, the compound can be prepared by a method described in a document and known to a person skilled in the art (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., 1978, p.1332-1399, for example). The compound (25b) may be commercially available as an optically active compound or prepared by a method known to a person skilled in the art as an optically active compound (see "Tetrahedron Letters", 1996, vol.37, p.3219-3222, for example). The compound of the present invention can be prepared as an optically active compound from this material as a starting material.

[Preparation of carbonyl compound (25e)]

The carbonyl compound (25e) is commercially available or can be prepared by a method known to a person skilled in the art. If not commercially available, the compound can be prepared by a method described in a document and known to a person skilled in the art (see Shin Jikken Kagaku Koza (New Courses in Experimental Chemistry), vol. 14, Yuki Kagobutsu No Gosei To Hannou (Synthesis and Reaction of Organic Compounds) [II], edited by The Chemical Society of Japan, Maruzen Co., Ltd., 1977, p.633-875, for example).

[Conversion of compound (25b) into compound (25c)]

Step 7-3 varies according to the starting material and is not particularly limited insofar as the conditions are similar to those in this reaction. A method known to a person skilled in the art may be used for the reaction. Examples of the method include reductive amination reaction of the compound (25b) with the carbonyl compound (25e) (see Shin Jikken Kagaku Koza (New Courses in Experimental Chemistry), vol.14, Yuki Kagobutsu No Gosei To Hannou (Synthesis and Reaction of Organic Compounds) [III], edited by The Chemical Society of Japan, Maruzen Co., Ltd., 1978, p. 1380-1384, for example). For example, it is possible to employ (i) a method of heating under reflux the carbonyl compound (25e) and 0.5 to 5.0 equivalents of the compound (25b) in the presence of an acid catalyst such as a typical inorganic acid such as hydrochloric acid or sulfuric acid, an organic acid such as methanesulfonic acid, p-toluenesulfonic acid or camphorsulfonic acid or an organic acid salt such as pyridinium p-toluenesulfonate (preferably 0.01 to 0.5 equivalent, for example) to cause dehydration reaction, and reducing the resulting imine derivative to the desired amine derivative by 1.0 to 10 equivalents of a metal hydride such as lithium aluminum hydride or sodium borohydride with respect to the imine derivative; (ii) a method of treating the carbonyl compound (25e) and 0.5 to 5.0 equivalents of the compound (25b) in an inert solvent such as tetrahydrofuran in the presence of a Lewis acid catalyst such as titanium tetraisopropoxide (preferably 0.01 to 0.5 equivalent, for example), and then reducing the compound to the desired amine derivative by 1.0 to 10 equivalents of a metal hydride such as sodium borohydride; or (iii) a method of reducing the carbonyl compound (25e) and 0.5 to 5.0 equivalents of the compound (25b) to the desired amine compound by 1.0 to 10 equivalents of a metal hydride such as sodium triacetoxyborohydride or sodium cyanoborohydride in an inert solvent such as dichloromethane, 1,2-dichloroethane, tetrahydrofuran, methanol or ethanol containing 1.0 to 10 equivalents of an acidic substance such as acetic acid or hydrochloric acid. The reaction temperature varies according to the starting material and is not particularly limited. However, the reaction temperature must be a temperature that can complete the reaction without promoting formation of an undesirable by-product, and is preferably room temperature to 100°C, for example. Under preferable reaction conditions, the reaction is completed in 0.5 to 24 hours, and the progress of the reaction can be monitored by a known chromatography technique. An undesirable by-product can be removed by a technique known to a person skilled in the art such as a conventional chromatography technique, extraction or/and crystallization.

[General Preparation Method 11]

Typically used General Preparation Method 11 for the compound of the general formula (VIII) of the present invention will be described below.

[Formula 68]

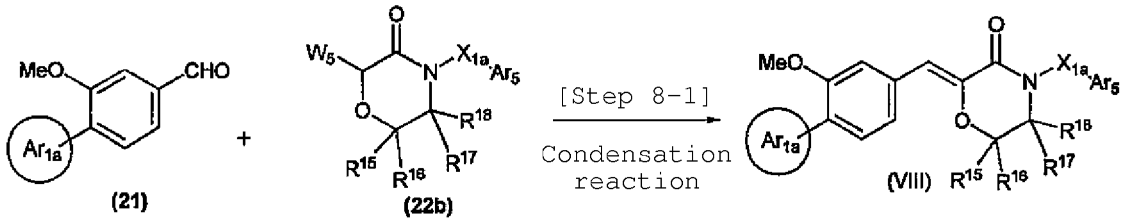


(21) (22b) (VIII)

In the formula, $Ar_{1a}$, $Ar_5$, $R^{15}$, $R^{16}$, $R^{17}$, $R^{18}$ and $X_{1a}$ are as defined above; and $W_5$ represents a phosphite group such as a diethylphosphonyl group, a phosphonium salt such as triphenylphosphonium bromide, a silyl group such as a trimethylsilyl group, or a carboxyl group.

The above General Preparation Method 11 is an example of a method for preparing the compound of the general formula (VIII) comprising condensing the aldehyde compound (21) and an oxomorpholine compound (22b) in Step 8-1.

[Conversion into compound of general formula (VIII)]

The condensation reaction in Step 8-1 varies according to the starting material and is not particularly limited insofar as the conditions are similar to those in this reaction. A known method described in many documents may be used for the reaction. Wittig reaction, Horner-Emmons reaction, Peterson reaction or Knoevenagel reaction is preferable, for example (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.57-85; or H.O. House, "Modern synthetic reactions", W.A. Benjamin, Inc., 1972, p.629-653, for example).

Wittig reaction is preferably performed using the compound (22b), wherein $W_5$ is a phosphonium salt, and 1.0 to 5.0 equivalents of a base with respect to the aldehyde compound (21), for example. This reaction may be a method of first treating the compound (22b) and a base to form a phosphorus ylide and then adding the aldehyde compound (21) to the ylide; or a method of adding a base in the presence of the compound (22b) and the aldehyde compound (21). This reaction is preferably performed in the presence of a solvent from the viewpoint of handleability and stirring efficiency. The solvent used varies according to the starting material and the base used, and is not particularly limited insofar as it does not inhibit the reaction and allows the starting material to be dissolved therein to a certain extent. Preferable examples of the solvent used include polar solvents such as nitromethane, acetonitrile, 1-methyl-2-pyrrolidone, N,N-dimethylformamide and dimethyl sulfoxide; ether solvents such as tetrahydrofuran, 1,4-dioxane and 1,2-dimethoxyethane; non-polar solvents such as benzene, toluene and xylene; alcohol solvents such as ethanol and methanol; halogenated solvents such as chloroform and methylene chloride; and water. A mixed solvent thereof is used in some cases. The base used varies according to the starting material and the solvent. Preferable examples of the base include alkali metal hydroxides such as sodium hydroxide and lithium hydroxide; alkali metal carbonates such as sodium carbonate; alkali metal salts of alcohols such as sodium methoxide and potassium tert-butoxide; organic bases such as triethylamine, pyridine and diazabicyclononene; organic metals such as butyl lithium and lithium diisobutylamide; and alkali metal hydrides such as sodium hydride. The reaction temperature must be a temperature that can complete the reaction without promoting formation of an undesirable by-product, and is preferably -78 to 150°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

Horner-Emmons reaction is preferably performed using the compound (22b), wherein $W_5$ is a phosphite group, and 1.0 to 5.0 equivalents of a base with respect to the aldehyde compound (21), for example. This reaction may be a method of first treating the compound (22b) and a base to form a phosphonate carbanion and then adding the aldehyde compound (21) to the carbanion; or a method of adding a base in the presence of the compound (22b) and the aldehyde

compound (21). This reaction is preferably performed in the presence of a solvent from the viewpoint of handleability and stirring efficiency. The solvent used varies according to the starting material and the base used, and is not particularly limited insofar as it does not inhibit the reaction and allows the starting material to be dissolved therein to a certain extent. Preferable examples of the solvent include polar solvents such as 1-methyl-2-pyrrolidone, N,N-dimethylformamide and dimethyl sulfoxide; ether solvents such as tetrahydrofuran, 1,4-dioxane and 1,2-dimethoxyethane; non-polar solvents such as benzene, toluene and xylene; alcohol solvents such as ethanol and methanol; and water. A mixed solvent thereof is used in some cases. The base used varies according to the starting material and the solvent. Preferable examples of the base include alkali metal hydroxides such as sodium hydroxide and lithium hydroxide; alkali metal carbonates such as sodium carbonate; alkali metal salts of alcohols such as sodium methoxide and potassium tert-butoxide; organic bases such as triethylamine, pyridine and diazabicyclononene; organic metals such as butyl lithium and lithium diisobutylamide; alkali metal hydrides such as sodium hydride; and alkali metal ammonia salts such as sodium amide. The reaction temperature must be a temperature that can complete the reaction without promoting formation of an undesirable by-product, and is preferably -78 to 150°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

Peterson reaction is preferably performed using the compound (22b), wherein $W_5$ is a silyl group, and 1.0 to 5.0 equivalents of a base with respect to the aldehyde compound (21), for example. This reaction may be a method of first treating the compound (22b) and a base to form an $\alpha$-silyl carbanion and then adding the aldehyde compound (21) to the carbanion; or a method of adding a base in the presence of the compound (22b) and the aldehyde compound (21). This reaction is preferably performed in the presence of a solvent from the viewpoint of handleability and stirring efficiency. The solvent used varies according to the starting material and the base used, and is not particularly limited insofar as it does not inhibit the reaction and allows the starting material to be dissolved therein to a certain extent. Preferable examples of the solvent include polar solvents such as 1-methyl-2-pyrrolidone, N,N-dimethylformamide and dimethyl sulfoxide; ether solvents such as tetrahydrofuran, 1,4-dioxane and 1,2-dimethoxyethane; non-polar solvents such as benzene, toluene and xylene; alcohol solvents such as ethanol and methanol; and water. A mixed solvent thereof is used in some cases. The base used varies according to the starting material and the solvent. Preferable examples of the base include alkali metal hydroxides such as sodium hydroxide and lithium hydroxide; alkali metal carbonates such as sodium carbonate; alkali metal salts of alcohols such as sodium methoxide and potassium tert-butoxide; organic bases such as triethylamine, pyridine and diazabicyclononene; organic metals such as butyl lithium and lithium diisobutylamide; alkali metal hydrides such as sodium hydride; and alkali metal ammonia salts such as sodium amide. The reaction temperature must be a temperature that can complete the reaction without promoting formation of an undesirable by-product, and is preferably -78 to 150°C. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

Knoevenagel reaction is performed using the compound (6), wherein $W_5$ is a carboxyl group, and 0.1 to 1.0 equivalents of a base with respect to the aldehyde compound (21), for example. Preferable examples of the base used in this reaction include piperidine, pyrrolidine, dimethylamine and N-methylaniline. The solvent used in this reaction varies according to the starting material and the base, and is not particularly limited insofar as it does not inhibit the reaction and allows the starting material to be dissolved therein to a certain extent. Preferable examples of the solvent include tetrahydrofuran, benzene, toluene, xylene, pyridine and dimethylformamide. The reaction temperature must be a temperature that can complete the reaction without promoting formation of an undesirable by-product, and is preferably room temperature to 150°C. Under preferable reaction conditions, the reaction is completed in 1 to 24 hours, and the progress of the reaction can be monitored by a known chromatography technique.

[Preparation of oxomorpholine compound (22b)-1]

[Formula 69]

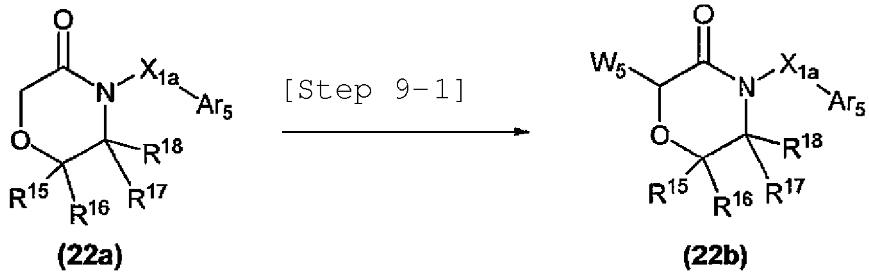


In the formula, $Ar_{1a}$, $Ar_5$, $R^{15}$, $R^{16}$, $R^{17}$, $R^{18}$ and $X_{1a}$ are as defined above; and $W_5$ represents a phosphite group such as a diethylphosphonyl group, a phosphonium salt such as triphenylphosphonium bromide, a silyl group such as a trimethylsilyl group, or a carboxyl group.

The above Step 9-1 shows an example of preparation of the oxomorpholine compound (22b). Step 9-1 varies according to the starting material and is not particularly limited insofar as the conditions are similar to those in this reaction. A method known to a person skilled in the art may be used for the reaction. Preferably, for example, (i) the Wittig reagent (22b), wherein $W_5$ is a phosphonium salt, can be prepared by halogenating the oxomorpholine compound (22a) by a method known to a person skilled in the art (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.430-438; and "J. Org. Chem.", 1993, vol.58, p.3384-3386, for example); and then reacting the compound with a triarylphosphine (see "Organic Reaction", 1965, vol.14, p.270, for example). (ii) The Horner-Emmons reagent (22b), wherein $W_5$ is a phosphite, can be prepared by halogenating the oxomorpholine compound (22a) by a method known to a person skilled in the art (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.430-438, for example); and then reacting the compound with a trialkyl phosphite by Arbuzov reaction (see "Chemical Review", 1981, vol.81, p. 415, for example) or with a metal phosphonite by Becker reaction (see "Journal of the American Chemical Society", 1945, vol.67, p.1180, for example). The Horner-Emmons reagent can also be prepared from the oxomorpholine compound (22a) and a chlorophosphate in the presence of a base (see "Journal of Organic Chemistry", 1989, vol.54, p. 4750, for example). (iii) The Peterson reagent (22b), wherein $W_5$ is a silyl group, can be prepared from the oxomorpholine compound (2a) and a trialkylsilyl chloride in the presence of a base, for example (see "Journal of Organometallic Chemistry", 1983, vol.248, p.51, for example). (iv) The Knoevenagel reagent (22b), wherein $W_5$ is a carboxyl group, can be prepared from the oxomorpholine compound (2a) and carbon dioxide in the presence of a base, for example.

[Preparation of oxomorpholine compound (22b)-2]

[Formula 70]

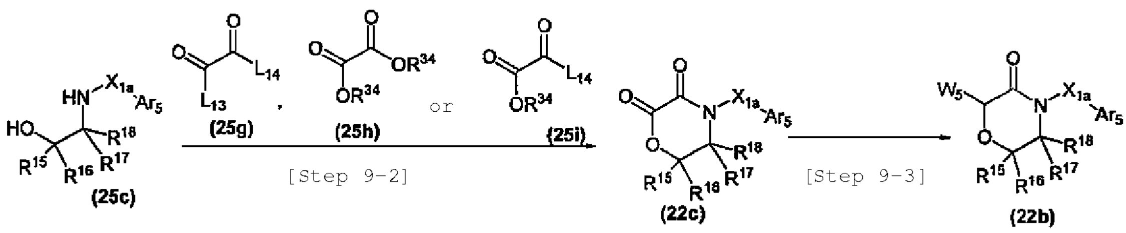


In the formula, $Ar_{1a}$, $Ar_5$, $R^{15}$, $R^{16}$, $R^{17}$, $R^{18}$, $L_{13}$, $L_{14}$, $W_5$ and $X_{1a}$ are as defined above; and $R^{34}$ represents a C1-4 alkyl group.

The above reaction formula shows an example of a method for preparing the oxomorpholine compound (22b).

Specifically, the oxomorpholine compound (22b) can be prepared by a method known to a person skilled in the art. The method is preferably a method of converting the compound (25c) as a starting material into a compound (2c) in Step 9-2 and then subjecting the compound (2c) to Step 9-3.

[Preparation of oxomorpholine compound (2c)]

Preferably, in Step 9-2, the reaction conveniently proceeds (i) when vigorously stirring the compound (25c) and 1.0 to 10 equivalents of a compound (25g) with respect to the compound (25c) in a two-phase reaction solvent composed of an organic solvent and a basic solution, for example. The solvent used is not particularly limited insofar as it does not inhibit the reaction and allows the starting material to be dissolved therein to a certain extent. Preferable examples of the organic solvent include ether solvents such as diethyl ether; halogenated solvents such as methylene chloride, 1,2-dichloroethane and chloroform; non-polar solvents such as toluene and xylene; and mixed solvents thereof. 2.0 or more equivalents of the basic solution is preferably used. Examples of the basic solution that can be used include solutions of alkali metal salts such as sodium hydroxide, potassium hydroxide, sodium carbonate, potassium carbonate, cesium carbonate and sodium bicarbonate. The reaction temperature must be a temperature that can complete the reaction without promoting formation of an undesirable by-product, and is preferably -78°C to room temperature, for example. (ii) It is also possible to employ a method of reacting the compound (25c) and 1.0 to 5.0 equivalents of a compound (25g) with respect to the compound (25c) in the presence of a base such as an organic amine such as triethylamine, isopropylethylamine, pyridine or 4-N,N-dimethylaminopyridine (preferably 2.0 to 5.0 equivalents, for example). The solvent used is not particularly limited insofar as it does not inhibit the reaction and allows the starting material to be dissolved therein to a certain extent. Examples of the solvent that can be used include ether solvents such as diethyl ether; halogenated solvents such as methylene chloride, 1,2-dichloroethane and chloroform; and non-polar solvents such as toluene and xylene. The reaction temperature must be a temperature that can complete the reaction without promoting formation of an undesirable by-product, and is preferably -78°C to 100°C, for example. (iii) The reaction may also conveniently proceed when heating the compound (25c) and 1.0 to 20 equivalents of a compound (25h) with respect to the compound (25c). The solvent used is not particularly limited insofar as it does not inhibit the reaction and allows the starting material to be dissolved therein to a certain extent. Examples of the solvent that can be used include ether solvents such as diethyl ether; halogenated solvents such as methylene chloride, 1,2-dichloroethane and 1,2-dichlorobenzene; non-polar solvents such as toluene and xylene; polar solvents such as dimethylformamide and N-methylpyrrolidone; and alcohol solvents such as methanol, ethanol, 2-propanol and tert-butanol. The compound (25h) may also be used as a solvent. The reaction temperature must be a temperature that can complete the reaction without promoting formation of an undesirable by-product, and is preferably 50°C to 200°C, for example.
In Step 9-2, the reaction may also conveniently proceed using the compound (25c) and 1.0 to 5.0 equivalents of a compound (25i) with respect to the compound (25c) under the above-described reaction conditions or a combination thereof.

[Preparation of compounds (25g), (25h) and (25i)]

The compounds (25g), (25h) and (25i) are commercially available or can be prepared by a method known to a person skilled in the art. If not commercially available, the compounds may be prepared by esterification or halogenation of a corresponding oxalic acid derivative by a method known to a person skilled in the art.

[Conversion of oxomorpholine compound (22c) into oxomorpholine compound (22b)]

Step 9-3 varies according to the starting material and is not particularly limited insofar as the conditions are similar to those in this reaction. A method known to a person skilled in the art may be used for the reaction. Preferably, for example, it is possible to (i) reduce an ester carbonyl moiety to an alcohol compound (see Jikken Kagaku Koza (Courses in Experimental Chemistry), 4th edition, vol.26, Yuki Gosei (Organic Synthesis) [VIII], edited by The Chemical Society of Japan, Maruzen Co., Ltd., 1992, p.159-266, for example); convert the alcohol compound into a halogen compound (see Shin Jikken Kagaku Koza (New Courses in Experimental Chemistry), vol.14, Yuki Kagobutsu No Gosei To Hannou (Synthesis and Reaction of Organic Compounds) [I], edited by The Chemical Society of Japan, Maruzen Co., Ltd., 1977, p.331-450, for example); and then prepare the Wittig reagent (22b) from the halogen compound (see "Organic Reaction", 1965, vol.14, p.270, for example), or (ii) prepare the Horner-Emmons reagent (22b) from the resulting halogen compound by Arbuzov reaction (see "Chemical Review", 1981, vol.81, p.415, for example), or (iii) convert the alcohol compound obtained by the reduction into the Wittig reagent (22b) by reaction with triallylphosphorus hydrobromide (see "Synth. Commun.", 1996, vol.26, p.3091-3095; and "Tetrahedron Lett.", 2001, vol.42, p.1309-1331, for example).

[Preparation of oxomorpholine compound (22b)-3]

[Formula 71]

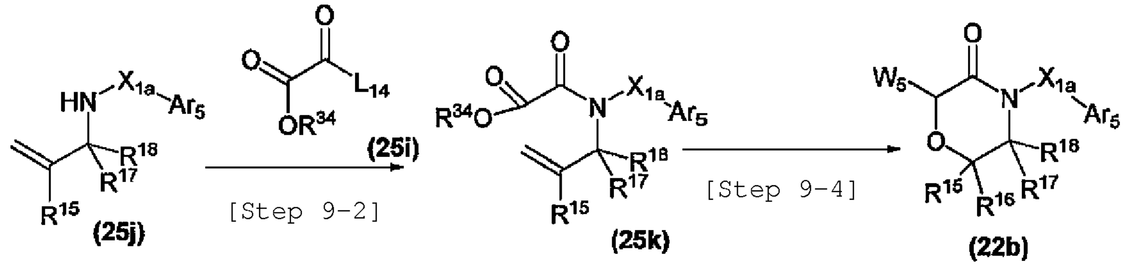


In the formula, $Ar_{1a}$, $Ar_5$, $R^{15}$, $R^{16}$, $R^{17}$, $R^{18}$, $R^{34}$, $W_5$, $L_{14}$ and $X_{1a}$ are as defined above.

The above reaction formula shows an example of a method for preparing the oxomorpholine compound (22b). Specifically, the oxomorpholine compound (22b) can be prepared by a method known to a person skilled in the art. The method is preferably a method of converting the compound (25j) as a starting material into a compound (25k) in Step 9-2 and then subjecting the compound (25k) to Step 9-4.

[Conversion of compound (25k) into oxomorpholine compound (22b)]

Step 9-4 varies according to the starting material and is not particularly limited insofar as the conditions are similar to those in this reaction. A known method described in many documents may be used for the reaction. A method known to a person skilled in the art may be used for the reaction. For example, the method is preferably a method of converting an olefin moiety of the compound (25k) into a hemiacetal derivative by oxidative cleavage reaction and intramolecular cyclization reaction; converting the hemiacetal derivative into a halogen compound (see Shin Jikken Kagaku Koza (New Courses in Experimental Chemistry), vol.14, Yuki Kagobutsu No Gosei To Hannou (Synthesis and Reaction of Organic Compounds) [I], edited by The Chemical Society of Japan, Maruzen Co., Ltd., November 1977, p. 331-450, for example); and converting the halogen compound into the Wittig reagent (22b) (see "Organic Reaction", 1965, vol.14, p.270, for example) or into the Horner-Emmons reagent (22b) by Arbuzov reaction (see "Chemical Review", 1981, vol.81, p.415, for example). Alternatively, the hemiacetal derivative can be converted into the Wittig reagent (22b) by reaction with triallylphosphorus hydrobromide (see "Synth. Commun.", 1996, vol.26, p.3091-3095; and "Tetrahedron Lett.", 2001, vol.42, p.1309-1331, for example). The oxidative cleavage reaction of an olefin moiety varies according to the starting material and is not particularly limited insofar as the conditions are similar to those in this reaction. A known method described in many documents may be used for the reaction. Ozone oxidation is preferable, for example (see Shin Jikken Kagaku Koza (New Courses in Experimental Chemistry), vol.15, Sanka To Kangen (Oxidation and Reduction) [I-2], edited by The Chemical Society of Japan, Maruzen Co., Ltd., September 1976, p.563-603, for example). The oxidative cleavage reaction and the intramolecular cyclization reaction may continuously proceed under suitable reaction conditions, and this is convenient for preparing a compound (22b).

[Preparation of compound (25k)]

The compound (25k) can be prepared from the compound (25j) and preferably 1.0 to 5.0 equivalents of the compound (25i) with respect to the compound (25j), for example, according to the above-described Step 9-2.

[Preparation of compound (25j)]

The compound (25j) is commercially available or can be prepared by a method known to a person skilled in the art. If not commercially available, the compound (25j) is preferably prepared by intramolecular hydroamination reaction of an amine compound or a sulfonylamide compound having an allenyl group using a metal catalyst, when $R^{18}$ and $X_{1a}$ are bonded to each other to form a nitrogen-containing heterocycle, for example (see "Journal of The American Chemical Society", 2003, vol.125, p.11956; and "Tetrahedron Lett.", 1998, vol.39, p.5421-5424, for example). This reaction varies according to the starting material and is not particularly limited insofar as the conditions are similar to

those in this reaction. The metal catalyst is preferably 0.001 to 0.1 equivalent of a palladium complex such as palladium (II) acetate, dichlorobis(triphenylphosphine)palladium (II), tetrakis(triphenylphosphine)palladium (0) or an allylpalladium chloride dimer, for example. The reaction may also conveniently proceed by addition of preferably 0.001 to 0.1 equivalent, for example, of a phosphorus ligand such as preferably 2,2'-bis(diphenylphosphino)-1,1'-binaphthyl or 1,1'-bis(diphenylphosphino)ferrocene. The reaction may also conveniently proceed by addition of preferably 0.001 to 10 equivalents of acetic acid or hydrochloric acid, for example. The solvent and reaction temperature used vary according to the starting material and are not particularly limited. The solvent is preferably a solvent that does not inhibit the reaction and allows the starting material to be dissolved therein to a certain extent, or a mixed solvent thereof. Preferable examples of the organic solvent that can be used include ether solvents such as diethyl ether and tetrahydrofuran; halogenated solvents such as methylene chloride and 1,2-dichloroethane; non-polar solvents such as toluene and xylene; polar solvents such as dimethylformamide and N-methylpyrrolidone; and alcohol solvents such as methanol, ethanol, 2-propanol and tert-butanol. The reaction temperature must be a temperature that can complete the reaction without promoting formation of an undesirable by-product, and is preferably 50°C to 200°C, for example. Under preferable reaction conditions, the reaction is completed in 0.5 to 24 hours, and the progress of the reaction can be monitored by a known chromatography technique. An undesirable by-product can be removed by a technique known to a person skilled in the art such as a conventional chromatography technique, extraction or/and crystallization.

Methods for preparing the compound of the general formula (IX) of the present invention will be described below. The compound represented by the general formula (IX):

[Formula 72]

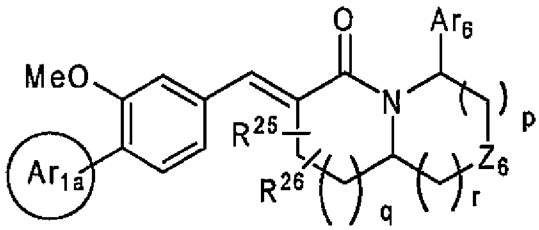

(IX)

wherein $Ar_{1a}$, $Ar_6$, $Z_6$, $R^{25}$, $R^{26}$, p, q and r are as defined above, is synthesized according to a method such as the following General Preparation Method 12 to General Preparation Method 15, for example. It is obvious that, in order to prepare the compound of the present invention conveniently, the method comprises a protection reaction step and a deprotection reaction step appropriately, using a protecting group known to a person skilled in the art which is suitably selected for each step (see T. Greene et al., "Protective Groups in Organic Synthesis", John Wiley & Sons, Inc., New York, 1981, for example).

[General Preparation Method 12]

Typically used General Preparation Method 12 for the compound of the general formula (IX) of the present invention will be described below.

[Formula 73]

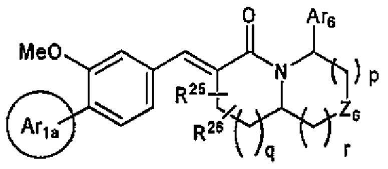

(IX)

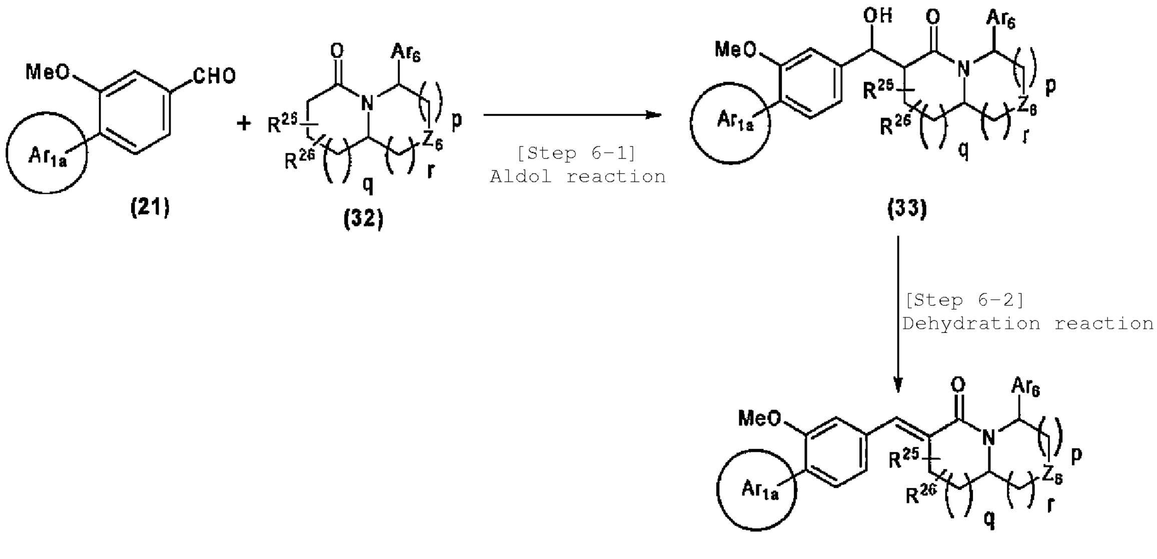


(21) (32) (33) (IX)

In the formula, $Ar_{1a}$, $Ar_6$, $Z_6$, $R^{25}$, $R^{26}$, p, q and r are as defined above.

The above General Production Method 12 is an example of a method for preparing the compound of the general formula (IX) comprising converting the aldehyde compound (21) obtained by General Preparation Method 10 and a lactam compound (32) into an aldol adduct (33) by aldol reaction in the above Step 6-1; and then dehydrating the adduct in the above Step 6-2.

[Preparation of lactam compound (32)-1]

[Formula 74]

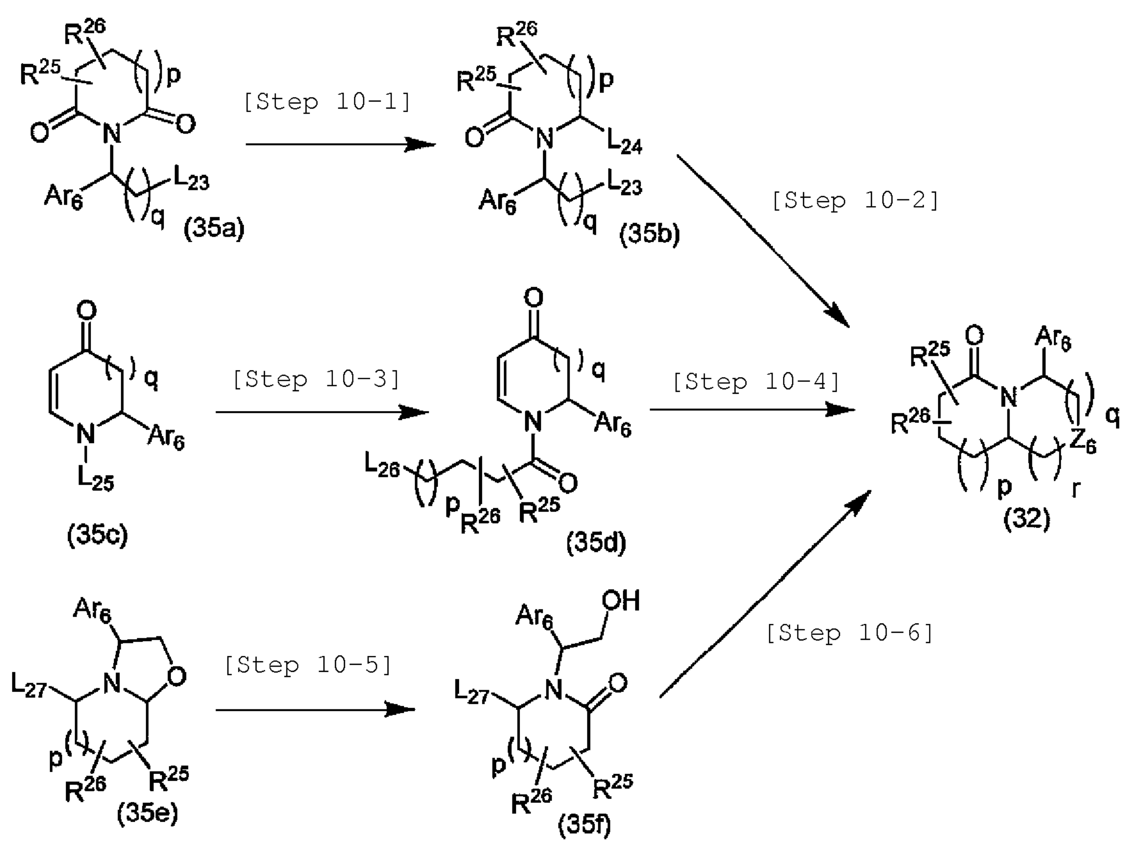


In the formula, $Ar_6$, $Z_6$, $R^{25}$, $R^{26}$, p, q and r are as defined above; $L_{23}$ represents an alkyl ester group such as a methyl ester group or an ethyl ester group, or an alkyl ketone group, an aryl ketone group or an aralkyl ketone group such as an acetyl group, a benzoyl group or an aryl methyl ketone group; $L_{24}$ represents an alkoxy group such as a methoxy group or an ethoxy group; $L_{25}$ represents a carbamate protecting group such as a methyl carbamate group, a benzyl carbamate group or a tert-butyl carbamate group, or an amide protecting group such as an acetyl group; $L_{26}$ represents a halogen atom such as a bromine atom or an iodine atom; and $L_{27}$ represents a nitrile group, an alkyl ester group such as a methyl ester group, or an alkyl ketone group such as an acetyl group.

The above reaction formula shows an example of a method for preparing the lactam compound (32). Specifically, the reaction formula shows (i) a method for preparing the lactam compound (32) comprising converting an imide compound (35a) commercially available or prepared by a method known to a person skilled in the art (see "Tetrahedron: Asymmetry", 1998, vol.9, p.4361, for example) as a starting material into an alkoxylactam compound (35b) according to Step 10-1; and then continuously performing carbon increasing reaction and cyclization reaction in Step 10-2, or (ii) a method for preparing the lactam compound (32) comprising converting a 4-pyridone compound (35c) commercially available or prepared by a method known to a person skilled in the art (see "Tetrahedron Letters", 1986, vol.27, p.4549, for example) as a starting material into an acylated compound (35d) according to Step 10-3; and then cyclizing the acylated compound (35d) in Step 10-4, or (iii) a method for preparing the lactam compound (32) comprising converting an oxazolidine compound (35e) commercially available or prepared by a method known to a person skilled in the art (see "European Journal of Organic Chemistry", 2004, vol.23, p.4823, for example) as a starting material into an amidoalcohol compound (35f) according to Step 10-5; and then cyclizing the amidoalcohol compound (35f) in Step 10-6.

[Conversion of imide compound (35a) into alkoxylactam compound (35b)]

Partial reduction of an imide group in Step 10-1 varies according to the starting material and can be performed by a method known to a person skilled in the art insofar as the conditions are similar to those in this reaction. The desired alkoxylactam compound (35b) can be obtained by reacting the imide compound (35a) with 1.0 to 5.0 equivalents of sodium borohydride, for example, in an alcohol solvent such as preferably methanol (see Jikken Kagaku Koza (Courses

in Experimental Chemistry), 4th edition, vol.26, Yuki Gosei (Organic Synthesis) [VIII], edited by The Chemical Society of Japan, Maruzen Co., Ltd., 1992, p.207-237, for example) or reacting the imide compound (35a) with 1.0 to 5.0 equivalents of borane, for example, in an ether solvent such as tetrahydrofuran (see Jikken Kagaku Koza (Courses in Experimental Chemistry), 4th edition, vol.26, Yuki Gosei (Organic Synthesis) [VIII], edited by The Chemical Society of Japan, Maruzen Co., Ltd., 1992, p.237-248, for example); and then treating the resulting compound in an alcohol solvent such as preferably methanol in the presence of 0.1 to 10.0 equivalents of an inorganic acid such as sulfuric acid, for example.
Alternatively, the desired alkoxylactam compound (35b) can be preferably obtained in one step using 1.0 to 5.0 equivalents of sodium borohydride, for example, in an alcohol solvent such as methanol in the presence of 0.1 to 5.0 equivalents of an inorganic acid such as sulfuric acid, for example (see "Tetrahedron: Asymmetry", 1998, vol.9, p.4361, for example). The reaction temperature must be a temperature that can complete the reaction without promoting formation of an undesirable by-product, and is preferably -78°C to 100°C, for example. Under preferable reaction conditions, the reaction is preferably completed in 1 to 24 hours, for example, and the progress of the reaction can be monitored by a known chromatography technique. An undesirable by-product can be removed by a technique known to a person skilled in the art such as a conventional chromatography technique, extraction or/and crystallization.

[Conversion of alkoxylactam compound (35b) into lactam compound (32)]

In Step 10-2, the desired lactam compound (32) can be obtained by converting $L_{23}$ of the alkoxylactam compound (35b) into an olefin by reaction with a Wittig reagent (see Jikken Kagaku Koza (Courses in Experimental Chemistry), 4th edition, vol.24, Yuki Gosei (Organic Synthesis) [VII], edited by The Chemical Society of Japan, Maruzen Co., Ltd., 1992, p.254-262, for example), a Grignard reagent (see Jikken Kagaku Koza (Courses in Experimental Chemistry), 4th edition, vol.24, Yuki Gosei (Organic Synthesis) [VI], edited by The Chemical Society of Japan, Maruzen Co., Ltd., 1991, p.59-72, for example) or an alkyllithium reagent (see Jikken Kagaku Koza (Courses in Experimental Chemistry), 4th edition, vol.24, Yuki Gosei (Organic Synthesis) [VI], edited by The Chemical Society of Japan, Maruzen Co., Ltd., 1991, p.9-51, for example); and then treating the olefin with an acid such as hydrochloric acid. Preferably, the desired lactam compound (32) can be obtained in a high yield by reacting the alkoxylactam compound (35b) with 1.0 to 10.0 equivalents of a Grignard reagent such as trimethylsilylmethylmagnesium chloride, for example, in an ether solvent such as preferably tetrahydrofuran in the presence of 1.0 to 10.0 equivalents of cerium chloride, for example; and then treating the resulting compound with an inorganic acid such as hydrochloric acid, for example (see "Tetrahedron: Asymmetry", 1998, vol.9, p.4361, for example). The reaction temperature must be a temperature that can complete the reaction without promoting formation of an undesirable by-product, and is preferably -78°C to 100°C, for example. Under preferable reaction conditions, the reaction is preferably completed in 1 to 24 hours, for example, and the progress of the reaction can be monitored by a known chromatography technique. An undesirable by-product can be removed by a technique known to a person skilled in the art such as a conventional chromatography technique, extraction or/and crystallization.

[Conversion of 4-pyridone compound (35c) into acylated compound (35d)]

Step 10-3 consists of deprotection reaction of an amine moiety and subsequent amidation reaction. A deprotection reaction described in many known documents may be used for deprotecting the compound (35c) (see T.W. Green, "Protective Groups in Organic Synthesis", John Wiley & Sons, Inc., 1981, for example). The amine compound can be obtained from a corresponding carbamate compound (preferably a tert-butyl carbamate compound, a benzyl carbamate compound or a 9-fluorenylmethyl carbamate compound, for example) or from a corresponding amide compound (preferably a formamide compound, an acetamide compound or a trifluoroacetamide compound, for example). The conditions for the deprotection reaction vary according to the starting material and are not particularly limited insofar as the conditions are similar to those in this reaction. A known method may be used for the reaction. Under preferable reaction conditions, the reaction is preferably completed in 1 to 24 hours, for example, and the progress of the reaction can be monitored by a known chromatography technique. An undesirable by-product can be removed by a technique known to a person skilled in the art such as a conventional chromatography technique or/and crystallization.
The acylated compound (35d) can be efficiently synthesized by amidation reaction according to the above Step 1-2 which varies according to the starting material.

[Conversion of acylated compound (35d) into lactam compound (32)]

Step 10-4 is cyclization reaction through radical formation. The desired lactam compound (32) can be preferably obtained in a high yield by treating with 1.0 to 2.0 equivalents of an alkyltin reagent such as tributyltin, for example, in a non-polar solvent such as toluene in the presence of 0.1 to 1.0 equivalent of a radical initiator such as 2,2-azobis (isobutyronitrile), for example. The reaction temperature must be a temperature that can complete the reaction without

promoting formation of an undesirable by-product, and is preferably 50°C to 150°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization. After ring formation, $Z_6$ may be converted in various manners using a ketone group as a scaffold by a method known to a person skilled in the art such as reduction reaction (see Jikken Kagaku Koza (Courses in Experimental Chemistry), 4th edition, vol.26, Yuki Gosei (Organic Synthesis) [VIII], edited by The Chemical Society of Japan, Maruzen Co., Ltd., 1992, p.159-266, for example), addition reaction (see Jikken Kagaku Koza (Courses in Experimental Chemistry), 4th edition, vol.25, Yuki Gosei (Organic Synthesis) [VII], edited by The Chemical Society of Japan, Maruzen Co., Ltd., 1991, p.9-72, for example) or addition dehydration reaction (see Jikken Kagaku Koza (Courses in Experimental Chemistry), 4th edition, vol.19, Yuki Gosei (Organic Synthesis) [I], edited by The Chemical Society of Japan, Maruzen Co., Ltd., 1992, p.57-85, for example).

[Conversion of oxazolidine compound (35e) into amidoalcohol compound (35f)]

Oxidative cleavage reaction of an oxazolidine ring in Step 10-5 varies according to the starting material and can be performed by a method known to a person skilled in the art insofar as the conditions are similar to those in this reaction. The desired amidoalcohol compound (35f) can be preferably obtained in a high yield by treating with 2.0 to 10.0 equivalents of potassium permanganate, for example, in an aqueous solvent such as a mixture of water and acetone (see "European Journal of Organic Chemistry", 2004, vol.23, p.4823, for example) or by treating with 1.0 to 10.0 equivalents of bromine, for example, in a halogenated solvent such as methylene chloride (see "Synlett", 1994, vol.2, p.143, for example). The solvent used in this step varies according to the starting material and the oxidizing agent used, and is not particularly limited insofar as the solvent does not inhibit the reaction and allows the starting material to be dissolved therein to a certain extent. The reaction temperature must be a temperature that can complete the reaction without promoting formation of an undesirable by-product, and is preferably ice-cold temperature to 100°C, for example. Under preferable reaction conditions, the reaction is preferably completed in 1 to 24 hours, for example, and the progress of the reaction can be monitored by a known chromatography technique. An undesirable by-product can be removed by a technique known to a person skilled in the art such as a conventional chromatography technique or/and crystallization.

[Conversion of amidoalcohol compound (35f) into lactam compound (32)]

Step 10-6 consists of conversion of $L_{27}$ of the amidoalcohol compound (35f) into an alcohol or amine and subsequent cyclization reaction. The conversion of $L_{27}$ of the amidoalcohol compound (35f) into an alcohol varies according to the starting material, and can be performed by a method known to a person skilled in the art insofar as the conditions are similar to those in this reaction (see Jikken Kagaku Koza (Courses in Experimental Chemistry), 4th edition, vol.20, Yuki Gosei Hannou (Organic Synthesis Reaction) [II], edited by The Chemical Society of Japan, Maruzen Co., Ltd., 1992, p.1-30, for example).
The conversion of $L_{27}$ of the amidoalcohol compound (35f) into an amine varies according to the starting material, and can be performed by a method known to a person skilled in the art insofar as the conditions are similar to those in this reaction (see Jikken Kagaku Koza (Courses in Experimental Chemistry), 4th edition, vol.20, Yuki Gosei Hannou (Organic Synthesis Reaction) [II], edited by The Chemical Society of Japan, Maruzen Co., Ltd., 1992, p.279-318, for example).
The cyclization reaction of the alcohol compound varies according to the starting material, and can be performed by a method known to a person skilled in the art insofar as the conditions are similar to those in this reaction (see "Journal of Fluorine Chemistry", 1997, vol.2, p.119; or "Scientia Pharmaceutica", 1996, vol.64, p.3, for example). Preferably, the lactam compound (32) can be obtained in a high yield by heating the alcohol compound in a solvent or without a solvent in the presence of 0.1 to 10 equivalents of an organic acid such as p-toluenesulfonic acid or camphorsulfonic acid or an inorganic acid such as sulfuric acid or hydrochloric acid, for example.
The cyclization reaction of the amine compound varies according to the starting material, and can be performed by a method known to a person skilled in the art insofar as the conditions are similar to those in this reaction (see "Petrochemia", 1990, vol.30, p.56; WO 2003076386; or "Tetrahedron Letters", 1982, vol.23, p.229, for example). Preferably, the lactam compound (32) can be obtained in a high yield by heating the amine compound in the presence of 0.1 to 1.0 equivalents of an organic metal such as tetrakistriphenylphosphine palladium or tristriphenylphosphine ruthenium, for example. The solvent used in this step varies according to the starting material and the reagent used, and is not particularly limited insofar as it does not inhibit the reaction and allows the starting material to be dissolved therein to a certain extent. The reaction temperature must be a temperature that can complete the reaction without promoting formation of an undesirable by-product, and is preferably ice-cold temperature to 100°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

[Preparation of lactam compound (32)-2]

[Formula 75]

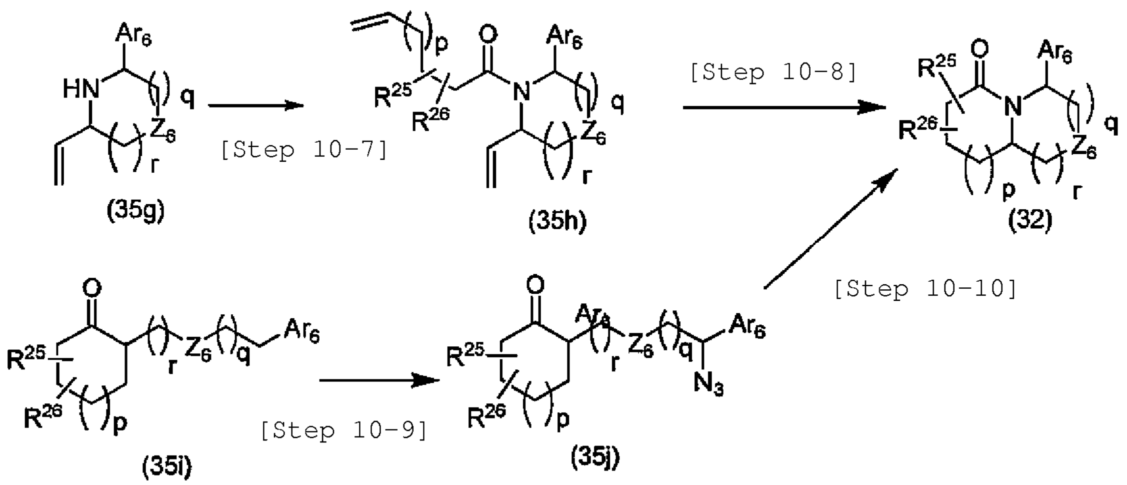


In the formula, $Ar_6$, $Z_6$, $R^{25}$, $R^{26}$, p, q and r are as defined above.

The above reaction formula also shows an example of a method for preparing the lactam compound (32). Specifically, the reaction formula shows (i) a method for preparing the lactam compound (32) comprising converting a vinyl group-substituted cyclic amine compound (35g) commercially available or prepared by a method known to a person skilled in the art (see "Tetrahedron Letters", 1998, vol.39, p.5421, for example) as a starting material into an acylated compound (35h) according to Step 10-7; and then cyclizing the acylated compound (35h) in Step 10-8 or (ii) a method for preparing the lactam compound (32) comprising converting a cycloalkyl ketone compound (35i) commercially available or prepared by a method known to a person skilled in the art (see "Journal of the Organic Chemistry", 2001, vol.66, p. 886, for example) as a starting material into an azide compound (35j) according to Step 10-9; and then cyclizing the azide compound (35j) in Step 10-10.

[Conversion of vinyl group-substituted cyclic amine compound (35g) into acylated compound (35h)]

The acylated compound (35h) can be prepared from the vinyl group substituted cyclic amine compound (35g) as a starting material in Step 10-7. Step 10-7 is performed by the same method as in the above Step 1-2.

[Conversion of acylated compound (35h) into lactam compound (32)]

Step 10-8 consists of ring closing metathesis reaction and subsequent double bond modification reaction. The ring closing metathesis reaction varies according to the starting material and can be performed by a method known to a person skilled in the art insofar as the conditions are similar to those in this reaction (see "Comprehensive Organometallic Chemistry", 1982, vol.8, p.499; or "Angewandte Chemie International Edition", 2000, vol.39, p.3012, for example). Preferably, the double bond modification reaction may be performed by, for example, i) catalytic hydrogenation (see Jikken Kagaku Koza (Courses in Experimental Chemistry), 4th edition, vol.26, Yuki Gosei Hannou (Organic Synthesis Reaction) [VIII], edited by The Chemical Society of Japan, Maruzen Co., Ltd., 1992, p.251-266, for example); ii) hydroboration (see Jikken Kagaku Koza (Courses in Experimental Chemistry), 4th edition, vol.25, Yuki Gosei Hannou (Organic Synthesis Reaction) [VII], edited by The Chemical Society of Japan, Maruzen Co., Ltd., 1991, p.83-134, for example); or iii) oxidation of a carbon-carbon double bond (see Jikken Kagaku Koza (Courses in Experimental Chemistry), 4th edition, vol.23, Yuki Gosei Hannou (Organic Synthesis Reaction) [V], edited by The Chemical Society of Japan, Maruzen Co., Ltd., 1991, p.237-267, for example).

In the first-stage ring closing metathesis reaction, the acylated compound (35h) is intramolecularly cyclized in the presence of preferably 0.01 to 0.2 equivalent of a metal catalyst with respect to the acylated compound (35h), for

example. This reaction is preferably performed in the presence of a solvent from the viewpoint of handleability and stirring efficiency. Preferable examples of the solvent used include halogenated solvents such as methylene chloride and chloroform; ether solvents such as tetrahydrofuran, 1,4-dioxane and 1,2-dimethoxyethane; non-polar solvents such as benzene, toluene and xylene; and mixed solvents thereof. The metal catalyst used varies according to the starting material and the solvent. Preferable examples of the metal catalyst used include ruthenium catalysts such as bis(tricyclohexylphosphine)benzylidene ruthenium (IV) dichloride, benzylidene[1,3-bis(2,4,6-trimethylphenyl)-2-imidazolidinylidene]dichloro(tricyclohexylphosphine)rut henium (IV) and [1,3-bis(2,4,6-trimethylphenyl)-2-imidazolidinylidene]dichloro (o-isopropoxyphenylmethylidene)ruthenium (IV); and molybdenum catalysts such as 2,6-diisopropylphenylimidoneophylidene biphen molybdenum (VI) and 2,6-diisopropylphenylimidoneophylidene molybdenum (VI) bis(hexafluoro-tert-butoxide). The reaction temperature must be a temperature that can complete the reaction without promoting formation of an undesirable by-product, and is preferably room temperature to 100°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

The second-stage double bond modification reaction is preferably catalytic hydrogenation, for example, in which the cyclized compound obtained by the ring closing metathesis reaction is preferably reduced in a hydrogen stream at 1 to 10 atm, for example, in the presence of 0.01 to 0.2 equivalent of a metal catalyst, for example. This reaction is preferably performed in the presence of a solvent from the viewpoint of handleability and stirring efficiency. Preferable examples of the solvent used include alcohol solvents such as ethanol and methanol; halogenated solvents such as methylene chloride and chloroform; ether solvents such as tetrahydrofuran, 1,4-dioxane and 1,2-dimethoxyethane; non-polar solvents such as benzene, toluene and xylene; polar solvents such as ethyl acetate and acetonitrile; and mixed solvents thereof. The metal catalyst used varies according to the starting material and the solvent. Preferable examples of the catalyst include platinum, platinum oxide, platinum black, Raney nickel and palladium-carbon. The reaction temperature must be a temperature that can complete the reaction without promoting formation of an undesirable by-product, and is preferably room temperature to 100°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

[Conversion of cycloalkyl ketone compound (35i) into azide compound (35j)]

Step 3-9 consists of ihalogenation reaction at the α-position of an aromatic ring and subsequent azide introduction reaction.

The first-step halogenation reaction at the α-position of an aromatic ring varies according to the starting material and can be performed by a method known to a person skilled in the art insofar as the conditions are the same for this reaction (see Jikken Kagaku Koza (Courses in Experimental Chemistry), 4th edition, vol.19, Yuki Gosei Hannou (Organic Synthesis Reaction) [I], edited by The Chemical Society of Japan, Maruzen Co., Ltd., 1992, p.422-458, for example). Preferably, 1.0 to 2.0 equivalents of a halogenating agent is used with respect to the cycloalkyl ketone compound (35i), for example. Preferable examples of the halogenating agent include N-bromosuccinimide and bromine. The reaction may be remarkably promoted by adding 0.01 to 0.5 equivalent of a radical initiator such as benzoyl peroxide or 2,2-azobis(isobutyronitrile) or 0.01 to 0.5 equivalent of an acid catalyst such as hydrobromic acid, for example. This reaction is preferably performed in the presence of a solvent from the viewpoint of handleability and stirring efficiency. The solvent used varies according to the starting material, and is not particularly limited insofar as it does not inhibit the reaction and allows the starting material to be dissolved therein to a certain extent. Preferable examples of the solvent include carbon tetrachloride and benzene. The reaction temperature must be a temperature that can complete the reaction without promoting formation of an undesirable by-product, and is preferably room temperature to 150°C, for example. Under preferable reaction conditions, the reaction is preferably completed in 1 to 24 hours, for example, and the progress of the reaction can be monitored by a known chromatography technique. An undesirable by-product can be removed by a technique known to a person skilled in the art such as a conventional chromatography technique, extraction or/and crystallization.

The second-step azidation reaction varies according to the starting material and can be performed by a method known to a person skilled in the art insofar as the conditions are similar to those in this reaction (see Jikken Kagaku Koza (Courses in Experimental Chemistry), 4th edition, vol.20, Yuki Gosei Hannou (Organic Synthesis Reaction) [II], edited by The Chemical Society of Japan, Maruzen Co., Ltd., 1992, p.415-420, for example). Preferably, 1.0 to 5.0 equivalents of an azidating agent is used with respect to the halogenated compound, for example. Examples of the azidating agent include sodium azide and trimethylsilyl azide. The reaction may be remarkably promoted using 0.1 to 5.0 equivalents of a quaternary amine salt such as tetrabutylammonium fluoride, for example. This reaction is preferably performed in the presence of a solvent from the viewpoint of handleability and stirring efficiency. The solvent used varies

according to the starting material, and is not particularly limited insofar as it does not inhibit the reaction and allows the starting material to be dissolved therein to a certain extent. Preferable examples of the solvent include ether solvents such as tetrahydrofuran and dioxane; halogenated solvents such as chloroform and methylene chloride; non-polar solvents such as benzene and toluene; and polar solvents such as acetone, acetonitrile, dimethylformamide, N-methylpyrrolidine and dimethyl sulfoxide. The reaction temperature must be a temperature that can complete the reaction without promoting formation of an undesirable by-product, and is preferably room temperature to 150°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

[Conversion of azide compound (35j) into lactam compound (32)]

Step 10-10 is a method for preparing the lactam compound (32) comprising treating the azide compound (35j) with an acid to cause rearrangement reaction. This step varies according to the starting material and can be performed by a method known to a person skilled in the art insofar as the conditions are similar to those in this reaction (see "Journal of the Organic Chemistry", 2001, vol.66, p.886, for example). Preferably, 1.0 to 10.0 equivalents of an acid such as trifluoromethanesulfonic acid, trifluoroacetic acid, sulfuric acid or hydrochloric acid is used, for example. Although the acid may be used as a solvent, this reaction is preferably performed in the presence of a separate solvent from the viewpoint of operativity and stirring efficiency. The solvent used varies according to the starting material, and is not particularly limited insofar as it does not inhibit the reaction and allows the starting material to be dissolved therein to a certain extent. Preferable examples of the solvent include halogenated solvents such as chloroform and methylene chloride; and non-polar solvents such as benzene and toluene. The reaction temperature must be a temperature that can complete the reaction without promoting formation of an undesirable by-product, and is preferably -78°C to 50°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

[General Preparation Method 13]

Typically used General Preparation Method 13 for the compound of the general formula (IX) of the present invention will be described below.
[Formula 76]

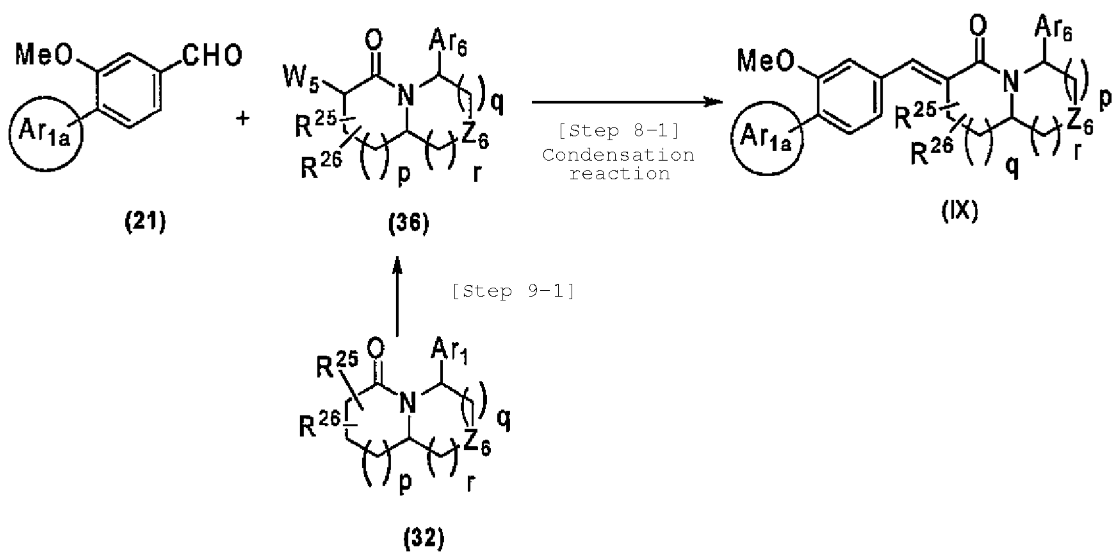


(21) (36) (IX) (32)

In the formula, $Ar_{1a}$, $Z_6$, $R^{25}$, $R^{26}$, p, q and r are as defined above; and $W_5$ represents a phosphite group such as a

diethylphosphonyl group, a phosphonium salt such as triphenylphosphonium bromide, a silyl group such as a trimethylsilyl group, or a carboxyl group.

The above General Preparation Method 13 is an example of a method for preparing the compound of the general formula (IX) comprising introducing a leaving group $W_5$ into the lactam compound (32) according to the above Step 9-1; and then condensing the compound with the aldehyde compound (21) obtained by General Preparation Method 10 by condensation reaction in the above Step 8-1 (such as Wittig reaction, Horner-Emmons reaction, Peterson reaction or Knoevenagel reaction).

[General Preparation Method 14]

Typically used General Preparation Method 14 for the compound of the general formula (IX) of the present invention will be described below.

[Formula 77]

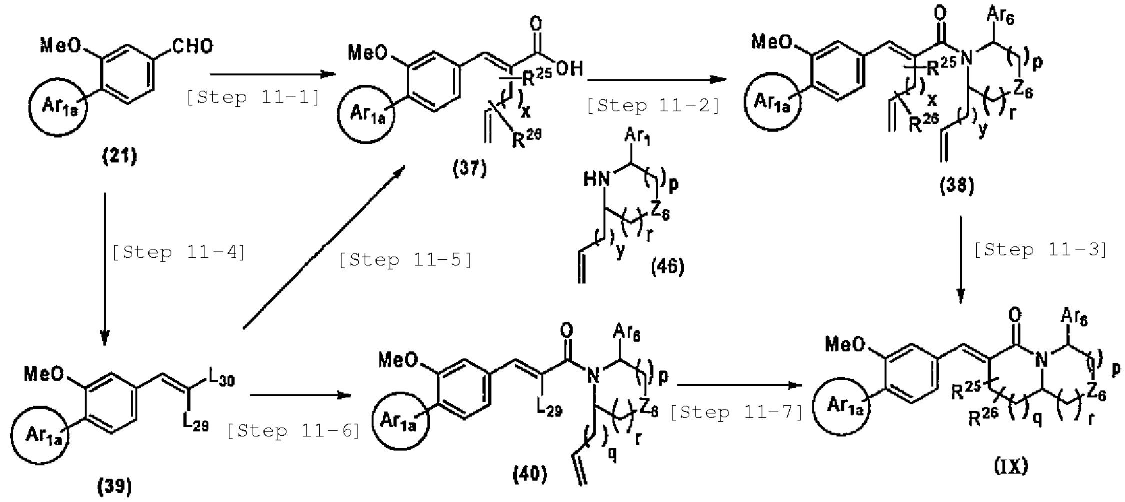


In the formula, $Ar_{1a}$, $Ar_6$, $Z_6$, $R^{25}$, $R^{26}$, p, q and r are as defined above; x and y each represent an integer of 0 to 2; $L_{29}$ represents a halogen atom such as chlorine, bromine or iodine, or a triflate group; and $L_{30}$ represents an ester group such as a methyl ester group or an ethyl ester group, or carboxylic acid.

The above General Preparation Method 14 is an example of i) a method for preparing the compound of the general formula (IX) comprising converting the aldehyde compound (21) into a cinnamic acid compound (37) according to Step 11-5 through Step 11-1 or Step 11-4; converting the cinnamic acid compound (37) into an amide compound (38) by condensation reaction with an amine compound (46) in Step 11-2; and then subjecting the amide compound (38) to ring closing metathesis reaction and subsequent double bond modification reaction in Step 11-3 or ii) a method for preparing the compound of the general formula (VIII) comprising converting the aldehyde compound (21) into a cinnamic acid compound (39) according to Step 11-4; converting the cinnamic acid compound (39) into an amide compound (40) in Step 11-6; and then subjecting the amide compound (40) to Heck reaction and subsequent double bond modification reaction in Step 11-7.

In the method i), the compound of the general formula (IX) can be prepared from the amide compound (38) according to Step 11-3. Step 11-3 consists of ring closing metathesis reaction and subsequent double bond modification reaction and is performed by the same method as in Step 10-8.

In the method ii), the compound of the general formula (IX) can be prepared from the amide compound (40) according to Step 11-7. Step 11-7 consists of Heck reaction and subsequent double bond modification reaction. Specifically, the first-stage Heck reaction varies according to the starting material and can be performed by a method known to a person skilled in the art insofar as the conditions are similar to those in this reaction (see Jikken Kagaku Koza (Courses in Experimental Chemistry), 4th edition, vol.19, Yuki Gosei Hannou (Organic Synthesis Reaction) [I], edited by The Chemical Society of Japan, Maruzen Co., Ltd., 1992, p.123-132, for example). The second-stage double bond modification reaction may be performed by the same method as in Step 10-8.

In the Heck reaction, coupling reaction is performed preferably in the presence of 0.01 to 0.2 equivalent of a transition metal catalyst with respect to the compound (40), for example. This reaction is preferably performed in the presence of a solvent from the viewpoint of handleability and stirring efficiency. The solvent used varies according to the starting material and the transition metal catalyst used, and is not particularly limited insofar as it does not inhibit the reaction and allows the starting material to be dissolved therein to a certain extent. Preferable examples of the solvent include acetonitrile, tetrahydrofuran, 1,4-dioxane, 1,2-dimethoxyethane, benzene, toluene, xylene, 1-methyl-2-pyrrolidone and N,N-dimethylformamide. The reaction temperature must be a temperature that can complete the coupling reaction, and is preferably room temperature to 150°C, for example. This reaction is performed preferably in an inert gas atmosphere, and more preferably in a nitrogen or argon atmosphere. The transition metal catalyst is preferably a palladium complex, for example, and more preferably a known palladium complex such as palladium (II) acetate, dichlorobis(triphenylphosphine)palladium (II), tetrakis(triphenylphosphine)palladium (0) or tris(dibenzylideneacetone)dipalladium (0). In addition, it is preferable to appropriately add preferably 1.0 to 5.0 equivalents of a phosphorus ligand (preferably triphenylphosphine, tri-o-tolylphosphine, tri-tert-butylphosphine or 2-(di-tert-butylphosphino)biphenyl, for example) with respect to the transition metal catalyst used, for example, in order to make the reaction efficiently proceed. A preferable result may be achieved in the presence of a base. The base used is not particularly limited insofar as it is used in a coupling reaction similar to this reaction. The base is preferably 0.1 to 5.0 equivalents of triethylamine, N,N-diisopropylethylamine, N,N-dicyclohexylmethylamine or tetrabutylammonium chloride, for example. Under preferable reaction conditions, the reaction is completed in 1 to 24 hours, and the progress of the reaction can be monitored by a known chromatography technique.

[Preparation of amide compound (38)]

The amide compound (38) can be efficiently synthesized by amidation reaction in Step 11-2 by the same method as in the above Step 1-2.

[Preparation of amine compound (46)]

The amine compound (46) used is commercially available or can be prepared by a method known to a person skilled in the art (see "Tetrahedron Letters", 1998, vol.39, p.5421, for example).

[Preparation of cinnamic acid compound (37)]

The cinnamic acid compound (37) can be prepared i) from the aldehyde compound (21) according to Step 11-1 or ii) by converting the aldehyde compound (21) into the cinnamate compound (39), wherein $L_{30}$ represents an ester group, according to Step 11-4; and then subjecting the cinnamate compound (39) to Step 11-5.

[Conversion of aldehyde compound (21) into cinnamate compound (37)]

Step 11-1 consists of a first stage of converting the aldehyde compound (21) into a cinnamate and a subsequent second stage of hydrolyzing the ester group into a carboxylic acid group. The cinnamate can be prepared from the aldehyde compound (21) and any of various Horner-Emmons reagents by a method known to a person skilled in the art (see W.S. Wadsworth, Jr., "Organic Reactions", 1997, vol.25, p.73, for example). Preferably, for example, the cinnamic acid compound (37) can be obtained in a high yield using the aldehyde compound (21), 1.0 to 2.0 equivalents of the Horner-Emmons reagent, for example, and 1.0 to 5.0 equivalents of a base, for example. The Horner-Emmons reagent can be prepared by a method known to a person skilled in the art. For example, the compound can be prepared by alkylation of commercially available trialkylphosphonoacetic acid (see "Synthetic Communication", 1991, vol.22, p.2391, for example), Arbuzov reaction using an alkylphosphinite of α-halogenoacetic acid derivative (see "Chemical Review", 1981, vol.81, p.415, for example) or Becker reaction using a metal phosphonite (see "Journal of the American Chemical Society", 1945, vol.67, p.1180, for example). Preferable examples of the solvent used include polar solvents such as 1-methyl-2-pyrrolidone, N,N-dimethylformamide and dimethyl sulfoxide; ether solvents such as tetrahydrofuran, 1,4-dioxane and 1,2-dimethoxyethane; non-polar solvents such as benzene, toluene and xylene; alcohol solvents such as ethanol and methanol; water; and mixed solvents thereof. The base used varies according to the starting material and the solvent. Preferable examples of the base include alkali metal hydroxides such as sodium hydroxide and lithium hydroxide; alkali metal carbonates such as sodium carbonate; alkali metal salts of alcohols such as sodium methoxide and potassium tert-butoxide; organic bases such as triethylamine, pyridine and diazabicyclononene; organic metals such as butyl lithium and lithium diisobutylamide; alkali metal hydrides such as sodium hydride; and alkali metal ammonium salts such as sodium amide. The reaction temperature must be a temperature that can complete the reaction without promoting formation of an undesirable by-product, and is preferably -78 to 150°C, for example. Under preferable reaction conditions,

the reaction is preferably completed in 1 to 24 hours, for example, and the progress of the reaction can be monitored by a known chromatography technique. An undesirable by-product can be removed by a technique known to a person skilled in the art such as a conventional chromatography technique, extraction or/and crystallization. A known deprotection method known to a person skilled in the art may be used for hydrolysis reaction from the cinnamate to the cinnamic acid compound (37) (see T.W. Green, "Protective Groups in Organic Synthesis", John Wiley & Sons, Inc., 1981, p.154-186).

[Conversion of compound (39) into cinnamic acid compound (37)]

The cinnamic acid compound (37) can be prepared by coupling the compound (39) as a starting material with a corresponding alkene compound according to Step 11-5. Specifically, a method known to a person skilled in the art may be used for the coupling reaction in Step 11-5. Preferable examples of the method include Heck reaction (see R.F. Heck, "Org. Reactions.", 1982, vol.27, p.345, for example), Suzuki reaction (see A. Suzuki, "Chem. Rev.", 1995, vol.95, p.2457, for example) and Stille coupling reaction (see J.K. Stille, "Angew. Chem. Int. Ed. Engl.", 1986, vol.25, p.508, for example).

For example, the Heck reaction can be preferably performed according to Step 11-7 using 1.0 to 5.0 equivalents of an alkene compound with respect to the halide or triflate compound (39), for example.

In the Suzuki reaction, for example, the halide or triflate compound (39) is preferably coupled with 1.0 to 5.0 equivalents of a boronic acid compound or a boronate compound, for example, in the presence of 0.01 to 0.5 equivalent of a transition metal catalyst with respect to the compound (39), for example. This reaction is preferably performed in the presence of a solvent from the viewpoint of handleability and stirring efficiency. The solvent used varies according to the starting material and the transition metal catalyst used, and is not particularly limited insofar as it does not inhibit the reaction and allows the starting material to be dissolved therein to a certain extent. Preferable examples of the solvent include acetonitrile, tetrahydrofuran, 1,4-dioxane, 1,2-dimethoxyethane, benzene, toluene, xylene, 1-methyl-2-pyrrolidone, N,N-dimethylformamide, water and a mixed solvent thereof. The reaction temperature must be a temperature that can complete the coupling reaction, and is preferably room temperature to 200°C, for example. This reaction is performed preferably in an inert gas atmosphere, and more preferably in a nitrogen or argon atmosphere. Under preferable reaction conditions, the reaction is completed in 1 to 24 hours, and the progress of the reaction can be monitored by a known chromatography technique. The transition metal catalyst is preferably a known palladium complex, and more preferably a known palladium complex such as palladium (II) acetate, dichlorobis(triphenylphosphine)palladium (II), tetrakis(triphenylphosphine)palladium (0) or tris(dibenzylideneacetone)dipalladium (0). A phosphorus ligand (preferably triphenylphosphine, tri-o-tolylphosphine, tricyclohexylphosphine or tri-tert-butylphosphine, for example) may be appropriately added in order to make the reaction efficiently proceed. A quaternary ammonium salt, preferably tetrabutylammonium chloride or tetrabutylammonium bromide, for example, may also be appropriately added in order to make the reaction efficiently proceed. In this reaction, a preferable result may be achieved in the presence of a base. The base used at this time varies according to the starting material and the solvent used, and is not particularly limited. Preferable examples of the base include sodium hydroxide, barium hydroxide, potassium fluoride, cesium fluoride, sodium carbonate, potassium carbonate, cesium carbonate and potassium phosphate. Under preferable reaction conditions, the reaction is completed in 1 to 24 hours, and the progress of the reaction can be monitored by a known chromatography technique.

In the Stille coupling reaction, the halide or triflate compound (39) is preferably coupled with 1.0 to 10.0 equivalents of a trialkyltin compound, for example, in the presence of 0.01 to 0.2 equivalent of a transition metal catalyst, for example. For example, 0.1 to 5.0 equivalents of copper (I) halide or/and lithium chloride may be appropriately used in order to make the reaction efficiently proceed. Preferable examples of the solvent used in this reaction include toluene, xylene, N,N-dimethylformamide, N,N-dimethylacetamide, 1-methyl-2-pyrrolidone and dimethyl sulfoxide. The reaction temperature must be a temperature that can complete the coupling reaction, and is preferably room temperature to 150°C, for example. The transition metal catalyst used is a palladium complex, preferably a known palladium complex such as palladium (II) acetate, dichlorobis(triphenylphosphine)palladium (II), tetrakis(triphenylphosphine)palladium (0) or tris(dibenzylideneacetone)dipalladium (0), for example, and more preferably tetrakis(triphenylphosphine)palladium (0) or tris(dibenzylideneacetone)dipalladium (0), for example. This reaction is performed preferably in an inert gas atmosphere, and more preferably in a nitrogen or argon atmosphere. Under preferable reaction conditions, the reaction is preferably completed in 1 to 24 hours, for example, and the progress of the reaction can be monitored by a known chromatography technique.

[Conversion of compound (21) into compound (39)]

The compound (39) can be prepared by reacting the compound (21) as a starting material with halogenated phosphonoacetic acid in Horner-Emmons reaction according to Step 11-4 (see "Organic Letter", 2000, vol.2, p.1975, for example).

[Conversion of compound (39) into compound (40)]

The compound (40) can be prepared from the compound (39) as a starting material according to Step 11-6. Step 11-6 and preparation of the amine compound used are the same as in the above Step 11-2.

[General Preparation Method 15]

Typically used General Preparation Method 15 for the compound of the general formula (IX) of the present invention will be described below.

[Formula 78]

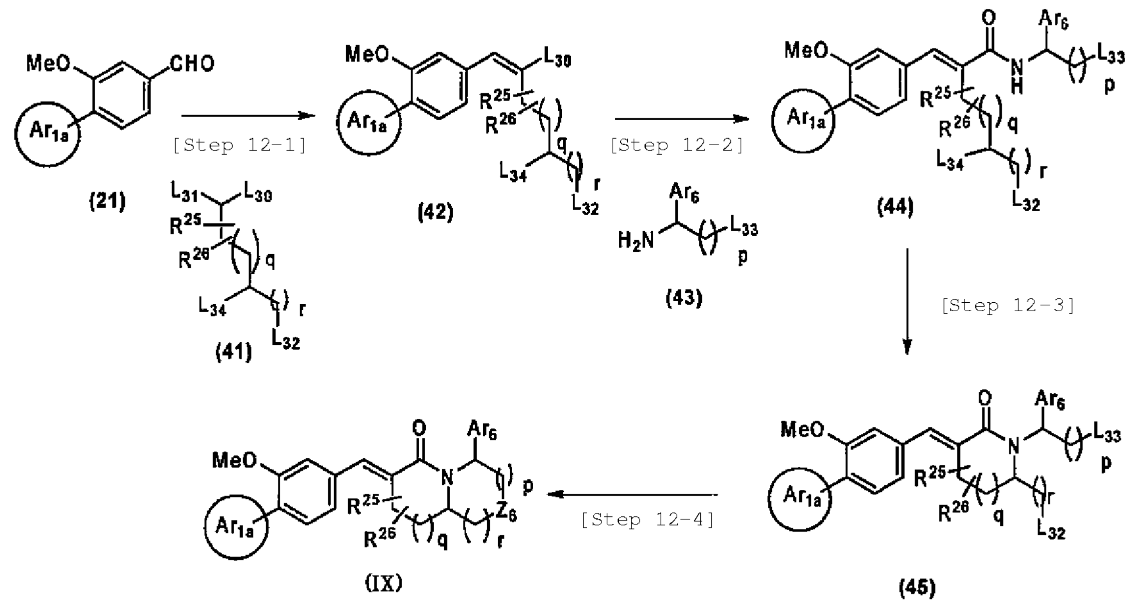

(21) (41) (42) (43) (44) (45) (IX)

In the formula, $Ar_{1a}$, $Ar_6$, $Z_6$, $R^{25}$, $R^{26}$, p, q and r are as defined above; $L_{30}$ represents an ester group such as a methyl ester group or an ethyl ester group, or a carboxylic acid group; $L_{31}$ represents a phosphite group such as a diethylphosphonyl group; $L_{32}$ and $L_{33}$ each represent an alcohol group, an amino group or a protected derivative thereof; and $L_{34}$ represents a halogen atom such as a chlorine atom or a bromine atom, or a sulfonate group such as a mesyl group or a tosyl group.

The above General Preparation Method 15 is an example of a method for preparing the compound of the general formula (IX) comprising converting the aldehyde compound (21) and a Horner-Emmons reagent (41) into a cinnamic acid compound (42) according to Step 12-1; amidating the cinnamic acid compound (42) in Step 12-2; then forming a lactam ring according to Step 12-3; and finally subjecting the lactam ring to second ring-forming reaction in Step 12-4.

[Preparation of compound of general formula (IX)]

The compound of the general formula (IX) can be prepared from a lactam compound (45) according to Step 12-4. Step 12-4 consists of deprotection reaction of an alcohol group or an amine group and subsequent cyclization reaction. A deprotection reaction described in many known documents may be used (see T.W. Green, "Protective Groups in Organic Synthesis", John Wiley & Sons, Inc., 1981). The cyclization reaction varies according to the starting material and is not particularly limited insofar as the conditions are similar to those in this reaction. A method known to a person skilled in the art may be used for the reaction. Preferable examples of the method include i) a method of forming a cyclic ether from a diol (see "Journal of Fluorine Chemistry", 1997, vol.2, p.119; or "Scientia Pharmaceutica", 1996, vol.64, p. 3, for example) and ii) a method of forming a cyclic amine from an aminoalcohol (see "Petrochemia", 1990, vol.30, p. 56; WO 2003076386; or "Tetrahedron Letters", 1982, vol.23, p.229, for example). More preferably, the compound of the

general formula (IX) can be obtained in a high yield by heating the lactam compound (45) in a solvent or without a solvent in the presence of 0.1 to 10 equivalents of an organic acid such as p-toluenesulfonic acid or camphorsulfonic acid or an inorganic acid such as sulfuric acid or hydrochloric acid, for example, or by heating the lactam compound (45) in the presence of 0.1 to 10 equivalents of an organic metal such as tetrakistriphenylphosphine palladium or tristriphenylphosphine ruthenium, for example. The solvent used in this step varies according to the starting material and the condensing agent used, and is not particularly limited insofar as it does not inhibit the reaction and allows the starting material to be dissolved therein to a certain extent. The reaction temperature must be a temperature that can complete the reaction without promoting formation of an undesirable by-product, and is preferably ice-cold temperature to 100°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique or/and crystallization.

[Preparation of lactam compound (45)]

The lactam compound (45) can be prepared from a cinnamide compound (44) as a starting material according to Step 12-3 by cyclization reaction involving elimination of $L_{34}$ of the cinnamide compound (44). Specifically, for example, the desired lactam compound (45) can be obtained in a high yield by treating the compound (44) with preferably 1.0 to 5.0 equivalents of a base, for example. This reaction is preferably performed in the presence of a solvent from the viewpoint of handleability and stirring efficiency. The solvent used varies according to the starting material and the base used, and is not particularly limited insofar as it does not inhibit the reaction and allows the starting material to be dissolved therein to a certain extent. Preferable examples of the solvent include polar solvents such as 1-methyl-2-pyrrolidone, N,N-dimethylformamide and dimethyl sulfoxide; ether solvents such as tetrahydrofuran, 1,4-dioxane and 1,2-dimethoxyethane; non-polar solvents such as benzene, toluene and xylene; alcohol solvents such as ethanol and methanol; water; and mixed solvents thereof. The base used varies according to the starting material and the solvent. Preferable examples of the base include alkali metal hydroxides such as sodium hydroxide and lithium hydroxide; alkali metal carbonates such as sodium carbonate; alkali metal salts of alcohols such as sodium methoxide and potassium tert-butoxide; organic bases such as triethylamine, pyridine and diazabicyclononene; organic metals such as butyl lithium and lithium diisobutylamide; alkali metal hydrides such as sodium hydride; and alkali metal ammonium salts such as sodium amide. The reaction temperature must be a temperature that can complete the reaction without promoting formation of an undesirable by-product, and is preferably -78 to 150°C, for example. Under preferable reaction conditions, the reaction is completed in 1 to 24 hours, and the progress of the reaction can be monitored by a known chromatography technique. An undesirable by-product can be removed by a technique known to a person skilled in the art such as a conventional chromatography technique, extraction or/and crystallization.

[Preparation of cinnamide compound (44)]

The cinnamide compound (44) can be prepared from the cinnamic acid compound (42) and preferably 1.0 to 5.0 equivalents of the amine compound (13), for example, according to amidation reaction in Step 12-2. The amidation reaction is the same reaction as in Step 1-2.

[Preparation of amine compound (43)]

The amine compound (43) is commercially available or can be prepared by a method known to a person skilled in the art. If not commercially available, the amine compound (43) can be prepared by converting a corresponding aldehyde group into a vinyl group and then aminohydroxylating the compound (see "Journal of the American Chemical Society", 2001, vol.123, p.1862, for example).

[Preparation of cinnamic acid compound (42)]

Step 12-1 consists of a step of synthesizing a cinnamate by condensation reaction of the aldehyde compound (21) with the Horner-Emmons reagent (41) and a subsequent step of deprotecting an ester group into carboxylic acid. This step is performed by the same method as in Step 11-1.

[Preparation of compound (41)]

The compound (41) is commercially available or can be prepared by a method known to a person skilled in the art if not commercially available. For example, the compound can be prepared by alkylation of commercially available trialkylphosphonoacetic acid (see "Synthetic Communication", 1991, vol.22, p.2391, for example), Arbuzov reaction

using an alkylphosphinite of α-halogenoacetic acid derivative (see "Chemical Review", 1981, vol.81, p.415, for example) or Becker reaction using a metal phosphonite (see "Journal of the American Chemical Society", 1945, vol.67, p.1180, for example).

The present inventors performed the following tests in order to exhibit utility of the compounds of the general formulas (I), (VIII) and (IX) of the present invention.

Test Example 1 [Quantification of Aβ peptide in neuronal culture from rat fetus brain]

(1) Rat primary neuronal culture

Primary neuronal cultures were prepared from the cerebral cortex of embryonic day 18 Wistar rats (Charles River Japan, Yokohama, Japan). Specifically, the embryos were aseptically removed from pregnant rats under ether anesthesia. The brain was isolated from the embryo and immersed in an ice-cold L-15 medium (such as Invitrogen Corp. Cat #11415-064, Carlsbad, CA, USA, or SIGMA L1518). The cerebral cortex was collected from the isolated brain under a stereoscopic microscope. The cerebral cortex fragments collected were enzymatically treated in an enzyme solution containing 0.25% trypsin (Invitrogen Corp. Cat #15050-065, Carlsbad, CA, USA) and 0.01% DNase (Sigma D5025, St. Louis, MO, USA) at 37°C for 30 minutes to disperse the cells. Here, the enzymatic reaction was stopped by adding inactivated horse serum to the solution. The enzymatically treated solution was centrifuged at 1,500 rpm for five minutes to remove the supernatant. 5 to 10 ml of a medium was added to the resulting cell mass. Neurobasal medium (Invitrogen Corp. Cat #21103-049, Carlsbad, CA, USA) supplemented with 2% B27 supplement (Invitrogen Corp. Cat #17504-044, Carlsbad, CA, USA), 25 μM 2-mercaptoethanol (2-ME, WAKO Cat #139-06861, Osaka, Japan), 0.5 mM L-glutamine (Invitrogen Corp. Cat #25030-081, Carlsbad, CA, USA), and Antibiotics-Antimycotics (Invitrogen Corp. Cat #15240-062, Carlsbad, CA, USA) was used as the medium (Neurobasal/B27/2-ME). However, the above Neurobasal medium not supplemented with 2-ME (Neurobasal/B27) was used for the assay. The cells were redispersed by mild pipetting of the cell mass to which the medium was added. The cell dispersion was filtered through a 40-μm nylon mesh (Cell Strainer, Cat #35-2340, Becton Dickinson Labware, Franklin Lakes, NJ, USA) to remove the remaining cell mass, and thus a neuronal cell suspension was obtained. The neuronal cell suspension was diluted with the medium and then plated in a volume of 100 μl/well at an initial cell density of 5 x $10^5$ cells/$cm^2$ in a 96-well polystyrene culture plate precoated with poly-L or D-lysine (Falcon Cat #35-3075, Becton Dickinson Labware, Franklin Lakes, NJ, USA coated with poly-L-lysine using the method shown below, or BIOCOAT™ cell environments Poly-D-lysine cell ware 96-well plate, Cat #35-6461, Becton Dickinson Labware, Franklin Lakes, NJ, USA). Poly-L-lysine coating was carried out as follows. 100 μg/ml of a poly-L-lysine (SIGMA P2636, St. Louis, MO, USA) solution was aseptically prepared with a 0.15 M borate buffer (pH 8.5). 100 μg/well of the solution was added to the 96-well polystyrene culture plate and incubated at room temperature for one or more hours or at 4°C overnight or longer. The coated 96-well polystyrene culture plate was washed with sterile water four or more times, and then dried or rinsed with, for example, sterile PBS or medium, and used for cell plating. The plated cells were cultured in the culture plate at 37°C in 5% $CO_2$-95% air for one day. Then, the total amount of the medium was replaced with a fresh Neurobasal™/B27/2-ME medium, and then the cells were cultured for further three days.

Addition of compounds

The drug was added to the culture plate on Day 4 of culture as follows. The total amount of the medium was removed from the wells, and 180 μl/well of Neurobasal medium not containing 2-ME and containing 2% B-27 (Neurobasal/B27) was added thereto. A solution of the test compound in dimethyl sulfoxide (hereinafter abbreviated as DMSO) was diluted with Neurobasal/B27 to a concentration 10-fold higher than the final concentration. 20 μl/well of the dilution was added to and sufficiently mixed with the medium. The final DMSO concentration was 1% or less. Only DMSO was added to the control group.

Sampling

The cells were cultured for three days after addition of the compound, and the total amount of the medium was collected. The resulting medium was used as an ELISA sample. The sample was not diluted for ELISA measurement of Aβx-42 and diluted to 5-fold with a diluent supplied with an ELISA kit for ELISA measurement of Aβx-40.

Evaluation of cell survival

Cell survival was evaluated by an MTT assay according to the following procedure. After collecting the medium, 100 μl/well of a pre-warmed medium was added to the wells. Further, 8 μl/well of a solution of 8 mg/ml of MTT (SIGMA M2128, St. Louis, MO, USA) in D-PBS(-) (Dulbecco's phosphate buffered Saline, SIGMA D8537, St. Louis, MO, USA)

was added to the wells. The 96-well polystyrene culture plate was incubated in an incubator at 37°C in 5% $CO_2$-95% air for 20 minutes. 100 μl/well of an MTT lysis buffer was added thereto, and MTT formazan crystals were sufficiently dissolved in the buffer in the incubator at 37°C in 5% $CO_2$-95% air. Then, the absorbance at 550 nm in each well was measured. The MTT lysis buffer was prepared as follows. 100 g of SDS (sodium dodecyl sulfate (sodium lauryl sulfate), WAKO 191-07145, Osaka, Japan) was dissolved in a mixed solution of 250 mL of N,N'-dimethylformamide (WAKO 045-02916, Osaka, Japan) and 250 mL of distilled water. 350 μl each of concentrated hydrochloric acid and acetic acid were further added to the solution to allow the solution to have a final pH of about 4.7.
Upon measurement, wells having no cells plated and containing only the medium and MTT solution were set as background (bkg). The measured values were respectively applied to the following formula including subtracting bkg values from them. Thus, the proportion against the control group (group not treated with the drug, CTRL) (% of CTRL) was calculated to compare and evaluate cell survival activities.

$$\% \text{ of CTRL} = (A550_sample-A550_bkg)/(A550_CTRL-bkg) \times 100$$

(A550_sample: absorbance at 550 nm of sample well, A550_bkg: absorbance at 550 nm of background well, A550_CTRL: absorbance at 550 nm of control group well)

Aβ ELISA

Aβ ELISA employed Human/Rat β Amyloid (42) ELISA Kit Wako (#290-62601) and Human/Rat β Amyloid (40) ELISA Kit Wako (#294-62501) from Wako Pure Chemical Industries, Ltd., or Human Amyloid beta (1-42) Assay Kit (#27711) and Human Amyloid beta (1-40) Assay Kit (#27713) from Immuno-Biological Laboratories, Co., Ltd. (IBL Co., Ltd.). Aβ ELISA was carried out according to the protocols recommended by the manufacturers (methods described in the attached documents). However, the Aβ calibration curve was created using beta-amyloid peptide 1-42, rat and beta-amyloid peptide 1-40, rat (Calbiochem, #171596 [$A\beta_{42}$], #171593 [$A\beta_{40}$]). The results are shown in Table 1 as percentage to the Aβ concentration in the medium of the control group (% of CTRL).

(2) Accordingly, the compound of the present invention was proved to have an Aβ42 production reducing effect. Consequently, since the compound of the general formula (I) or a pharmaceutically acceptable salt thereof have an Aβ42 production reducing effect, the present invention can particularly provide a prophylactic or therapeutic agent for a neurodegenerative disease caused by Aβ such as Alzheimer's disease and Down's syndrome.

[Table 1]

| Test compound | Aβ42 production reducing effect IC50 (μM) |
|---|---|
| Example 6 | 0.32 |
| Example 17 | 0.18 |
| Example 18 | 0.16 |
| Example 19 | 0.15 |
| Example 20 | 0.21 |
| Example 21 | 0.06 |
| Example 22 | 0.05 |
| Example 23 | 0.40 |
| Example 24 | 0.29 |
| Example 26 | 0.38 |
| Example 27 | 0.27 |
| Example 28 | 0.44 |

The "salt" refers to a pharmaceutically acceptable salt, and is not particularly limited insofar as it forms a

pharmaceutically acceptable salt with the compound of the general formula (I) as a prophylactic or therapeutic agent for a disease caused by Aβ. Preferable specific examples of the salt include hydrohalides (such as hydrofluorides, hydrochlorides, hydrobromides and hydroiodides), inorganic acid salts (such as sulfates, nitrates, perchlorates, phosphates, carbonates and bicarbonates), organic carboxylates (such as acetates, oxalates, maleates, tartrates, fumarates and citrates), organic sulfonates (such as methanesulfonates, trifluoromethanesulfonates, ethanesulfonates, benzenesulfonates, toluenesulfonates and camphorsulfonates), amino acid salts (such as aspartates and glutamates), quaternary amine salts, alkali metal salts (such as sodium salts and potassium salts) and alkali earth metal salts (such as magnesium salts and calcium salts).

The therapeutic agent for a disease caused by Aβ according to the present invention can be prepared by a conventional method. Preferable examples of the dosage form include tablets, powders, fine granules, granules, coated tablets, capsules, syrups, troches, inhalants, suppositories, injections, ointments, ophthalmic solutions, ophthalmic ointments, nasal drops, ear drops, cataplasms and lotions. The prophylactic or therapeutic agent can be prepared by using ingredients typically used such as an expicient, a binder, a lubricant, a colorant and a corrective, and ingredients used where necessary such as a stabilizer, an emulsifier, an absorbefacient, a surfactant, a pH adjuster, a preservative and an antioxidant, and can be prepared by blending ingredients generally used as materials for a pharmaceutical preparation. Examples of such ingredients include animal and vegetable oils such as soybean oil, beef tallow and synthetic glyceride; hydrocarbons such as liquid paraffin, squalane and solid paraffin; ester oils such as octyldodecyl myristate and isopropyl myristate; higher alcohols such as cetostearyl alcohol and behenyl alcohol; a silicone resin; silicone oil; surfactants such as polyoxyethylene fatty acid ester, sorbitan fatty acid ester, glycerin fatty acid ester, polyoxyethylene sorbitan fatty acid ester, polyoxyethylene hydrogenated castor oil and a polyoxyethylene-polyoxypropylene block copolymer; water-soluble polymers such as hydroxyethylcellulose, polyacrytic acid, a carboxyvinyl polymer, polyethylene glycol, polyvinylpyrrolidone and methylcellulose; lower alcohols such as ethanol and isopropanol; polyhydric alcohols such as glycerin, propylene glycol, dipropylene glycol and sorbitol; sugars such as glucose and sucrose; inorganic powders such as silicic anhydride, magnesium aluminum silicate and aluminum silicate; and purified water. Examples of the expicient used include lactose, corn starch, saccharose, glucose, mannitol, sorbitol, crystalline cellulose and silicon dioxide. Examples of the binder used include polyvinyl alcohol, polyvinyl ether, methylcellulose, ethylcellulose, gum arabic, tragacanth, gelatin, shellac, hydroxypropylmethylcellulose, hydroxypropylcellulose, polyvinylpyrrolidone, a polypropylene glycol-polyoxyethylene block copolymer and meglumine. Examples of the disintegrator used include starch, agar, gelatin powder, crystalline cellulose, calcium carbonate, sodium bicarbonate, calcium citrate, dextrin, pectin and carboxymethylcellulose calcium. Examples of the lubricant used include magnesium stearate, talc, polyethylene glycol, silica and hydrogenated vegetable oil. Examples of the colorant used include those permitted to be added to pharmaceuticals. Examples of the corrective used include cocoa powder, menthol, empasm, mentha oil, borneol and cinnamon powder.

For example, an oral preparation is prepared by adding an active ingredient compound or a salt thereof or a hydrate of the compound or salt, an excipient, and, where necessary, a binder, a disintegrant, a lubricant, a colorant and a corrective, for example, and then forming the mixture into powder, fine granules, granules, tablets, coated tablets or capsules, for example, by a conventional method. It is obvious that tablets or granules may be appropriately coated, for example, sugar coated, where necessary. A syrup or an injection preparation is prepared by adding a pH adjuster, a solubilizer and an isotonizing agent, for example, and a solubilizing agent, a stabilizer and the like where necessary by a conventional method. An external preparation may be prepared by any conventional method without specific limitations. As a base material, any of various materials usually used for a pharmaceutical, a quasi drug, a cosmetic or the like may be used. Examples of the base material include materials such as animal and vegetable oils, mineral oils, ester oils, waxes, higher alcohols, fatty acids, silicone oils, surfactants, phospholipids, alcohols, polyhydric alcohols, water-soluble polymers, clay minerals and purified water. A pH adjuster, an antioxidant, a chelator, a preservative and fungicide, a colorant, a flavor or the like may be added where necessary. Further, an ingredient having a differentiation inducing effect such as a blood flow enhancer, a bactericide, an antiphlogistic, a cell activator, vitamin, amino acid, a humectant or a keratolytic agent may be blended where necessary. The dose of the therapeutic or prophylactic agent of the present invention varies according to the degree of symptoms, age, sex, body weight, mode of administration, type of salt and specific type of disease, for example. Typically, the therapeutic or prophylactic agent is orally administered to an adult at about 30 μg to 10 g, preferably 100 μg to 5 g, and more preferably 100 μg to 100 mg per day, or is administered to an adult by injection at about 30 μg to 1 g, preferably 100 μg to 500 mg, and more preferably 100 βg to 30 mg per day, in one or several doses, respectively.

BEST MODE FOR CARRYING OUT THE INVENTION

The present invention will now be described in detail with reference to examples; however, the examples are provided only for illustration purposes. The prophylactic or therapeutic agent for a disease caused by Aβ according to the present invention is not limited to the following specific examples in any cases. A person skilled in the art can fully implement the present invention by making various modifications to not only the following reference examples and

examples but also the claims of the present specification, and such modifications are within the scope of the claims of the present specification.

The following abbreviations are used in the following examples.

DMF: Dimethylformamide
THF: Tetrahydrofuran
LAH: Lithium aluminum hydride
EDC: 1-Ethyl-3-(3-dimethylaminopropyl)carbodiimide hydrochloride
HOBT: 1-Hydroxybenzotriazole
IPEA: Diisopropylethylamine
DCC: 1,3-Dicyclohexylcarbodiimide
DMAP: 4-(Dimethylamino)pyridine
TEA: Triethylamine
DPPA: 1,1-Bis(diphenylphosphino)ferrocene
CDI: Carbonyldiimidazole
TBAF: Tetrabutylammonium fluoride
PYBOP: Benzotriazol-1-yloxytris(pyrrolidino)phosphonium hexafluorophosphate
DBU: 1,8-Diazabicyclo[5.4.0]undec-7-ene
t: Tertiary
DAST: Diethylaminosulfur trifluoride
BOP: Benzotriazol-1-yloxytris(dimethylamino)phosphonium hexafluorophosphate
DIBAL-H: Diisobutylaluminum hydride

The present invention will now be described in detail with reference to examples; however, the examples are provided only for illustration purposes. The prophylactic or therapeutic agent for a disease caused by Aβ according to the present invention is not limited to the following specific examples in any cases. A person skilled in the art can fully implement the present invention by making various modifications to not only the following reference examples and examples but also the claims of the present specification, and such modifications are within the scope of the claims of the present specification.

Example 1

Synthesis of (E)-1-[(S)-1-(4-fluorophenyl)ethyl]-3-[1-[3-methox-4-(5-methltetrazol-1-yl)phenyl]methylidene]piperidin-2-one

[Formula 79]

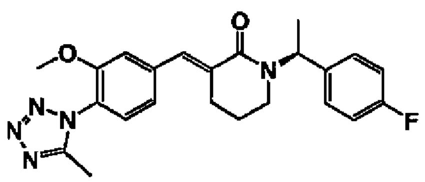

Synthesis of 1-[(S)-1-(4-fluorophenyl)ethyl]piperidin-2-one

A solution of 5-bromovaleryl chloride (1.0 mL) [CAS #4509-90-4] in toluene (2 mL) was added dropwise to a two-layer mixture of a vigorously stirred solution of (S)-1-(4-fluorophenyl)ethylamine (1.0 g) [CAS #66399-30-2] in toluene (5 mL) and a 50% sodium hydroxide solution (7 mL) under ice-cooling over 13 minutes. The reaction solution was stirred at the same temperature for 15 minutes. Benzyltriethylammonium chloride (164 mg) was added to the reaction solution, and the reaction solution was stirred at room temperature for four days. Ice water was added to the reaction solution and the organic layer was separated. Then, the aqueous layer is reextracted with toluene. The combined organic layers were sequentially washed with water, 1 N hydrochloric acid, water, a saturated sodium bicarbonate solution and brine,

dried over anhydrous magnesium sulfate and then concentrated under reduced pressure to obtain 1.38 g of a crude product of the title compound. The property values of the compound are as follows.
$^1$H-NMR ($CDCl_3$) δ (ppm): 1.48 (d, J = 7.2 Hz, 3H), 1.55-1.80 (m, 4H), 2.47 (m, 2H), 2.76 (m, 1H), 3.09 (m, 1H), 6.12 (q, J = 7.2 Hz, 1H), 7.01 (dd, J = 8.4, 8.4 Hz, 2H), 7.26 (m, 2H).

Synthesis of 1-[(S)-1-(4-fluorophenyl)ethyl]-3-iodopiperidin-2-one

Chlorotrimethylsilane (12.5 mL) was added dropwise to a solution of 1-[(S)-1-(4-fluorophenylethyl)piperidin-2-one (10 g) and N,N,N',N'-tetramethylethylenediamine (22.5 mL) in toluene (100 mL) at -20°C. Then, iodine (18.6 g) was introduced into the reaction solution in three portions. The reaction solution was gradually heated to 0°C and then stirred under ice-cooling for one hour. A mixed solution of a 10% sodium thiosulfate solution and 10% saline was added to the reaction solution, and the organic layer was separated. The organic layer was sequentially washed with a 10% sodium thiosulfate solution, water (twice), 1 N hydrochloric acid, water, a saturated sodium bicarbonate solution and brine, dried over anhydrous magnesium sulfate and then concentrated under reduced pressure. The resulting residue was purified by silica gel column chromatography (elution solvent: heptane:ethyl acetate = 3:1 -> 2:1) to obtain 15.1 g of the title compound. The property values of the compound are as follows.
$^1$H-NMR ($CDCl_3$) δ (ppm): 1.48, 1.50 (each d, J = 7.2 Hz, 3H), 1.65-1.81 (m, 1H,), 1.96-2.25 (m, 3H,), 2.82-3.00 (m, 1H), 3.20-3.35 (m, 1H), 4.88-4.96 (m, 1H), 6.04 (q, J = 7.2 Hz, 1H), 6.99-7.10 (m, 2H), 7.24-7.34 (m, 2H).

Synthesis of diethyl {1-[(S)-1-(4-fluorophenyl)ethyl]-2-oxopiperidin-3-yl}phosphonate

A mixture of 1-[(S)-1-(4-fluorophenyl)ethyl]-3-iodopiperidin-2-one (10 g) and triethyl phosphite (14.8 mL) was stirred at an external temperature of 80°C for seven hours. The reaction solution was left to cool to room temperature and triethyl phosphite was evaporated under reduced pressure. Then, the residue was purified by silica gel column chromatography (elution solvent: heptane:ethyl acetate = 1:1 -> ethyl acetate:ethanol = 19:1) to obtain 10.28 g of the title compound. The property values of the compound are as follows.
$^1$H-NMR (DMSO-$D_6$) δ (ppm): 1.17-1.27 (m, 6H), 1.42, 1.44 (each d, J = 7.2 Hz, 3H), 1.45-2.01 (m, 4H), 2.65-2.82 (m, 1H), 3.08-3.28 (m, 2H), 3.98-4.12 (m, 4H), 5.79-5.89 (m, 1H), 7.17 (dd, J = 8.8, 8.8 Hz, 2H), 7.28-7.36 (m, 2H).

Synthesis of 3-methoxy-4-(5-methyltetrazol-2-yl)benzaldehyde and 3-methoxy-4-(5-methyltetrazol-1-yl)benzaldehyde

Sodium hydroxide powder (260 mg) was added to a solution of 4-fluoro-3-methoxybenzaldehyde [CAS #128495-46-5] (1.0 g) and 5-methyltetrazole [CAS #4076-36-2] (546 mg) in DMF (10 mL). The reaction solution was stirred at 90°C for 1.5 hours and at 120°C for 2.5 hours and then concentrated under reduced pressure. Ethyl acetate and water were added to the residue and the organic layer was separated. The organic layer was sequentially washed with water, 1 N hydrochloric acid, a saturated sodium bicarbonate solution and brine, dried over anhydrous magnesium sulfate and then concentrated under reduced pressure. The resulting residue was purified by silica gel column chromatography (elution solvent: toluene:ethyl acetate = 49:1) to obtain 39 mg of 3-methoxy-4-(5-methyltetrazol-2-yl)benzaldehyde from the fraction of the elution solvent. The property values of the compound are as follows.
$^1$H-NMR ($CDCl_3$) δ (ppm): 2.67 (s, 3H), 3.98 (s, 3H), 7.62 (dd, J = 1.6, 8.0 Hz, 1H), 7.65 (d, J = 1.6 Hz, 1H), 7.78 (d, J = 8.0 Hz, 1H), 10.08 (s, 1H).
Further, 146 mg of 3-methoxy-4-(5-methyltetrazol-1-yl)benzaldehyde was obtained from the fraction of the elution solvent (toluene:ethyl acetate = 4:1 -> 3:1). The property values of the compound are as follows.
$^1$H-NMR ($CDCl_3$) δ (ppm): 2.49 (s, 3H), 3.94 (s, 3H), 7.60 (d, J = 8.0 Hz, 1H), 7.64 (d, J = 1.6 Hz, 1H), 7.66 (dd, J = 1.6, 8.0 Hz, 1H), 10.10 (s, 3H).

Synthesis of (E)-1-[(S)-1-(4-fluorophenyl)ethyl]-3-[1-[3-methox-4-(5-methltetrazol-1-yl)phenyl]methylidene]piperidin-2-one

Lithium hydroxide monohydrate powder (97 mg) was added to a solution of 3-methoxy-4-(5-methyltetrazol-1-yl)benzaldehyde (100 mg) and diethyl {1-[(S)-1-(4-fluorophenyl)ethyl]-2-oxopiperidin-3-yl}phosphonate (205 mg) in THF (3 mL)-ethanol (0.3 mL). The reaction solution was stirred at room temperature for five hours. Ethyl acetate and water were added to the reaction solution, and the organic layer was separated. The organic layer was washed with brine, dried over anhydrous magnesium sulfate and then concentrated under reduced pressure. The resulting residue was purified by silica gel column chromatography using NH silica gel (elution solvent: heptane:ethyl acetate = 2:1 -> 1:1) and then triturated with ethyl acetate-diisopropyl ether to obtain 162 mg of the title compound. The property values of the compound are as follows.
$^1$H-NMR ($CDCl_3$) δ (ppm): 1.56 (d, J = 7.2 Hz, 3H), 1.64-1.76 (m, 1H), 1.79-1.90 (m, 1H), 2.47 (s, 3H), 2.70-2.86 (m,

2H), 2.92-3.00 (m, 1H), 3.22-3.30 (m, 1H), 3.83 (s, 3H), 6.23 (q, J = 7.2 Hz, 1H), 7.04 (dd, J = 8.0, 8.8 Hz, 2H), 7.07 (d, J = 1.6 Hz, 1H), 7.12 (dd, J = 1.6, 8.0 Hz, 1H), 7.33 (dd, J = 5.6, 8.0 Hz, 2H), 7.37 (d, J = 8.0 Hz, 1H), 7.92 (s, 1H).

Example 2

Synthesis of (E)-1-[(S)-1-(4-fluorophenyl)ethyl]-3-[1-[3-methox-4-(5-methltetrazol-2-yl)phenyl]methylidene]piperidin-2-one

[Formula 80]

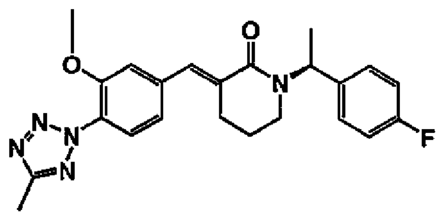

Lithium hydroxide monohydrate powder (34 mg) was added to a solution of 3-methoxy-4-(5-methyltetrazol-2-yl)benzaldehyde obtained in Example 1 (35 mg) and diethyl {1-[(S)-1-(4-fluorophenyl)ethyl]-2-oxopiperidin-3-yl}phosphonate obtained in Example 1 (72 mg) in THF (1 mL)-ethanol (0.1 mL). The reaction solution was stirred at room temperature for 7.5 hours. Ethyl acetate and water were added to the reaction solution, and the organic layer was separated. The resulting organic layer was washed with brine, dried over anhydrous magnesium sulfate and then concentrated under reduced pressure. The resulting residue was purified by silica gel column chromatography using NH silica gel (elution solvent: heptane:ethyl acetate = 1:0 -> 3:1) and then purified again by LC-MS. The organic solvent of the objective fraction was evaporated under reduced pressure and then the aqueous layer was extracted with ethyl acetate. The organic layer was sequentially washed with a saturated sodium bicarbonate solution and brine, dried over anhydrous magnesium sulfate and then concentrated under reduced pressure to obtain 35 mg of the title compound. The property values of the compound are as follows. $^1$H-NMR ($CDCl_3$) $\delta$ (ppm): 1.56 (d, J = 7.2 Hz, 3H), 1.64-1.76 (m, 1H), 1.79-1.90 (m, 1H), 2.66 (s, 3H), 2.70-2.86 (m, 2H), 2.91-2.99 (m, 1H), 3.21-3.30 (m, 1H), 3.88 (s, 3H), 6.23 (q, J = 7.2 Hz, 1H), 7.04 (dd, J = 8.0, 8.4 Hz, 2H), 7.09 (s, 1H), 7.11 (d, J = 8.8 Hz, 1H), 7.33 (dd, J = 5.6, 8.4 Hz, 2H), 7.53 (d, J = 8.8 Hz, 1H), 7.92 (s, 1H). ESI-MS; m/z 422 [$M^+$ + H].

Example 3

Synthesis of (E)-N-(9H-fluoren-9-yl)-3-[3-methoxy-4-(3-methyl-1H-[1,2,4]triazol-1-yl)phenyl]acrylamide

[Formula 81]

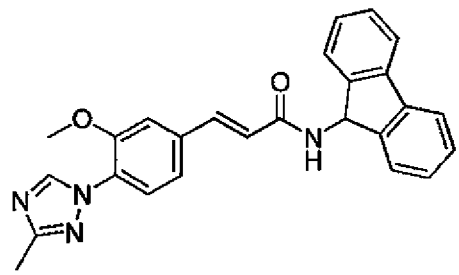

Synthesis of 3-methoxy-4-(3-methyl-1H-[1,2,4]triazol-1-yl)benzaldehyde

A solution of 3-methyl-1H-[1,2,4]triazole [described in Collect. Czech. Chem. Commun., 1984, vol.49, p.2492] (383 mg) and 4-fluoro-3-methoxybenzaldehyde (711 mg) in DMF (10 mL) was stirred at 90°C overnight. Then, potassium carbonate (1.20 g) was added to the reaction solution, and the reaction solution was stirred at 110°C for 6.5 hours. Water and ethyl acetate were added to the reaction solution, and the organic layer was separated. The resulting organic layer was washed with brine, dried over anhydrous magnesium sulfate and then concentrated under reduced pressure. The residue was purified by silica gel column chromatography (elution solvent: heptane-ethyl acetate system) to obtain 82.5 mg of the title compound. The property values of the compound are as follows.
$^1$H-NMR ($CDCl_3$) δ (ppm): 2.50 (s, 3H), 4.05 (s, 3H), 7.59 (d, J = 8.4 Hz, 1H), 7.60 (s, 1H), 8.07 (d, J = 8.4 Hz, 1H), 8.84 (s, 1H), 9.99 (s, 1H).

Synthesis of (E)-N-(9H-fluoren-9-yl)-3-[3-methoxy-4-(3-methyl-1H-[1,2,4]triazol-1-yl)phenyl]acrylamide

Triethyl phosphonoacetate (88 μL) and lithium hydroxide monohydrate (18.5 mg) were added to a solution of 3-methoxy-4-(3-methyl-1H-[1,2,4]triazol-1-yl)benzaldehyde (80.0 mg) in THF (3.0 mL), and the reaction solution was stirred at room temperature for one hour and 45 minutes. After confirming that the raw materials were eliminated, a 2 N sodium hydroxide solution (3.0 mL) was added to the reaction solution. The reaction solution was stirred at room temperature overnight and then stirred at 60°C for five hours and 15 minutes. 2 N hydrochloric acid and ethyl acetate were added to the reaction solution, and the organic layer was separated. The resulting organic layer was washed with brine, dried over anhydrous magnesium sulfate and then concentrated under reduced pressure to obtain 125 mg of a crude cinnamic acid compound.
9-Aminofluorene monohydrochloride [CAS #5978-75-6] (55.2 mg), IPEA (147 μL), HOBT (34.4 mg) and EDC (48.6 mg) were sequentially added to a solution of the resulting crude cinnamic acid compound (125 mg) in DMF (1.0 mL), and the reaction solution was stirred at room temperature overnight. The reaction solution was purified by LC-MS to obtain 12.0 mg of the title compound. The property values of the compound are as follows.
$^1$H-NMR (DMSO-d6) δ (ppm): 2.35 (s, 3H), 3.94 (s, 3H), 6.17 (d, J = 8.0 Hz, 1H), 6.79 (d, J = 16 Hz, 1H), 7.30-7.37 (m, 3H), 7.43-7.50 (m, 3H), 7.54-7.65 (m, 2H), 7.65 (d, J = 16 Hz, 1H), 7.70 (d, J = 8.4 Hz, 1H), 7.89 (d, J = 7.6 Hz, 2H), 8.80 (d, J = 8.0 Hz, 1H), 8.86 (s, 1H).

Example 4

Synthesis of (E)-1-[(S)-1-(4-fluorophenyl)ethyl]-3-[1-[3-methoxy-4-(3-methyl[1,2,4]triazol-1-yl)phenyl]methylidene]piperidin-2-one

[Formula 82]

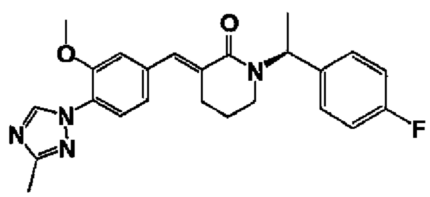

Synthesis of methyl 4-hydrazino-3-methoxybenzoate

A solution of sodium nitrite (3.36 g) in water was added dropwise to a suspension of methyl 4-amino-3-methoxybenzoate [CAS #41608-64-4] (8.4 g) in concentrated hydrochloric acid (84 mL) at -20°C while maintaining the internal temperature at -7°C or less. The reaction solution was stirred at -20°C for 15 minutes and at 0°C for 20 minutes. Then, the reaction solution was added dropwise to a solution of Tin(II) chloride dihydrate (39.3 g) in concentrated hydrochloric acid (285 mL) cooled to -20°C while maintaining the internal temperature at -5°C or less. The reaction solution was stirred at -20°C for 10 minutes and at room temperature for 40 minutes. After cooling the reaction solution with ice, the

precipitate was collected by filtration and the collected product was washed with ice-cold water and then with diethyl ether. The collected product was suspended in ethyl acetate and a potassium carbonate solution was added. After stirring, the insoluble matter was removed by filtration through celite. The organic layer of the filtrate was separated and then the aqueous layer was extracted with ethyl acetate (twice). The combined organic layers were dried over anhydrous magnesium sulfate and then concentrated under reduced pressure. The resulting powder was triturated with ethyl acetate-diisopropyl ether to obtain 6.12 g of the title compound. The property values of the compound are as follows.
$^1$H-NMR ($CDCl_3$) δ (ppm): 3.87 (s, 3H), 3.88 (s, 3H), 6.96 (d, J = 8.4 Hz, 1H), 7.42 (d, J = 2.0 Hz, 1H), 7.68 (dd, J = 2.0, 8.4 Hz, 1H).

Synthesis of methyl thioacetimidate hydroiodide

Methyl iodide (14.3 mL) was added dropwise to a suspension of thioacetamide [CAS #62-55-5] (7.5 g) in diethyl ether (200 mL), and the reaction solution was stirred at room temperature for four days. The precipitated crystals were collected by filtration, washed with diethyl ether and then dried under reduced pressure to obtain 21.35 g of the title compound. The property values of the compound are as follows.
$^1$H-NMR ($CDCl_3$) δ (ppm): 2.83 (s, 3H), 2.95 (s, 3H).

Synthesis of methyl 3-methoxy-4-(3-methyl[1,2,4]triazol-1-yl)benzoate

Methyl thioacetimidate hydroiodide (5.66 g) was added to a suspension of methyl 4-hydrazino-3-methoxybenzoate (5.1 g) in methanol (50 ml). The reaction solution was stirred at room temperature for 30 minutes and then concentrated under reduced pressure. Trimethyl orthoformate (25 mL) and pyridine (50 mL) were added to a suspension of the resulting residue in toluene (50 mL), and the reaction solution was stirred at 100°C overnight. The reaction solution was left to cool to room temperature and concentrated under reduced pressure. A half-saturated sodium bicarbonate solution and ethyl acetate were added to the residue, and the organic layer was separated. The resulting organic layer was sequentially washed with water and brine, dried over anhydrous magnesium sulfate and then concentrated under reduced pressure. The resulting residue was purified by silica gel column chromatography (elution solvent: toluene:ethyl acetate = 9:1 -> 4:1) to obtain 5.56 g of the title compound. The property values of the compound are as follows.
$^1$H-NMR ($CDCl_3$) δ (ppm): 2.50 (s, 3H), 3.96 (s, 3H), 4.02 (s, 3H), 7.74-7.80 (m, 2H), 7.93 (d, J = 8.0 Hz, 1H), 8.81 (s, 1H).

Synthesis of 3-methoxy-4-(3-methyl[1,2,4]triazol-1-yl)benzaldehyde

A solution of methyl 3-methoxy-4-(3-methyl[1,2,4]triazol-1-yl)benzoate (65 mg) in THF (3 mL) was added dropwise to a suspension of LAH (15 mg) in THF (1 mL) under ice-cooling. The reaction solution was stirred at room temperature for 50 minutes. The reaction solution was ice-cooled and then quenched with water and a 5 N sodium hydroxide solution. After adding methylene chloride to the reaction solution, the insoluble matter was removed by filtration through celite and the filtrate was concentrated under reduced pressure. Dess-Martin reagent (203 mg) was added to a solution of the resulting residue in methylene chloride (2 mL), and the reaction solution was stirred at room temperature for two hours. A saturated sodium bicarbonate solution and sodium thiosulfate were sequentially added to the reaction solution, followed by extraction with ethyl acetate. The organic layer was dried over anhydrous magnesium sulfate and then concentrated under reduced pressure. The resulting residue was purified by silica gel column chromatography (elution solvent: toluene:ethyl acetate = 3:1) to obtain 45 mg of the title compound. The property values of the compound are as follows.
$^1$H-NMR ($CDCl_3$) δ (ppm): 2.51 (s, 3H), 4.05 (s, 3H), 7.58-7.64 (m, 2H), 8.08 (d, J = 7.2 Hz, 1H), 8.85 (s, 1H), 10.01 (s, 1H).

Synthesis of tert-butyl 5-chloro-2-(diethoxyphosphoryl)valerate

Oily 60% sodium hydride (4.36 g) was washed with hexane (three times) to remove the oily component. A solution of tert-butyl diethylphosphonoacetate [CAS# 27784-76-5] (25 g) in THF (35 mL) was added dropwise to a suspension of the sodium hydride in THF (150 mL) at room temperature, and the reaction solution was stirred at the same temperature for three hours. A solution of 1-bromo-3-chloropropane [CAS #109-70-6] (31.2 g) in THF (35 mL) was added dropwise to the reaction solution, and then the reaction solution was heated under reflux overnight. The reaction solution was left to cool to room temperature. A saturated ammonium chloride solution was added, followed by extraction with ethyl acetate. The organic layer was sequentially washed with water and brine, dried over anhydrous magnesium sulfate and then concentrated under reduced pressure. The resulting residue was purified by silica gel column chromatography (elution solvent: heptane:ethyl acetate = 2:1) to obtain 17.2 g of the title compound. The property values of the compound are as follows.
$^1$H-NMR ($CDCl_3$) δ (ppm): 1.31-1.48 (m, 6H), 1.48 (s, 9H), 1.79-2.14 (m, 4H), 2.73-2.91 (m, 1H), 3.55 (t, J = 6.4 Hz, 2H),

4.10-4.19 (m, 4H).

Synthesis of tert-butyl (E)-5-chloro-2-[1-[3-methoxy-4-(3-methyl[1,2,4]triazol-1-yl)phenyl]methylidene]valerate

Lithium hydroxide monohydrate powder (0.95 g) was added to a solution of 3-methoxy-4-(3-methyl[1,2,4]triazol-1-yl)benzaldehyde (1.5 g) and tert-butyl 5-chloro-2-(diethoxyphosphoryl)valerate (2.4 g) in THF (15 mL)-ethanol (15 mL). The reaction solution was stirred at room temperature for 1.5 hours. Ethyl acetate and water were added to the reaction solution, and the organic layer was separated. The resulting organic layer was sequentially washed with water and brine, dried over anhydrous magnesium sulfate and then concentrated under reduced pressure. Diisopyl ether and hexane were added to the resulting residue. After removing the insoluble matter, the filtrate was concentrated under reduced pressure. The resulting residue was purified by silica gel column chromatography using NH silica gel (elution solvent: heptane:ethyl acetate = 19:1 -> 4:1). The resulting crystals were triturated with diethyl ether-hexane to obtain 1.15 g of the title compound. The property values of the compound are as follows.
$^1$H-NMR ($CDCl_3$) δ (ppm): 1.56 (s, 9H), 1.98-2.08 (m, 2H), 2.50 (s, 3H), 2.65-2.72 (m, 2H), 3.59 (t, J = 6.0 Hz, 2H), 3.96 (s, 3H), 7.04 (d, J = 1.2 Hz, 1H), 7.09 (dd, J = 1.2, 8.4 Hz, 1H), 7.61 (s, 1H), 7.81 (d, J = 8.4 Hz, 1H), 8.71 (s, 1H).

Synthesis of (E)-5-chloro-2-[1-[3-methoxy-4-(3-methyl[1,2,4]triazol-1-yl)phenyl]methylidene]valeric acid trifluoroacetate

Trifluoroacetic acid (2 mL) was added to a solution of tert-butyl (E)-5-chloro-2-[1-[3-methoxy-4-(3-methyl[1,2,4] triazol-1-yl)phenyl]methylidene]valerate (1.11 g) in methylene chloride (4 mL). The reaction solution was stirred at room temperature for one day. The reaction solution was concentrated under reduced pressure. The resulting powder was triturated with diethyl ether to obtain 1.26 g of the title compound. The property values of the compound are as follows.
$^1$H-NMR (DMSO-d6) δ (ppm): 1.92-2.02 (m, 2H), 2.36 (s, 3H), 2.58-2.66 (m, 2H), 3.70 (t, J = 6.4 Hz, 2H), 3.93 (s, 3H), 7.20 (d, J = 8.0 Hz, 1H), 7.30 (s, 1H), 7.67 (s, 1H), 7.71 (d, J = 8.0 Hz, 1H), 8.87 (s, 1H).

Synthesis of (E)-5-chloro-2-[1-[3-methoxy-4-(3-methyl[1,2,4]triazol-1-yl)phenyl]methylidene]valeric acid [(S)-1-(4-fluorophenyl)ethyl]amide

HOBT (90 mg), IPEA (0.25 ml) and EDC (128 mg) were sequentially added to a solution of (E)-5-chloro-2-[1-[3-methoxy-4-(3-methyl[1,2,4]triazol-1-yl)phenyl]methylidene]valeric acid trifluoroacetate (200 mg) and (S)-1-(4-fluorophenyl)ethylamine (93 mg) in DMF (3 mL). Then, the reaction solution was stirred at room temperature for 2.5 hours. Ethyl acetate and a half-saturated sodium bicarbonate solution were added to the reaction solution and then the organic layer was separated. The resulting organic layer was sequentially washed with a saturated sodium bicarbonate solution and brine, dried over anhydrous magnesium sulfate and then concentrated under reduced pressure. The resulting residue was purified by silica gel column chromatography using NH silica gel (elution solvent: heptane:ethyl acetate = 3:1 -> 1:1) to obtain 176 mg of the title compound. The property values of the compound are as follows.
$^1$H-NMR ($CDCl_3$) δ (ppm): 1.56 (d, J = 7.2 Hz, 3H), 1.95-2.05 (m, 2H), 2.49 (s, 3H), 2.65-2.75 (m, 2H), 3.57 (t, J = 6.0 Hz, 2H), 3.94 (s, 3H), 5.21 (qd, J = 7.2, 7.2 Hz, 1H), 6.15 (d, J = 7.2 Hz, 1H), 6.98 (d, J = 2.0 Hz, 1H), 7.02 (dd, J = 2.0, 8.0 Hz, 1H), 7.05 (dd, J = 8.4, 8.8 Hz, 2H), 7.18 (s, 1H), 7.35 (dd, J = 5.2, 8.4 Hz, 2H), 7.79 (d, J = 8.0 Hz, 1H), 8.67 (s, 1H).

Synthesis of (E)-1-[(S)-1-(4-trifluorophenyl)ethyl]-3-[1-[3-methoxy-4-(3-methyl[1,2,4]triazol-1-yl)phenyl]methylidene] piperidin-2-one

Oily 60% sodium hydride (22 mg) was added to a solution of (E)-5-chloro-2-[1-[3-methoxy-4-(3-methyl[1,2,4] triazol-1-yl)phenyl]methylidene]valeric acid [(S)-1-(4-fluorophenyl)ethyl]amide (231 mg) in DMF (5 mL) under ice-cooling, and then the reaction solution was stirred at room temperature for 15 minutes. Water was added to the reaction solution under ice-cooling, followed by extraction with ethyl acetate. The resulting organic layer was washed with brine, dried over anhydrous magnesium sulfate and then concentrated under reduced pressure. The resulting residue was purified by silica gel column chromatography using NH silica gel (elution solvent: heptane: ethyl acetate = 3:1 -> 1:1) to obtain 142 mg of the title compound. The property values of the compound are as follows.
$^1$H-NMR ($CDCl_3$) δ (ppm): 1.55 (d, J = 7.2 Hz, 3H), 1.62-1.88 (m, 2H), 2.49 (s, 3H), 2.70-2.87 (m, 2H), 2, 90-2.98 (m, 1H), 3.20-3.28 (m, 1H), 3.93 (s, 3H), 6.22 (q, J = 7.2 Hz, 1H), 7.04 (dd, J = 8.4, 8.8 Hz, 2H), 7.06 (d, J = 1.6 Hz, 1H), 7.10 (dd, J = 1.6, 8.4 Hz, 1H), 7.31 (dd, J = 5.2, 8.4 Hz, 2H), 7.77 (d, J = 8.4 Hz, 1H), 7.88 (s, 1H), 8.67 (s, 1H).

Example 5

Synthesis of (E)-1-[(S)-1-(4-fluorophenyl)ethyl]-3-{1-[3-methoxy-4-(4-methyl[1,2,3]triazol-1-yl)phenyl]methylidene}piperidin-2-one

[Formula 83]

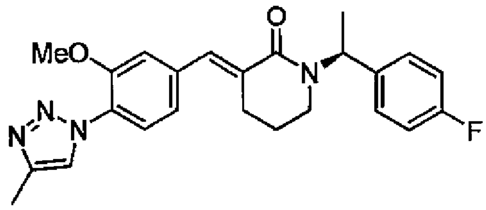

Synthesis of methyl 3-methoxy-4-(4-methyl[1,2,3]triazol-1-yl)benzoate

10 mg of the title compound was obtained from α,α-dichloroacetone tosylhydrazone (100 mg) and methyl 4-amino-3-methoxybenzoate (185 mg) according to the method described in Bull. Chem. Soc. Jpn., 1986, vol.59, p. 179-183. The property values of the compound are as follows.
$^1$H-NMR ($CDCl_3$) δ (ppm): 2.45 (d, J = 0.8 Hz, 3H), 3.97 (s, 3H), 3.98 (s, 3H), 7.75-7.85 (m, 1H), 7.79 (dd, J = 8.0, 1.6 Hz, 1H), 7.95 (d, J = 8.0 Hz, 1H), 7.97 (d, J = 0.8 Hz, 1H).

Synthesis of 3-methoxy-4-(4-methyl[1,2,3]triazol-1-yl)benzaldehyde

74 mg of the title compound was obtained from methyl 3-methoxy-4-(4-methyl[1,2,3]triazol-1-yl)benzoate (90 mg) by the same method as in Example 4. The property values of the compound are as follows.
$^1$H-NMR ($CDCl_3$) δ (ppm): 2.46 (s, 3H), 4.01 (s, 3H), 7.60-7.64 (m, 2H), 8.02 (d, J = 0.8 Hz, 1H), 8.10 (d, J = 8.4 Hz, 1H), 10.0 (s, 1H).

Synthesis of (E)-1-[(S)-1-(4-fluorophenyl)ethyl]-3-{1-[3-methoxy-4-(4-methyl[1,2,3]triazol-1-yl)phenyl]methylidene}piperidin-2-one

116 mg of the title compound was obtained from 3-methoxy-4-(4-methyl[1,2,3]triazol-1-yl)benzaldehyde (74 mg) and diethyl {1-[(S)-1-(4-fluorophenyl)ethyl]-2-oxopiperidin-3-yl}phosphonate obtained in Example 1 (122 mg) by the same method as in Example 1. The property values of the compound are as follows.
$^1$H-NMR ($CDCl_3$) δ (ppm): 1.57 (d, J = 7.2 Hz, 3H), 1.62-1.75 (m, 1H), 1.78-1.88 (m, 1H), 2.45 (d, J = 0.4 Hz, 3H), 2.71-2.98 (m, 3H), 3.21-3.29 (m, 1H), 3.91 (s, 3H), 6.24 (q, J = 7.2 Hz, 1H), 7.01-7.08 (m, 3H), 7.12 (dd, J = 8.4, 1.6 Hz, 1H), 7.30-7.35 (m, 2H), 7.81 (d, J = 8.4 Hz, 1H), 7.88 (d, J = 0.4 Hz, 1H), 7.91 (brs, 1H).

Example 6

Synthesis of (E)-3-[1-[3-methoxy-4-(3-methyl[1,2,4]triazol-1-yl)phenyl]methylidene]-1-[(S)-1-(3,4,5-trifluorophenyl)ethyl]piperidin-2-one

[Formula 84]

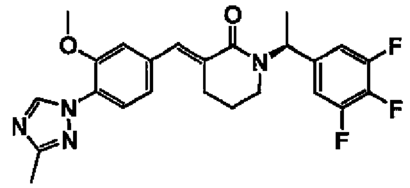

Synthesis of (R)-1-(3,4,5-trifluorophenyl)ethanol

3,4,5-Trifluoroacetophenone [CAS #220141-73-1] (5.0 g) was added dropwise to a solution of (+)-DIP-chloride™ [CAS #112246-73-8] (11.8 g) in THF (200 mL) at -30°C. The reaction solution was stirred at the same temperature for five hours and at room temperature for one hour and then concentrated under reduced pressure. Diethanolamine (6.5 mL) was added dropwise to a solution of the resulting residue in diethyl ether (150 mL), and the reaction solution was stirred at room temperature overnight. The insoluble matter was removed by filtration and then the filtrate was concentrated under reduced pressure. Hexane was added to the resulting residue, and the insoluble matter was removed by filtration again. Then, the filtrate was purified by silica gel column chromatography (developing solvent: heptane:ethyl acetate = 19:1 to 4:1) to obtain 3.69 g of the title compound. The property values of the compound are as follows.
$^1$H-NMR ($CDCl_3$) δ (ppm): 1.46 (d, J = 6.8 Hz, 3H), 4.85 (q, J = 6.8, 1H), 6.98-7.05 (m, 2H).

Synthesis of 5-[(S)-1-azidoethyl]-1,2,3-trifluorobenzene

DBU (4.1 mL) was added dropwise to a solution of (R)-1-(3,4,5-trifluorophenyl)ethanol (3.6 g) and diphenylphosphoryl azide (6.0 mL) in toluene (70 mL) under ice-cooling. Then, the reaction solution was stirred at the same temperature for one hour and at room temperature overnight. Water was added to the reaction solution and the organic layer was separated. Then, the aqueous layer was reextracted with toluene. The combined organic layers were sequentially washed with 1 N hydrochloric acid, water, a saturated sodium bicarbonate solution and brine, dried over anhydrous magnesium sulfate and then concentrated under reduced pressure. The resulting residue was purified by silica gel column chromatography (elution solvent: heptane:ethyl acetate = 49:1) to obtain 858 mg of the title compound. The property values of the compound are as follows.
$^1$H-NMR ($CDCl_3$) δ (ppm): 1.50 (d, J = 6.8 Hz, 3H), 4.56 (q, J = 6.8, 1H), 6.92-7.01 (m, 2H).

Synthesis of (S)-1-(3,4,5-trifluorophenyl)ethylamine

Triphenylphosphine (1.23 g) was added to a solution of 5-[(S)-1-azidoethyl]-1,2,3-trifluorobenzene (858 mg) in THF (20 mL), and the reaction solution was stirred at room temperature for five minutes. Thereafter, water (2.5 mL) was added to the reaction solution, and the reaction solution was then stirred at 60°C for 2.5 hours. Ethyl acetate was added to the reaction solution, followed by extraction with 2 N hydrochloric acid (twice). The hydrochloric acid extraction layer was washed with ethyl acetate and then the aqueous layer was made basic with a 5 N sodium hydroxide solution, followed by extraction with methylene chloride (twice). The methylene chloride layer was dried over anhydrous magnesium sulfate and concentrated under reduced pressure to obtain 348 mg of a crude product of the title compound.
Further, the following operation was performed in order to collect the title compound remaining in the reaction solution diluted with ethyl acetate. Diethyl ether was added to the reaction solution diluted with ethyl acetate, followed by extraction with water. The water extraction layer was washed with diethyl ether and then the aqueous layer was made basic with a 5 N sodium hydroxide solution, followed by extraction with methylene chloride (twice). The methylene chloride layer was dried over anhydrous magnesium sulfate and concentrated under reduced pressure to obtain 413 mg of a crude product of the title compound.
The property values of the compounds are as follows.
$^1$H-NMR ($CDCl_3$) δ (ppm): 1.33 (d, J = 6.4 Hz, 3H), 4.08 (q, J = 6.4, 1H), 6.95-7.04 (m, 2H).

Synthesis of (E)-3-[1-[3-methoxy-4-(3-methyl[1,2,4]triazol-1-yl)phenyl]methylidene]-1-[(S)-1-(3,4,5-trifluorophenyl)ethyl]piperidin-2-one

199 mg of the title compound was obtained from (S)-1-(3,4,5-trifluorophenyl)ethylamine (172 mg) and (E)-5-chloro-2-[1-[3-methoxy-4-(3-methyl[1,2,4]triazol-1-yl)phenyl]methylidene]valeric acid trifluoroacetate obtained in Example 4 (300 mg) by the same method as in Example 4. The property values of the compound are as follows.
$^1$H-NMR ($CDCl_3$) δ (ppm): 1.53 (d, J = 7.2 Hz, 3H), 1.65-1.85 (m, 2H), 2.50 (s, 3H), 2.70-2.80 (m, 1H), 2, 84-3.00 (m, 2H), 3.25-3.33 (m, 1H), 3.95 (s, 3H), 6.16 (q, J = 7.2 Hz, 1H), 6.93-7.04 (m, 2H), 7.07 (s, 1H), 7.11 (d, J = 8.4 Hz, 1H), 7.80 (d, J = 8.4 Hz, 1H), 7.89 (s, 1H), 8.70 (s, 1H).

Examples 7 and 8

Synthesis of (E)-1-[(R)-1-(2,6-difluoropyridin-3-yl)ethyl]-3-[1-[3-methoxy-4-(3-methyl[1,2,4]triazol-1-yl)phenyl]methylidene]piperidin-2-one (Example 7) and synthesis of (E)-1-[(S)-1-(2,6-difluoropyridin-3-yl)ethyl]-3-[1-[3-methoxy-4-(3-methyl[1,2,4]triazol-1-yl)phenyl]methylidene]piperidin-2-one (Example 8)

[Formula 85]

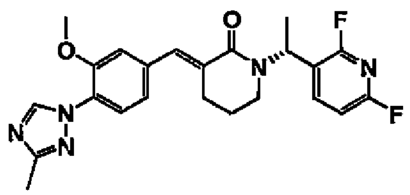

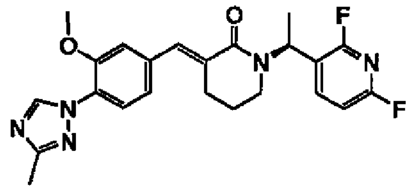

Synthesis of 2,6-difluoronicotinic acid

A 2.62 M solution of n-butyllithium in THF (29.1 mL) was added dropwise to a solution of diisopropylamine (11.7 mL) in tetrahydrofuran (310 mL) under ice-cooling in a nitrogen atmosphere, and the reaction solution was stirred under ice-cooling for one hour. After cooling the reaction solution to -78°C, a solution of 2,6-difluoropyridine [CAS# 1513-65-1] (8 g) in tetrahydrofuran (10 mL) was added dropwise to the reaction solution, and the reaction solution was stirred at -78°C for three hours. Then, an excessive amount of crushed dry ice was added to the reaction solution in a nitrogen stream, and the reaction solution was stirred at -78°C for 20 minutes and at room temperature for three hours. Water and diethyl ether were added to the reaction solution, and the aqueous layer was separated. The aqueous layer was adjusted to pH 1 with concentrated hydrochloric acid. Then, ethyl acetate was added and the organic layer was separated. The ethyl acetate layer was dried over anhydrous magnesium sulfate and concentrated under reduced pressure to obtain 10.4 g of a crude product of the title compound. The property values of the compound are as follows.
$^1$H-NMR ($CD_3OD$) δ (ppm): 7.08 (dd, J = 8.4, 2.8 Hz, 1H) 8.58 (dd, J = 17.2, 8.4 Hz, 1H).

Synthesis of 2,6-difluoro-N-methoxy-N-methylnicotinamide

N,O-dimethylhydroxylamine hydrochloride (14.7 g), HOBT (20.4 g) and EDC (28.9 g) were sequentially added to a solution of 2,6-difluoronicotinic acid (6 g) and IPEA (10 mL) in DMF (100 mL), and the reaction solution was stirred at room temperature for two days. Water and ethyl acetate were added to the reaction solution, and the organic layer was separated. The organic layer was dried over anhydrous magnesium sulfate and concentrated under reduced pressure. The resulting residue was purified by silica gel column chromatography using NH silica gel (elution solvent: ethyl acetate) to obtain 7.01 g of the title compound. The property values of the compound are as follows.
$^1$H-NMR ($CDCl_3$) δ (ppm): 3.37 (s, 3H), 3.58 (brs, 3H), 6.90 (dd, J = 8.0, 2.8 Hz, 1H) 8.02 (dd, J = 16.0, 8.0 Hz, 1H).

1-(2,6-Difluoropyridin-3-yl)ethanone

A 0.96 M solution of methylmagnesium bromide in THF (88.1 mL) was added to a solution of 2,6-difluoro-N-methoxy-N-methyl-nicotinamide (7.01 g) in THF (180 mL) under ice-cooling, and the reaction solution was stirred at the same temperature for two hours. A saturated ammonium chloride solution and ethyl acetate were added to the reaction solution under ice-cooling, and the organic layer was separated. The organic layer was washed with brine, dried over anhydrous magnesium sulfate and then concentrated under reduced pressure. The residue was purified by silica gel

column chromatography (elution solvent: hexane:ethyl acetate = 7:3) to obtain 4.74 g of the title compound. The property values of the compound are as follows.
$^1$H-NMR ($CDCl_3$) δ (ppm): 2.05 (s, 3H), 6.93-6.97 (m, 1H), 8.46-8.52 (m, 1H).

Synthesis of (S)-1-(2,6-difluoropyridin-3-yl)ethylamine

1.70 g of the title compound was obtained from 1-(2,6-difluoropyridin-3-yl)ethanone (3.1 g) by the same method as in Example 6.
The compound had an optical purity of >85% ee. The property values of the compound are as follows.
$^1$H-NMR ($CDCl_3$) δ (ppm): 1.42 (d, J = 6.8 Hz, 1H), 4.39 (q, J = 6.8 Hz, 1H), 6.82 (dd, J = 8.0, 2.8 Hz, 1H), 8.02 (dd, J = 17.2, 8.0 Hz, 1H).

Synthesis of (E)-1-[(R)-1-(2,6-difluoropyridin-3-yl)ethyl]-3-[1-[3-methoxy-4-(3-methyl-[1,2,4]triazol-1-yl)phenyl]methylidenelpiperidin-2-one and synthesis of (E)-1-[(S)-1-(2,6-difluoropyridin-3-yl)ethyl]-3-[1-[3-methoxy-4-(3-methyl-[1,2,4]triazol-1-yl)phenyl]methylidene]piperidin-2-one

26 mg of an enantiomeric mixture of the title compound was obtained from (S)-1-(2,6-difluoropyridin-3-yl)ethylamine (17 mg) and (E)-5-chloro-2-[1-[3-methoxy-4-(3-methyl[1,2,4]triazol-1-yl)phenyl]methylidene]valeric acid trifluoroacetate obtained in Example 4 (40 mg) by the same method as in Example 4. The resulting enantiomeric mixture was separated by CHIRALPAK™ AD-H manufactured by Daicel Chemical Industries, Ltd. (2 cm x 25 cm; mobile phase: ethanol) to obtain 1.3 mg of the title compound with a retention time of 17 minutes (Example 7) and 17 mg of the title compound with a retention time of 31 minutes (Example 8).
The title optically active compound with a retention time of 17 minutes has a positive optical rotation, and the title optically active compound with a retention time of 31 minutes has a negative optical rotation. The property values of the compounds are as follows.
$^1$H-NMR ($CDCl_3$) δ (ppm): 1.66 (d, J = 7.2 Hz, 3H), 1.80-1.95 (m, 2H), 2.50 (s, 3H), 2.75-2.82 (m, 2H), 3.17-3.25 (m, 1H), 3.40-3.48 (m, 1H), 3.93 (s, 3H), 5.84 (q, J = 7.2 Hz, 1H), 6.85 (dd, J = 2.8, 8.0 Hz, 1H), 7.03 (d, J = 1.6 Hz, 1H), 7.08 (dd, J = 1.6, 8.0 Hz, 1H), 7.78 (d, J = 8.0 Hz, 1H), 7.80 (s, 1H), 7.98 (ddd, J = 8.0, 8.0, 8.0 Hz, 1H), 8.70 (s, 1H).

Example 9

Synthesis of (Z)-4-[(S)-1-(4-fluorophenyl)ethyl]-2-[1-[3-methoxy-4-(3-methyl[1,2,4]triazol-1-yl)phenyl]methylidene]-6,6-dimethylmorpholin-3-one

[Formula 86]

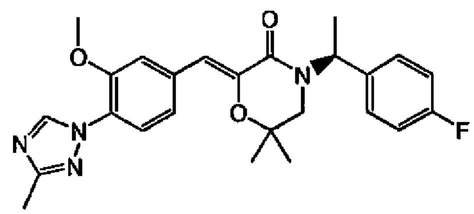

Synthesis of 1-[(S)-1-(4-fluorophenyl)ethylamino]-2-methylpropan-2-ol

Isobutylene oxide [CAS #558-30-5] (1.0 g) and (S)-1-(4-fluorophenyl)ethylamine (2.25 mL) were added to a solution of lithium perchlorate (14.8 g) in diethyl ether (27.8 mL) at room temperature, and the reaction solution was stirred at the same temperature for 1.5 hours. Isobutylene oxide (0.5 mL) was further added to the reaction solution, and the reaction solution was stirred overnight. Ice water and chloroform were added to the reaction solution, and the organic layer was separated. The organic layer was dried over anhydrous magnesium sulfate and then concentrated under reduced pressure. The resulting residue was purified by silica gel column chromatography (elution solvent: chloroform:2-propanol = 100:1 -> 1:1) to obtain 2.13 g of the title compound. The property values of the compound are as

follows.
$^{1}$H-NMR ($CDCl_3$) δ (ppm): 1.13 (s, 3H), 1.16 (s, 3H), 1.35 (d, J = 6.8 Hz, 3H), 2.32 (d, J = 11.6 Hz, 1H), 2.44 (d, J = 11.6 Hz, 1H), 3.75 (q, J = 6.8 Hz, 1H), 6.99-7.10 (m, 2H), 7.23-7.30 (m, 2H).

Synthesis of 4-[(S)-1-(4-fluorophenyl)ethyl]-6,6-dimethylmorpholine-2,3-dione

A mixture of 1-[(S)-1-(4-fluorophenyl)ethylamino]-2-methylpropan-2-ol (2.13 g) and diethyl oxalate (7.0 mL) was heated at 170°C for one hour. The reaction solution was concentrated under reduced pressure. Then, diethyl ether was added to the residue, and the precipitated crystals were collected by filtration. The crystals were air-dried to obtain 1.44 g of the title compound. The property values of the compound are as follows.
$^{1}$H-NMR ($CDCl_3$) δ (ppm): 1.19 (s, 3H), 1.44 (s, 3H), 1.56 (d, J = 6.8 Hz, 3H), 3.00 (d, J = 13.6 Hz, 1H), 3.31 (d, J = 13.6 Hz, 1H), 6.02 (q, J = 6.8 Hz, 1H), 7.06-7.10 (m, 2H), 7.30-7.36 (m, 2H).

Synthesis of 4-[(S)-1-(4-fluorophenyl)ethyl]-2-hydroxy-6,6-dimethylmorpholin-3-one

A 1 M solution of lithium tri-sec-butylborohydride in THF (4.97 mL) was added dropwise to a solution of 4-[(S)-1-(4-fluorophenyl)ethyl]-6,6-dimethylmorpholine-2,3-dione (1.20 g) in THF at -15°C, and the reaction solution was stirred at the same temperature for two hours. A 5 N sodium hydroxide solution (0.45 mL) and 30% aqueous hydrogen peroxide (154 uL) were added dropwise to the reaction solution at 20°C or less, and the reaction solution was then stirred at 10°C for one hour. Sodium bisulfite (141 mg) was added to the reaction solution, and the reaction solution was stirred for 30 minutes. Brine and chloroform were added to the reaction solution, and the organic layer was separated. The organic layer was dried over anhydrous magnesium sulfate and then concentrated under reduced pressure. The residue was purified by silica gel column chromatography (elution solvent: heptane:ethyl acetate = 1:1 -> 0:1) to obtain 1.22 g of the title compound. The property values of the compound are as follows.
$^{1}$H-NMR ($CDCl_3$) δ (ppm): 0.97 (s, 1.5H), 1.08 (s, 1.5H), 1.24 (s, 1.5H), 1.31 (s, 1.5H), 1.52 (d, J = 6.8 Hz, 1.5H), 1.53 (d, J = 6.8 Hz, 1.5H), 2.05 (s, 3H), 2.79 (d, J = 12.8 Hz, 0.5H), 2.87 (d, J = 12.8 Hz, 0.5H), 3.08 (d, J = 12.8 Hz, 0.5H), 3.13 (d, J = 12.8 Hz, 0.5H), 3.77 (brs, 1H), 5.26 (d, J = 4.0 Hz, 0.5H), 5.29 (d, J = 4.0 Hz, 0.5H), 5.93 (q, J = 6.8 Hz, 0.5H), 5.99 (q, J = 6.8 Hz, 0.5H), 7.03-7.07 (m, 2H), 7.26-7.35 (m, 2H).

Synthesis of (Z)-4-[(S)-1-(4-fluorophenyl)ethyl]-2-[1-[3-methoxy-4-(3-methyl[1,2,4]triazol-1-yl)phenyl]methylidene]-6,6-dimethylmorpholin-3-one

Thionyl chloride (0.66 mL) was added to a solution of 4-[(S)-1-(4-fluorophenyl)ethyl]-2-hydroxy-6,6-dimethylmorpholin-3-one (161 mg) in methylene chloride, and the reaction solution was stirred at 50°C for two hours. The reaction solution was concentrated under reduced pressure. The residue was diluted with methylene chloride and then triphenylphosphine (212 mg) was added under ice-cooling. The reaction solution was stirred at room temperature for three hours and then concentrated under reduced pressure. Ethanol (1.0 mL), TEA (0.07 mL) and 3-methoxy-4-(3-methyl [1,2,4]triazol-1-yl)benzaldehyde obtained in Example 4 (43 mg) were added to one-third of the resulting residue. The reaction solution was heated under reflux for two hours and then concentrated under reduced pressure. The residue was purified by silica gel column chromatography using NH silica gel (elution solvent: heptane:ethyl acetate = 1:1 -> 0: 1) and purified again by HPLC using CHIRALPAK™ IA column manufactured by Daicel Chemical Industries, Ltd. to obtain 25 mg of the title compound. The property values of the compound are as follows.
$^{1}$H-NMR ($CDCl_3$) δ (ppm): 1.19 (s, 3H), 1.42 (s, 3H), 1.55 (d, J = 7.2 Hz, 3H), 2.49 (s, 3H), 2.89 (d, J = 12.8 Hz, 1H), 3.24 (d, J = 12.8 Hz, 1H), 3.92 (s, 3H), 6.18 (q, J = 7.2 Hz, 1H), 6.92 (s, 1H), 7.04-7.08 (m, 2H), 7.32-7.36 (m, 2H), 7.38 (dd, J = 8.4, 2.0 Hz, 1H), 7.57 (d, J = 2.0 Hz, 1H), 7.72 (d, J = 8.4 Hz, 1H), 8.66 (s, 1H).

Example 10

Synthesis of (Z)-4-(4-fluorobenzyl)-2-[l-[3-methoxy-4-(3-methyl[1,2,4]triazol-1-yl)phenyl]methylidene]-6,6-dimethylmorpholin-3-one

[Formula 87]

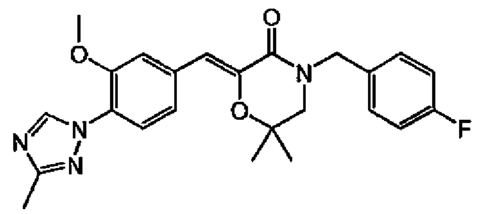

29 mg of the title compound was obtained by the same method as in Example 9 from 133 mg of 4-(4-fluorobenzyl)-2-hydroxy-6,6-dimethylmorpholin-3-one prepared from 4-fluorobenzylamine [CAS #149-75-0] and isobutylene oxide by the same method as in Example 9 as an intermediate material. The property values of the compound are as follows. $^1$H-NMR ($CDCl_3$) δ (ppm): 1.38 (s, 6H), 2.51 (s, 3H), 3.33 (s, 2H), 3.93 (s, 3H), 4.68 (s, 2H), 6.94 (s, 1H), 7.02-7.07 (m, 2H), 7.29-7.33 (m, 2H), 7.40 (dd, J = 8.4, 2.0 Hz, 1H), 7.58 (d, J = 2.0 Hz, 1H), 7.74 (d, J = 8.4 Hz, 1H), 8.74 (s, 1H). ESI-MS; m/z 437 [$M^+$ + H].

Example 11

Synthesis of (Z)-(6S)-4-(4-fluorobenz1)-2-[1-[3-methoxy-4-(3-methyl[1,2,4]triazol-1-yl)phenyl]methylidene]-6-methylmorpholin-3-one

[Formula 88]

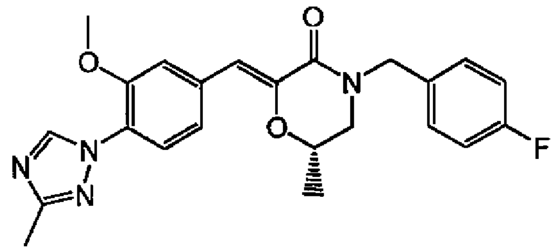

7.7 mg of the title compound was obtained by the same method as in Example 9 from 104 mg of (6S)-4-(4-fluorobenzyl)-2-hydroxy-6-methylmorpholin-3-one prepared from 4-fluorobenzylamine and (S)-(-)-propylene oxide [CAS #16088-62-3] by the same method as in Example 9 as an intermediate material. The property values of the compound are as follows. $^1$H-NMR ($CDCl_3$) δ (ppm): 1.42 (d, J = 6.0 Hz, 3H), 2.49 (s, 3H), 3.26 (dd, J = 12.8, 2.8 Hz, 1H), 3.43 (dd, J = 12.8, 10.4 Hz, 1H), 3.93 (s, 3H), 4.35 (ddq, J = 10.4, 6.0, 2.8 Hz, 1H), 4.63 (d, J = 14.8 Hz, 1H), 4.73 (d, J = 14.8 Hz, 1H), 6.91 (s, 1H), 7.01-7.08 (m, 2H), 7.27-7.32 (m, 2H), 7.40 (dd, J = 8.4, 1.6 Hz, 1H), 7.56 (d, J = 1.6 Hz, 1H), 7.74 (d, J = 8.4 Hz, 1H), 8.69 (s, 1H).

Example 12

Synthesis of (Z)-4-[(S)-chroman-4-yl]-2-[1-[3-methoxy-4-(3-methyl[1,2,4]triazol-1-yl)phenyl]methylidene]-6,6-dimethylmorpholin-3-one

[Formula 89]

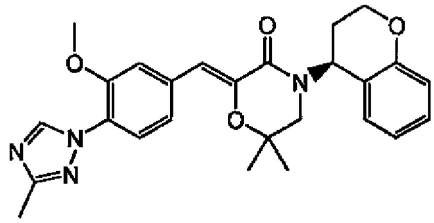

1.1 g of 4-[(S)-chroman-4-yl]-2-hydroxy-6,6-dimethylmorpholin-3-one was obtained from (S)-chroman-4-ylamine [CAS #188198-38-1] (4.13 g) and isobutylene oxide by the same method as in Example 9.
A solution of 196 mg of the compound and triphenylphosphine hydrobromide (292 mg) in acetonitrile (10 mL) was heated under reflux for two hours. The reaction solution was concentrated under reduced pressure, and a part of the resulting residue (61 mg) was dissolved in ethanol (1.0 mL). 3-Methoxy-4-(3-methyl[1,2,4]triazol-1-yl)benzaldehyde (22 mg) obtained in Example 4 and TEA (0.035 mL) were added. The reaction solution was stirred at 70 to 80°C for two hours and then concentrated under reduced pressure. The resulting residue was purified by silica gel column chromatography using NH silica gel (elution solvent: heptane:ethyl acetate = 1:1 -> 0:1) and purified again by HPLC using CHIRALPAK™ AD-H manufactured by Daicel Chemical Industries, Ltd. (2 cm x 25 cm) to obtain 28 mg of the title compound. The property values of the compound are as follows.
$^1$H-NMR ($CDCl_3$) δ (ppm): 1.42 (s, 3H), 1.44 (s, 3H), 2.10-2.25 (m, 2H), 2.50 (s, 3H), 3.12 (d, J = 12.8 Hz, 1H), 3.19 (d, J = 12.8 Hz, 1H), 3.94 (s, 3H), 4.21-4.35 (m, 2H), 6.13 (t, J = 7.2 Hz, 1H), 6.86-6.95 (m, 3H), 7.10 (d, J = 7.6 Hz, 1H), 7.20 (t, J = 7.6 Hz, 1H), 7.41 (dd, J = 8.0, 1.2 Hz, 1H), 7.59 (d, J = 1.2 Hz, 1H), 7.75 (d, J = 8.0 Hz, 1H), 8.69 (s, 1H).
Examples 13 and 14

Synthesis of (Z)-(65)-4-[(S)-chroman-4-yl]-2-[1-[3-methoxy-4-(3-methyl[1,2,4]triazol-1-yl)phenyl]methylidene]-6-methylmorpholin-3-one (Example 13) and synthesis of (Z)-(6R)-4-[(S)-chroman-4-1]-2-[1-[3-methoxy-4-(3-methyl[1,2,4]triazol-1-yl)phenyl]methylidene]-6-methylmorpholin-3-one (Example 14)

[Formula 90]

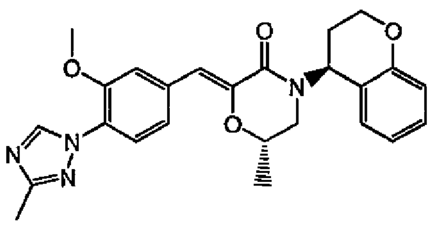

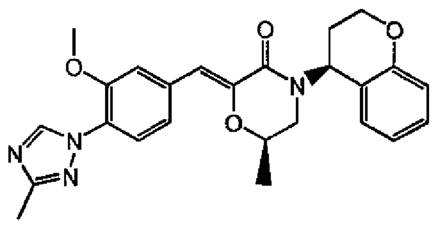

The title compound was obtained as a diastereomeric mixture by the same method as in Example 12 from 65 mg of 4-[(S)-chroman-4-yl]-2-hydroxy-6-methylmorpholin-3-one prepared from (S)-chroman-4-ylamine and (±)-propylene oxide [CAS #75-56-9] by the same method as in Example 9 as an intermediate material. The mixture was separated by CHIRALPAK™ IA column manufactured by Daicel Chemical Industries, Ltd. (2 cm x 25 cm; mobile phase: ethanol) to obtain 7.9 mg of the isomer with a retention time of 20 minutes and 12 mg of the isomer with a retention time of 24 minutes. The property values of the title isomer with a retention time of 20 minutes (Example 13) are as follows.
$^1$H-NMR ($CDCl_3$) δ (ppm): 1.41 (d, J = 6.0 Hz, 3H), 2.10-2.28 (m, 2H), 2.49 (s, 3H), 3.08 (dd, J = 13.2, 3.2 Hz, 1H), 3.17 (dd, J = 13.2, 9.6 Hz, 1H), 3.94 (s, 3H), 4.24-4.38 (m, 3H), 6.07 (t, J = 5.6 Hz, 1H), 6.86-6.95 (m, 3H), 7.07 (d, J = 7.2 Hz, 1H), 7.20 (t, J = 7.2 Hz, 1H), 7.42 (dd, J = 8.4, 2.0 Hz, 1H), 7.58 (d, J = 2.0 Hz, 1H), 7.75 (d, J = 8.4 Hz, 1H), 8.67 (s, 1H).
The property values of the title isomer with a retention time of 24 minutes (Example 14) are as follows.
$^1$H-NMR ($CDCl_3$) δ (ppm): 1.41 (d, J = 6.4 Hz, 3H), 2.11-2.20 (m, 2H), 2.51 (s, 3H), 3.09 (dd, J = 12.8, 2.4 Hz, 1H), 3.33

(dd, J = 12.8, 10.4 Hz, 1H), 3.94 (s, 3H), 4.23-4.37 (m, 3H), 6.14 (t, J = 8.8 Hz, 1H), 6.86-6.94 (m, 3H), 7.06 (d, J = 7.6 Hz, 1H), 7.19 (t, J = 7.6 Hz, 1H), 7.42 (dd, J = 8.4, 1.6 Hz, 1H), 7.59 (d, J = 1.6 Hz, 1H), 7.76 (d, J = 8.4 Hz, 1H), 8.72 (s, 1H).

Example 15

Synthesis of (Z)-(6S)-4-(6-chloropyridin-2-ylmethyl)-2-[1-[3-methoxy-4-(3-methyl[1,2,4]triazol-1-yl)phenyl]methylidene]-6-methylmorpholin-3-one

[Formula 91]

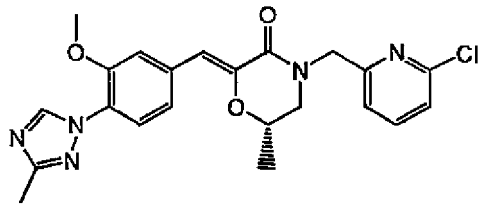

21 mg of the title compound was obtained by the same method as in Example 9 from 59 mg of (6S)-4-(6-chloropyridin-2-ylmethyl)-2-hydroxy-6-methylmorpholin-3-one prepared from (6-chloropyridin-2-yl)methylamine [CAS# 188637-75-4] and (S)-(-)-propylene oxide by the same method as in Example 9 as an intermediate material. The property values of the compound are as follows.
$^1$H-NMR ($CDCl_3$) δ (ppm): 1.47 (d, J = 6.4 Hz, 3H), 2.49 (s, 3H), 3.56 (dd, J = 12.8, 2.8 Hz, 1H), 3.68 (dd, J = 12.8, 10.0 Hz, 1H), 3.93 (s, 3H), 4.44 (ddq, J = 10.0, 6.4, 2.8 Hz, 1H), 4.76 (s, 2H), 6.86 (s, 1H), 7.27 (d, J = 8.0 Hz, 1H), 7.33 (d, J = 8.0 Hz, 1H), 7.38 (dd, J = 8.4, 1.2 Hz, 1H), 7.57 (d, J = 1.2 Hz, 1H), 7.66 (t, J = 8.0 Hz, 1H), 7.74 (d, J = 8.4 Hz, 1H), 8.68 (s, 1H).

Example 16

Synthesis of (Z)-(6S)-4-[(S)-1-(6-chloropyridin-3-yl)ethyl]-2-[1-[3-methoxy-4-(3-methyl[1,2,4]triazol-1-yl)phenyl]methylidene]-6-methylmorpholin-3-one

[Formula 92]

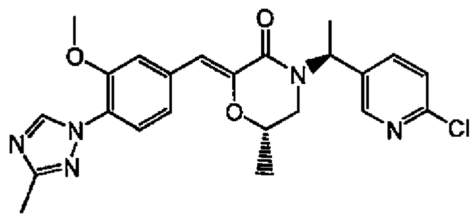

11 mg of the title compound was obtained by the same method as in Example 12, from 66 mg of (6S)-4-[(S)-1-(6-chloropyridin-3-yl)ethyl]-2-hydroxy-6-methylmorpholin-3-one prepared from (S)-1-(6-chloropyridin-3-yl)ethylamine [CAS: 579515-26-] and (S)-(-)-propylene oxide by the same method as in Example 9 as an intermediate material. The property values of the compound are as follows. $^1$H-NMR ($CDCl_3$) δ (ppm): 1.39 (d, J = 6.8 Hz, 3H), 1.62 (d, J = 7.2 Hz, 3H), 2.49 (s, 3H), 2.96 (dd, J = 12.8, 9.6 Hz, 1H), 3.25 (dd, J = 12.8, 2.8 Hz, 1H), 3.93 (s, 3H), 4.37 (m, 1H), 6.13 (q, J = 7.2 Hz, 1H), 6.89 (s, 1H), 7.34 (d, J = 8.4 Hz, 1H), 7.39 (dd, J = 8.4, 1.2 Hz, 1H), 7.54 (d, J = 1.2 Hz, 1H), 7.63 (dd, J

= 8.0, 2.8 Hz, 1H), 7.74 (d, J = 8.0 Hz, 1H), 8.38 (d, J = 2.8 Hz, 1H), 8.67 (s, 1H).
ESI-MS; m/z 454 [$M^+$ + H].

Example 17

Synthesis of (Z)-(6S)-4-[1-(4-fluorophenyl)-1-methylethyl]-2-[1-[3-methoxy-4-(3-methyl[1,2,4]triazol-1-yl)phenyl]methylidene]-6-methylmorpholin-3-one

[Formula 93]

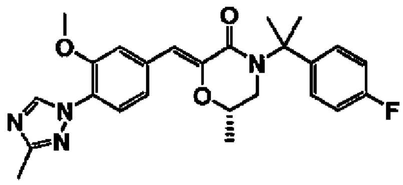

synthesis of (6S)-4-[1-(4-fluorophenyl)-1-methylethyl]-2-hydroxy-6-methylmorpholin-3-one

22 mg of the title compound was obtained from 1-(4-fluorophenyl)-1-methylethylamine [CAS# 17797-10-3] (153 mg) and (S)-(-)-propylene oxide (0.11 mL) by the same method as in Example 9. The property values of the compound are as follows.
$^1$H-NMR ($CDCl_3$) δ (ppm): 1.29 and 1.34 (each d, J = 6.4 Hz, 3H), 1.66 and 1.71 and 1.74 (each s, 6H), 3.20-3.40 (m, 2H), 3.52 (br s, 1H), 4.10-4.35 (m, 1H), 5.05 and 5.15 (d and s, J = 2.8 Hz, 1H), 6.99 (dd, J = 8.8, 8.8 Hz, 2H), 7.27 (dd, J = 8.8, 6.8 Hz, 2H).

Synthesis of (Z)-(6S)-4-[1-(4-fluorophenyl)-1-methylethyl]-2-[1-[3-methoxy-4-(3-methyl[1,2,4]triazol-1-yl)phenyl]methylidene]-6-methylmorpholin-3-one

4.99 mg of the title compound was obtained from (6S)-4-[1-(4-fluorophenyl)-1-methylethyl]-2-hydroxy-6-methylmorpholin-3-one (11 mg) and 3-methoxy-4-(3-methyl[1,2,4]triazol-1-yl)benzaldehyde obtained in Example 4 (11 mg) by the same method as in Example 12. The property values of the compound are as follows.
$^1$H-NMR ($CDCl_3$) δ (ppm): 1.50 (d, J = 6.4 Hz, 3H), 1.76 (s, 3H), 1.77 (s, 3H), 2.49 (s, 3H), 3.50 (dd, J = 13.2, 8.8 Hz), 3.57 (dd, J = 13.2, 3.2 Hz), 3.90 (s, 3H), 4.40 (dqd, J = 8.8, 6.4, 3.2 Hz, 1H), 6.67 (s, 1H), 7.01 (dd, J = 8.8, 8.8 Hz, 2H), 7.26-7.35 (m, 3H), 7.49 (d, J = 1.6 Hz, 1H), 7.70 (d, J = 8.4 Hz, 1H), 8.67 (s, 1H).

Example 18

Synthesis of (Z)-(6S)-4-[(1R,2R)-2-hydroxy-1-(3,4,5-trifluorophenyl)propyl]-2-[1-[3-methoxy-4-(3-methyl[1,2,4]triazol-1-yl)phenyl]methylidene]-6-methylmorpholin-3-one

[Formula 94]

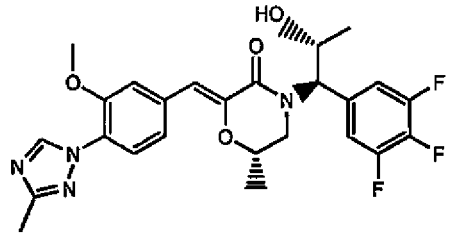

Synthesis of (E)-1,2,3-trifluoro-5-(1-propenyl)benzene

Tetrakistriphenylphosphine palladium (0) (4.66 g) and cesium fluoride (21.4 g) were added to a solution of 1-bromo-3,4,5-trifluorobenzene [CAS #138526-69-9] (8.5 g) and trans-1-propen-1-ylboronic acid (4.1 g) [CAS# 7547-97-9] in dioxane (95 mL) and water (5 mL) in a nitrogen atmosphere, and the reaction solution was stirred at 80°C for five hours. The reaction solution was left to cool to room temperature and water and hexane were added to the reaction solution. The insoluble matter was removed by filtration and then the organic layer was separated. Water was added to the organic layer. The insoluble matter was removed by filtration again and then the organic layer was separated. The organic layer was sequentially washed with water and brine, dried over anhydrous magnesium sulfate and then concentrated under reduced pressure. The resulting crude product was purified by silica gel column chromatography (elution solvent: hexane) to obtain 5.83 g of the title compound. The property values of the compound are as follows.
$^1$H-NMR ($CDCl_3$) δ (ppm): 1.88 (d, J = 6.0 Hz, 3H), 6.18 (qd, J = 6.0, 16.0 Hz, 1H), 6.24 (d, J = 16.0 Hz, 1H), 6.85-6.96 (m, 2H).

Synthesis of (1S,2S)-1-(3,4,5-trifluorophenyl)propane-1,2-diol

(E)-1,2,3-Trifluoro-5-(1-propenyl)benzene (5.83 g) was added to a mixed solution of AD-Mix-α (47.5 g) and methanesulfonamide (3.22 g) in tert-butanol (170 mL) and water (170 mL) under ice-cooling, and the reaction solution was stirred at 5°C overnight. Then, sodium sulfite (51 g) was added to the reaction solution. The reaction solution was stirred at room temperature for one hour and then extracted with methylene chloride three times. The combined organic layers were washed with a 2 N sodium hydroxide solution, and then the sodium hydroxide layer was reextracted with methylene chloride. The combined organic layers were dried over anhydrous magnesium sulfate and then concentrated under reduced pressure. The resulting residue was purified by silica gel column chromatography (elution solvent: hexane: ethyl acetate = 9:1 -> 1:1) to obtain 5.54 g of the title compound. The property values of the compound are as follows.
$^1$H-NMR ($CDCl_3$) δ (ppm): 1.12 (d, J = 6.4 Hz, 3H), 2.20 (br.s, 1H), 2.79 (br.s, 1H), 3.78 (qd, J = 6.4, 6.4 Hz, 1H), 4.34 (d, J = 6.4 Hz, 1H), 6.96-7.05 (m, 2H).

Synthesis of (1R,2S)-1-azido-1-(3,4,5-trifluorophenyl)-propan-2-ol

A sodium hydroxide pellet (110 mg) was added to a mixture of (1S,2S)-1-(3,4,5-trifluorophenyl)-propane-1,2-diol (5.54 g) and dimethyl carbonate (15 mL), and the reaction solution was stirred at 70°C for 45 minutes. Then, the temperature was raised to 100°C and dimethyl carbonate was removed with a nitrogen stream. Further, dimethyl carbonate (5 mL) was added to the residue and then dimethyl carbonate was removed with a nitrogen stream. THF was added to the residue and the insoluble matter was removed by filtration through celite. Then, the filtrate was concentrated under reduced pressure to obtain 6.13 g of a carbonate compound.
Water (0.5 mL) and sodium azide (1.92 g) were added to a solution of the carbonate compound (6.13 g) in DMF (20 mL), and the reaction solution was stirred at 110°C overnight. The reaction solution was returned to room temperature. Diethyl ether and water were added to the reaction solution, and the organic layer was separated. The organic layer was sequentially washed with water (twice) and brine, dried over anhydrous magnesium sulfate and then concentrated under reduced pressure. The resulting residue was purified by silica gel column chromatography (elution solvent: hexane: ethyl acetate = 19:1 -> 9:1) to obtain 5.16 g of the title compound. The property values of the compound are as follows.
$^1$H-NMR ($CDCl_3$) δ (ppm): 1.14 (d, J = 6.4 Hz, 3H), 1.79 (br.s, 1H), 3.97 (qd, J = 6.4, 4.8 Hz, 1H), 4.42 (d, J = 4.8 Hz, 1H), 6.96-7.05 (m, 2H).

Synthesis of tert-butyl [(1R,2S)-2-hydroxy-1-(3,4,5-trifluorophenyl)propyl]carbamate

Triphenylphosphine (5.85 g) was added to a solution of (1R,2S)-1-azido-1-(3,4,5-trifluorophenyl)-propan-2-ol (5.16 g) in THF (75 mL), and the reaction solution was stirred at room temperature for 10 minutes. Thereafter, water (5 ml) was added to the reaction solution, and the reaction solution was stirred at 60°C for 3.5 hours. Then, the reaction solution was left to cool to room temperature. Di-tert-butyl dicarbonate (5.35 g) was added to the reaction solution, and the reaction solution was stirred at the same temperature for 45 minutes. Thereafter, the reaction solution was concentrated under reduced pressure. The resulting residue was purified by silica gel column chromatography (elution solvent: toluene:ethyl acetate = 9:1) to obtain 5.88 g of the title compound. The property values of the compound are as follows.
$^1$H-NMR ($CDCl_3$) δ (ppm): 1.07 (d, J = 6.4 Hz, 3H), 1.41 (s, 9H), 4.10 (br.s, 1H), 4.47 (br.s, 1H), 5.44 (br.s, 1H), 6.92-7.01 (m, 2H).

Synthesis of [(1R,2R)-2-tert-butoxycarbonylamino-1-methyl-2-(3,4,5-trifluorophenyl)ethyl] 4-nitro-benzoate

Diisopropyl azodicarboxylate (6 mL) was added dropwise to a solution of tert-butyl [(1R,2S)-2-hydroxy-1-(3,4,5-trifluorophenyl)propyl]carbamate (5.88 g), 4-nitrobenzoic acid (4.84 g) and triphenylphosphine (7.59 g) in THF (100 mL) under ice-cooling, and then the reaction solution was stirred at room temperature for two hours. The reaction solution was concentrated under reduced pressure. The resulting residue was purified by silica gel column chromatography (elution solvent: toluene:ethyl acetate = 97:3). Then, the resulting powder was triturated with toluene-hexane to obtain 6.69 g of the title compound. The property values of the compound are as follows.
$^1$H-NMR ($CDCl_3$) δ (ppm): 1.37 (s, 9H), 1.38 (d, J = 6.4 Hz, 3H), 4.85 (br.s, 1H), 5.16 (d, J = 9.2 Hz, 1H), 5.41 (qd, J = 6.4, 6.0 Hz, 1H), 6.92-7.01 (m, 2H), 8.16 (d, J = 8.8 Hz, 2H), 8.29 (d, J = 8.8 Hz, 2H).

Synthesis of tert-butyl [(1R,2R)-2-hydroxy-1-(3,4,5-trifluorophenyl)propyl]carbamate

Potassium carbonate powder (6.43 g) was added to a mixed solution of [(1R,2R)-2-tert-butoxycarbonylamino-1-methyl-2-(3,4,5-trifluorophenyl)ethyl] 4-nitrobenzoate (7.03 g) in methanol (90 mL)-THF (10 mL), and the reaction solution was stirred at room temperature for one hour. Ethyl acetate and water were added to the reaction solution, and the organic layer was separated. The organic layer was washed with brine (twice), dried over anhydrous magnesium sulfate and then concentrated under reduced pressure. Diethyl ether was added to the resulting residue, and the insoluble matter was removed by filtration. The filtrate was concentrated and the resulting residue was purified by silica gel column chromatography (elution solvent: toluene:ethyl acetate = 6:1) to obtain 4.49 g of the title compound. The property values of the compound are as follows.
$^1$H-NMR ($CDCl_3$) δ (ppm): 1.28 (d, J = 6.4 Hz, 3H), 1.44 (s, 9H), 4.01 (br.s, 1H), 4.48 (br.s, 1H), 5.35 (br.s, 1H), 6.90-7.00 (m, 2H).
The tert-butyl [(1R,2R)-2-hydroxy-1-(3,4,5-trifluorophenyl)propyl]carbamate was reacted with Mosher's acid chloride to confirm that the compound was approximately a single optically active compound.

Synthesis of tert-butyl [(1R,2R)-2-(tert-butyldiphenylsilanyloxy)-1-(3,4,5-trifluorophenyl)propyl]carbamate

tert-Butyldiphenylsilyl chloride (2.0 mL) was added in four portions to a solution of tert-butyl [(1R,2R)-2-hydroxy-1-(3,4,5-trifluorophenyl)propyl]carbamate (610 mg) and imidazole (817 mg) in DMF (3 mL), and the reaction solution was stirred at room temperature for three hours. Ethyl acetate and water were added to the reaction solution, and the organic layer was separated. The organic layer was sequentially washed with 1 N hydrochloric acid, water, a saturated sodium bicarbonate solution and brine, dried over anhydrous magnesium sulfate and then concentrated under reduced pressure. The resulting residue was purified by silica gel column chromatography (elution solvent: hexane:diethyl ether = 49:1 -> 19:1) to obtain 684 mg of the title compound. The property values of the compound are as follows.
$^1$H-NMR ($CDCl_3$) δ (ppm): 0.95 (s, 9H) 1.13 (d, J = 6.4 Hz, 3H), 1.47 (s, 9H), 4.02 (br.s, 1H), 4.46 (br.s, 1H), 5.34 (br.s, 1H), 6.69-6.80 (m, 2H), 7.28-7.46 (m, 8H), 7.55 (d, J = 8.4 Hz, 2H).

Synthesis of (1R,2R)-2-(tert-butyldiphenylsilanyloxy)-1-(3,4,5-trifluorophenyl)propylamine

Trifluoroacetic acid (0.5 mL) was added to a solution of tert-butyl [(1R,2R)-2-(tert-butyldiphenylsilanyloxy)-1-(3,4,5-trifluorophenyl)propyl]carbamate (370 mg) in methylene chloride (2 mL), and the reaction solution was stirred at room temperature for 11 hours. A saturated sodium bicarbonate solution was added to the reaction solution, followed by extraction with ethyl acetate. The organic layer was washed with a saturated sodium bicarbonate solution and brine, dried over anhydrous magnesium sulfate and then concentrated under reduced pressure to obtain 275 mg of a crude product of the title compound. The property values of the compound are as follows.

$^1$H-NMR ($CDCl_3$) δ (ppm): 0.93 (d, J = 6.4 Hz, 3H), 1.02 (s, 9H), 3.81 (d, J = 4.8 Hz, 1H), 3.91 (dq, J = 4.8, 6.0 Hz, 1H), 6.88-6.97 (m, 2H), 7.32-7.46 (m, 6H), 7.57 (d, J = 8.0 Hz, 2H), 7.55 (d, J = 8.0 Hz, 2H).

Synthesis of (2S)-1-[(1R,2R)-2-(tert-butyldiphenylsilanyloxy)-1-(3,4,5-trifluorophenyl)propylamino]propan-2-ol

A solution of (S)-(-)-propylene oxide (0.1 ml) and (1R,2R)-2-(tert-butyldiphenylsilanyloxy)-1-(3,4,5-trifluorophenyl)propylamine (212 mg) in diethyl ether (1 mL) was added to a suspension of lithium perchlorate (750 mg) in diethyl ether (1 mL), and the reaction solution was stirred in a nitrogen atmosphere at room temperature overnight. Ice water and methylene chloride were added to the reaction solution, and the organic layer was separated. The aqueous layer was reextracted with methylene chloride. Then, the combined organic layers were dried over anhydrous magnesium sulfate and concentrated under reduced pressure. The resulting residue was purified by silica gel column chromatography (elution solvent: heptane:ethyl acetate = 9:1 -> 4:1) to obtain 172 mg of the title compound. The property values of the compound are as follows.
$^1$H-NMR ($CDCl_3$) δ (ppm): 0.83 (d, J = 6.0 Hz, 3H), 1.06 (s, 9H), 1.08 (m, 3H), 2.20-2.50 (m, 3H), 3.47 (br.s, 1H), 3.59 (br.s, 1H), 3.86 (br.s, 1H), 6.78-6.95 (m, 2H), 7.36-7.48 (m, 6H), 7.67 (d, J = 6.8 Hz, 4H).

Synthesis of (6S)-4-[(1R,2R)-2-(tert-butyldiphenylsilanyloxy)-1-(3,4,5-trifluorophenyl)propyl]-6-methylmorpholine-2,3-dione

Oxalyl chloride (45 ul) was added dropwise to a solution of (2S)-1-[(1R,2R)-2-(tert-butyldiphenylsilanyloxy)-1-(3,4,5-trifluorophenyl)propylamino]-propan-2-ol (171 mg), TEA (0.17 mL) and 4-(N,N-dimethylamino)pyridine (8 mg) in methylene chloride (2 mL) under ice-cooling, and the reaction solution was stirred at the same temperature for two hours. Ice water and ethyl acetate were added to the reaction solution, and the organic layer was separated. The organic layer was sequentially washed with water, 1 N hydrochloric acid, water, a saturated sodium bicarbonate solution and brine, dried over anhydrous magnesium sulfate and then concentrated under reduced pressure. The resulting residue was purified by silica gel column chromatography (elution solvent: heptane:ethyl acetate = 9:1 -> 3:1) to obtain 96 mg of the title compound. The property values of the compound are as follows.
$^1$H-NMR ($CDCl_3$) δ (ppm): 1.02 (s, 9H), 1.19 (d, J = 6.0 Hz, 3H), 1.28 (d, J = 6.4 Hz, 3H), 3.20 (dd, J = 5.6, 13.2 Hz, 1H), 3.68 (dd, J = 2.4, 13.2 Hz, 1H), 4.42 (dq, J = 5.6, 6.0 Hz, 1H) 4.62 (ddq, J = 2.4, 5.6, 6.4 Hz, 1H), 5.51 (d, J = 5.6 Hz, 1H), 6.82-6.94 (m, 2H), 7.40-7.54 (m, 6H), 7.62 (d, J = 8.0 Hz, 2H), 7.67 (d, J = 8.0 Hz, 2H).

Synthesis of (6S)-4-[(1R,2R)-2-(tert-butyldiphenylsilanyloxy)-1-(3,4,5-trifluorophenyl)propyl]-2-hydroxy-6-methylmorpholin-3-one

A 1.06 M solution of lithium tri-sec-butylborohydride in THF (0.25 mL) was added dropwise to a solution of (6S)-4-[(1R,2R)-2-(tert-butyldiphenylsilanyloxy)-1-(3,4,5-trifluorophenyl)propyl]-6-methylmorpholine-2,3-dione (95 mg) in THF (3 mL) at -20°C, and the reaction solution was stirred at the same temperature for 30 minutes. A 5 N sodium hydroxide solution (0.03 mL) and 30% aqueous hydrogen peroxide (0.07 mL) were added to the reaction solution, and the reaction solution was stirred under ice-cooling for one hour. Thereafter, sodium bisulfite powder (20 mg) was added to the reaction solution, and the reaction solution was stirred at room temperature for 30 minutes. Brine and ethyl acetate were added to the reaction solution, and the organic layer was separated. The organic layer was washed with brine, dried over anhydrous magnesium sulfate and then concentrated under reduced pressure. The resulting residue was purified by silica gel column chromatography (elution solvent: heptane:ethyl acetate = 1:1) to obtain 93 mg of the title compound. The property values of the compound are as follows.
$^1$H-NMR ($CDCl_3$) δ (ppm): 1.01 (s, 9H), 1.11 (d, J = 6.0 Hz, 3H), 1.19 (d, J = 6.4 Hz, 3H), 2.88 and 2.99 (dd, J = 12.0, 12.0 Hz, 1H), 3.12 and 3.48 (dd, J = 2.4, 12.0 Hz, 1H), 3.16 and 3.91 (d, J = 2.8 Hz, 1H), 4.35-4.55 (m, 2H), 5.11 and 5.30 (d, J = 3.6 Hz, 1H), 5.40 and 5.49 (d, J = 6.8 Hz, 1H), 6.79-6.94 (m, 2H), 7.38-7.54 (m, 6H), 7.65 (d, J = 8.0 Hz, 2H), 7.69 (d, J = 8.0 Hz, 2H).

Synthesis of (Z)-(6S)-4-[(1R,2R)-2-hydroxy-1-(3,4,5-trifluorophenyl)propyl]-2-[1-[3-methoxy-4-(3-methyl[1,2,4]triazol-1-yl)phenyl]methylidene]-6-methylmorpholin-3-one

A solution of (6S)-4-[(1R,2R)-2-(tert-butyldiphenylsilanyloxy)-1-(3,4,5-trifluorophenyl)propyl]-2-hydroxy-6-methylmorpholin-3-one (28.9 mg) and triphenylphosphine hydrobromide (20.8 mg) in acetonitrile (0.6 mL) was heated under reflux for one hour. The reaction solution was concentrated under reduced pressure and the resulting residue was dissolved in ethanol (1 mL).
3-Methoxy-4-(3-methyl[1,2,4]triazol-1-yl)benzaldehyde obtained in Example 4 (3.38 mg) and TEA (0.06 mL) were added to the ethanol solution (0.3 ml), and the reaction solution was stirred at 70 to 80°C for one hour.

The reaction solution was concentrated under reduced pressure. The resulting residue was dissolved in trifluoroacetic acid (1 ml), and the reaction solution was stirred at room temperature for two hours. The reaction solution is poured into a saturated sodium bicarbonate solution, followed by extraction with chloroform. The organic layer was concentrated under reduced pressure. The resulting residue was purified by silica gel column chromatography using NH silica gel (elution solvent: heptane:ethyl acetate = 1:1 -> 0:1) and purified again by HPLC using CHIRALPAK™ IA column manufactured by Daicel Chemical Industries, Ltd. (2 cm x 25 cm) to obtain 3.70 mg of the title compound. The property values of the compound are as follows.

$^1$H-NMR ($CDCl_3$) δ (ppm): 1.33 (d, J = 6.4 Hz, 3H), 1.42 (d, J = 6.4 Hz, 3H), 2.49 (s, 3H), 3.20 (dd, J = 12.8, 10.0 Hz, 1H), 3.59 (dd, J = 12.8, 2.4 Hz, 1H), 3.90 (s, 3H), 4.43-4.49 (m, 2H), 5.35 (d, J = 6.8 Hz, 1H), 6.85 (s, 1H), 7.06-7.13 (m, 2H), 7.37 (dd, J = 8.4, 1.6 Hz, 1H), 7.55 (d, J = 1.6 Hz, 1H), 7.74 (d, J = 8.4 Hz, 1H), 8.67 (s, 1H).

ESI-MS; m/z 503 [$M^+$ + H].

Example 19

Synthesis of (Z)-4-[(1R,2R)-2-hydroxy-1-(3,4,5-trifluorophenyl)propyl]-2-[1-[3-methoxy-4-(3-methyl[1,2,4]triazol-1-yl) phenyl]methylidene]-6,6-dimethylmorpholin-3-one

[Formula 95]

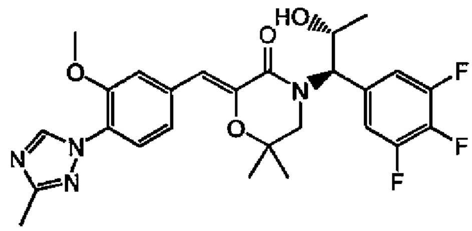

4-[(1R, 2R)- 2-(tert- Butyldiphenylsilanyloxy)- 1-(3,4,5- trifluorophenyl) propyl]- 2- hydroxy- 6,6- dimethylmorpholin- 3- one (520 mg) was obtained from (1R,2R)-2-(tert-butyldiphenylsilanyloxy)-1-(3,4,5-trifluorophenyl)propylamine (704 mg) and isobutylene oxide (212 μL) by the same method as in Example 18 as an intermediate material. 6.75 mg of the title compound was obtained from 50 mg of the compound by the same method as in Example 18. The property values of the compound are as follows.

$^1$H-NMR ($CDCl_3$) δ (ppm): 1.28 (s, 3H), 1.33 (d, J = 6.4 Hz, 3H), 1.46 (s, 3H), 2.29 (d, J = 6.4 Hz, 1H), 2.48 (s, 3H), 3.18 (d, J = 12.8 Hz, 1H), 3.59 (d, J = 12.8 Hz, 1H), 3.91 (s, 3H), 4.45 (sex, J = 6.4 Hz, 1H), 5.37 (d, J = 6.4 Hz, 1H), 6.91 (s, 1H), 7.10-7.13 (m, 2H), 7.36 (dd, J = 8.4, 2.8 Hz, 1H), 7.55 (d, J = 2.8 Hz, 1H), 7.72 (d, J = 8.4 Hz, 1H), 8.65 (s, 1H).

ESI-MS; m/z 517 [$M^+$ + H].

Example 20

Synthesis of (Z)-4-[(1R,2R)-2-hydroxy-1-(3,4,5-trifluorophenyl)propyl]-2-[1-[3-methoxy-4-(3-methyl[1,2,4]triazol-1-yl) phenyl]methylidene]morpholin-3-one

[Formula 96]

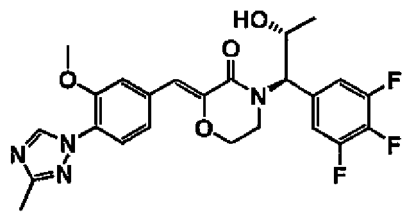

Synthesis of ethyl [(1R,2R)-2-(tert-butyldiphenylsilanyloxy)-1-(3,4,5-trifluorophenyl)propylamino]acetate

Cesium carbonate (1.1 g) and ethyl bromoacetate [CAS #105-36-2] (0.38 g) are added to a solution of (1R, 2R)-2-(tert-butyldiphenylsilanyloxy)-1-(3,4,5-trifluoro-phenyl)propylamine obtained in Example 18 (1.0 g) in DMF (15 ml), and the reaction solution was stirred at room temperature for four hours. Ice water and ethyl acetate were added to the reaction solution, and the organic layer was separated. The organic layer was sequentially washed with half-saturated brine and brine and dried over anhydrous magnesium sulfate, and then the solvent was evaporated under reduced pressure. The resulting residue was purified by silica gel column chromatography (elution solvent: hexane: diethyl ether = 19:1) to obtain 811 mg of the title compound. The property values of the compound are as follows.
$^1$H-NMR ($CDCl_3$) δ (ppm): 0.76 (d, J = 6.4 Hz, 3H), 1.09 (s, 9H), 1.26 (t, J = 7.2 Hz, 3H), 2.97-3.12 (m, 1H), 3.15-3.35 (br s, 1H), 3.50-3.65 (br s, 1H), 3.75-3.95 (br s, 1H), 4.20 (q.J = 7.2 Hz, 2H), 6.85-7.05 (br s, 2H), 7.34-7.48 (m, 6H), 7.63-7.80 (m, 4H).

Synthesis of 2-[(1R,2R)-2-(tert-butyldiphenylsilanyloxy)-1-(3,4,5-trifluorophenyl)propylamino]ethanol

Lithium borohydride (100 mg) was added to a solution of ethyl [(1R,2R)-2-(tert-butyldiphenylsilanyloxy)-1-(3,4,5-trifluorophenyl)propylamino]acetate (810 mg) in THF (15 ml), and the reaction solution was stirred at room temperature for 20 hours. A saturated ammonium chloride solution was added to the reaction solution under ice-cooling. The reaction solution was stirred until foaming stopped, followed by extraction with ethyl acetate (twice). The combined organic layers were washed with brine and dried over anhydrous magnesium sulfate. Then, the solvent was evaporated under reduced pressure. The resulting residue was purified by silica gel column chromatography (elution solvent: heptane:ethyl acetate = 9:1 -> 3:1) to obtain 534 mg of the title compound. The property value of the compound is as follows.
ESI-MS; m/z 488 [$M^+$ + H]

Synthesis of 4-[(1R,2R)-2-(tert-butyldiphenylsilanyloxy)-1-(3,4,5-trifluorophenyl)propyl]morpholine-2,3-dione

A mixture of 2-[(1R,2R)-2-(tert-butyldiphenylsilanyloxy)-1-(3,4,5-trifluorophenyl)propylamino]ethanol (483 mg) and diethyl oxalate (6 ml) was stirred at 170°C for 5.5 hours. The reaction solution was left to cool to room temperature and diethyl oxalate was evaporated under reduced pressure. The resulting residue was purified by silica gel column chromatography (elution solvent: heptane:ethyl acetate = 6:1) to obtain 165 mg of the title compound. The property values of the compound are as follows.
$^1$H-NMR ($CDCl_3$) δ (ppm): 0.99 (s, 9H), 1.18 (d, J = 6.4 Hz, 3H), 1.57 (s, 9H), 3.47 (ddd, J = 8.4, 5.6, 2.8 Hz, 1H), 3.83 (ddd, J = 11.2, 8.4, 3.2 Hz, 1H), 4.29-4.45 (m, 1H), 5.54 (d, J = 5.6 Hz, 1H), 6.81-6.90 (m, 2H), 7.38-7.54 (m, 6H), 7.62 (d, J = 6.8 Hz, 2H), 7.66 (d, J = 6.8 Hz, 2H).

Synthesis of (Z)-4-[(1R,2R)-2-hydroxy-1-(3,4,5-trifluorophenyl)propyl]-2-[1-[3-methoxy-4-(3-methyl[1,2,4]triazol-1-yl) phenyl]methylidene]morpholin-3-one

3.37 mg of the title compound was obtained from 4-[(1R,2R)-2-(tert-butyldiphenylsilanyloxy)-1-(3,4,5-trifluorophenyl)propyl]morpholine-2,3-dione (8 mg) by the same method as in Example 18.
$^1$H-NMR ($CDCl_3$) δ (ppm): 1.34 (d, J = 6.0 Hz, 3H), 2.27 (br s, 1H), 2.50 (s, 3H), 3.40 (m, 1H), 3.78 (m, 1H), 3.93 (s, 3H), 4.16 (m, 1H), 4.29 (m, 1H), 4.49 (dq, J = 6.4, 6.0 Hz, 1H), 5.39 (d, J = 6.4 Hz, 1H), 6.88 (s, 1H), 7.06-7.16 (m, 2H), 7.40-7.45 (m, 2H), 7.74 (d, J = 8.8 Hz, 1H), 8.71 (s, 1H).

Example 21

Synthesis of (E)-1-[(1R,2R)-2-hydroxy-1-(3,4,5-trifluorophenyl)propyl]-3-[1-[3-methoxy-4-(3-methyl[1,2,4]triazol-1-yl)phenyl]methylidene]piperidin-2-one

[Formula 97]

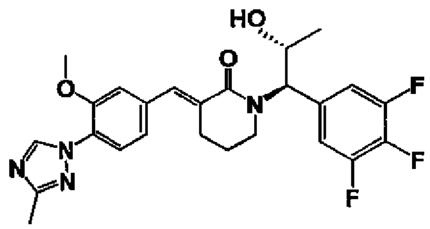

Synthesis of (1R,2R)-1-amino-1-(3,4,5-trifluorophenyl)propan-2-ol hydrochloride

A solution of 4 N hydrogen chloride in dioxane (10 mL) was added to a solution of tert-butyl [(1R,2R)-2-hydroxy-1-(3,4,5-trifluorophenyl)propyl]carbamate obtained in Example 18 (2.85 g) in dioxane (10 mL), and the reaction solution was stirred at room temperature for six hours. Hexane (80 mL) was added dropwise to the reaction suspension, and then the suspension was stirred at room temperature for 20 minutes. The precipitate is collected by filtration and dried under reduced pressure to obtain 2.16 g of the title compound. The property values of the compound are as follows.
$^1$H-NMR (DMSO-$D_6$) δ (ppm): 0.97 (d, J = 6.8 Hz, 3H), 3.93 (qd, J = 6.8, 6.4 Hz, 1H), 4.06 (br s, 1H), 5.73 (br s, 1H), 7.59 (t, J = 7.6 Hz, 2H), 8.51 (br s, 3H).

Synthesis of (E)-1-[(1R,2R)-2-hydroxy-1-(3,4,5-trifluorophenyl)propyl]-3-[1-[3-methoxy-4-(3-methyl[1,2,4]triazol-1-yl)phenyl]methylidene]piperidin-2-one

149 mg of the title compound was obtained from (1R,2R)-1-amino-1-(3,4,5-trifluorophenyl)propan-2-ol hydrochloride (130 mg) and (E)-5-chloro-2-[1-[3-methoxy-4-(3-methyl[1,2,4]triazol-1-yl)phenyl]methylidene]valeric acid trifluoroacetate obtained in Example 4 (200 mg) by the same method as in Example 4. The property values of the compound are as follows.
$^1$H-NMR ($CDCl_3$) δ (ppm): 1.31 (d, J = 6.4 Hz, 3H), 1.75-1.96 (m, 2H), 2.50 (s, 3H), 2.78-2.85 (m, 2H), 3.23-3.30 (m, 1H), 3.50-3.57 (m, 1H), 3.94 (s, 3H), 4.47 (qd, J = 6.4, 6.8 Hz, 1H), 5.26 (d, J = 6.8 Hz, 1H), 7.04-7.14 (m, 4H), 7.80 (d, J = 8.4 Hz, 1H), 7.86 (s, 1H), 8.70 (s, 1H).

Example 22

Synthesis of (E)-1-[(1R,2R)-1-(4-fluorophenyl)-2-hydroxypropyl]-3-{1-[3-methoxy-4-(3-methyl[1,2,4]triazol-1-yl)phenyl]methylidene}piperidin-2-one

[Formula 98]

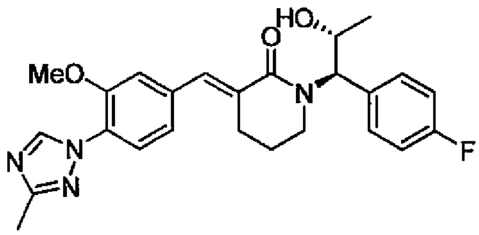

Synthesis of tert-butyl [(1R,2R)-1-(4-fluorophenyl)-2-hydroxypropyl]carbamate

4.44 g of the title compound was obtained from 4-bromofluorobenzene [CAS #460-00-4] (10 g) by the same method as in Example 18. The property values of the compound are as follows.
$^1$H-NMR ($CDCl_3$) δ (ppm): 1.23 (d, J = 6.4 Hz, 3H), 1.42 (brs, 9H), 1.88 (brs, 1H), 4.00 (brs, 1H), 4.53 (brs, 1H), 5.29 (brs, 1H), 7.00-7.06 (m, 2H), 7.22-7.27 (m, 2H).

Synthesis of (1R,2R)-1-amino-1-(4-fluorophenyl)propan-2-ol hydrochloride

81.5 mg of the title compound was obtained from tert-butyl [(1R,2R)-1-(4-fluorophenyl)-2-hydroxypropyl]carbamate (100 mg) by the same method as in Example 21. The property value of the compound is as follows.
ESI-MS; m/z 170 [$M^+$ + H]

Synthesis of 1-[(1R,2R)-1-(4-fluorophenyl)-2-hydroxypropyl]-3-{1-[3-methoxy-4-(3-methyl-[1,2,4]triazol-1-yl)phenyl]-(E)-methylidene}piperidin-2-one

125.7 mg of the title compound was obtained from (1R,2R)-1-amino-1-(4-fluorophenyl)propan-2-ol hydrochloride (80 mg) and (E)-5-chloro-2-[1-[3-methoxy-4-(3-methyl[1,2,4]triazol-1-yl)phenyl]methylidene]valeric acid trifluoroacetate obtained in Example 4 (160 mg) by the same method as in Example 18. The property values of the compound are as follows.
$^1$H-NMR ($CDCl_3$) δ (ppm): 1.29 (d, J = 6.4 Hz, 3H), 1.76-1.88 (m, 2H), 2.49 (s, 3H), 2.74-2.79 (m, 2H), 3.14-3.21 (m, 1H), 3.43-3.50 (m, 1H), 3.93 (s, 3H), 4.51 (dq, J = 8.4, 6.4 Hz, 1H), 5.41 (d, J = 8.4 Hz, 1H), 7.02-7.10 (m, 4H), 7.26-7.37 (m, 2H), 7.78 (d, J = 8.4 Hz, 1H), 7.85 (s, 1H), 8.66 (s, 1H).

Example 23

Synthesis of (Z)-4-[(1R,2R)-1-(4-fluorophenyl)-2-hydroxypropyl]-2-[1-[3-methoxy-4-(3-methyl[1,2,4]triazol-1-yl)phenyl]methylidene]-6,6-dimethylmorpholin-3-one

[Formula 99]

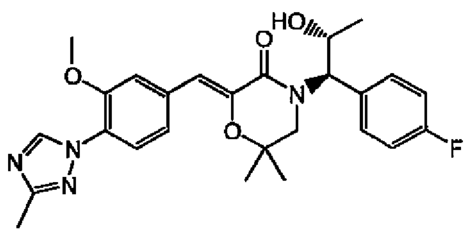

Synthesis of (1R,2R)-2-(tert-butyldiphenylsilanyloxy)-1-(4-fluorophenyl)-2-propylamine

1.04 g of the title compound was obtained from tert-butyl [(1R,2R)-1-(4-fluorophenyl)-2-hydroxypropyl]carbamate obtained in Example 22 (1 g) by the same method as in Example 18. The property values of the compound are as follows.
$^1$H-NMR ($CDCl_3$) δ (ppm): 0.83 (d, J = 6.4 Hz, 3H), 1.03 (s, 9H), 3.85 (d, J = 6.0 Hz, 1H), 3.91 (dq, J = 6.4, 6.0 Hz, 1H), 6.92-6.97 (m, 2H), 7.21-7.25 (m, 2H), 7.31-7.44 (m, 6H), 7.60 (dd, J = 6.0, 1.6 Hz, 2H), 7.67 (dd, J = 6.0, 1.6 Hz, 2H).

Synthesis of (Z)-4-[(1R,2R)-1-(4-fluorophenyl)-2-hydroxypropyl]-2-[1-[3-methoxy-4-(3-methyl[1,2,4]triazol-1-yl)phenyl] methylidene]-6,6-dimethylmorpholin-3-one

30 mg of the title compound was obtained by the same method as in Example 18 from 56 mg of 4-[(1R,2R)-2-(tert-butyldiphenylsilanyloxy)-1-(4-fluorophenyl)propyl]-2-hydroxy-6,6-dimethylmorpholin-3-one prepared from (1R, 2R)-2-(tert-butyldiphenylsilanyloxy)-1-(4-fluorophenyl)propylamine and isobutylene oxide by the same method as in Example 18 as an intermediate material. The property values of the compound are as follows.
$^1$H-NMR ($CDCl_3$) δ (ppm): 1.19 (s, 3H), 1.31 (d, J = 6.0 Hz, 3H), 1.43 (s, 3H), 2.49 (s, 3H), 3.16 (d, J = 12.4 Hz, 1H), 3.54 (d, J = 12.4 Hz, 1H), 3.90 (s, 3H), 4.47 (dq, J = 8.0, 6.0 Hz, 1H), 5.49 (d, J = 8.0 Hz, 1H), 6.90 (s, 1H), 7.03-7.10 (m, 2H), 7.27-7.41 (m, 3H), 7.56 (s, 1H), 7.72 (d, J = 8.4 Hz, 1H), 8.67 (s, 1H). ESI-MS; m/z 481 [$M^+$ + H].

Example 24

Synthesis of (65)-(Z)-4-[(1R,2R)-1-(4-fluorophenyl)-2-hydroxypropyl]-2-[1-[3-methoxy-4-(3-methyl[1,2,4]triazol-1-yl) phenyl]methylidene]-6-methylmorpholin-3-one

[Formula 100]

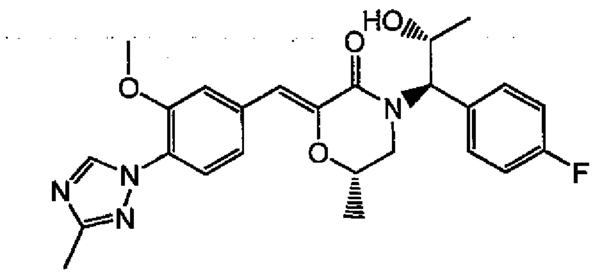

6.57 mg of the title compound was obtained by the same method as in Example 18 from 89 mg of (6S)-4-[(1R,2R)-2-(tert-butyldiphenylsilanyloxy)-1-(4-fluorophenyl)propyl]-2-hydroxy-6-methylmorpholin-3-one prepared from (1R,2R)-2-(tert-butyldiphenylsilanyloxy)-1-(4-fluorophenyl)propylamine obtained in Example 23 and (S)-(-)-propylene oxide by the same method as in Example 18 as an intermediate material. The property values of the compound are as follows.
$^1$H-NMR ($CDCl_3$) δ (ppm): 1.30 (d, J = 5.6 Hz, 3H), 1.38 (d, J = 6.4 Hz, 3H), 2.49 (s, 3H), 3.12 (dd, J = 12.8, 9.6 Hz, 1H), 3.53 (dd, J = 12.8, 2.8 Hz, 1H), 3.88 (s, 3H), 4.42-4.51 (m, 2H), 5.45 (d, J = 8.0 Hz, 1H), 6.83 (s, 1H), 7.05-7.09 (m, 2H), 7.34-7.39 (m, 3H), 7.54 (d, J = 1.2 Hz, 1H), 7.73 (d, J = 8.4 Hz, 1H), 8.68 (s, 1H).
ESI-MS; m/z 467 [$M^+$ + H].

Example 25

Synthesis of (Z)-4-[(1R)-1-(4-fluorophenyl)-2-hydroxyethyl]-2-[1-[3-methoxy-4-(3-methyl[1,2,4]triazol-1-yl)phenyl] methylidene]-6,6-dimethylmorpholin-3-one

[Formula 101]

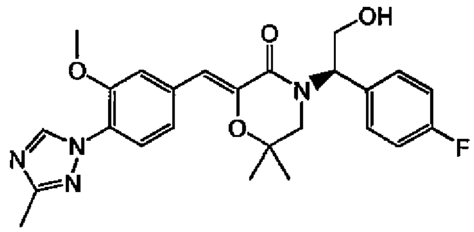

36 mg of the title compound was obtained by the same method as in Example 18 from 93 mg of 4-[(1R)-2-(tert-butyldiphenylsilanyloxy)-1-(4-fluorophenyl)ethyl]-2-hydroxy-6,6-dimethylmorpholin-3-one prepared from (R)-2-amino-2-(4-fluorophenyl)ethanol [CAS: 174770-74-2] by the same method as in Example 18 as an intermediate material. The property values of the compound are as follows.
$^1$H-NMR ($CDCl_3$) δ (ppm): 1.26 (s, 3H), 1.43 (s, 3H), 2.49 (s, 3H), 3.06 (d, J = 12.8 Hz, 1H), 3.38 (d, J = 12.8 Hz, 1H), 3.91 (s, 3H), 4.10-4.23 (m, 2H), 5.87 (dd, J = 8.4, 5.2 Hz, 1H), 6.91 (s, 1H), 7.04-7.10 (m, 2H), 7.28-7.38 (m, 3H), 7.55 (s, 1H), 7.72 (d, J = 8.4 Hz, 1H), 8.68 (s, 1H).
ESI-MS; m/z 467 [$M^+$ + H].

Example 26

Synthesis of (E)-(3S,8aS)-3-(3,4-difluorophenyl)-6-[3-methoxy-4-(3-methyl[1,2,4]triazol-1-yl)benzylidene]hexahydroindolizin-5-one

[Formula 102]

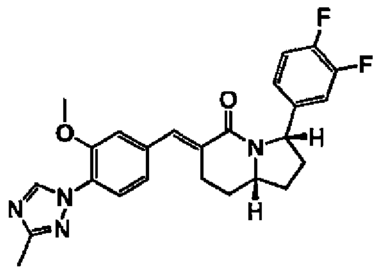

Synthesis of (2R,5S)-5-(2,4-difluorophenyl)pyrrolidine-1,2-dicarboxylic acid 1-tert-butyl ester 2-ethyl ester

A 0.5 M solution of 3,4-difluorophenylmagnesium bromide in THF (80 mL) was added to a solution of (R)-5-oxopyrrolidine-1,2-dicarboxylic acid 1-tert-butyl ester 2-ethyl ester [CAS #144978-35-8] (9 g) in THF (200 mL) at -78°C, and the reaction solution was stirred at -40°C for three hours. Ethyl acetate and a saturated sodium bicarbonate solution were added to the reaction solution, and the organic layer was separated. The resulting organic layer was dried over anhydrous sodium sulfate and concentrated under reduced pressure. The residue was purified by silica gel column chromatography (elution solvent: heptane:ethyl acetate = 1:1) to obtain 9.32 g of ethyl (R)-2-tert-butoxycarbonylamino-5-(3,4-difluorophenyl)-5-oxovalerate.
A solution of 4 N hydrogen chloride in ethyl acetate (120 mL) was added to a solution of the resulting ethyl (R)-2-tert-butoxycarbonylamino-5-(3,4-difluorophenyl)-5-oxovalerate (9.32 g) in ethyl acetate (120 mL), and the reaction solution was stirred at room temperature for 16 hours. The reaction solution was concentrated under reduced pressure. Ethyl acetate and a saturated sodium bicarbonate solution were added to the residue, and the organic layer was separated. The resulting organic layer was dried over anhydrous sodium sulfate and concentrated under reduced pressure. 10% palladium-carbon (50% wet, 200 mg) was added to a solution of the resulting oil in ethyl acetate (50 mL), and the reaction

solution was stirred in a hydrogen atmosphere at room temperature for five hours. The reaction solution was filtered through celite, and the filtrate was concentrated under reduced pressure. Di-tert-butyl dicarbonate (11 g) and TEA (10.5 mL) were added to a solution of the resulting residue in DMF (100 mL), and the reaction solution was stirred at room temperature for 11 hours. Ethyl acetate and 1 N hydrochloric acid were added to the reaction solution, and the organic layer was separated. The resulting organic layer was washed with saturated sodium bicarbonate, dried over anhydrous sodium sulfate and then concentrated under reduced pressure. The residue was purified by silica gel column chromatography (elution solvent: heptane:ethyl acetate = 1:0 -> 3:1) to obtain 6.9 g of the title compound. The property values of the compound are as follows.
$^1$H-NMR ($CDCl_3$) δ (ppm): 1.19 (s, 4.5H), 1.30-1.38 (m, 3H), 1.41 (s, 4.5H), 1.85-1.93 (m, 1H), 1.95-2.08 (m, 1H), 2.17-2.36 (m, 2H), 4.26 (q, J = 7.2 Hz, 2H), 4.33 (t, J = 7.6 Hz, 0.5H), 4.45 (dd, J = 8.0, 4.8 Hz, 0.5H), 4.70 (t, J = 6.8 Hz, 0.5H), 4.91 (dd, J = 7.2, 3.6 Hz, 0.5H), 7.08 (q, J = 8.4 Hz, 1H), 7.20-7.29 (m, 1H), 7.45-7.53 (m, 1H).

Synthesis of tert-butyl (2R,5S)-2-(3,4-difluorophenyl)-5-hydroxymethylpyrrolidine-1-carboxylate

Lithium borohydride (1.7 g) was added to a solution of (2R,5S)-5-(3,4-difluorophenyl)pyrrolidine-1,2-dicarboxylic acid 1-tert-butyl ester 2-ethyl ester (6.9 g) in THF (100 mL) at 0°C, and the reaction solution was stirred at room temperature for two hours. The reaction solution was cooled to 0°C, and a saturated ammonium chloride solution was added to the reaction solution until foaming stopped. Ethyl acetate was added to the solution and the organic layer was separated. The resulting organic layer was dried over anhydrous sodium sulfate and then concentrated under reduced pressure. The residue was purified by silica gel column chromatography (elution solvent: heptane:ethyl acetate = 1:0 -> 1:1) to obtain 5.9 g of the title compound. The property values of the compound are as follows.
$^1$H-NMR ($CDCl_3$) δ (ppm): 1.24 (s, 9H), 1.58-1.70 (m, 1H), 1.77-1.86 (m, 1H), 1.98-2.08 (m, 1H), 2.23-2.32 (m, 1H), 3.52-3.83 (m, 2H), 4.10-4.20 (m, 1H), 4.55-4.68 (m, 1H), 4.78 (t, J = 6.8 Hz, 1H), 6.93-6.99 (m, 1H), 7.02-7.13 (m, 2H).

Synthesis of tert-butyl (2S,5R)-2-(3,4-difluorophenyl)-5-[(E)-2-methoxycarbonylvinyl]pyrrolidine-1-carboxylate

A solution of dimethyl sulfoxide (1.56 g) in methylene chloride (10 mL) was added dropwise to a solution of oxalyl chloride (2.38 g) in methylene chloride (60 mL) at -78°C, and the reaction solution was stirred at the same temperature for 20 minutes. A solution of tert-butyl (2R,5S)-2-(3,4-difluorophenyl)-5-hydroxymethylpyrrolidine-1-carboxylate (3.92 g) in methylene chloride (30 mL) was added dropwise to the reaction solution, and the reaction solution was stirred at -78°C for one hour. TEA (6.8 mL) was added to the reaction solution, and the reaction solution was stirred at 78°C for one hour. The reaction solution was heated to room temperature. Ethyl acetate and 1 N hydrochloric acid were added to the reaction solution, and the organic layer was separated. The resulting organic layer was washed with a saturated sodium bicarbonate solution, dried over anhydrous sodium sulfate and then concentrated under reduced pressure to obtain 3.67 g of a crude product of tert-butyl (2S,5R)-2-(3,4-difluorophenyl)-5-formylpyrrolidine-1-carboxylate. Trimethylphosphonoacetate (4 mL) was added to a mixed suspension of oily 60% sodium hydride (1 g) in THF (70 mL) and DMF (10 mL), and the reaction solution was stirred at room temperature for one hour. A solution of the crude product of tert-butyl (2S,5R)-2-(3,4-difluorophenyl)-5-formylpyrrolidine-1-carboxylate obtained above (3.67 g) in THF (30 mL) was added dropwise to the reaction solution, and the reaction solution was stirred at room temperature for 13 hours. A saturated ammonium chloride solution and ethyl acetate were added to the reaction solution, and the organic layer was separated. The resulting organic layer was dried over anhydrous sodium sulfate and concentrated under reduced pressure. The residue was purified by silica gel column chromatography (elution solvent: heptane:ethyl acetate = 1:0 -> 1: 1) to obtain 3.6 g of the title compound. The property values of the compound are as follows.
$^1$H-NMR ($CDCl_3$) δ (ppm): 1.13-1.50 (brs, 9H), 1.57-1.80 (m, 2H), 2.08-2.18 (m, 1H), 2.24-2.35 (m, 1H), 3.77 (s, 3H), 4.40-4.65 (m, 1H), 4.67-4.92 (m, 1H), 6.02 (brd, J = 15.6 Hz, 1H), 6.93-6.98 (m, 2H), 7.00-7.06 (m, 1H), 7.07-7.14 (m, 1H).

Synthesis of methyl (E)-3-[(2S,5R)-5-(3,4-difluorophenyl)pyrrolidin-2-yl]acrylate

tert-Butyl (2S,5R)-2-(3,4-difluorophenyl)-5-[(E)-2-methoxycarbonylvinyl]pyrrolidine-1-carboxylate (3.6 g) was dissolved in a solution of 4 N hydrogen chloride in ethyl acetate (100 mL), and the reaction solution was stirred at room temperature for one hour. The reaction solution was concentrated under reduced pressure. Ethyl acetate and a saturated sodium bicarbonate solution were added to the residue, and the organic layer was separated. The resulting organic layer was dried over anhydrous sodium sulfate and concentrated under reduced pressure to obtain 2.6 g of a crude product of the title compound. The property values of the compound are as follows.
$^1$H-NMR ($CDCl_3$) δ (ppm): 1.59-1.75 (m, 2H), 2.03-2.21 (m, 2H), 3.75 (s, 3H), 3.92 (q, J = 6.4 Hz, 1H), 4.24 (t, J = 7.2 Hz, 1H), 6.09 (d, J = 15.2 Hz, 1H), 7.02 (dd, J = 15.2, 6.4 Hz, 1H), 7.04-7.12 (m, 2H), 7.23-7.27 (m, 1H).

Synthesis of methyl (E)-3-[(2R,5S)-1-(3-buteno1)-5-(3,4-difluorophenyl)pyrrolidin-2-yl]acrylate

Diethyl cyanophosphonate (4.5 mL) was added to a solution of methyl (E)-3-[(2S,5R)-5-(3,4-difluorophenyl) pyrrolidin-2-yl]acrylate (2.6 g), 3-butenoic acid (2.5 mL) and TEA (8.2 mL) in DMF at 0°C, and the reaction solution was stirred at room temperature for 16 hours. Ethyl acetate and 1 N hydrochloric acid were added to the reaction solution, and the organic layer was separated. The resulting organic layer was washed with a saturated sodium bicarbonate solution, dried over anhydrous sodium sulfate and then concentrated under reduced pressure. The residue was purified by silica gel column chromatography (elution solvent: heptane:ethyl acetate = 4:1 -> 1:1) to obtain 2.5 g of the title compound. The property value of the compound is as follows.
ESI-MS; m/z336 [$M^+$ + H].

Synthesis of (3S,8aR)-3-(3,4-difluorophenyl)-[2,3,6,8a]-tetrahydro-1H-indolizin-5-one

A solution of methyl (E)-3-[(2R,5S)-1-(3-butenoyl)-5-(3,4-difluorophenyl)pyrrolidin-2-yl]acrylate (2.5 g) and tricyclohexylphosphine[1,3-bis(2,4,6-trimethylphenyl)-4,5-dihydroimidazol-2-ylidene][benzylidene]ruthenium (IV) dichloride (318 mg) in methylene chloride (500 mL) was heated under reflux for four hours. The reaction solution was left to cool to room temperature. TEA (1 mL) was added to the reaction solution, and the reaction solution was stirred for one hour. The reaction solution was concentrated under reduced pressure, and the residue was purified by silica gel column chromatography (elution solvent: heptane:ethyl acetate = 1:0 -> 1:1) to obtain 1.77 g of the title compound. The property values of the compound are as follows.
$^1$H-NMR ($CDCl_3$) δ (ppm): 1.77-1.88 (m, 2H), 2.07-2.14 (m, 1H), 2.29-2.41 (m, 1H), 2.74-2.78 (m, 2H), 4.23-4.32 (m, 1H), 5.06 (d, J = 9.2 Hz, 1H), 5.98-6.04 (m, 1H), 6.07-6.12 (m, 1H), 6.80-6.86 (m, 1H), 6.87-6.91 (m, 1H), 7.01-7.08 (m, 1H).

Synthesis of (3S,8aR)-3-(3,4-difluorophenyl)hexahydroindolizin-5-one

Platinum oxide (20 mg) was added to a solution of (3S,8aR)-3-(3,4-difluorophenyl)-[2,3,6,8a]-tetrahydro-1H-indolizin-5-one (1.77 g) in methanol (50 mL), and the reaction solution was stirred in a hydrogen atmosphere at room temperature for 12 hours. The reaction solution was filtered through celite, and the filtrate was concentrated under reduced pressure to obtain 1.87 g of a crude product of the title compound. The property values of the compound are as follows.
$^1$H-NMR ($CDCl_3$) δ (ppm): 1.52-1.70 (m, 2H), 1.71-1.87 (m, 2H), 1.96-2.09 (m, 2H), 2.16-2.25 (m, 2H), 2.27-2.47 (m, 2H), 3.54-3.64 (m, 1H), 5.04 (d, J = 8.8 Hz, 1H), 6.83-6.87 (m, 1H), 6.88-6.94 (m, 1H), 7.02-7.10 (m, 1H).
ESI-MS; m/z 252 [$M^+$ + H].

Synthesis of (3S,8aR)-3-(3,4-difluorophenyl)-6-iodohexahydroindolizin-5-one

Iodotrimethylsilane (0.74 mL) was added to a solution of (3S,8aR)-3-(3,4-difluorophenyl)hexahydroindolizin-5-one (875 mg) and N,N,N',N'-tetramethylethylenediamine (1.8 mL) in methylene chloride (30 mL) at 0°C, and the reaction solution was stirred at 0°C for 30 minutes. Iodine (1.32 g) was added to the reaction solution, and the reaction solution was stirred at 0°C for one hour. A saturated sodium thiosulfate solution and ethyl acetate were added to the reaction solution, and the organic layer was separated. The resulting organic layer was washed with brine, dried over anhydrous sodium sulfate and then concentrated under reduced pressure to obtain 1.3 g of a crude product of the title compound. The property value of the compound is as follows.
ESI-MS; m/z 378 [$M^+$ + H].

Synthesis of diethyl [(3S,8aR)-3-(3,4-difluorophenyl)-5-oxooctahydroindolizin-6-yl]phosphonate

A mixture of (3S,8aR)-3-(3,4-difluorophenyl)-6-iodohexahydroindolizin-5-one (1.3 g) and triethyl phosphite (7 mL) was stirred at 120°C for two hours. The reaction solution was left to cool to room temperature and concentrated under reduced pressure to obtain 1.3 g of a crude product of the title compound. The property value of the compound is as follows.
ESI-MS; m/z 388 [$M^+$ + H].

Synthesis of (E)-(3S,8aS)-3-(3,4-difluorophenyl)-6-[3-methoxy-4-(3-methyl[1,2,4]triazol-1-yl)benzylidene]hexahydroindolizin-5-one

Lithium hydroxide monohydrate powder (34 mg) was added to a mixed solution of diethyl [(3S,8aR)-3-(3,4-difluorophenyl)-5-oxooctahydroindolizin-6-yl]phosphonate (20 mg) and 3-methoxy-4-(3-methyl[1,2,4]triazol-1-yl)benzaldehyde obtained in Example 4 (11 mg) in THF (1 mL)-ethanol (0.1 mL). The reaction solution was stirred at room temperature for one hour. Water was added to the reaction solution, followed by extraction with ethyl acetate (three times). The combined organic layers were concentrated in a nitrogen stream. The resulting residue was purified by LC-MS, and the objective fraction was concentrated under reduced pressure. Ethyl acetate and a saturated sodium bicarbonate solution were added to the resulting residue, and the organic layer was separated. The organic layer was washed with brine, dried over anhydrous magnesium sulfate and then concentrated under reduced pressure to obtain 8.95 mg of the title compound. The property values of the compound are as follows. $^1$H-NMR ($CDCl_3$) δ (ppm): 1.70-1.86 (m, 3H), 2.05-2.12 (m, 1H), 2.27-2.40 (m, 2H), 2.51 (s, 3H), 2.70-2.82 (m, 1H), 3.10-3.20 (m, 1H), 3.75-3.86 (m, 1H), 3.93 (s, 3H), 5.17 (d, J = 8.8 Hz, 1H), 6.89-7.00 (m, 2H), 7.05-7.17 (m, 3H), 7.75 (d, J = 2.0 Hz, 1H), 7.81 (d, J = 8.0 Hz, 1H), 8.72 (s, 1H).
ESI-MS; m/z 451 [$M^+$ + H].

Example 27

Synthesis of (E)-1-[(S)-1-(4-fluorophenyl)ethyl]-3-[1-[3-methox-4-(3-meth1[1,2,4]triazol-1-yl)phenyl]methylidene]-5,5-dimethylpiperidin-2-one

[Formula 103]

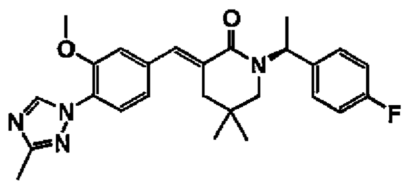

Synthesis of 4-[(S)-1-(4-fluorophenyl)ethylcarbamoyl]-2,2-dimethylbutyric acid

A solution of 2,2-dimethylglutaric anhydride (1.2 g) [CAS# 2938-48-9] in toluene (10 mL) was added dropwise to a solution of (S)-1-(4-fluorophenyl)ethylamine (1.76 g) in toluene (50 mL), and then TEA (1.27 mL) was added dropwise in a nitrogen atmosphere at -78°C. Thereafter, the reaction solution was stirred overnight with heating to room temperature. 1 N hydrochloric acid (20 mL) and ethyl acetate were added to the reaction mixture, and the organic layer was separated. The organic layer was washed with brine, dried over anhydrous magnesium sulfate and then concentrated under reduced pressure. The residue was purified by silica gel column chromatography (elution solvent: heptane-ethyl acetate system) to obtain 2.4 g of the title compound. The property values of the compound are as follows.
$^1$H-NMR ($CDCl_3$) δ (ppm): 1.20 (s, 3H), 1.22 (s, 3H), 1.46 (d, J = 6.8 Hz, 3H), 1.87-1.92 (m, 2H), 2.18-2.22 (m, 2H), 5.05-5.12 (m, 1H), 5.76 (d, J = 7.6 Hz, 1H), 6.98-7.04 (m, 2H), 7.24-7.30 (m, 2H).

Synthesis of 1-[(S)-1-(4-fluorophenyl)ethyl]-5,5-dimethylpiperidin-2-one

Oxalyl chloride (2.3 mL) was added to a solution of 4-[(S)-1-(4-fluorophenyl)ethylcarbamoyl]-2,2-dimethylbutyric acid (2.4 g) in methylene chloride (30 mL) under ice-cooling, and the reaction solution was stirred for 10 minutes. The reaction mixture was concentrated under reduced pressure and the residue was dissolved in methanol (10 mL). The reaction solution was stirred at room temperature for 10 minutes and then concentrated under reduced pressure. Water and ethyl acetate were added to the residue, and the organic layer was separated. The organic layer was washed with brine, dried over anhydrous magnesium sulfate and then concentrated under reduced pressure. The residue was purified by silica gel column chromatography (elution solvent: heptane-ethyl acetate system) to obtain a methyl ester compound (846 mg).
A solution of the methyl ester compound (846 mg) in THF (5 mL) was added dropwise to a suspension of LAH (95 mg) in THF (5 mL) in a nitrogen atmosphere under ice-cooling, and the reaction solution was stirred for 15 minutes. Water (0.095 mL), a 5 N sodium hydroxide solution (0.095 mL) and water (0.285 mL) were sequentially added to the reaction mixture, and the precipitated insoluble matter was filtered through celite. The filtrate was concentrated under reduced pressure to a crude alcohol compound (226 mg).

Methanesulfonyl chloride (0.079 mL) was added to a solution of the crude alcohol compound (226 mg) in pyridine (4 mL), and the reaction solution was stirred at room temperature for 30 minutes. Water and ethyl acetate were added to the reaction mixture, and the organic layer was separated. The organic layer was sequentially washed with 1 N hydrochloric acid and brine, dried over anhydrous magnesium sulfate and then concentrated under reduced pressure. Potassium tert-butoxide (142 mg) was added to a solution of the residue in THF (4 mL), and the reaction solution was stirred at room temperature for one hour. A saturated ammonium chloride solution and ethyl acetate were added to the reaction mixture, and the organic layer was separated. The organic layer was washed with brine, dried over anhydrous magnesium sulfate and then concentrated under reduced pressure. The residue was purified by silica gel column chromatography (elution solvent: heptane-ethyl acetate system) to obtain 118 mg of the title compound. The property values of the compound are as follows.
$^1$H-NMR ($CDCl_3$) δ (ppm): 0.78 (s, 3H), 0.95 (s, 3H), 1.45 (d, J = 7.2 Hz, 3H), 1.49-1.60 (m, 2H), 2.42 (dd, J = 12 Hz, 1.2 Hz, 1H), 2.47 (t, J = 7.2 Hz, 2H), 2.78 (d, J = 12 Hz, 1H), 6.14 (q, J = 7.2 Hz, 1H), 6.98-7.04 (m, 2H), 7.24-7.29 (m, 2H).

Synthesis of diethyl {1-[(S)-1-(4-fluorophenyl)ethyl]-5,5-dimethyl-2-oxopiperidin-3-yl}phosphonate

111 mg of the title compound was obtained from 1-[(S)-1-(4-fluorophenyl)ethyl]-5,5-dimethylpiperidin-2-one (80 mg) by the same method as in Example 26. The property value of the compound is as follows.
ESI-MS; M/Z 386 ($M^+$+H)

Synthesis of (E)-1-[(S)-1-(4-fluorophenyl)ethyl]-3-[1-[3-methoxy-4-(3-methyl[1,2,4]triazol-1-yl)phenyl]methylidene]-5,5-dimethylpiperidin-2-one

5.84 mg of the title compound was obtained from diethyl {1-[(S)-1-(4-fluorophenyl)ethyl]-5,5-dimethyl-2-oxopiperidin-3-yl}phosphonate (10 mg) and 3-methoxy-4-(3-methyl[1,2,4]triazol-1-yl)benzaldehyde obtained in Example 4 (5 mg) by the same method as in Example 26. The property values of the compound are as follows.
$^1$H-NMR ($CDCl_3$) δ (ppm): 0.74 (s, 3H), 0.95 (s, 3H), 1.53 (d, J = 7.2 Hz, 3H), 2.49 (s, 3H), 2.54 (m, 2H), 2.60 (d, J = 12.4 Hz, 1H), 2.94 (d, J = 12.4 Hz, 1H), 3.93 (s, 3H), 6.26 (q, J = 7.2 Hz, 1H), 6.98-7.08 (m, 4H), 7.32 (dd, J = 8.8, 5.6 Hz, 2H), 7.76 (d, J = 8.0 Hz, 1H), 7.95 (s, 1H), 8.67 (s, 1H).
ESI-MS; m/z 449 [$M^+$ + H].

Example 28

Synthesis of (E)-(6S,9aR)-6-(4-fluorophenyl)3-{1-[3-methoxy-4-(3-methyl[1,2,4]triazol-1-yl)phenyl]methylideneloctahydroquinolizin-4-one

[Formula 104]

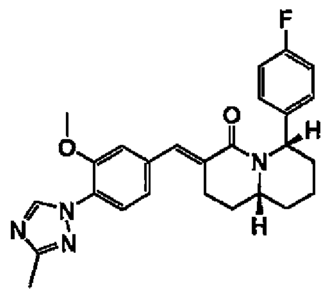

Synthesis of methyl (2R,6S)-1-(3-buteno1)-6-(4-fluorophenyl)piperidine-2-carboxylate

A 1 M solution of 4-fluorophenylmagnesium bromide in THF (15.7 mL) was added to a solution of (R)-6-oxopiperidine-1,2-dicarboxylic acid 1-tert-butyl ester 2-methyl ester [CAS #183890-36-0] (3.65 g) in THF (50 mL) in a nitrogen atmosphere at -78°C. The reaction solution was heated to -10°C with stirring for two hours. A ammonium chloride solution and ethyl acetate were added to the reaction solution, and the organic layer was separated. The resulting

organic layer was dried over anhydrous magnesium sulfate and concentrated under reduced pressure. The residue was purified by silica gel column chromatography (elution solvent: heptane-ethyl acetate system) to obtain 3.30 g of methyl (R)-2-tert-butoxycarbonylamino-6-(4-fluorophenyl)-6-oxohexanoate.
A solution of 4 N hydrogen chloride in ethyl acetate (40 mL) was added to a solution of the resulting methyl (R)-2-tert-butoxycarbonylamino-6-(4-fluorophenyl)-6-oxohexanoate (3.30 g) in ethyl acetate (40 mL), and the reaction solution was stirred at room temperature for 16 hours. The reaction solution was concentrated under reduced pressure. Chloroform and a saturated sodium bicarbonate solution were added to the residue, and the solution was stirred at room temperature for two hours. The organic layer was separated, dried over anhydrous magnesium sulfate and then concentrated under reduced pressure. 10% palladium-carbon (50% wet, 270 mg) was added to a solution of the resulting oil in methanol (60 mL), and the reaction solution was stirred in a hydrogen atmosphere at room temperature for two hours. The reaction solution was filtered through celite, and the filtrate was concentrated under reduced pressure to obtain 2.05 g of a crude product of methyl (2R,6S)-6-(4-fluorophenyl)piperidine-2-carboxylate.
Diethyl cyanophosphonate (1.0 mL) was added to a solution of the resulting crude product of methyl (2R,6S)-6-(4-fluorophenyl)piperidine-2-carboxylate (500 mg), 3-butenoic acid (0.56 mL) and TEA (1.8 mL) in DMF (15 mL), and the reaction solution was stirred at room temperature for 17 hours. Ethyl acetate and 0.5 N hydrochloric acid were added to the reaction solution, and the organic layer was separated. The resulting organic layer was sequentially washed with a saturated sodium bicarbonate solution and brine, dried over anhydrous magnesium sulfate and then concentrated under reduced pressure. The residue was purified by silica gel column chromatography (elution solvent: heptane-ethyl acetate system) to obtain 392 mg of the title compound. The property value of the compound is as follows.
ESI-MS; m/z 306 [$M^+$ + H].

Synthesis of 1-[(2S,6R)-2-(4-fluorophenyl)-6-(hydroxymethyl)piperidin-1-yl]-3-buten-1-one

Lithium borohydride (84 mg) was added to a solution of methyl (2R, 6S)-1-(3-butenoyl)-6-(4-fluorophenyl) piperidine-2-carboxylate (392 mg) in THF (10 mL) at 0°C, and the reaction solution was stirred at 0°C for two hours and at room temperature for 5.5 hours. The reaction solution was cooled to 0°C. A saturated ammonium chloride solution and ethyl acetate were added to the reaction solution, and the organic layer was separated. The resulting organic layer was dried over anhydrous magnesium sulfate and concentrated under reduced pressure. The residue was purified by silica gel column chromatography (elution solvent: heptane-ethyl acetate system) to obtain 300 mg of the title compound. The property value of the compound is as follows.
ESI-MS; m/z 278 [$M^+$ + H].

Synthesis of methyl (EZ)-3-[(2R,6S)-1-(3-buteno1)-6-(4-fluorophenyl)piperidin-2-yl]acrylate

Dimethyl sulfoxide (0.12 mL) was added to a solution of oxalyl chloride (0.14 mL) in methylene chloride (6 mL) in a nitrogen atmosphere at -78°C, and the reaction solution was stirred at -78°C for five minutes. A solution of 1-[(2S, 6R)-2-(4-fluorophenyl)-6-(hydroxymethyl)piperidin-1-yl]-3-buten-1-one (300 mg) in methylene chloride (3 mL) was added dropwise to the reaction solution, and the reaction solution was stirred at -78°C for 20 minutes. TEA (0.89 mL) was added to the reaction solution, and the reaction solution was stirred at -78°C for 10 minutes. The reaction solution was heated to room temperature.
Ethyl acetate and water were added to the reaction solution, and the organic layer was separated. The resulting organic layer was sequentially washed with 0.5 N hydrochloric acid and brine, dried over anhydrous magnesium sulfate and then concentrated under reduced pressure to obtain 297 mg of a crude product of (2R,6S)-1-(3-butenoyl)-6-(4-fluorophenyl)piperidine-2-carbaldehyde. Trimethylphosphonoacetate (0.45 mL) was added to a mixed suspension of oily 60% sodium hydride (72 mg) in THF (5 mL) and DMF (1 mL), and the reaction solution was stirred at room temperature for 30 minutes. A solution of the crude product of (2R,6S)-1-(3-butenoyl)-6-(4-fluorophenyl)piperidine-2-carbaldehyde obtained above (297 mg) in THF (3 mL) was added to the reaction solution, and the reaction solution was stirred at room temperature for 30 minutes. The reaction solution was added to a saturated ammonium chloride solution, followed by extraction with ethyl acetate. The resulting organic layer was dried over anhydrous magnesium sulfate and concentrated under reduced pressure. The residue was purified by silica gel column chromatography (elution solvent: heptane-ethyl acetate system) to obtain 146 mg of an E-isomer of the title compound and 91 mg of a Z-isomer of the title compound. The property values of the compound are as follows.
E-isomer of title compound
$^1$H-NMR ($CDCl_3$) δ (ppm): 1.62-1.71 (m, 1H), 1.78-2.00 (m, 4H), 2.40-2.48 (m, 1H), 3.17-3.30 (m, 2H), 3.61 (s, 3H), 5.00-5.24 (m, 3H), 5.46-5.66 (m, 1H), 5.70 (brd, J = 15.6 Hz, 1H), 5.96-6.06 (m, 1H), 6.47 (dd, J = 15.6, 6.4 Hz, 1H), 6.95-7.02 (m, 2H), 7.18-7.26 (m, 2H).
ESI-MS; m/z 332 [$M^+$ + H]
Z-isomer of title compound

$^1$H-NMR ($CDCl_3$) δ (ppm): 1.62-1.83 (m, 3H), 1.83-1.97 (m, 2H), 2.45-2.53 (m, 1H), 3.17-3.24 (m, 1H), 3.33 (dd, J = 16.0, 6.8 Hz, 1H), 3.71 (s, 3H), 5.16-5.23 (m, 2H), 5.50 (dd, J = 11.2, 1.2 Hz, 1H), 5.87 (dd, J = 11.2, 9.6 Hz, 1H), 5.92-6.07 (m, 3H), 6.97-7.03 (m, 2H), 7.28-7.33 (m, 2H).
ESI-MS; m/z 332 [$M^+$ + H]

Synthesis of (6S,9aR)-6-(4-fluorophenyl)-[3,6,7,8,9,9a]-hexahydroquinolizin-4-one

A solution of methyl (E)-3-[(2R,6S)-1-(3-butenoyl)-6-(4-fluorophenyl)piperidin-2-yl]acrylate (146 mg) and tricyclohexylphosphine[1,3-bis(2,4,6-trimethylphenyl)-4,5-dihydroimidazol-2-ylidene][benzylidene]ruthenium (IV) dichloride (37 mg) in methylene chloride (30 mL) was heated under reflux in a nitrogen atmosphere for three hours. The reaction solution was left to cool to room temperature. TEA (0.06 mL) was added to the reaction solution, and the reaction solution was stirred at room temperature for 10 minutes. The reaction solution was concentrated under reduced pressure. The residue was purified by silica gel column chromatography (elution solvent: heptane-ethyl acetate system) to obtain 83 mg of the title compound. The property values of the compound are as follows.
$^1$H-NMR ($CDCl_3$) δ (ppm): 1.39-1.52 (m, 1H), 1.60-1.75 (m, 2H), 1.84-1.94 (m, 1H), 1.97-2.06 (m, 1H), 2.19-2.30 (m, 1H), 2.93-3.10 (m, 2H), 4.27-4.36 (m, 1H), 5.30 (brt, J = 4.0 Hz, 1H), 5.63-5.71 (m, 1H), 5.83-5.90 (m, 1H), 6.93-7.01 (m, 2H), 7.14-7.21 (m, 2H).
ESI-MS; m/z 246 [$M^+$ + H].

Synthesis of (6S, 9aR)-6-(4-fluorophenyl)octahydroquinolizin-4-one

A solution of (6S,9aR)-6-(4-fluorophenyl)-[3,6,7,8,9,9a]-hexahydroquinolizin-4-one (130 mg) and platinum oxide (12 mg) in methanol (5 mL) was stirred in a hydrogen atmosphere at room temperature for three hours. The reaction solution was filtered through celite, and the filtrate was concentrated under reduced pressure. The residue was purified by silica gel column chromatography (elution solvent: heptane-ethyl acetate system) to obtain 125 mg of the title compound. The property values of the compound are as follows.
$^1$H-NMR ($CDCl_3$) δ (ppm): 1.24-1.41 (m, 1H), 1.46-1.68 (m, 3H), 1.68-1.87 (m, 2H), 1.91-2.00 (m, 2H), 2.00-2.09 (m, 1H), 2.13-2.24 (m, 1H), 2.43-2.57 (m, 2H), 3.58-3.68 (m, 1H), 5.38 (brs, 1H), 6.94-7.02 (m, 2H), 7.13-7.20 (m, 2H).
ESI-MS; m/z 248 [$M^+$ + H].

Synthesis of (6S,9aR)-6-(4-fluorophenyl)-3-iodooctahydroquinolizin-4-one

Iodotrimethylsilane (0.11 mL) was added to a solution of (6S,9aR)-6-(4-fluorophenyl)octahydroquinolizin-4-one (125 mg) and N,N,N',N'-tetramethylethylenediamine (0.27 mL) in methylene chloride (4 mL) in a nitrogen atmosphere at 0°C, and the reaction solution was stirred at 0°C for 30 minutes. Iodine (0.19 g) was added to the reaction solution, and the reaction solution was stirred at 0°C for one hour. A saturated sodium thiosulfate solution and ethyl acetate were added to the reaction solution, and the organic layer was separated. The resulting organic layer was washed with brine, dried over anhydrous magnesium sulfate and then concentrated under reduced pressure to obtain 188 mg of a crude product of the title compound. The property value of the compound is as follows.
ESI-MS; m/z 374 [$M^+$ + H].

Synthesis of diethyl [(6S,9aR)-6-(4-fluorophenyl)-4-oxooctahydroquinolizin-3-yl]phosphonate

A mixture of the crude product of (6S,9aR)-6-(4-fluorophenyl)-3-iodooctahydroquinolizin-4-one (188 mg) and triethyl phosphite (3 mL) was stirred at 120°C for five hours. The reaction solution was left to cool to room temperature and concentrated under reduced pressure to obtain 180 mg of a crude product of the title compound. The property value of the compound is as follows.
ESI-MS; m/z 384 [$M^+$ + H].

Synthesis of (E)-(6S,9aR)-6-(4-fluorophenyl) 3-{1-[3-methoxy-4-(3-methyl[1,2,4]triazol-1-yl)phenyl]methylideneloctahydroquinolizin-4-one

Lithium hydroxide monohydrate powder (4 mg) was added to a solution of diethyl [(6S,9aR)-6-(4-fluorophenyl)-4-oxooctahydroquinolizin-3-yl]phosphonate (14.2 mg) and 3-methoxy-4-(3-methyl[1,2,4]triazol-l-yl)benzaldehyde obtained in Example 4 (4.8 mg) in THF (0.25 ml)-ethanol (0.25 ml). The reaction solution was stirred at room temperature for four hours. 2 ml of water was added to the reaction solution, followed by extraction with ethyl acetate (three times). The combined organic layers were concentrated in a nitrogen stream. The resulting residue was purified by LC-MS, and the objective fraction was concentrated under reduced pressure. Ethyl acetate and a saturated sodium bicarbonate

solution were added to the resulting residue, and the organic layer was separated. The organic layer was washed with brine, dried over anhydrous magnesium sulfate and then concentrated under reduced pressure to obtain 3.53 mg of the title compound. The property values of the compound are as follows.
$^1$H-NMR ($CDCl_3$) δ (ppm): 1.33-1.46 (m, 1H), 1.48-1.78 (m, 4H), 1.99-2.08 (m, 1H), 2.17-2.26 (m, 2H), 2.54 (s, 3H), 2.66-2.78 (m, 1H), 3.05-3.14 (m, 1H), 3.75-3.85 (m, 1H), 3.93 (s, 3H), 5.52 (br s, 1H), 7.00 (dd, J = 8.8, 8.8 Hz, 2H), 7.05 (d, J = 2.4 Hz, 1H), 7.11 (dd, J = 8.4, 2.4 Hz, 1H), 7.22 (dd, J = 8.8, 5.6 Hz, 2H), 7, 78 (d, J = 8.4 Hz, 1H), 7.80 (s, 1H), 8.71 (s, 1H).
ESI-MS; m/z 447 [$M^+$ + H].

Example 29

Synthesis of (E)-1-[(S)-1-(4-fluorophenyl)ethyl]-3-[1-[3-methoxy-4-([1,2,4]triazol-1-yl)phenyl]methylidene]-5,5-dimethylpiperidin-2-one

[Formula 105]

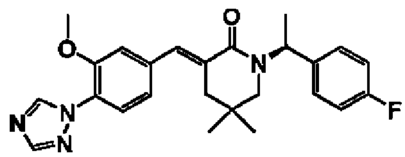

Synthesis of 3-methoxy-4-([1,2,4]triazol-1-yl)benzaldehyde and 3-methoxy-4-([1,2,4]triazol-4-yl)benzaldehyde

744 mg of 3-methoxy-4-([1,2,4]triazol-1-yl)benzaldehyde and 65 mg of 3-methoxy-4-([1,2,4]triazol-4-yl)benzaldehyde were obtained from 4-fluoro-3-methoxybenzaldehyde (1.0 g) and [1,2,4]triazole [CAS #288-88-0] (448 mg) by the same method as in Example 3.
The property values of 3-methoxy-4-([1,2,4]triazol-1-yl)benzaldehyde are as follows.
$^1$H-NMR ($CDCl_3$) δ (ppm): 4.06 (s, 3H), 7.60-7.65 (m, 2H), 8.09 (d, J = 8.8 Hz, 1H), 8.10 (s, 1H), 8.96 (s, 1H), 10.01 (s, 1H) .
The property values of 3-methoxy-4-([1,2,4]triazol-4-yl)benzaldehyde are as follows.
$^1$H-NMR ($CDCl_3$) δ (ppm): 3.99 (s, 3H), 7.51 (d, J = 7.6 Hz, 1H), 7.59-7.65 (m, 2H), 8.53 (s, 2H), 10.03 (s, 1H) .

Synthesis of (E)-1-[(S)-1-(4-fluorophenyl)ethyl]-3-[1-[3-methoxy-4-([1,2,4]triazol-1-yl)phenyl]methylidene]-5,5-dimethylpiperidin-2-one

5.34 mg of the title compound was obtained from 3-methoxy-4-([1,2,4]triazol-1-yl)benzaldehyde (7 mg) and diethyl {1-[(S)-1-(4-fluorophenyl)ethyl]-5,5-dimethyl-2-oxopiperidin-3-yl}phosphonate obtained in Example 27 (11 mg) by the same method as in Example 26. The property values of the compound are as follows.
$^1$H-NMR ($CDCl_3$) δ (ppm): 0.75 (s, 3H), 0.95 (s, 3H), 1.53 (d, J = 7.2 Hz, 3H), 2.55 (d, J = 2.0 Hz, 2H), 2.60 (d, J = 12.0 Hz, 1H), 2.94 (d, J = 12.0 Hz, 1H), 3.94 (s, 3H), 6.26 (q, J = 7.2 Hz, 1H), 6.98-7.10 (m, 4H), 7.32 (dd, J = 8.4, 5.2 Hz, 2H), 7.80 (d, J = 8.4 Hz, 1H), 7.96 (s, 1H), 8.07 (s, 1H), 8.79 (s, 1H).

Example 30

Synthesis of (E)-1-[(S)-1-(4-fluorophenyl)ethyl]-3-[1-[3-methoxy-4-([1,2,4]triazol-1-yl)phenyl]methylidene]-5,5-dimethylpiperidin-2-one

[Formula 106]

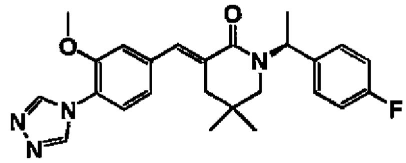

5.05 mg of the title compound was obtained from 3-methoxy-4-([1,2,4]triazol-4-yl)benzaldehyde obtained in Example 29 (7 mg) and diethyl {1-{1-[(S)-1-(4-fluorophenyl)ethyl]-5,5-dimethyl-2-oxopiperidin-3-yl}phosphonate obtained in Example 27 (11 mg) by the same method as in Example 26. The property values of the compound are as follows. $^1$H-NMR ($CDCl_3$) δ (ppm): 0.75 (s, 3H), 0.96 (s, 3H), 1.53 (d, J = 7.2 Hz, 3H), 2.53 (d, J = 2.0 Hz, 2H), 2.62 (d, J = 12.4 Hz, 1H), 2.95 (d, J = 12.4 Hz, 1H), 3.88 (s, 3H), 6.25 (q, J = 7.2 Hz, 1H), 6.98-7.07 (m, 4H), 7.29 (d, J = 8.4 Hz, 1H), 7.32 (dd, J = 8.4, 5.6 Hz, 2H), 7.95 (s, 1H), 8.45 (s, 2H).

INDUSTRIAL APPLICABILITY

The compound of the general formula (I) of the present invention has an Aβ40 and Aβ42 production reducing effect, and thus is particularly useful as a prophylactic or therapeutic agent for a neurodegenerative disease caused by Aβ such as Alzheimer's disease or Down's syndrome.

**Claims**

1. A compound represented by the formula (I):

[Formula 1]

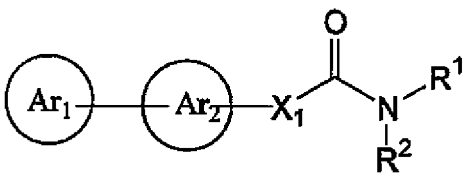

(I)

or a pharmacologically acceptable salt thereof,
wherein $Ar_1$ represents a triazolyl group or a tetrazolyl group which may be substituted with 1 to 3 substituents selected from Substituent Group A1 shown below;
$Ar_2$ represents a pyridinyl group, a pyrimidinyl group or a phenyl group which may be substituted with 1 to 3 substituents selected from Substituent Group A2 shown below;
$X_1$ represents (1) -C=C- or (2) -$CR^3$=$CR^4$- (wherein $R^3$ and $R^4$ are the same or different and each represent a substituent selected from Substituent Group A3 shown below); and

(1) $R^1$ and $R^2$ are the same or different and each represent a group selected from Substituent Group A4 shown below; or $R^1$ and $R^2$, together with a nitrogen atom to which they are bonded, form:
(2-1) a 5- to 11-membered heterocyclic group which may be substituted with 1 to 4 substituents selected from Substituent Group A4 and is represented by the formula (II):

[Formula 2]

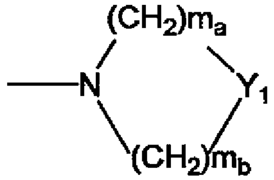

(II)

wherein $Y_1$ represents (1) -NH-, (2) -O-, (3) -S-, (4) -SO-, (5) -$SO_2$-, (6) -$CH_2$-, (7) -CO-, (8) -CONH-, (9) -NHCO-, (10) -$CR^5$=$CR^6$- (wherein $R^5$ and $R^6$ are the same or different and each represent a substituent selected from Substituent Group A4 shown below), (11) a single bond or (12) >C=$CR^{13}R^{14}$ (wherein $R^{13}$ and $R^{14}$ are the same or different and each represent a substituent selected from Substituent Group A4 shown below); and $m_a$ and $m_b$ are the same or different and each represent an integer of 0 to 4;

(2-2) a 6- to 20-membered non-aromatic heterocyclic group which may be substituted with 1 to 4 substituents selected from Substituent Group A4 and is represented by the formula (III):

[Formula 3]

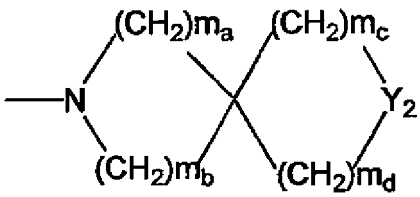

(III)

wherein $Y_2$ represents (1) -NH-, (2) -O-, (3) -S-, (4) -SO-, (5) -$SO_2$-, (6) -$CH_2$-, (7) -CO-, (8) -CONH-, (9)- NHCO-, (10) -$CR^{5a}$=$CR^{6a}$- (wherein $R^{5a}$ and $R^{6a}$ are the same or different and each represent a substituent selected from Substituent Group A4 shown below or $R^{5a}$ and $R^{6a}$, together with a carbon atom to which they are bonded, form a 6- to 14-membered aromatic hydrocarbon ring group or a 6- to 14-membered non-aromatic hydrocarbon ring group) or (11) a single bond; and $m_a$, $m_b$, $m_c$ and $m_d$ are the same or different and each represent an integer of 0 to 4;

(2-3) a 9- to 16-membered non-aromatic heterocyclic group which may be substituted with 1 to 4 substituents selected from Substituent Group A4 and is represented by the formula (IV):

[Formula 4]

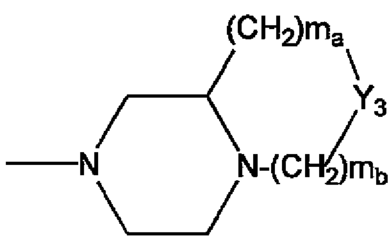

(IV)

wherein $Y_3$ represents (1) -NH-, (2) -O-, (3) -S-, (4) -SO-, (5) -$SO_2$-, (6) -$CH_2$-, (7) -CO-, (8) -CONH-, (9) -NHCO- or (10) a single bond; and $m_a$ and $m_b$ are as defined above;

(2-4) a group which may be substituted with 1 to 4 substituents selected from Substituent Group A4 and is represented by the following formula:

[Formula 5]

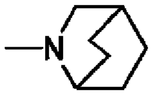, 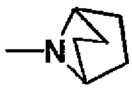, 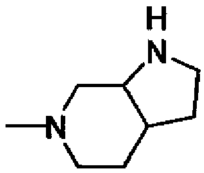 or 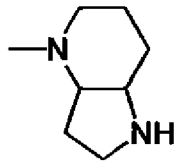

; or
(2-5) a group which may be substituted with 1 to 4 substituents selected from Substituent Group A4 and is represented by the following formula:

[Formula 6]

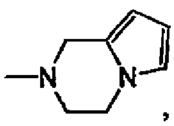, 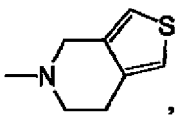, 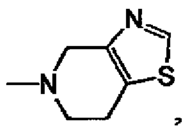, 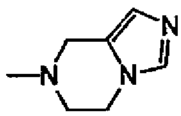, 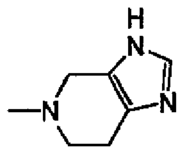, 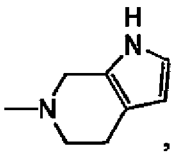, 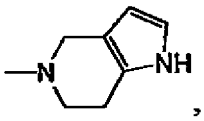, 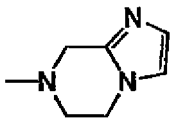, 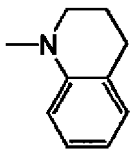, 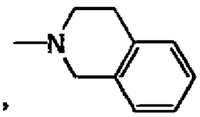,

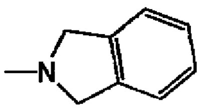 or 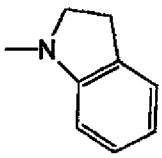

; or
$R^1$ and $R^2$, together with $-X_1$-CO-N, form:
(3-1) a cyclic group which may be substituted with 1 to 4 substituents selected from Substituent Group A4 and is represented by the formula (V):

[Formula 7]

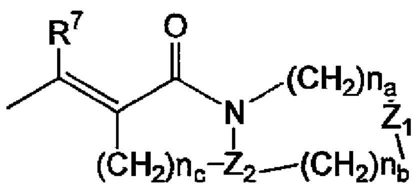 (V)

wherein $Z_1$ represents (1) -NH-, (2) -O-, (3) -S-, (4) -SO-, (5) $-SO_2-$, (6) $-CH_2-$, (7) -CO-, (8) -CONH-, (9) -NHCO- or (10) a single bond; $Z_2$ represents (1) a methine group or (2) a nitrogen atom; $R^7$ represents a substituent

selected from Substituent Group A3 shown below; and $n_a$, $n_b$ and $n_c$ are the same or different and each represent an integer of 0 to 4;
(3-2) a cyclic group represented by the formula (VI):

[Formula 8]

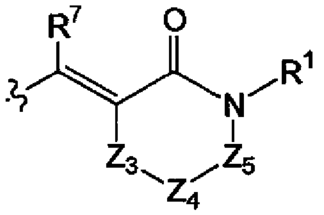

(VI)

wherein $Z_3$ represents (1) a single bond, (2) -CO-, (3) $-(CH_2)n_d-$ (wherein $n_d$ represents an integer of 1 to 3) or (4) $-CR^8R^9-$ (wherein $R^8$ and $R^9$ are the same or different and each represent a substituent selected from Substituent Group A4 shown below); $Z_4$ represents (1) a single bond, (2) -O-, (3) -NRCO-, (4) -CONR-, (5) -CSNR-, (6) -NRCS- (wherein R represents a substituent selected from Substituent Group A4 shown below) or (7)- S-; $Z_5$ represents (1) a single bond, (2) an imino group which may be substituted with a substituent selected from Substituent Group A4 shown below, (3) $-(CH_2)n_e-$(wherein $n_e$ represents an integer of 1 to 3), (4) $-CR^8R^9-$ (wherein $R^8$ and $R^9$ are as defined above) or (5) -O-; and $R^1$ and $R^7$ are as defined above; or
(3-3) a cyclic group which may be substituted with 1 to 4 substituents selected from Substituent Group A4 and is represented by the following formula:

[Formula 9]

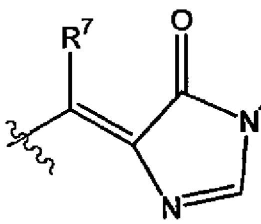

,

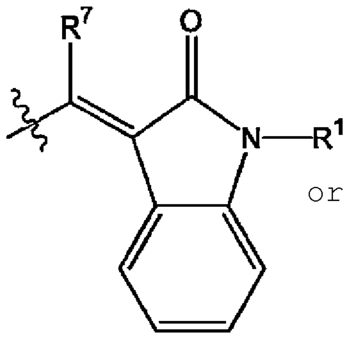

or

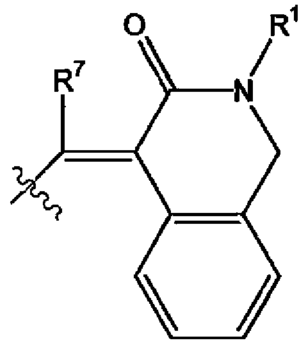

wherein $R^1$ and $R^7$ are as defined above.
Substituent Group A1: (1) a hydrogen atom, (2) a halogen atom, (3) a cyano group, (4) a nitro group, (5) a C3-8 cycloalkyl group, (6) a C2-6 alkenyl group, (7) a C2-6 alkynyl group, (8) a C1-6 alkoxy group, (9) a C3-8 cycloalkoxy group, (10) a formyl group, (11) a C1-6 alkylcarbonyl group and (12) a C1-6 alkyl group (wherein the C1-6 alkyl group may be substituted with 1 to 3 substituents selected from the group consisting of a halogen atom, a hydroxyl group, a cyano group, a C1-6 alkoxy group, a C3-8 cycloalkyl group and a C1-6 alkylcarbonyl group).
Substituent Group A2: (1) a hydrogen atom, (2) a halogen atom, (3) a hydroxyl group, (4) a cyano group, (5) a C1-6 alkoxy group (wherein the C1-6 alkoxy group may be substituted with 1 to 3 substituents selected from the group consisting of a halogen atom, a cyano group, a C1-6 alkoxy group, a C2-6 alkenyl group, a C2-6 alkynyl group and a C3-8 cycloalkyl group), (6) a C3-8 cycloalkoxy group, (7) a C2-6 alkenyloxy group and (8) a C2-6 alkynyloxy group.
Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to

14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**2.** The compound or pharmacologically acceptable salt thereof according to claim 1, wherein $Ar_1$ is a triazolyl group or a tetrazolyl group which may be substituted with 1 or 2 substituents selected from the group consisting of (1) a hydrogen atom, (2) a halogen atom, (3) a C3-8 cycloalkyl group, (4) a C2-6 alkenyl group, (5) a C2-6 alkynyl group and (6) a C1-6 alkyl group (wherein the C1-6 alkyl group may be substituted with 1 to 3 halogen atoms).

**3.** The compound or pharmacologically acceptable salt thereof according to claim 2, wherein $Ar_1$ is a triazolyl group which may be substituted with 1 or 2 substituents selected from the group consisting of (1) a hydrogen atom, (2) a halogen atom, (3) a C3-8 cycloalkyl group, (4) a C2-6 alkenyl group, (5) a C2-6 alkynyl group and (6) a C1-6 alkyl group (wherein the C1-6 alkyl group may be substituted with 1 to 3 halogen atoms).

**4.** The compound or pharmacologically acceptable salt thereof according to claim 2, wherein $Ar_1$ is a tetrazolyl group which may be substituted with 1 or 2 substituents selected from the group consisting of (1) a hydrogen atom, (2) a halogen atom, (3) a C3-8 cycloalkyl group, (4) a C2-6 alkenyl group, (5) a C2-6 alkynyl group and (6) a C1-6 alkyl group (wherein the C1-6 alkyl group may be substituted with 1 to 3 halogen atoms).

**5.** The compound or pharmacologically acceptable salt thereof according to claim 3 or 4, wherein $Ar_1$ is a triazolyl group or a tetrazolyl group which may be substituted with a C1-6 alkyl group.

**6.** The compound or pharmacologically acceptable salt thereof according to claim 1, wherein $Ar_2$ is a phenyl group which may be substituted with 1 to 3 substituents selected from the group consisting of (1) a hydrogen atom, (2) a halogen atom, (3) a hydroxyl group, (4) a cyano group, (6) a C1-6 alkoxy group (wherein the C1-6 alkoxy group may be substituted with 1 to 3 substituents selected from a C2-6 alkenyl group, a C2-6 alkynyl group and a C3-8 cycloalkyl group), (7) a C2-6 alkenyloxy group and (8) a C2-6 alkynyloxy group.

7. The compound or pharmacologically acceptable salt thereof according to any one of claims 1 to 6, wherein $Ar_2$ is a phenyl group which may be substituted with 1 to 3 substituents selected from the group consisting of (1) a hydrogen atom, (2) a halogen atom, (3) a cyano group and (4) a C1-6 alkoxy group.

8. The compound or pharmacologically acceptable salt thereof according to claim 1, wherein $X_1$ is -C≡C-.

9. The compound or pharmacologically acceptable salt thereof according to claim 1, wherein $X_1$ represents $-CR^3=CR^4-$ (wherein $R^3$ and $R^4$ are the same or different and each represent a substituent selected from Substituent Group A3 shown below).
Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

10. The compound or pharmacologically acceptable salt thereof according to any one of claims 1 to 9, wherein $X_1$ is $-CR^{31}=CR^{41}-$ (wherein $R^{31}$ is a group selected from the group consisting of (1) a hydrogen atom, (2) a halogen atom, (3) a C1-6 alkyl group and (4) a C1-6 alkoxy group; and $R^{41}$ represents a substituent selected from the group consisting of (1) a hydrogen atom, (2) a halogen atom, (3) a 6- to 14-membered aromatic hydrocarbon ring group which may be substituted with 1 to 3 substituents selected from Substituent Group A5, (4) a 5- to 14-membered aromatic heterocyclic group which may be substituted with 1 to 3 substituents selected from Substituent Group A5 and (5) a C1-6 alkyl group (wherein the C1-6 alkyl group may be substituted with 1 to 3 substituents selected from the group consisting of a halogen atom, a hydroxyl group, a cyano group, a C3-8 cycloalkyl group, a C1-6 alkyl group, a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A5, a 5- to 14-membered aromatic heterocyclic group which may be substituted with 1 to 3 substituents selected from Substituent Group A5, a 5-to 14-membered non-aromatic heterocyclic group which may be substituted with 1 to 3 substituents selected from Substituent Group A5 and $-O-A^1$ (wherein $A^1$ represents a 6- to 14-membered aromatic hydrocarbon ring group which may be substituted with 1 to 3 substituents selected from Substituent Group A5 or a 5-to 14-membered aromatic heterocyclic group

which may be substituted with 1 to 3 substituents selected from Substituent Group A5))).
Substituent Group A5: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkyl group (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) an amino group (wherein the amino group may be substituted with a C1-6 alkyl group optionally having 1 to 5 halogen atoms).

**11.** The compound or pharmacologically acceptable salt thereof according to claim 1, 9 or 10, wherein $X_1$ is $-CR^{32}=CR^{42}-$ (wherein $R^{32}$ represents a hydrogen atom or a halogen atom; and $R^{42}$ represents a substituent selected from the group consisting of a hydrogen atom, a halogen atom, a C1-6 alkyl group (wherein the C1-6 alkyl group may be substituted with a C3-8 cycloalkyl group or a phenyl group) and a phenyl group).

**12.** The compound or pharmacologically acceptable salt thereof according to claim 1, wherein $R^1$ and $R^2$ are the same or different and each represent a group selected from Substituent Group A4 shown below. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**13.** The compound or pharmacologically acceptable salt thereof according to any one of claims 1 to 12, wherein $R^1$ is a group selected from Substituent Group A8 shown below and $R^2$ is a group selected from Substituent Group A6 shown below.
Substituent Group A6: (1) a hydrogen atom, (2) a C3-8 cycloalkyl group, (3) a C3-8 cycloalkoxy group, (4) a C1-6 alkyl group (wherein the C1-6 alkyl group may be substituted with 1 to 3 substituents selected from the group consisting of a halogen atom, a hydroxyl group, a cyano group, a C3-8 cycloalkyl group, a C3-8 cycloalkoxy group, a formyl group, a C1-6 alkylthio group, a hydroxyimino group, a C1-6 alkoxyimino group, a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7 shown below, a 5- to 14-membered aromatic heterocyclic group which may be substituted with 1 to 3 substituents selected from Substituent Group A7 shown below, a 5- to 14-membered non-aromatic heterocyclic group which may be substituted with 1 to 3 substituents selected from Substituent Group A7 and $-O-A^2$ (wherein $A^2$ represents a 6- to 14-membered aromatic hydrocarbon ring group which may be substituted with 1 to 3 substituents selected from Substituent Group A7 shown below or a 5- to 14-membered aromatic heterocyclic group which may be substituted with 1 to 3 substituents selected from Substituent Group A7 shown below)) and (5) a C1-6 alkoxy group (wherein the C1-6 alkoxy group may be substituted with 1 to 3 substituents selected from the group consisting of a halogen atom, a hydroxyl group, a cyano group, a C3-8 cycloalkyl group, a C3-8 cycloalkoxy group, a formyl group, a C1-6 alkylthio group, a hydroxyimino group, a C1-6 alkoxyimino group, a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7 shown below, a 5- to 14-membered aromatic heterocyclic group which may be substituted with 1 to 3 substituents selected from Substituent Group A7 shown below, a 5- to 14-membered non-aromatic heterocyclic group which may be substituted with 1 to 3 substituents selected from Substituent Group A7 and $-O-A^2$ (wherein $A^2$ is as defined above)).
Substituent Group A7: (1) a hydrogen atom, (2) a halogen atom, (3) a hydroxyl group, (4) a cyano group, (5) a C3-8

cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above).
Substituent Group A8: (1) a hydrogen atom, (2) a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7, a 5-to 14-membered non-aromatic heterocyclic group which may be substituted with 1 to 3 substituents selected from Substituent Group A7 and -X-$A^2$ (wherein X represents an imino group, -O- or -S- and $A^2$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7)), (3) a 6- to 14-membered aromatic hydrocarbon ring group which may be substituted with 1 to 3 substituents selected from Substituent Group A7, (4) a 5- to 14-membered aromatic heterocyclic group which may be substituted with 1 to 3 substituents selected from Substituent Group A7 and (5) -X-$A^2$ (wherein X and $A^2$ are as defined above).

**14.** The compound or pharmacologically acceptable salt thereof according to claim 1, 12 or 13, wherein $R^1$ is a C1-6 alkyl group (wherein the C1-6 alkyl group is a hydrogen atom, a C3-8 cycloalkoxy group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, a 6- to 14-membered aromatic hydrocarbon ring group which may be substituted with 1 to 3 substituents selected from Substituent Group A9, a 5- to 14-membered aromatic heterocyclic group which may be substituted with 1 to 3 substituents selected from Substituent Group A9 and -O-$A^4$ (wherein $A^4$ represents a 6- to 14-membered aromatic hydrocarbon ring group which may be substituted with 1 to 3 substituents selected from Substituent Group A9 or a 5- to 14-membered aromatic heterocyclic group which may be substituted with 1 to 3 substituents selected from Substituent Group A9)); and $R^2$ is (1) a hydrogen atom or (2) a C1-6 alkyl group (wherein the C1-6 alkyl group may be substituted with 1 to 3 substituents selected from the group consisting of a hydroxyl group, a C3-8 cycloalkyl group, a C3-8 cycloalkoxy group, a C1-6 alkylthio group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A9, a 5- to 14-membered aromatic heterocyclic group which may be substituted with 1 to 3 substituents selected from Substituent Group A9 and a 5- to 14-membered non-aromatic heterocyclic group which may be substituted with 1 to 3 substituents selected from Substituent Group A9).
Substituent Group A9: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) -CO-$A^3$ (wherein $A^3$ represents a 6- to 14-membered aromatic hydrocarbon ring group), (10) a 6- to 14-membered aromatic hydrocarbon ring group which may be substituted with 1 to 3 substituents selected from Substituent Group

A9 and (11) a 5- to 14-membered aromatic heterocyclic group which may be substituted with 1 to 3 substituents selected from Substituent Group A9.

**15.** The compound or pharmacologically acceptable salt thereof according to claim 1, wherein $R^1$ and $R^2$, together with a nitrogen atom to which they are bonded, form a 5- to 11-membered heterocyclic group which may be substituted with 1 to 4 substituents selected from Substituent Group A4 shown below and is represented by the formula (II):

[Formula 10]

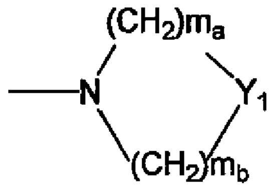
(II)

wherein $Y_1$ represents (1) -NH-, (2) -O-, (3) -S-, (4) -SO-, (5) $-SO_2-$, (6) $-CH_2-$, (7) -CO-, (8) -CONH-, (9) -NHCO-, (10) $-CR^5=CR^6-$ (wherein $R^5$ and $R^6$ are the same or different and each represent a substituent selected from Substituent Group A4 shown below), (11) a single bond or (12) $>C=CR^{13}R^{14}$ (wherein $R^{13}$ and $R^{14}$ are the same or different and each represent a substituent selected from Substituent Group A4 shown below); and $m_a$ and $m_b$ are the same and different and each represent an integer of 0 to 4.
Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**16.** The compound or pharmacologically acceptable salt thereof according to any one of claims 1 to 15, wherein the 5- to 11-membered heterocyclic group is a piperidinyl group, a pyrrolidinyl group, an azepinyl group, an azocanyl group, a piperazinyl group, a 1,4-diazepanyl group, a morpholinyl group or a thiomorpholinyl group.

**17.** The compound or pharmacologically acceptable salt thereof according to claim 1, 15 or 16, wherein $R^1$ and $R^2$, together with a nitrogen atom to which are bonded, form a piperidinyl group, a pyrrolidinyl group, an azepinyl group, an azocanyl group, a piperazinyl group, a 1,4-diazepanyl group, a morpholinyl group or a thiomorpholinyl group which may be substituted with 1 to 3 substituents selected from the group consisting of (1) a hydrogen atom, (2) a halogen atom, (3) a hydroxyl group, (4) a formyl group, (5) a hydroxyimino group, (6) a C1-6 alkoxyimino group, (7) a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A7 shown below and a 5- to 14-membered aromatic heterocyclic group which may be substituted with 1 to 3 substituents selected from Substituent Group A7 shown below), (8) a 6- to 14-membered aromatic hydrocarbon ring group which may be substituted with 1 to 3 substituents

selected from Substituent Group A7 shown below, (9) a 5- to 14-membered aromatic heterocyclic group which may be substituted with 1 to 3 substituents selected from Substituent Group A7 shown below, (10) -O-$A^2$ (wherein $A^2$ represents a 6- to 14-membered aromatic hydrocarbon ring group which may be substituted with 1 to 3 substituents selected from Substituent Group A7 shown below or a 5- to 14-membered aromatic heterocyclic group which may be substituted with 1 to 3 substituents selected from Substituent Group A7 shown below), (11) -CO-$A^2$ (wherein $A^2$ is as defined above) and (12) =CH-$A^2$ (wherein $A^2$ is as defined above).
Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above).

**18.** The compound or pharmacologically acceptable salt thereof according to claim 1, 15, 16 or 17, wherein $R^1$ and $R^2$, together with a nitrogen atom to which they are bonded, form a piperidinyl group, a pyrrolidinyl group, an azepinyl group, an azocanyl group, a piperazinyl group, a 1,4-diazepanyl group, a morpholinyl group or a thiomorpholinyl group which may be substituted with 1 to 4 substituents selected from the group consisting of (1) a hydrogen atom, (2) a halogen atom, (3) a hydroxyl group, (4) a C1-6 alkyl group (wherein the C1-6 alkyl group may be substituted with 1 to 3 hydroxyl groups or 1 to 3 substituents selected from the group consisting of a 6- to 14-membered aromatic hydrocarbon ring group which may be substituted with 1 to 3 substituents selected from Substituent Group A10 shown below), (5) a 6- to 14-membered aromatic hydrocarbon ring group which may be substituted with 1 to 3 substituents selected from Substituent Group A10 shown below, (6) a 5- to 14-membered aromatic heterocyclic group which may be substituted with 1 to 3 substituents selected from Substituent Group A10 shown below, (7) -O-$A^6$ (wherein $A^6$ represents a 6- to 14-membered aromatic hydrocarbon ring group which may be substituted with 1 to 3 substituents selected from Substituent Group A10 shown below) and (8) =CH-$A^6$ (wherein $A^6$ is as defined above). Substituent Group A10: (1) a hydrogen atom, (2) a halogen atom, (3) a C1-6 alkyl group (wherein the C1-6 alkyl group may be substituted with 1 to 5 halogen atoms), (4) a C1-6 alkoxy group and (5) a 6- to 14-membered aromatic hydrocarbon ring group.

**19.** The compound or pharmacologically acceptable salt thereof according to claim 1, wherein $R^1$ and $R^2$, together with a nitrogen atom to which they are bonded, form a 6- to 20-membered non-aromatic heterocyclic group which may be substituted with 1 to 4 substituents selected from Substituent Group A4 shown below and is represented by the formula (III):

[Formula 11]

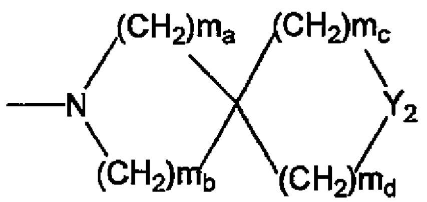

(III)

wherein $Y_2$ represents (1) -NH-, (2) -O-, (3) -S-, (4) -SO-, (5) -$SO_2$-, (6) -$CH_2$-, (7) -CO-, (8) -CONH-, (9) -NHCO-, (10) -$CR^5$=$CR^6$- (wherein $R^5$ and $R^6$ are the same or different and each represent a substituent selected from Substituent Group A4 shown below or $R^5$ and $R^6$, together with a carbon atom to which they are bonded, form a 6-

to 14-membered aromatic hydrocarbon ring group or a 6- to 14-membered non-aromatic hydrocarbon ring group) or (11) a single bond; and $m_a$, $m_b$, $m_c$ and $m_d$ are the same or different and each represent an integer of 0 to 4. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**20.** The compound or pharmacologically acceptable salt thereof according to claim 1, wherein $R^1$ and $R^2$, together with a nitrogen atom to which they are bonded, form a 9- to 16-membered non-aromatic heterocyclic group which may be substituted with 1 to 4 substituents selected from Substituent Group A4 and is represented by the formula (IV):

[Formula 12]

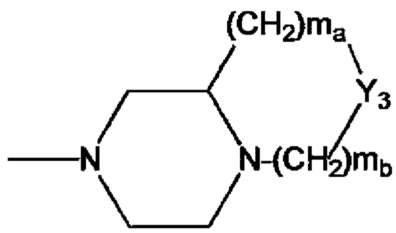

(IV)

wherein $Y_3$ represents (1) -H-, (2) -O-, (3) -S-, (4) -SO-, (5) $-SO_2-$, (6) $-CH_2-$, (7) -CO-, (8) -CONH-, (9) -NHCO- or (10) a single bond; and $m_a$ and $m_b$ are the same or different and each represent an integer of 0 to 4. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above)

and (32) =CH-A (wherein A is as defined above).

**21.** The compound or pharmacologically acceptable salt thereof according to claim 1, wherein $R^1$ and $R^2$, together with a nitrogen atom to which they are bonded, form a group which may be substituted with 1 to 4 substituents selected from Substituent Group A4 and is represented by the following formula:

[Formula 13]

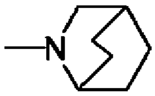 , 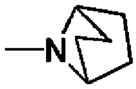 , 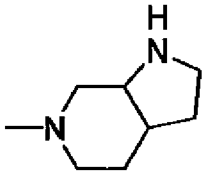 or 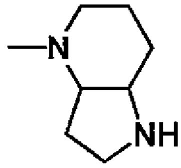

.

Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**22.** The compound or pharmacologically acceptable salt thereof according to claim 1, wherein $R^1$ and $R^2$, together with a nitrogen atom to which they are bonded, form a group which may be substituted with 1 to 4 substituents selected from Substituent Group A4 and is represented by the following formula:

[Formula 14]

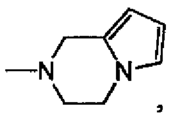, 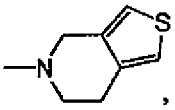, 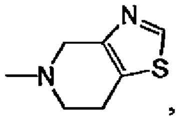, 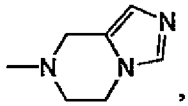, 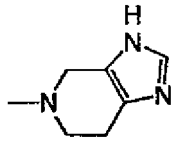, 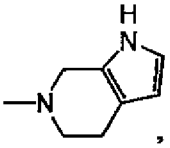, 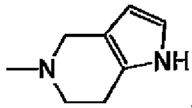, 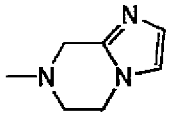, 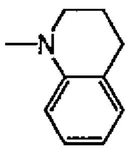, 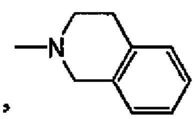, 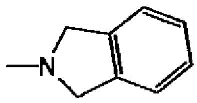 or 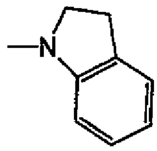

.

Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**23.** The compound or pharmacologically acceptable salt thereof according to claim 1 or 22, wherein $R^1$ and $R^2$, together with a nitrogen atom to which they are bonded, form a group which may be substituted with 1 to 4 substituents selected from Substituent Group A4 and is represented by the following formula:

[Formula 15]

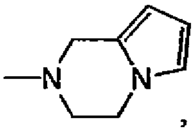, 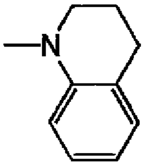, 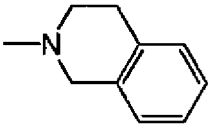, 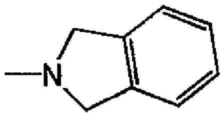

or 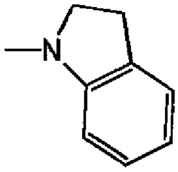

.

Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**24.** The compound or pharmacologically acceptable salt thereof according to claim 1, 22 or 23, wherein $R^1$ and $R^2$, together with a nitrogen atom to which they are bonded, form a group which may be substituted with 1 to 4 fluorine atoms.

**25.** The compound or pharmacologically acceptable salt thereof according to claim 1, wherein $R^1$ and $R^2$, together with $-X_1$-CO-N, form a cyclic group which may be substituted with 1 to 4 substituents selected from Substituent Group A4 and is represented by the formula (V):

[Formula 16]

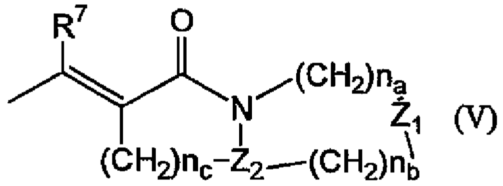

wherein $Z_1$ represents (1) -NH-, (2) -O-, (3) -S-, (4) -SO-, (5) $-SO_2-$, (6) $-CH_2-$, (7) -CO-, (8) -CONH-, (9) -NHCO- or (10) a single bond; $Z_2$ represents (1) a methine group or (2) a nitrogen atom; $R^7$ represents a substituent selected from Substituent Group A3 shown below; and $n_a$, $n_b$ and $n_c$ are the same or different and each represent an integer of 0 to 4.

Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**26.** The compound or pharmacologically acceptable salt thereof according to any one of claims 1 to 7, wherein $R^1$ and $R^2$, together with $-X_1$-CO-N, form a cyclic group which may be substituted with 1 to 4 substituents selected from Substituent Group A4 and is represented by the formula (VI):

[Formula 17]

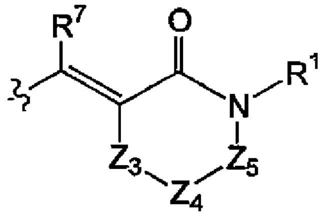

(VI)

wherein $Z_3$ represents (1) a single bond, (2) -CO-, (3) $-(CH_2)n_d-$ (wherein $n_d$ represents an integer of 1 to 3) or (4) $-CR^8R^9-$ (wherein $R^8$ and $R^9$ are the same or different and each represent a substituent selected from Substituent Group A4 shown below); $Z_4$ represents (1) a single bond, (2) -O-, (3) -NRCO-, (4) -CONR-, (5) -CSNR-, (6) -NRCS- (wherein R represents a substituent selected from Substituent Group A4 shown below) or (7) -S-; $Z_5$ represents (1) a single bond, (2) an imino group which may be substituted with a substituent selected from Substituent Group A4 shown below, (3) $-(CH_2)n_e-$(wherein $n_e$ represents an integer of 1 to 3), (4) $-CR^8R^9-$ (wherein $R^8$ and $R^9$ are as defined above) or (5) -O-; $R^1$ represents a substituent selected from Substituent Group A4; and $R^7$ represents a substituent selected from Substituent Group A3.
Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**27.** The compound or pharmacologically acceptable salt thereof according to claim 1 or 26, wherein the formula (VI) represents a cyclic group which may be substituted with 1 to 4 substituents selected from Substituent Group A7 and is represented by the following formula:

[Formula 18]

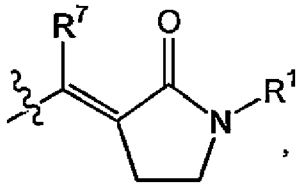
,
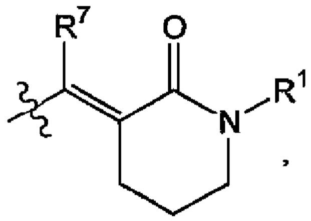
,
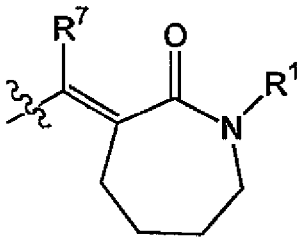

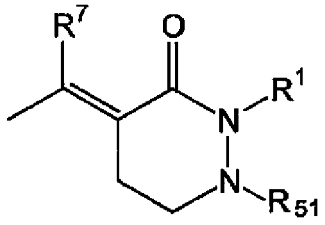
or
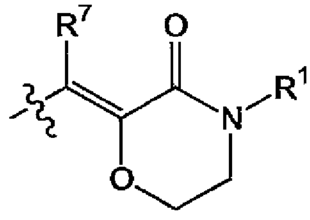

wherein $R^1$ and $R_{51}$ are the same or different and each represent a substituent selected from Substituent Group A4; and $R^7$ represents a substituent selected from Substituent Group A3.

Substituent Group A3: (1) a hydrogen atom, (2) a halogen atom, (3) a 6- to 14-membered aromatic hydrocarbon ring group which may be substituted with 1 to 3 substituents selected from Substituent Group A4, (4) a 5- to 14-membered aromatic heterocyclic group which may be substituted with 1 to 3 substituents selected from Substituent Group A4, (5) a C1-6 alkyl group (wherein the C1-6 alkyl group may be substituted with 1 to 3 substituents selected from the group consisting of a formyl group, a halogen atom, a hydroxyl group, a hydroxyl group having a protecting group, a cyano group, a C2-6 alkenyl group, a C2-6 alkynyl group, a C3-8 cycloalkyl group, a C1-6 alkoxy group, a C1-6 alkylthio group, a C1-6 alkylsulfinyl group, a C1-6 alkylsulfonyl group, a C1-6 alkylcarbonyl group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A4, a 5- to 14-membered aromatic heterocyclic group which may be substituted with 1 to 3 substituents selected from Substituent Group A4, a 6- to 14-membered non-aromatic hydrocarbon ring group which may be substituted with 1 to 3 substituents selected from Substituent Group A4, a 5- to 14-membered non-aromatic heterocyclic group which may be substituted with 1 to 3 substituents selected from Substituent Group A4 and -X-A (wherein X represents an imino group, -O- or -S- and A represents a 6- to 14-membered aromatic hydrocarbon ring group or 5- to 14-membered aromatic heterocyclic group which may be substituted with 1 to 3 substituents selected from Substituent Group A4)) and (6) a C1-6 alkoxy group. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6

alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above).

**28.** The compound or pharmacologically acceptable salt thereof according to claim 1, wherein $R^1$ and $R^2$, together with -$X_1$-CO-N, form a cyclic group which may be substituted with 1 to 4 substituents selected from Substituent Group A4 and is represented by the following formula:

[Formula 19]

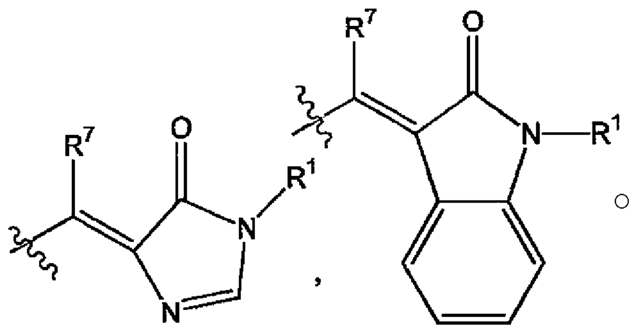

or

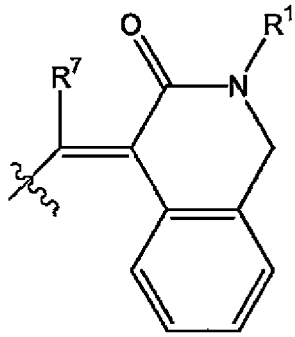

wherein $R^1$ and $R^7$ are as defined above.

Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**29.** The compound or pharmacologically acceptable salt thereof according to any one of claims 1, 12, 13, 15 and 17 to 28, wherein $R^1$ is a substituent selected from Substituent Group A8.

Substituent Group A8: (1) a hydrogen atom, (2) a C1-6 alkyl group (wherein the C1-6 alkyl group may be substituted with 1 to 3 substituents selected from the group consisting of a hydrogen atom, a halogen atom, a hydroxyl group,

a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7, a 5-to 14-membered non-aromatic heterocyclic group which may be substituted with 1 to 3 substituents selected from Substituent Group A7 and -X-$A^2$ (wherein X represents an imino group, -O- or -S- and $A^2$ represents a 6- to 14-membered aromatic hydrocarbon ring group which may be substituted with 1 to 3 substituents selected from Substituent Group A7 or a 5- to 14-membered aromatic heterocyclic group which may be substituted with 1 to 3 substituents selected from Substituent Group A7)), (3) a 6- to 14-membered aromatic hydrocarbon ring group which may be substituted with 1 to 3 substituents selected from Substituent Group A7, (4) a 5- to 14-membered aromatic heterocyclic group which may be substituted with 1 to 3 substituents selected from Substituent Group A7 and (5) -X-$A^2$ (wherein X and $A^2$ are as defined above). Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above).

**30.** The compound or pharmacologically acceptable salt thereof according to any one of claims 1, 12, 13, 14, 15 and 17 to 29, wherein $R^1$ is a C1-6 alkyl group (wherein the C1-6 alkyl group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A9, a 5- to 14-membered aromatic heterocyclic group which may be substituted with 1 to 3 substituents selected from Substituent Group A9, a 5-to 14-membered non-aromatic heterocyclic group which may be substituted with 1 to 3 substituents selected from Substituent Group A9 and -X-$A^4$ (wherein X represents an imino group, -O- or -S- and $A^4$ represents a 6- to 14-membered aromatic hydrocarbon ring group which may be substituted with 1 to 3 substituents selected from Substituent Group A9 or a 5- to 14-membered aromatic heterocyclic group which may be substituted with 1 to 3 substituents selected from Substituent Group A9)).
Substituent Group A9: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) -CO-$A^3$ (wherein $A^3$ represents a 6- to 14-membered aromatic hydrocarbon ring group), (10) a 6- to 14-membered aromatic hydrocarbon ring group which may be substituted with 1 to 3 substituents selected from Substituent Group A9 and (11) a 5- to 14-membered aromatic heterocyclic group which may be substituted with 1 to 3 substituents selected from Substituent Group A9.

**31.** The compound or pharmacologically acceptable salt thereof according to claim 1, 10, 26 or 28,

wherein $R^1$ is $-X_{21}-X_{22}-Ar_3$
(wherein $X_{21}$ represents 1) a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with a carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group) and a 5- to 14-membered non-aromatic heterocyclic group which may be substituted with 1 to 3 substituents selected from Substituent Group A7) or 2) a single bond; $X_{22}$ represents a single bond, an imino group which may be substituted with a substituent selected from Substituent Group A7, -O- or -S-; and $Ar_3$ represents a 6- to 14-membered aromatic hydrocarbon which may be substituted with 1 to 3 substituents selected from Substituent Group A7 or a 5- to 14-membered aromatic heterocyclic group which may be substituted with 1 to 3 substituents selected from Substituent Group A7).
Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and $-O-A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) $-CO-A^3$ (wherein $A^3$ is as defined above).

**32.** The compound or pharmacologically acceptable salt thereof according to claim 1, 10, 26, 28 or 31, wherein $R^1$ is $-X_{21a}-X_{22a}-Ar_{3a}$
(wherein $X_{21a}$ represents a C1-6 alkylene group (wherein the C1-6 alkylene group may be substituted with 1 to 3 substituents selected from the group consisting of a hydrogen atom, a halogen atom, a hydroxyl group, a cyano group, a C3-8 cycloalkyl group, a C3-8 cycloalkoxy group, a formyl group, a C1-6 alkyl group (wherein the one or two C1-6 alkyl groups may substitute the same carbon atom in the C1-6 alkylene group and the two C1-6 alkyl groups, together with the carbon atom to which they are bonded, may form a cyclic group (wherein a methylene group in the cyclic group which constitutes the ring may be substituted with one oxygen atom)), a C1-6 alkoxy group, an amino group (wherein the amino group may be substituted with a C1-6 alkyl group optionally having 1 to 5 halogen atoms) and a 5- to 14-membered non-aromatic heterocyclic group which may be substituted with 1 to 3 substituents selected from Substituent Group A9); $X_{22a}$ represents a single bond or an oxygen atom; and $Ar_{3a}$ represents a 6- to 14-membered aromatic hydrocarbon ring group which may be substituted with 1 to 3 substituents selected from Substituent Group A9 or a 5- to 14-membered aromatic heterocyclic group which may be substituted with 1 to 3 substituents selected from Substituent Group A9).
Substituent Group A9: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) $-CO-A^3$ (wherein $A^3$ represents a 6- to 14-membered aromatic hydrocarbon ring group), (10) a 6- to 14-membered aromatic hydrocarbon ring group which may be substituted with 1 to 3 substituents selected from Substituent Group A9 and (11) a 5- to 14-membered aromatic heterocyclic group which may be substituted with 1 to 3 substituents selected from Substituent Group A9.

**33.** The compound or pharmacologically acceptable salt thereof according to claim 32, wherein $Ar_{3a}$ is a 6-to 14-membered aromatic hydrocarbon ring group selected from the group consisting of a phenyl group, a naphthyl group and a fluorenyl group or a 5- to 14-membered aromatic heterocyclic group selected from the group consisting of a thienyl group, a pyridinyl group, a quinolinyl group, an isoquinolinyl group, an indolyl group, a benzothiazolyl group,

a benzoxazolyl group and a furyl group, which may be substituted with 1 to 3 substituents selected from Substituent Group A9. Substituent Group A9: (1) a hydrogen atom, (2) a halogen atom, (3) a C3-8 cycloalkyl group, (4) a C3-8 cycloalkoxy group, (5) a C1-6 alkyl group (wherein the C1-6 alkyl group may be substituted with 1 to 5 substituents selected from the group consisting of a halogen atom and a C1-6 alkyl group), (6) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (7) an amino group (wherein the amino group may be substituted with a C1-6 alkyl group optionally having 1 to 5 halogen atoms), (8) a 5- to 14-membered non-aromatic heterocyclic group which may be substituted with 1 to 3 substituents selected from Substituent Group A9, (9) -CO-$A^3$ (wherein $A^3$ represents a 6- to 14-membered aromatic hydrocarbon ring group), (10) a 6- to 14-membered aromatic hydrocarbon ring group which may be substituted with 1 to 3 substituents selected from Substituent Group A9 and (11) a 5- to 14-membered aromatic heterocyclic group which may be substituted with 1 to 3 substituents selected from Substituent Group A9.

**34.** The compound or pharmacologically acceptable salt thereof according to claim 1, wherein $R^1$ is a 6- to 14-membered non-aromatic hydrocarbon ring group or a 5-to 14-membered non-aromatic heterocyclic group represented by the formula (VII):

[Formula 20]

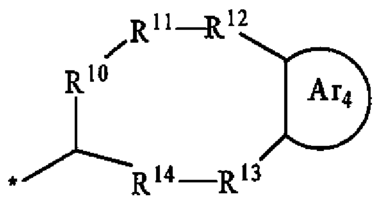

(VII)

wherein $R^{10}$ to $R^{14}$ represent 1) a single bond, 2) -CO-, 3) a methylene group which may be substituted with 1 or 2 substituents selected from Substituent Group A4, 4) -O-, 5) an imino group which may have a substituent selected from Substituent Group A4 or 6) -S-; and $Ar_4$ represents a 6- to 14-membered aromatic hydrocarbon ring group which may be substituted with 1 to 3 substituents selected from Substituent Group A4 shown below or a 5- to 14-membered aromatic heterocyclic group which may be substituted with 1 to 3 substituents selected from Substituent Group A4 shown below. Substituent Group A4: (1) a hydrogen atom, (2) a halogen atom, (3) a hydroxyl group, (4) a cyano group, (5) a nitro group, (6) a C3-8 cycloalkyl group, (7) a C2-6 alkenyl group, (8) a C2-6 alkynyl group, (9) a C3-8 cycloalkoxy group, (10) a C3-8 cycloalkylthio group, (11) a formyl group, (12) a C1-6 alkylcarbonyl group, (13) a C1-6 alkylthio group, (14) a C1-6 alkylsulfinyl group, (15) a C1-6 alkylsulfonyl group, (16) a hydroxyimino group, (17) a C1-6 alkoxyimino group, (18) a C1-6 alkyl group which may be substituted with 1 to 3 substituents selected from Substituent Group A4, (19) a C1-6 alkoxy group which may be substituted with 1 to 3 substituents selected from Substituent Group A4, (20) an amino group which may be substituted with 1 or 2 substituents selected from Substituent Group A4, (21) a carbamoyl group which may be substituted with 1 or 2 substituents selected from Substituent Group A4, (22) a 6- to 14-membered aromatic hydrocarbon ring group which may be substituted with 1 to 3 substituents selected from Substituent Group A4, (23) a 5- to 14-membered aromatic heterocyclic group which may be substituted with 1 to 3 substituents selected from Substituent Group A4, (24) a 6- to 14-membered non-aromatic hydrocarbon ring group which may be substituted with 1 to 3 substituents selected from Substituent Group A4, (25) a 5- to 14-membered non-aromatic heterocyclic group which may be substituted with 1 to 3 substituents selected from Substituent Group A4, (26) a C2-6 alkenyloxy group, (27) a C2-6 alkynyloxy group, (28) a C3-8 cycloalkylsulfinyl group, (29) a C3-8 cycloalkylsulfonyl group, (30) -X-A (wherein X represents an imino group, -O- or -S- and A represents a 6- to 14-membered aromatic hydrocarbon ring group or a 5- to 14-membered aromatic heterocyclic group which may be substituted with 1 to 3 substituents selected from Substituent Group A4), (31) -CO-A (wherein A is as defined above) and (32) =CH-A (wherein A is as defined above).

**35.** The compound or pharmacologically acceptable salt thereof according to claim 34, wherein $Ar_4$ is a phenyl group or a 5- to 14-membered aromatic heterocyclic group selected from the group consisting of a pyridinyl group, a pyrimidinyl group, a pyrazinyl group, a thienyl group, an oxazolyl group, a pyrrolyl group, a thiazolyl group and a furyl group, which may be substituted with 1 to 3 substituents selected from the group consisting of a halogen atom, a C1-6 alkyl group (wherein the C1-6 alkyl group may be substituted with 1 to 3 substituents selected from the group

consisting of a halogen atom and a C1-6 alkyl group), a C1-6 alkoxy group (wherein the C1-6 alkoxy group may be substituted with 1 to 3 halogen atoms), an amino group (wherein the amino group may be substituted with a C1-6 alkyl group optionally having 1 to 5 halogen atoms), a 6- to 14-membered aromatic hydrocarbon ring group which may be substituted with 1 to 3 substituents selected from Substituent Group A7, a 5- to 14-membered aromatic heterocyclic group which may be substituted with 1 to 3 substituents selected from Substituent Group A7, a 5-to 14-membered non-aromatic heterocyclic group which may be substituted with 1 to 3 substituents selected from Substituent Group A7 and -CO-$A^2$ (wherein $A^2$ represents a 6- to 14-membered aromatic hydrocarbon ring group which may be substituted with 1 to 3 substituents selected from Substituent Group A7 shown below or a 5- to 14-membered aromatic heterocyclic group which may be substituted with 1 to 3 substituents selected from Substituent Group A7 shown below). Substituent Group A7: (1) a hydrogen atom, (2) a halogen atom, (3) a hydroxyl group, (4) a cyano group, (5) a C3-8 cycloalkyl group, (6) a C3-8 cycloalkoxy group, (7) a C1-6 alkylcarbonyl group, (8) a C1-6 alkylthio group, (9) a C1-6 alkylsulfinyl group, (10) a C1-6 alkylsulfonyl group, (11) a C1-6 alkyl group (wherein the C1-6 alkyl group may be substituted with 1 to 5 substituents selected from the group consisting of a halogen atom, a C1-6 alkyl group, a 6- to 14-membered aromatic hydrocarbon ring group, a 5- to 14-membered aromatic heterocyclic group and -O-$A^3$ (wherein $A^3$ represents a 6- to 14-membered aromatic hydrocarbon ring group or a 5- to 14-membered aromatic heterocyclic group)), (12) a C1-6 alkoxy group (wherein the C1-6 alkoxy group may be substituted with 1 to 5 halogen atoms or the adjacent C1-6 alkoxy groups, together with a carbon atom to which they are bonded, may form a cyclic group), (13) an amino group (wherein the amino group may be substituted with a C1-6 alkyl group optionally having 1 to 5 halogen atoms), (14) a 6- to 14-membered aromatic hydrocarbon ring group which may be substituted with 1 to 3 substituents selected from Substituent Group A7, (15) a 5- to 14-membered aromatic heterocyclic group which may be substituted with 1 to 3 substituents selected from Substituent Group A7, (16) a 5- to 14-membered non-aromatic heterocyclic group which may be substituted with 1 to 3 substituents selected from Substituent Group A7 and (17) -CO-$A^3$ (wherein $A^3$ is as defined above).

**36.** The compound or pharmacologically acceptable salt thereof according to claim 35, wherein $R^1$ is an indanyl group, an azaindanyl group, a tetrahydronaphthyl group, an azatetrahydronaphthyl group, a chromanyl group, an azachromanyl group, a tetrahydrobenzofuranyl group or a tetrahydrobenzothienyl group, which may be substituted with 1 to 3 substituents selected from the group consisting of (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 3 halogen atoms or C1-6 alkyl groups), (7) a C1-6 alkoxy group (wherein the C1-6 alkoxy group may be substituted with 1 to 3 halogen atoms), (8) an amino group (wherein the amino group may be substituted with a C1-6 alkyl group optionally having 1 to 5 halogen atoms) and (9) a 5- to 14-membered non-aromatic heterocyclic group.

**37.** A compound represented by the formula (VIII):

[Formula 21]

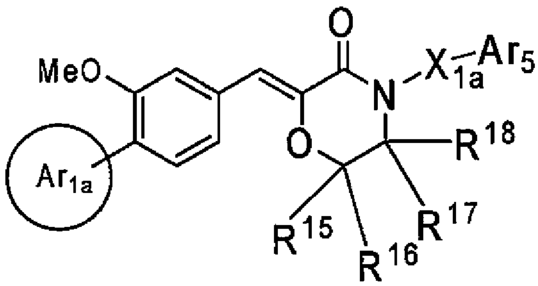

(VIII)

or a pharmacologically acceptable salt thereof,
wherein $Ar_{1a}$ represents a triazolyl group or a tetrazolyl group which may be substituted with a C1-6 alkyl group; and

(a) $R^{15}$, $R^{16}$, $R^{17}$ and $R^{18}$ are the same or different and each represent a hydrogen atom or a C1-6 alkyl group; $X_{1a}$ represents a C1-6 alkylene group (wherein the C1-6 alkylene group may be substituted with 1 to 3 hydroxyl groups or C1-6 alkyl groups (wherein the C1-6 alkyl group may be substituted with 1 to 3 hydroxyl groups));

and $Ar_5$ represents an aryl group, a pyridinyl group, an aryloxy group or a pyridinyloxy group which may be substituted with 1 to 3 substituents selected from Substituent Group All; or
(b) one of $R^{15}$ and $R^{16}$ and one of $R^{17}$ and $R^{18}$ are the same or different and each represent a hydrogen atom or a C1-6 alkyl group; the other of $R^{15}$ and $R^{16}$ and the other of $R^{17}$ and $R^{18}$, together with carbon atoms to which they are respectively bonded, form a C3-8 cycloalkyl group (wherein the C3-8 cycloalkyl group may be substituted with 1 to 3 substituents selected from Substituent Group A11); and $X_{1a}$ and $Ar_5$ are as defined in (a); or
(c) $Ar_5$-$X_{1a}$- represents a C3-8 cycloalkyl group (wherein one methylene group in the C3-8 cycloalkyl group may be substituted with an oxygen atom) condensed with a benzene ring (wherein the benzene ring may be substituted with 1 to 3 substituents selected from Substituent Group A11) ; and $R^{15}$, $R^{16}$, $R^{17}$ and $R^{18}$ are as defined in (a); or
(d) $Ar_5$-$X_{1a}$- and $R^{18}$, together with a nitrogen atom to which $Ar_5$-$X_{1a}$- is bonded and a carbon atom to which $R^{18}$ is bonded, form a 4- to 8-membered nitrogen-containing heterocyclic group (wherein one methylene group in the 4- to 8-membered nitrogen-containing heterocyclic group may be substituted with a methylene group or a vinylene group which may be substituted with 1 or 2 substituents selected from Substituent Group A11, an oxygen atom or an imino group which may be substituted with a C1-6 alkyl group or a C1-6 acyl group) which may be substituted with an aryl group or a pyridinyl group (wherein the aryl group or pyridinyl group may be substituted with 1 to 3 substituents selected from Substituent Group A11) ; and $R^{15}$, $R^{16}$ and $R^{17}$ are as defined in (a); or
(e) $R^{15}$ and $R^{16}$ form a C3-8 cycloalkyl group together; and $R^{17}$ , $R^{18}$, $X_{1a}$ and $Ar_5$ are as defined in (a) and (c); or
(f) $R^{17}$ and $R^{18}$ form a C3-8 cycloalkyl group together; and $R^{15}$, $R^{16}$, $X_{1a}$ and $Ar_5$ are as defined in (a) and (c).
Substituent Group A11: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 5 halogen atoms or 1 to 3 C1-6 alkoxy groups), (7) an amino group which may be substituted with 1 or 2 C1-6 alkyl groups (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) a carbamoyl group which may be substituted with 1 or 2 C1-6 alkyl groups (wherein the C1-6 alkyl group may be substituted with 1 to 3 halogen atoms).

**38.** The compound or pharmacologically acceptable salt thereof according to claim 37, wherein the compound is represented by the formula (VIII-a):

[Formula 22]

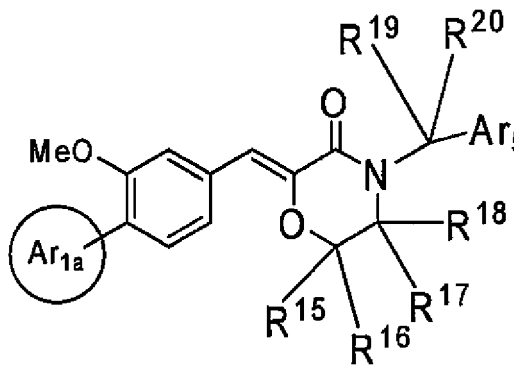

(VIII-a)

wherein $Ar_{1a}$ represents a triazolyl group or a tetrazolyl group which may be substituted with a C1-6 alkyl group; $R^{15}$, $R^{16}$, $R^{17}$ and $R^{18}$ are the same or different and each represent a hydrogen atom or a C1-6 alkyl group; $R^{19}$ and $R^{20}$ are the same or different and each represent a hydrogen atom or a C1-6 alkyl group (wherein the C1-6 alkyl group may be substituted with 1 to 3 hydroxyl groups); and
$Ar_{5\text{-}a}$ represents a phenyl group or a pyridinyl group which may be substituted with 1 to 3 substituents selected from Substituent Group A11.
Substituent Group A11: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 5 halogen atoms or 1 to 3 C1-6 alkoxy groups), (7) an amino group which may be substituted with 1 or 2 C1-6 alkyl

groups (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) a carbamoyl group which may be substituted with 1 or 2 C1-6 alkyl groups (wherein the C1-6 alkyl group may be substituted with 1 to 3 halogen atoms).

**39.** The compound or pharmacologically acceptable salt thereof according to claim 38, wherein $Ar_{5-a}$ may be substituted with 1 to 3 halogen atoms.

**40.** The compound or pharmacologically acceptable salt thereof according to claim 37, wherein the formula (VIII) is represented by the formula (VIII-b):

[Formula 23]

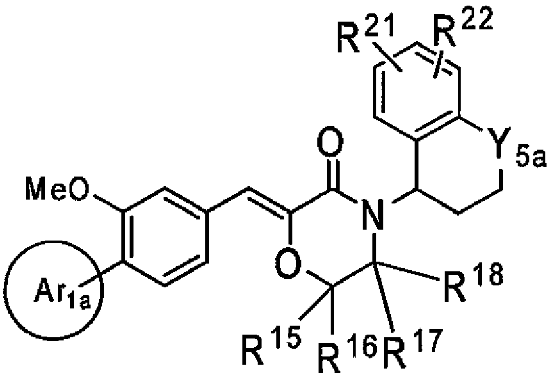

(VIII-b)

wherein $Ar_{1a}$ represents a triazolyl group or a tetrazolyl group which may be substituted with a C1-6 alkyl group; $R^{15}$, $R^{16}$, $R^{17}$ and $R^{18}$ are the same or different and each represent a hydrogen atom or a C1-6 alkyl group; $R^{21}$ and $R^{22}$ are the same or different and each represent a substituent selected from a hydrogen atom and Substituent Group All; and $Y_{5a}$ represents a methylene group or an oxygen atom.

Substituent Group A11: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 5 halogen atoms or 1 to 3 C1-6 alkoxy groups), (7) an amino group which may be substituted with 1 or 2 C1-6 alkyl groups (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) a carbamoyl group which may be substituted with 1 or 2 C1-6 alkyl groups (wherein the C1-6 alkyl group may be substituted with 1 to 3 halogen atoms).

**41.** The compound or pharmacologically acceptable salt thereof according to claim 40, wherein $R^{21}$ and $R^{22}$ are the same or different and each represent a hydrogen atom, a halogen atom or a C1-6 alkoxy group.

**42.** The compound or pharmacologically acceptable salt thereof according to claim 37, wherein the formula (VIII) is represented by the formula (VIII-c):

[Formula 24]

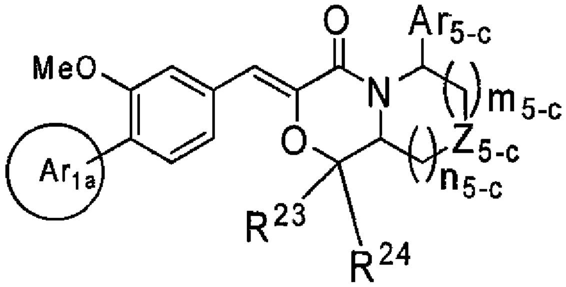

(VIII-c)

wherein $Ar_{1a}$ represents a triazolyl group or a tetrazolyl group which may be substituted with a C1-6 alkyl group; $R^{23}$ and $R^{24}$ are the same or different and each represent a hydrogen atom or a C1-6 alkyl group;
$Ar_{5-c}$ represents a phenyl group or a pyridinyl group which may be substituted with 1 to 3 substituents selected from Substituent Group All;
$Z_{5-c}$ represents a methylene group or a vinylene group which may be substituted with 1 or 2 substituents selected from Substituent Group A11, an oxygen atom or an imino group which may be substituted with a C1-6 alkyl group or a C1-6 acyl group; and
$n_{5-c}$ and $m_{5-c}$ are the same or different and each represent an integer of 0 to 2.
Substituent Group A11: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 5 halogen atoms or 1 to 3 C1-6 alkoxy groups), (7) an amino group which may be substituted with 1 or 2 C1-6 alkyl groups (wherein the C1-6 alkyl group may be substituted with 1 to 5 halogen atoms), (8) a C1-6 alkoxy group (wherein the C1-6 alkoxy group may be substituted with 1 to 5 halogen atoms) and (9) a carbamoyl group which may be substituted with 1 or 2 C1-6 alkyl groups (wherein the C1-6 alkyl group may be substituted with 1 to 3 halogen atoms).

**43.** The compound or pharmacologically acceptable salt thereof according to claim 42, wherein $Z_{5-c}$ represents a methylene group (wherein the methylene group may be substituted with 1 or 2 substituents which are the same or different and are selected from the group consisting of a C1-6 alkyl group and a hydroxyl group); and $n_{5-c}$ and $m_{5-c}$ each represent 1.

**44.** The compound or pharmacologically acceptable salt thereof according to claim 42 or 43, wherein $Ar_{5-c}$ has 1 to 3 halogen atoms.

**45.** A compound represented by the formula (IX):

[Formula 25]

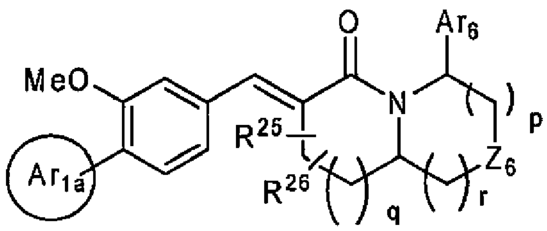

(IX)

or a pharmacologically acceptable salt thereof,
wherein $Ar_{1a}$ represents a triazolyl group or a tetrazolyl group which may be substituted with a C1-6 alkyl group;
$Ar_6$ represents a phenyl group which may be substituted with 1 to 3 substituents selected from Substituent Group A12 or a pyridinyl group which may be substituted with 1 to 3 substituents selected from Substituent Group A12;

$R^{25}$ and $R^{26}$ are the same or different and each represent a group selected from Substituent Group A12 shown below; $Z_6$ represents a methylene group or a vinylene group which may be substituted with 1 or 2 substituents selected from Substituent Group A11, an oxygen atom or an imino group which may be substituted with a C1-6 alkyl group or a C1-6 acyl group; and
p, q and r are the same or different and each represent an integer of 0 to 2.
Substituent Group A12: (1) a halogen atom, (2) a hydroxyl group, (3) a cyano group, (4) a C3-8 cycloalkyl group, (5) a C3-8 cycloalkoxy group, (6) a C1-6 alkyl group (wherein the C1-6 alkyl group may be substituted with 1 to 3 substituents selected from the group consisting of a halogen atom, a hydroxyl group, a cyano group, a C3-8 cycloalkyl group, a C1-6 alkoxy group and a C3-8 cycloalkoxy group), (7) a C1-6 alkoxy group (wherein the C1-6 alkoxy group may be substituted with 1 to 3 substituents selected from the group consisting of a halogen atom, a hydroxyl group, a cyano group, a C3-8 cycloalkyl group and a C3-8 cycloalkoxy group), (8) an amino group which may be substituted with 1 or 2 C1-6 alkyl groups (wherein the C1-6 alkyl group may be substituted with 1 to 3 halogen atoms) and (9) a carbamoyl group which may be substituted with 1 or 2 C1-6 alkyl groups (wherein the C1-6 alkyl group may be substituted with 1 to 3 halogen atoms).

**46.** The compound or pharmacologically acceptable salt thereof according to claim 45, wherein $Z_6$ represents a methylene group (wherein the methylene group may be substituted with 1 or 2 substituents which are the same or different and are selected from the group consisting of a C1-6 alkyl group and a hydroxyl group); and p, q and r each represent 1.

**47.** The compound or pharmacologically acceptable salt thereof according to claim 46, wherein $Z_6$ represents a methylene group (wherein the methylene group may be substituted with 1 or 2 substituents which are the same or different and are selected from the group consisting of a C1-6 alkyl group and a hydroxyl group); p, q and r each represent 1; and $Ar_6$ represents a phenyl group substituted with 1 to 3 halogen atoms.

**48.** The compound or pharmacologically acceptable salt thereof according to claim 45, wherein $Z_6$ represents a methylene group (wherein the methylene group may be substituted with 1 or 2 substituents which are the same or different and are selected from the group consisting of a C1-6 alkyl group and a hydroxyl group); p and q each represent 1; and r represents 0.

**49.** The compound or pharmacologically acceptable salt thereof according to claim 48, wherein $Z_6$ represents a methylene group (wherein the methylene group may be substituted with 1 or 2 substituents which are the same or different and are selected from the group consisting of a C1-6 alkyl group and a hydroxyl group); p and q each represent 1; r represents 0; and $Ar_6$ represents a phenyl group substituted with 2 or 3 halogen atoms.

**50.** The compound or pharmacologically acceptable salt thereof according to claim 45, wherein $Z_6$ represents an oxygen atom; and p, q and r each represent 1.

**51.** The compound or pharmacologically acceptable salt thereof according to claim 50, wherein $Z_6$ represents an oxygen atom; p, q and r each represent 1; and $Ar_6$ represents a phenyl group substituted with 1 to 3 halogen atoms.

**52.** The compound or pharmacologically acceptable salt thereof according to claim 45, 47, 49 or 51, wherein the halogen atom is a fluorine atom.

**53.** The compound or pharmacologically acceptable salt thereof according to claim 45, wherein $Z_6$ represents a methylene group (wherein the methylene group may be substituted with 1 or 2 substituents which are the same or different and are selected from the group consisting of a C1-6 alkyl group and a hydroxyl group); p represents 1; and q and r each represent 0.

**54.** The compound or pharmacologically acceptable salt thereof according to claim 45, wherein $Z_6$ represents a methylene group (wherein the methylene group may be substituted with 1 or 2 substituents which are the same or different and are selected from the group consisting of a C1-6 alkyl group and a hydroxyl group); p and r each represent 1; and q represents 0.

**55.** The compound or pharmacologically acceptable salt thereof according to claim 45, wherein $Z_6$ represents a methylene group (wherein the methylene group may be substituted with 1 or 2 substituents which are the same or different and are selected from the group consisting of a C1-6 alkyl group and a hydroxyl group); p represents 1; q represents 2; and r represents 0.

**56.** The compound or pharmacologically acceptable salt thereof according to claim 45, wherein $Z_6$ represents a methylene group (wherein the methylene group may be substituted with 1 or 2 substituents which are the same or different and are selected from the group consisting of a C1-6 alkyl group and a hydroxyl group); p and r each represent 1; and q represents 2.

**57.** The compound or pharmacologically acceptable salt thereof according to claim 1, wherein $Z_6$ represents a vinylene group (wherein the vinylene group may be substituted with 1 or 2 C1-6 alkyl groups); p represents 0; and q and r each represent 1.

**58.** The compound or pharmacologically acceptable salt thereof according to claim 45, wherein $Z_6$ represents a vinylene group (wherein the vinylene group may be substituted with 1 or 2 C1-6 alkyl groups); p and q each represent 1; and r represents 0.

**59.** A medicine comprising the compound or pharmacologically acceptable salt thereof according to any one of claims 1 to 58 as an active ingredient.

**60.** A prophylactic or therapeutic agent for a disease caused by amyloid-β, comprising the compound or pharmacologically acceptable salt thereof according to any one of claims 1 to 58 as an active ingredient.

**61.** The prophylactic or therapeutic agent according to claim 60, wherein the disease caused by amyloid-β is Alzheimer's disease, dementia, Down's syndrome or amyloidosis.

## INTERNATIONAL SEARCH REPORT

International application No.
PCT/JP2007/060188

A. CLASSIFICATION OF SUBJECT MATTER
*C07D249/08*(2006.01)i, *A61K31/4196*(2006.01)i, *A61K31/437*(2006.01)i, *A61K31/4375*(2006.01)i, *A61K31/454*(2006.01)i, *A61K31/4545*(2006.01)i, *A61K31/5377*(2006.01)i, *A61P25/28*(2006.01)i, *A61P43/00*(2006.01)i,
According to International Patent Classification (IPC) or to both national classification and IPC

B. FIELDS SEARCHED
Minimum documentation searched (classification system followed by classification symbols)
C07D249/08, A61K31/4196, A61K31/437, A61K31/4375, A61K31/454, A61K31/4545, A61K31/5377, A61P25/28, A61P43/00, C07D401/10, C07D401/14, C07D413/10, C07D413/14, C07D471/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CAOLD(STN), CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | WO 2005/020921 A2 (EXELIXIS, INC.), 10 March, 2005 (10.03.05), Full text; particularly, Claim 27, table 3; Entry 22, 65 & JP 2007-504160 A & EP 1663204 A2 & AU 2004268621 A & CA 2536954 A | 1,2,4-7, 9-14,16, 19-33,59 |
| X | JP 2005-518371 A (Amgen Inc.), 23 June, 2005 (23.06.05), Full text; particularly, Claims; the 5th compound from the last in column [1281] & WO 2003/049702 A2 & EP 1463714 A2 & US 2003/195201 A1 & AU 2002364549 A & CA 2468544 A | 1,2,4-7, 9-14,16, 19-33,59 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 July, 2007 (05.07.07) | 17 July, 2007 (17.07.07) |

| Name and mailing address of the ISA/ | Authorized officer |
|---|---|
| Japanese Patent Office | |
| Facsimile No. | Telephone No. |

Form PCT/ISA/210 (second sheet) (April 2005)

**INTERNATIONAL SEARCH REPORT**

International application No.
PCT/JP2007/060188

C (Continuation). DOCUMENTS CONSIDERED TO BE RELEVANT

| Category* | Citation of document, with indication, where appropriate, of the relevant passages | Relevant to claim No. |
|---|---|---|
| X | JP 7-002780 A (Fujisawa Pharmaceutical Co., Ltd.), 06 January, 1995 (06.01.95), Full text; particularly, Claims; example 70 & EP 622361 A1 & US 5563162 A & AU 9460525 A & CA 2122236 A & CN 1097417 A | 1,2,4-7, 9-12,16, 19-33,59 |
| A | WO 2005/115990 A1 (Eisai Co., Ltd.), 08 December, 2005 (08.12.05), Full text; particularly, Claims & EP 1757591 A1 & US 2006/004013 A1 & AU 2005247746 A & CA 2566094 A & CN 1972916 A | 1-61 |

Form PCT/ISA/210 (continuation of second sheet) (April 2005)

**INTERNATIONAL SEARCH REPORT**

International application No.
PCT/JP2007/060188

Continuation of A. CLASSIFICATION OF SUBJECT MATTER
(International Patent Classification (IPC))

*C07D401/10*(2006.01)i, *C07D401/14*(2006.01)i, *C07D413/10*(2006.01)i, *C07D413/14*(2006.01)i, *C07D471/04*(2006.01)i

(According to International Patent Classification (IPC) or to both national classification and IPC)

Form PCT/ISA/210 (extra sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2003076386 A


### Non-patent literature cited in the description


- **KLEIN WL.** Alzheimer's disease-affected brain: Presence of oligomeric Aβ ligands (ADDLs) suggests a molecular basis for reversible memory loss. *Proceeding National Academy of Science USA,* 02 September 2003, vol. 100 (18), 10417-10422
- **NITSCH RM.** Antibodies against β-amyloid slow cognitive decline in Alzheimer's disease. *Neuron,* 22 May 2003, vol. 38, 547-554
- **JARRETT JT.** The carboxy terminus of the β amyloid protein is critical for the seeding of amyloid formation: Implications for the pathogenesis of Alzheimer's disease. *Biochemistry,* 1993, vol. 32 (18), 4693-4697
- **GLENNER GG.** Alzheimer's disease: initial report of the purification and characterization of a novel cerebrovascular amyloid protein. *Biochemical and biophysical research communications,* 16 May 1984, vol. 120 (3), 885-890
- **MASTERS CL.** Amyloid plaque core protein in Alzheimer disease and Down syndrome. *Proceeding National Academy of Science USA,* 08 June 1985, vol. 2 (12), 4245-4249
- **GOURAS GK.** Intraneuronal Aβ42 accumulation in human brain. *American Journal of Pathology,* 15 January 2000, vol. 6 (1), 15-20
- **SCHEUNER D.** Secreted amyloid β-protein similar to that in the senile plaques of Alzheimer's disease is increased in vivo by the presenilin 1 and 2 and APP mutations linked to familial Alzheimer's disease. *Nature Medicine,* 19 December 1997, vol. 2 (8), 864-870
- **FORMAN MS.** Differential effects of the swedish mutant amyloid precursor protein on β-amyloid accumulation and secretion in neurons and nonneuronal cells. *The Journal of Biological Chemistry,* 19 December 1997, vol. 272 (51), 32247-32253
- **SHEARMAN MS.** L-685,458, an Aspartyl Protease Transition State Mimic, Is a Potent Inhibitor of Amyloid β-Protein Precursor γ-Secretase Activity. *Biochemistry,* 01 August 2000, vol. 39 (30), 8698-8704
- **SHEARMAN MS.** Catalytic Site-Directed γ-Secretase Complex Inhibitors Do Not Discriminate Pharmacologically betweeen Notch S3 and β-APP Clevages. *Biochemistry,* 24 June 2003, vol. 42 (24), 7580-7586
- **LANZ TA.** Studies of Aβ pharmacodynamics in the brain, cerebrospinal fluid, and plasma in young (plaque-free) Tg2576 mice using the γ-secretase inhibitor N2-[(2S)-2-(3,5-difluorophenyl)-2-hydroxyethanoyl]-N1-[(7S)-5-methyl-6-oxo-6,7-dihydro-5H-dibenzo[b,d]azepin-7-yl]-L-alaninamide (LY-411575. *The journal of pharmacology and experimental therapeutics,* 30 April 2004, vol. 9 (1), 49-55
- **WONG GT.** Chronic treatment with the γ-secretase inhibitor LY-411,575 inhibits β-amyloid peptide production and alters lymphopoiesis and intestinal cell differentiation. *The journal of biological chemistry,* 26 March 2004, vol. 279 (13), 12876-12882
- **NITSCH RM.** Antibodies against β-amyloid slow cognitive decline in Alzheimer's disease. *Neuron,* 22 May 2003, vol. 38 (4), 547-554
- **JARRETT JT.** The carboxy terminus of the β amyloid protein is critical for the seeding of amyloid formation: Implications for the pathogenesis of Alzheimers' disease. *Biochemistry,* 11 May 1993, vol. 32 (18), 4693-4697
- **GLENNER GG.** Alzheimer's disease; initial report of the purification and characterization of a novel cerebrovascular amyloid protein. *Biochemical and biophysical research communications,* 16 May 1984, vol. 120 (3), 885-890
- **GOURAS GK.** Intraneuronal Aβ42 accumulation in human brain. *American journal of pathology,* 15 January 2000, vol. 6 (1), 15-20
- **SCHEUNER D.** Secreted amyloid β-protein similar to that in the senile plaques of Alzheimer's disease is increased in vivo by the presenilin 1 and 2 and APP mutations linked to familial Alzheimer's disease. *Nature Medicine,* August 1996, vol. 2 (8), 864-870

- **FORMAN MS.** Differential effects of the swedish mutant amyloid precursor protein on β-amyloid accumulation and secretion in neurons and nonneuronal cells. *The journal of biological chemistry,* 19 December 1997, vol. 272, 32247-32253
- **BLASS JP.** Brain metabolism and brain disease: Is metabolic deficiency the proximate cause of Alzheimer dementia?. *Journal of Neuroscience Research,* 01 December 2001, vol. 66 (5), 851-856
- **EVIN G.** Alternative transcripts of presenilin-1 associated with frontotemporal dementia. *Neuroreport,* 16 April 2002, vol. 13 (5), 719-723
- **YASUHARA O.** Accumulation of amyloid precursor protein in brain lesions of patients with Pick disease. *Neuroscience Letters,* 25 April 1994, vol. 171 (1-2), 63-66
- **TELLER JK.** Presence of soluble amyloid β-peptide precedes amyloid plaque formation in Down's syndrome. *Nature Medicine,* January 1996, vol. 2 (1), 93-95
- **TOKUDA T.** Plasma levels of amyloid β proteins Aβ1-40 and Aβ1-42(43) are elevated in Down's syndrome. *Annals of Neurology,* February 1997, vol. 41 (2), 271-273
- **HAYASHI Y.** Evidence for presenilin-1 involvement in amyloid angiopathy in the Alzheimer's disease-affected brain. *Brain Research,* 13 April 1998, vol. 789 (2), 307-314
- **BARELLI H.** Characterization of new polyclonal antibodies specific for 40 and 42 amino acid-long amyloid β peptides: their use to examine the cell biology of presenilins and the immunohistochemistry of sporadic Alzheimer's disease and cerebral amyloid angiopathy cases. *Molecular Medicine,* October 1997, vol. 3 (10), 695-707
- **CALHOUN ME.** Neuronal overexpression of mutant amyloid precursor protein results in prominent deposition of cerebrovascular amyloid. *Proceeding National Academy of Science USA,* 23 November 1999, vol. 96 (24), 14088-14093
- **DERMAUT B.** Cerebral amyloid angiopathy is a pathogenic lesion in Alzheimer's Disease due to a novel presenilin-1 mutation. *Brain,* 12 December 2001, vol. 4 (12), 2383-2392
- **CRAS P.** Presenile Alzheimer dementia characterized by amyloid angiopathy and large amyloid core type senile plaques in the APP 692A1a-->Gly mutation. *Acta Neuropathologica(Berl,* September 1998, vol. 96 (3), 253-260
- **HERZIG MC.** Aβ is targeted to the vasculature in a mouse model of hereditary cerebral hemorrhage with amyloidosis. *Nature Neuroscience,* September 2004, vol. 7 (9), 954-960
- **VAN DUINEN SG.** Hereditary cerebral hemorrhage with amyloidosis in patients of Dutch origin is related to Alzheimer disease. *Proceeding National Academy of Science USA,* August 1987, vol. 84 (16), 5991-5994
- **LEVY E.** Mutation of the Alzheimer's disease amyloid gene in hereditary cerebral hemorrhage, Dutch type. *Science,* 01 June 1990, vol. 248 (4959), 1124-1126
- **LAWS SM.** Association between the presenilin-1 mutation Glu318Gly and complaints of memory impairment. *Neurobiology of Aging,* January 2002, vol. 23 (1), 55-58
- **VAUCHER E.** Object recognition memory and cholinergic parameters in mice expressing human presenilin 1 transgenes. *Experimental Neurology,* June 2002, vol. 175 (2), 398-406
- **MORGAN D.** Aβ peptide vaccination prevents memory loss in an animal model of Alzheimer's disease. *Nature,* 21 December 2000, vol. 408 (6815), 982-985
- **MORAN PM.** Age-related learning deficits in transgenic mice expressing the 751-amino acid isoform of human β-amyloid precursor protein. *Proceeding National Academy of Science USA,* 06 June 1995, vol. 92 (12), 5341-5345
- **KOISTINAHO M.** β-amyloid precursor protein transgenic mice that harbor diffuse Aβ deposits but do not form plaques show increased ischemic vulnerability: Role of inflammation. *Proceeding National Academy of Science USA,* 05 February 2002, vol. 99 (3), 1610-1615
- **ZHANG F.** Increased susceptibility to ischemic brain damage in transgenic mice overexpressing the amyloid precursor protein. *The journal of neuroscience,* 15 October 1997, vol. 17 (20), 7655-7661
- **SADOWSKI M.** Links between the pathology of Alzheimer's disease and vascular dementia. *Neurochemical Research,* June 2004, vol. 29 (6), 1257-1266
- **O'RIORDAN S.** Presenilin-1 mutation(E280G), spastic paraparesis, and cranial MRI white-matter abnormalities. *Neurology,* 08 October 2002, vol. 59 (7), 1108-1110
- **GEHRMANN J.** Amyloid precursor protein (APP) expression in multiple sclerosis lesions. *Glia,* October 1995, vol. 15 (2), 141-51
- **REYNOLDS WF.** Myeloperoxidase polymorphism is associated with gender specific risk for Alzheimer's disease. *Experimental Neurology,* January 1999, vol. 155 (1), 31-41
- **SMITH DH.** Protein accumulation in traumatic brain injury. *NeuroMolecular Medicine,* 2003, vol. 4 (1-2), 59-72
- **MATSUBARA-TSUTSUI M.** Molecular evidence of presenilin 1 mutation in familial early onset dementia. *American journal of Medical Genetics,* 08 April 2002, vol. 114 (3), 292-298
- **KIRKITADZE MD.** Paradigm shifts in Alzheimer's disease and other neurodegenerative disorders: the emerging role of oligomeric assemblies. *Journal of Neuroscience Research,* 01 September 2002, vol. 69 (5), 567-577

- **EVERT BO.** Inflammatory genes are upreglulated in expanded ataxin-3-expressing cell lines and spinocerebellar ataxia type 3 brains. *The Journal of Neuroscience,* 01 August 2001, vol. 21 (15), 5389-5396
- **MANN DM.** Deposition of amyloid(A4) protein within the brains of persons with dementing disorders other than Alzheimer's disease and Down's syndrome. *Neuroscience Letters,* 05 February 1990, vol. 109 (1-2), 68-75
- **PRIMAVERA J.** Brain accumulation of amyloid-β in Non-Alzheimer Neurodegeneration. *Jornal of Alzheimer's Disease,* October 1999, vol. 1 (3), 183-193
- **GIASSON BI.** Interactions of amyloidogenic proteins. *NeuroMolecular Medicine,* 2003, vol. 4 (1-2), 49-58
- **MASLIAH E.** β-amyloid peptides enhance α-synuclein accumulation and neuronal deficits in a trancgenic mouse model linking Alzheimer's disease and Parkinson's disease. *Proceeding National Academy of Science USA,* 09 October 2001, vol. 98 (21), 12245-12250
- **BARRACHINA M.** Amyloid-β deposition in the cerebral cortex in Dementia with Lewy bodies is accompanied by a relative increase in AβPP mRNA isoforms containing the Kunitz protease inhibitor. *Neurochemistry International,* February 2005, vol. 46 (3), 253-260
- **SCHMIDT ML.** Amyloid plaques in Guam amyotrophic lateral sclerosis/ parkinsonism-dementia complex contain species of Aβ similar to those found in the amyloid plaques of Alzheimer's disease and pathological aging. *Acta Neuropathologica (Berl,* February 1998, vol. 95 (2), 117-122
- **ITO H.** Demonstration of β amyloid protein-containing neurofibrillary tangles in parkinsonism-dementia complex on Guam. *Neuropathology and applied neurobiology,* October 1991, vol. 17 (5), 365-373
- **ROSSO SM.** Coexistent tau andamyloid pathology in hereditary frontotemporal dementia with tau mutations. *Annals of the New York academy of sciences,* 2000, vol. 920, 115-119
- **TOLNAY M.** Low amyloid(Aβ) plaque load and relative predominance of diffuse plaques distinguish argyrophilic grain disease from Alzheimer's disease. *Neuropathology and applied neurobiology,* August 1999, vol. 25 (4), 295-305
- **JIN LW.** Intracellular accumulation of amyloidogenic fragments of amyloid-β precursor protein in neurons with Niemann-Pick type C defects is associated with endosomal abnormalities. *American Journal of Pathology,* March 2004, vol. 164 (3), 975-985
- **SASAKI S.** Immunoreactivity of β-amyloid precursor protein in amyotrophic lateral sclerosis. *Acta Neuropathologica(Berl,* May 1999, vol. 97 (5), 463-468
- **TAMAOKA A.** Increased amyloid β protein in the skin of patients with amyotrophic lateral sclerosis. *Journal of neurology,* August 2000, vol. 247 (8), 633-635
- **HAMILTON RL.** Alzheimer disease pathology in amyotrophic lateral sclerosis. *Acta Neuropathologica,* June 2004, vol. 107 (6), 515-522
- **TURNER BJ.** Brain β-amyloidaccumulation in transgenic mice expressing mutant superoxide dismutase 1. *Neurochemical Research,* December 2004, vol. 29 (12), 2281-2286
- **WELLER RO.** Pathology of cerebrospinal fluid and interstitial fluid of the CNS: Significance for Alzheimer disease, prion disorders and multiple sclerosis. *Journal of Neuropathology and Experimental Neurology,* October 1998, vol. 57 (10), 885-894
- **SILVERBERG GD.** Alzheimer's disease, normal-pressure hydrocephalus, and senescent changes in CSF circulatory physiology: a hypothesis. *Lancet neurology,* August 2003, vol. 2 (8), 506-511
- **WELLER RO.** Cerebral amyloid angiopathy: Accumulation of Aβ in interstitial fluid drainage pathways in Alzheimer's disease. *Annals of the New York academy of sciences,* April 2000, vol. 903, 110-117
- **YOW HY.** A role for cerebrovascular disease in determining the pattern of β-amyloid deposition in Alzheimer's disease. *Neurology and applied neurobiology,* 2002, vol. 28, 149
- **WELLER RO.** Cerebrovasculardisease is a major factor in the failure of elimination of Aβ from the aging human brain. *Annals of the New York academy of sciences,* November 2002, vol. 977, 162-168
- **SMITH MJ.** Variable phenotype of Alzheimer's disease with spastic paraparesis. *Annals of Neurology,* 2001, vol. 49 (1), 125-129
- **CROOK R.** A variant of Alzheimer's disease with spastic pararesis and unusual plaques due to deletion of exon 9 of presenilin 1. *Nature Medicine,* April 1998, vol. 4 (4), 452-455
- **ATWOOD CS.** Cerebrovascular requirement for sealant, anti-coagulant and remodeling molecules that allow for the maintenance of vascular integrity and blood supply. *Brain Research Reviews,* September 2003, vol. 43 (1), 164-78
- **LOWENSON JD.** Protein aging: Extracellular amyloid formation and intracellular repair. *Trends in cardiovascular medicine,* 1994, vol. 4 (1), 3-8
- **SINGLETON AB.** Pathology of early-onset Alzheimer's disease cases bearing the Thr113-114ins presenilin-1 mutation. *Brain,* December 2000, vol. 123 (12), 2467-2474
- **GATTAZ WF.** Platelet phospholipase A2 activity in Alzheimer's disease and mild cognitive impairment. *Journal of Neural Transmission,* May 2004, vol. 111 (5), 591-601

- **ASSINI A.** Plasma levels of amyloid β-protein 42 are increased in women with mild cognitive impariment. *Neurology,* 14 September 2004, vol. 63 (5), 828-831
- **DE MEYER GR.** Platelet phagocytosis and processing of β-amyloid precursor protein as a mechanism of macrophage activation in atherosclerosis. *Circulation Research,* 14 June 2002, vol. 90 (11), 1197-1204
- **T. GREENE et al.** Protective Groups in Organic Synthesis. John Wiley & Sons, Inc, 1981
- **T.W. GREEN.** Protective Groups in Organic Synthesis. John Wiley & Sons, Inc, 1981, 154-186
- Yuki Kagobutsu No Gosei To Hannou. Shin Jikken Kagaku Koza. Maruzen Co., Ltd, 1978, vol. 14, 930-943
- Yuki Kagobutsu No Gosei To Hannou. Shin Jikken Kagaku Koza. Maruzen Co., Ltd, 1978, vol. 14, 1136-1162
- Yuki Kagobutsu No Gosei To Hannou. Shin Jikken Kagaku Koza. Maruzen Co., Ltd, 1978, vol. 14, 1142-1145
- Yukikagaku Jikken No Tebiki. Kagaku-Dojin Publishing Company, Inc, 1990, 27-52
- **O. MITSUNOBU.** *Synthesis,* 1981, 1
- **M.M.S. GIBSON et al.** *Angew. Chem.,* 1968, vol. 80, 986
- Yuki Kagobutsu No Gosei To Hannou. Shin Jikken Kagaku Koza. Maruzen Co., Ltd, 1978, vol. 14, 1333-1341
- Yuki Kagobutsu No Gosei To Hannou. Shin Jikken Kagaku Koza. Maruzen Co., Ltd, 1978, vol. 14, 1380-1384
- **T.W. GREEN.** Protective Groups in Organic Synthesis. John Wiley & Sons, Inc, 1981
- **B.E. HUFF et al.** *Tetrahedron Letter,* 1993, vol. 50, 8011-8014
- **T. VANEK et al.** *Collect. Czech. Chem. Commun.,* 1984, vol. 49, 2492
- **J. MICHEL et al.** *Tetrahedron Letter,* 2001, vol. 42, 9117-9118
- **D.D. DAVEY et al.** *J. Med. Chem.,* 1991, vol. 39, 2671-2677
- **C. PATOIS et al.** *Synth. Commun.,* 1991, vol. 22, 2391
- **J.A. JACKSON et al.** *J. Org. Chem.,* 1989, vol. 20, 5556
- **H.O. HOUSE.** Modern synthetic reactions. W.A. Benjamin Inc, 1972, 629-733
- **W. CARRTHERS.** Some modern methods of organic synthsis. Cambridge University Press, 1986, 125-144
- **W.S. WADSWORTH, JR.** *Org. Reactions.,* 1997, vol. 25, 73
- Yuki Kagobutsu No Gosei To Hannou. Shin Jikken Kagaku Koza. Maruzen Co., Ltd, 1978, vol. 14, 1261-1300
- Yuki Kagobutsu No Gosei To Hannou. Shin Jikken Kagaku Koza. Maruzen Co., Ltd, 1978, vol. 14, 1333-1335
- **K. SAKAI et al.** *Bull. Chem. Soc. Jpn.,* 1986, vol. 59, 179-183
- Jikken Kagaku Koza. Yuki Gosei. Maruzen Co., Ltd, 1991, vol. 21, 83-85
- **T. ABE et al.** *Tetrahedron,* 2001, vol. 57, 2701-2710
- Jikken Kagaku Koza. Yuki Gosei. Maruzen Co., Ltd, 1991, vol. 21, 89-92
- Jikken Kagaku Koza. Yuki Gosei. Maruzen Co., Ltd, 1992, vol. 20, 1-29
- Jikken Kagaku Koza. Yuki Gosei. Maruzen Co., Ltd, 1991, vol. 21, 2-22
- *Teterahedron Letter,* 1981, vol. 22, 3815-3818
- Jikken Kagaku Koza. Yuki Gosei. Maruzen Co., Ltd, 1991, vol. 21, 289-298
- **S.L. BUCHWALD et al.** *Tetrahedron Lett.,* 1997, vol. 38, 6367-6370
- **J.F. HARTWIG et al.** *J. Am. Chem. Soc.,* 1998, vol. 120, 827-828
- Yuki Kagobutsu No Gosei To Hannou. Shin Jikken Kagaku Koza. Maruzen Co., Ltd, 1977, vol. 14, 354-360
- **S.L. BUCHWALD et al.** *J. Am. Chem. Soc.,* 2002, vol. 124, 14844-14845
- **J. MARTI et al.** *Synth. Commun.,* 2000, vol. 30, 3023-3030
- **N. MIYAURA et al.** *J. Org. Chem.,* 1995, vol. 60, 7508-7510
- **R.F. HECK.** *Org. Reactions.,* 1982, vol. 27, 345
- **A. SUZUKI.** *Chem. Rev.,* 1995, vol. 95, 2457
- **K. SONOGASHIRA.** *Comprehensive Organic Synthesis,* 1991, vol. 3, 521
- **J.K. STILLE.** *Angew. Chem. Int. Ed. Engl.,* 1986, vol. 25, 508
- **C.R. DELOGE et al.** *Bull. Soc. Chim. Fr.,* 1992, vol. 129, 285-290
- **B. DUPRE et al.** *J. Org. Chem.,* 1991, vol. 56, 3197-3198
- **S.S. CHANDRAN et al.** *Bioorg. Med. Chem. Lett.,* 2001, vol. 11, 1493-1496
- **T. OKANO et al.** *Bull. Chem. Soc. Jpn.,* 1994, vol. 67, 2329-2332
- **O. PAMIES et al.** *J. Org. Chem.,* 2003, 4815-4818
- **T.-J. TSAI.** *Tetrahedron Letters,* 1996, vol. 37 (5), 629-632
- Yuki Kagobutsu No Gosei To Hannou. Shin Jikken Kagaku Koza. Maruzen Co., Ltd, 1977, vol. 14, 383-388

- Jikken Kagaku Koza. Yuki Gosei. Maruzen Co., Ltd, 1992, vol. 19, 194-196
- Jikken Kagaku Koza. Yuki Gosei. Maruzen Co., Ltd, 1992, vol. 19, 198-205
- **H.O. HOUSE.** Modern synthetic reactions. W.A. Benjamin, Inc, 1972, 629-653
- **J.A. CAMPBELL et al.** *J. Org. Chem.,* 1995, vol. 60, 4602-4616
- Jikken Kagaku Koza. Yuki Gosei. Maruzen Co., Ltd, 1992, vol. 19, 430-438
- *Chemical Review,* 1981, vol. 81, 415
- *Journal of the American Chemical Society,* 1945, vol. 67, 1180
- *The Journal of Organic Chemistry,* 1989, vol. 54, 4750
- Jikken Kagaku Koza. Yuki Gosei. Maruzen Co., Ltd, 1992, vol. 20, 300-302
- Jikken Kagaku Koza. Yuki Gosei. Maruzen Co., Ltd, July 1992, vol. 20, 94-100
- Jikken Kagaku Koza. Yuki Gosei. Maruzen Co., Ltd, 1992, vol. 20, 97-98
- Jikken Kagaku Koza. Yuki Gosei. Maruzen Co., Ltd, 1992, vol. 20, 96-97
- Jikken Kagaku Koza. Yuki Gosei. Maruzen Co., Ltd, 1992, vol. 19, 194-226
- Jikken Kagaku Koza. Maruzen Co., Ltd, 1992, vol. 19, 198-205
- Yuki Kagobutsu No Gosei To Hannou. Shin Jikken Kagaku Koza. Maruzen Co., Ltd, 1978, vol. 14, 1332-1399
- *Angew. Chem. Int. Ed.,* 2003, vol. 42, 5472-5474
- *Tetrahedron,* 1999, vol. 55, 7555-7562
- *Chem. Rev,* 1994, vol. 94, 2483-2547
- *Tetrahedron Letters,* 1996, vol. 37, 3219-3222
- Yuki Kagobutsu No Gosei To Hannou. Shin Jikken Kagaku Koza. Maruzen Co., Ltd, 1977, vol. 14, 567-611
- **K.B. SHARPLESS ; B.M. TROST et al.** Comprehensive Organic Synthesis. Pergamon, 1991, vol. 7
- *Synthesis,* 2004, vol. 10, 1563-1565
- Yuki Kagobutsu No Gosei To Hannou. Shin Jikken Kagaku Koza. Maruzen Co., Ltd, 1977, vol. 14, 633-875
- Jikken Kagaku Koza. Yuki Gosei. Maruzen Co., Ltd, 1992, vol. 19, 57-85
- *J. Org. Chem.,* 1993, vol. 58, 3384-3386
- *Organic Reaction,* 1995, vol. 14, 270
- *Journal of Organic Chemistry,* 1989, vol. 54, 4750
- *Journal of Organometallic Chemistry,* 1983, vol. 248, 51
- Jikken Kagaku Koza. Yuki Gosei. Maruzen Co., Ltd, 1992, vol. 26, 159-266
- Yuki Kagobutsu No Gosei To Hannou. Shin Jikken Kagaku Koza. Maruzen Co., Ltd, 1977, vol. 14, 331-450
- *Organic Reaction,* 1965, vol. 14, 270
- *Synth. Commun.,* 1996, vol. 26, 3091-3095
- *Tetrahedron Lett.,* 2001, vol. 42, 1309-1331
- Yuki Kagobutsu No Gosei To Hannou. Shin Jikken Kagaku Koza. Maruzen Co., Ltd, November 1977, vol. 14, 331-450
- Shin Jikken Kagaku Koza. Sanka To Kangen. Maruzen Co., Ltd, September 1976, vol. 15, 563-603
- *Journal of The American Chemical Society,* 2003, vol. 125, 11956
- *Tetrahedron Lett.,* 1998, vol. 39, 5421-5424
- *Tetrahedron: Asymmetry,* 1998, vol. 9, 4361
- *Tetrahedron Letters,* 1986, vol. 27, 4549
- *European Journal of Organic Chemistry,* 2004, vol. 23, 4823
- Jikken Kagaku Koza. Yuki Gosei. Maruzen Co., Ltd, 1992, vol. 26, 207-237
- Jikken Kagaku Koza. Yuki Gosei. Maruzen Co., Ltd, 1992, vol. 26, 237-248
- Jikken Kagaku Koza. Yuki Gosei. Maruzen Co., Ltd, 1992, vol. 24, 254-262
- Jikken Kagaku Koza. Yuki Gosei. Maruzen Co., Ltd, 1991, vol. 24, 59-72
- Jikken Kagaku Koza. Yuki Gosei. Maruzen Co., Ltd, 1991, vol. 24, 9-51
- Jikken Kagaku Koza. Yuki Gosei. Maruzen Co., Ltd, 1991, vol. 25, 9-72
- *Synlett,* 1994, vol. 2, 143
- Jikken Kagaku Koza. Yuki Gosei Hannou. Maruzen Co., Ltd, 1992, vol. 20, 1-30
- Jikken Kagaku Koza. Yuki Gosei Hannou. Maruzen Co., Ltd, 1992, vol. 20, 279-318
- *Journal of Fluorine Chemistry,* 1997, vol. 2, 119
- *Scientia Pharmaceutica,* 1996, vol. 64, 3
- *Petrochemia,* 1990, vol. 30, 56
- *Tetrahedron Letters,* 1982, vol. 23, 229
- *Tetrahedron Letters,* 1998, vol. 39, 5421
- *Journal of the Organic Chemistry,* 2001, vol. 66, 886
- *Comprehensive Organometallic Chemistry,* 1982, vol. 8, 499
- Angewandte Chemie. 2000, vol. 39, 3012
- Jikken Kagaku Koza. Yuki Gosei Hannou. Maruzen Co., Ltd, 1992, vol. 26, 251-266
- Jikken Kagaku Koza. Yuki Gosei Hannou. Maruzen Co., Ltd, 1991, vol. 25, 83-134
- Jikken Kagaku Koza. Yuki Gosei Hannou. Maruzen Co., Ltd, 1991, vol. 23, 237-267

- Jikken Kagaku Koza. Yuki Gosei Hannou. Chemical Society of Japan, 1992, vol. 19, 422-458
- Jikken Kagaku Koza. Yuki Gosei Hannou. Maruzen Co., Ltd, 1992, vol. 20, 415-420
- Jikken Kagaku Koza. Yuki Gosei Hannou. Maruzen Co., Ltd, 1992, vol. 19, 123-132
- **W.S. WADSWORTH, JR.** *Organic Reactions,* 1997, vol. 25, 73
- *Synthetic Communication,* 1991, vol. 22, 2391
- *Organic Letter,* 2000, vol. 2, 1975
- *Journal of the American Chemical Society,* 2001, vol. 123, 1862
- *Collect. Czech. Chem. Commun.,* 1984, vol. 49, 2492
- *Bull. Chem. Soc. Jpn.,* 1986, vol. 59, 179-183